# EUROPEAN PATENT APPLICATION

(11) **EP 4 851 140 A1**
(43) Date of publication of application: **09.12.2026**
(21) Application number: 25180300.3
(22) Date of filing: 02.06.2025
(51) Int. Cl.: C07D 215/22, C07D 401/12, C07D 401/14, C07D 409/12, C07D 409/14, C07D 413/14, A61P 31/00, A61K 31/395, A61P 35/00

(54) **QUINOLINE COMPOUNDS FOR THE TREATMENT OF PROLIFERATIVE DISEASES, PREVENTION OF METASTASIS, AND RESISTANCE**

(71) Applicant: BST Bavarian Systems and Technologies GmbH, 82031 Grünwald (München) (DE)
(72) Inventor: ILLYÉS, Eszter, H-1045 Budapest (HU); HORVÁTH, Zoltán, H-1045 Budapest (HU); KREKÓ, Marcell, H-1045 Budapest (HU); ÖRFI, Erik, H-1045 Budapest (HU); ÖRFI, László, H-1045 Budapest (HU); ÖRFI, Zoltán, H-1045 Budapest (HU); SZOKOL, Bálint, H-1045 Budapest (HU); SZABADKAI, István, H-1045 Budapest (HU); ISAEV, Artur, D-80807 Munich (DE); KNYAZEV, Petr G., D-82131 Munich (DE)
(74) Representative: Danubia Patent & Law Office LLC

(57) **Abstract**

The invention relates to quinolinyloxyphenyl-amides, -sulfonamides and stereoisomeric forms, solvates, hydrates and/or pharmaceutically acceptable salts of these compounds as well as pharmaceutical compositions comprising at least one of these compounds, together with at least one pharmaceutically acceptable carrier, excipient and/or diluent. The quinolinyloxyphenyl-amides and -sulfonamides are useful for the treatment of proliferative diseases, in particular prevention, treatment and/or after-treatment of hyperproliferative disorders, such as cancers, tumors, as well as cancer metastases, and resistance.

## Description

### FIELD OF THE INVENTION

The present invention relates to quinolinyloxyphenyl-amides, -sulfonamides and stereoisomeric forms, solvates, hydrates and/or pharmaceutically acceptable salts of these compounds as well as pharmaceutical compositions comprising at least one of these compounds, together with at least one pharmaceutically acceptable carrier, excipient and/or diluent. The quinolinyloxyphenyl-amides and -sulfonamides are useful for the treatment of proliferative diseases, in particular prevention, treatment and/or after-treatment of hyperproliferative disorders, such as cancers, tumors, as well as cancer metastases, and resistance.

### SCIENTIFIC BACKGROUND

AXL is a receptor tyrosine kinase (RTK) belonging to the TAM kinase family along with MER and Tyro3. Overexpression of AXL is associated with poor prognosis in many types of cancer, linked to tumor malignancy and metastasis, and is also associated with resistance to various cancer therapies. The activation of this kinase induces signaling pathways that lead to proliferation and anti-apoptotic processes, effectively protecting tumor cells from suppression of growth and elimination. This receptor may also heterodimerize with MER and Tyro3, as well as with non-TAM kinases, such as EGFR, PDGFR or MET, which together activate AXL-dependent signaling, while, at the same time, attenuate the effect of inhibitors of these kinases. Due to its role in cancer development, AXL and MER are regarded promising therapeutic targets in various malignancies. Inhibition of the the AXL kinase promotes apoptosis of tumor cells, and it has been proven to augment anti-tumor effect of some anticancer drugs, as well as restoring sensitivity to certain kinase inhibitors once resistance emerges.

### BACKGROUND OF THE INVENTION

Several known kinase inhibitors, including sunitinib, cabozantinib, foretinib, bosutinib, crizotinib, gilteritinib, merestinib, amuvatinib, BMS777607 have been shown to block the activity of AXL besides potently inhibiting other kinases as well. A handful of compounds that have been developed specifically against AXL, as TAM-kinase-specific inhibitors, have reached clinical trials, such as bemcentinib, sitravatinib, dubermatinib, ONO-7475, INCB081776, DS-1205 or SCL-391. AXL inhibitors are used in trials primarily as therapies against solid tumors, including non-small cell lung cancer, renal cell carcinoma, melanoma, and pancreatic cancer, and against certain types of acute myeloid leukemia (AML).

Recently, covalent inhibitors have emerged as valuable tools for targeted cancer therapy. These compounds possess the ability to block kinase activity through formation of covalent adducts, resulting in prolonged residual time of the inhibitor in the active site, or even inactivating the kinase irreversibly. The advantages of covalent inhibitors lie in their strong affinity, enhanced selectivity, and the ability to overcome various resistance mechanisms, just to name a few. As opposed to conventional chemotherapeutic agents, covalent inhibitors have a scaffold that is selective for kinases specifically, and are equipped with specific structural elements called covalent "warheads." Upon initial, typically reversible binding of the inhibitor to the receptor, the warhead is positioned proximal to a targeted amino acid in or near the binding site, forming a covalent bond with it, thereby reinforcing the binding of the molecule through an additional strong interaction with the kinase. While off-target reactivity may be an undesired property of drugs exhibiting covalent mechanisms, the reactivity of the covalent warhead can be fine-tuned by using a structural environment that enables the warhead to react with the desired amino acid upon initial binding, which can also minimize adduct formation with off-site amino acids, or other proteins in general.

The international patent applications PCT/EP2009/002798 (WO2009/127417), WO2011/045084 and WO2012/028332 refer to similar compounds which are inhibitors of receptor tyrosine kinases of the AXL receptor family. These compounds are suitable for the treatment or prevention of disorders associated with, accompanied by, or caused by hyperfunction of a receptor of the AXL family, preferably AXL and MER, AXL in particular. These compounds are suitable for the treatment of hyperproliferative disorders, such as cancer and cancer metastases in particular.

The object of the present invention is to provide novel compounds and pharmaceutically acceptable salts thereof which can be used as pharmaceutically active agents, in particular for cancer or tumor therapy and treatment, as well as prophylaxis and after-treatment or follow-up treatment of cancers, tumors and cancer metastases in particular.

The object of the present invention is solved by the teaching of the independent claims. Further advantageous features, aspects, and details of the invention are disclosed in the description, the examples, and the claims of the present application.

### BRIEF DESCRIPTION OF THE INVENTION

**1.** The novel quinolinyloxyphenyl compounds or pharmaceutically acceptable salts thereof according to the present invention are represented by the following general formula (I) 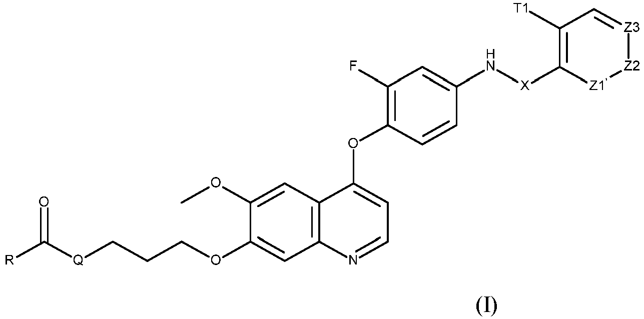 wherein
R is -CR¹=CR²R³, -C≡CH, -C≡C-C₁₋₂ alkyl, -CH₂-halogen, -CH₂-CH₂-halogen, -CH₂-C≡N, -CH₂-CH₂-C≡N, -CH₂-CH₂-O-C₁₋₂ alkyl, -CH₂-CH₂-CH₂-O-C₁₋₂ alkyl, -CH₂-CH₂-R⁴, -CH₂-CH₂-CH₂-R⁴, -CH₂-CH₂-C(O)R⁴ or -CH₂-CH₂-CH₂-C(O)R⁴;
R¹ is H, halogen or -C₁₋₂ alkyl;
R² is H, halogen or -C₁₋₂ alkyl;
R³ is H; -C₁₋₂ alkyl; -CF₃; -CH₂-R⁴; phenyl optionally substituted with 1 or 2 substituents selected independently from hydroxy, alkoxy, halogen or -CF₃; 5-membered heteroaryl containing 1 or 2 heteroatom selected from O, N and S; -C(O)-phenyl, -CH₂-O-benzotriazol, -C(O)R⁴
R⁴ is -OH, -NC₁₋₃ alkyl₂, -NH-OH, -Nallyl₂, or a N-containing 4-, 5- or 6-membered saturated heterocyclic ring which may further comprise an additional heteroatom selected from N, O or S, said heterocyclic ring is optionally substituted with 1 or 2 substituent selected from hydroxy, methyl, allyl, -NC₁₋₂ alkyl₂, said heterocyclic ring is connected to the rest of the compound through the N-atom;

Q is -NH- or 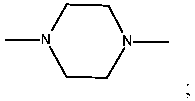
X is -S(O)₂- or -C(O)-;
Z¹ is C-H or -C(O)-;
Z² is C-T² or N-T³; as valency permits
the bond between Z¹ and Z² is a double bond when Z¹ is C-H and Z² is C-T² or a single bond when Z¹ is -C(O)- and Z² is N-T³;
Z³ is C-H or N;
T¹ is H, F, CF₃, methoxy or ethoxy; preferably H, F, CF₃ or ethoxy;
   T² is H or F;
   T³ is phenyl substituted in para position with F or CF₃.
**2.** Compound according to point 1 or pharmaceutically acceptable salt thereof, wherein
R is -CR¹=CR²R³, -C≡C-C₁₋₂ alkyl, -CH₂halogen, -CH₂-C≡N, -CH₂-CH₂-C(O)R⁴ or -CH₂-CH₂-CH₂-C(O)R⁴, preferably -CR¹=CR²R³, -C≡C-Me, -CH₂-Cl, -CH₂-C≡N, -CH₂-CH₂-C(O)R⁴ or -CH₂-CH₂-CH₂-C(O)R⁴;
R¹ is H or methyl, preferably H;
R² is H or methyl, preferably H;
R³ is H; -C₁₋₂ alkyl; -CF₃; -CH₂-R⁴; phenyl optionally substituted with 1 or 2 substituents selected independently from hydroxy, methoxy, chloro or -CF₃; 5-membered heteroaryl containing 1 or 2 heteroatom selected from N and S, preferably the heteroaryl is selected from thienyl and 1H-imidazolyl; -C(O)-phenyl, -CH₂-O-benzotriazol, -C(O)R⁴; and/or
R⁴ is -OH, -NMe₂, -NEt₂, -NH-OH, -Nallyl₂, or a N-containing heterocyclic ring selected from azetidinyl, pyrrolidinyl, piperidynyl, morpholinyl, thiomorpholinyl, or piperazinyl; said heterocyclic ring is optionally substituted with 1 or 2 substituent selected from hidroxy, methyl, allyl, -NMe₂, - NEt₂, said heterocyclic ring is connected to the rest of the compound through the N-atom.
**3.** Compound according to point 1 or 2 or pharmaceutically acceptable salt thereof, wherein
R⁴ is -OH, -NMe₂, -NEt₂, -NH-OH, -Nallyl₂, or a N-containing heterocyclic ring selected from azetidinyl, pyrrolidinyl, 3-hydroxypyrrolidinyl, 3-(dimethylamino)pyrrolidinyl, piperidynyl, 3-methylpiperidyl, 4-methylpiperidyl, thiomorpholinyl, morpholinyl, 2-methylmorpholinyl, methylpiperazinyl, allylpiperazinyl, 4-(diethylamino)piperidyl; said heterocyclic ring is connected to the rest of the compound through the N-atom.
**4.** Compound of any one of the previous points or pharmaceutically acceptable salt thereof, wherein R³ is H; -C₁₋₂ alkyl; -CF₃; -CH₂-NMe₂; -CH₂-NEt₂; -CH₂-piperidine; -CH₂-piperidine substituted with methyl in position 3; -CH₂-morpholine; -CH₂-piperazine; -CH₂-piperazine substituted with methyl in para position; phenyl; 2-hydroxyphenyl, 3,4-dihydroxyphenyl; 2-methoxyphenyl; 2,3-dimethoxyphenyl; 2,5-dimethoxyphenyl; 2-chlorophenyl; 3-(trifluoromethyl)phenyl; thienyl; 1H-imidazolyl; -C(O)-phenyl, -CH₂-O-benzotriazol, -C(O)R⁴.
**5.** Compound of any one of the previous points or pharmaceutically acceptable salt thereof, wherein R is -CR¹=CR²R³, -CH₂-CH₂-C(O)R⁴ or -CH₂-CH₂-CH₂-C(O)R⁴; preferably -CR¹=CR²R³.
**6.** Compound of any one of the previous points or pharmaceutically acceptable salt thereof, wherein
Q is 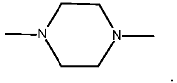 **7.** Compound of any one of the previous points or pharmaceutically acceptable salt thereof, wherein Q is -NH-.
**8.** Compound of any one of the previous points or pharmaceutically acceptable salt thereof, wherein
X is -S(O)₂-, Z¹ is C-H, Z² is C-T² and Z³ is C-H, or
X is -C(O)-, Z¹ is -C(O)-, Z² is N-T³ and Z³ is C-H or N.
**9.** Compound according to point 6 or pharmaceutically acceptable salt thereof, wherein
X is -S(O)₂-; T¹ is F and T² is F or T¹ is CF₃ and T² is H.
**10.** Compound according to point 6 or pharmaceutically acceptable salt thereof, wherein
X is -C(O)-; T¹ is ethoxy, Z² is N-T³, T³ is phenyl substituted in para position with F, and Z³ is C-H.
**11.** Compound according to point 8 or pharmaceutically acceptable salt thereof, wherein
Q is 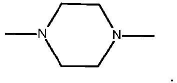 **12.** Compound according to point 6 or pharmaceutically acceptable salt thereof, wherein
X is -C(O)-; T¹ is H, Z² is N-T³, T³ is phenyl substituted in para position with F or CF₃, and Z³ is N.
**13.** Compound according to any of claims 1 to 12, wherein
R is 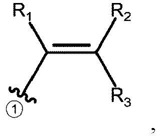
   R¹ is H, halogen or -C₁₋₂ alkyl; preferably H or methyl, more preferably H;
   R² is H, halogen or -C₁₋₂ alkyl; preferably H or methyl, more preferably H;
   R³ is H; -C₁₋₂ alkyl; -CF₃; -CH₂-R⁴; phenyl optionally substituted with 1 or 2 substituents selected independently from hydroxy, alkoxy, halogen or -CF₃; 5-membered heteroaryl containing 1 or 2 heteroatom selected from O, N and S; -C(O)-phenyl, -CH₂-O-benzotriazol, -C(O)R⁴; preferably -C₁₋₂ alkyl; -CF₃; -CH₂-R⁴; -C(O)R⁴; more preferably -CF₃; and -CH₂-R⁴;
   R⁴ is -OH, -NC₁₋₃ alkyl₂, -NH-OH, -N-allyl₂, or a N-containing 4-, 5- or 6-membered saturated heterocyclic ring which may further comprise an additional heteroatom selected from N, O or S, said heterocyclic ring is optionally substituted with 1 or 2 substituent selected from hydroxy, methyl, allyl, -NC₁₋₂ alkyl₂, said heterocyclic ring is connected to the rest of the compound through the N-atom; preferably -NC₁₋₃ alkyl₂, or a N-containing 5- or 6-membered saturated heterocyclic ring, said heterocyclic ring is optionally substituted with 1 or 2, preferably 1 substituent selected from hydroxy, methyl, allyl, -NC₁₋₂ alkyl₂, said heterocyclic ring is connected to the rest of the compound through the N-atom; more preferably -NC₁₋₂ alkyl₂, or 1-piperidyl optionally substituted with a methyl;
X is -S(O)₂- or -C(O)-, preferably -C(O)-
preferably wherein X is -S(O)₂- then
Z¹ is C-H;
Z² is C-T²;
the bond between Z¹ and Z² is a double bond when Z¹ is C-H and Z² is C-T²;
Z³ is C-H or N; preferably CH;
T¹ is H, F, CF₃, methoxy or ethoxy; preferably H, F, CF₃ or ethoxy; preferably F;
   T² is H or F; preferably F;
   or
preferably wherein X is - -C(O)-, then
Z¹ is C-H or -C(O)-; preferably -C(O)-;
Z² is C-T² or N-T³; as valency permits; preferably N-T³;
the bond between Z¹ and Z² is a double bond when Z¹ is C-H and Z² is C-T² or a single bond when Z¹ is -C(O)- and Z² is N-T³;
Z³ is C-H or N;
T¹ is H, F, CF₃, methoxy or ethoxy; preferably H, or ethoxy;
   T² is H or F;
   T³ is phenyl substituted in para position with F or CF₃;
preferably Q is 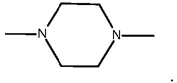
Preferably, the compound is selected from the group of the following compounds:

| | |
|---|---|
| **Example no.** | |
| Example 56 [AXL22074] | 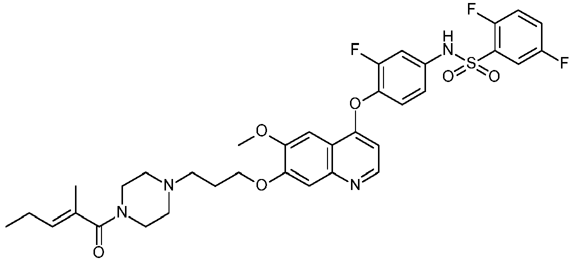 |
| Example 78 [AXL22101] | 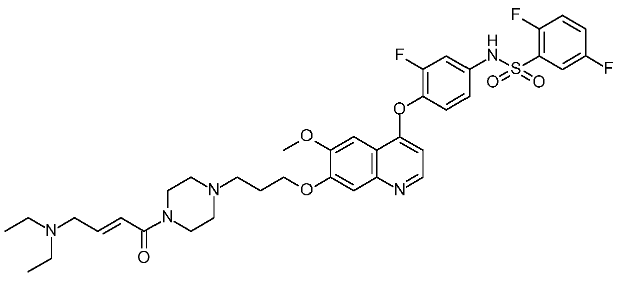 |
| Example 97 [AXL22123] | 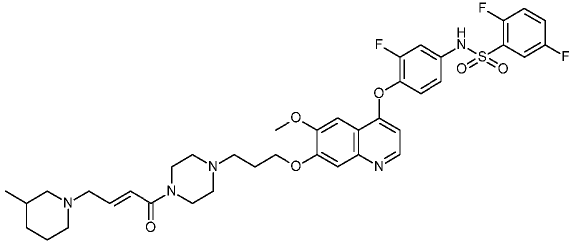 |
| Example 151 [AXL22134] | 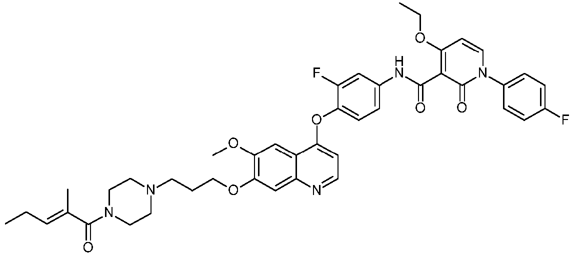 |
| Example 154 [AXL22139] | 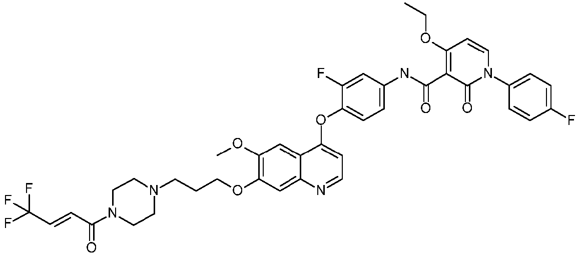 |
| Example 174 [AXL22160] | 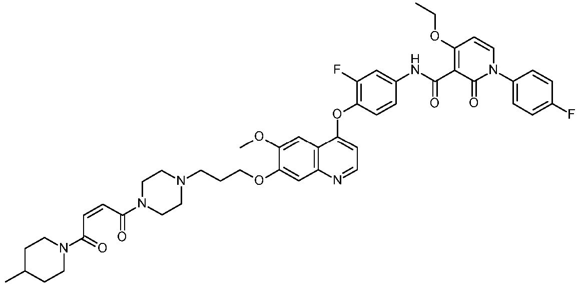 |
| Example 179 [AXL22064] | 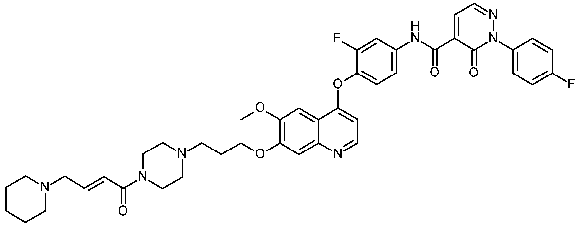 |
| Example 182 [AXL22108] | 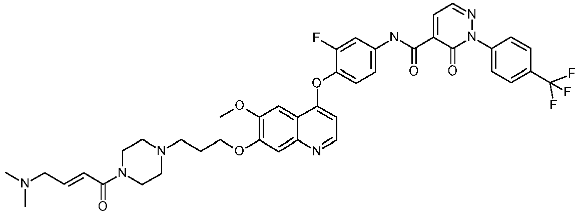 |
| Example 188 [AXL22176] | 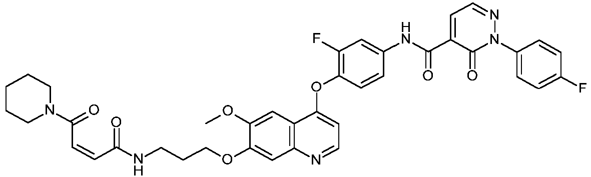 |

Highly preferably the compound is selected from the group of the following compounds:

| **Example no.** | |
|---|---|
| Example 154 [AXL22139] | 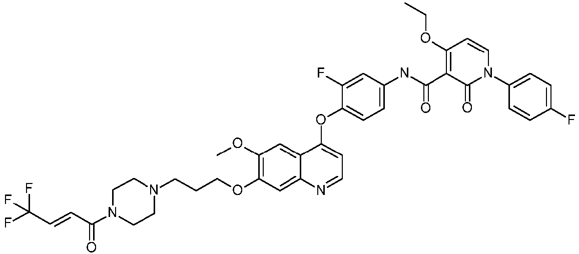 |
| Example 179 [AXL22064] | 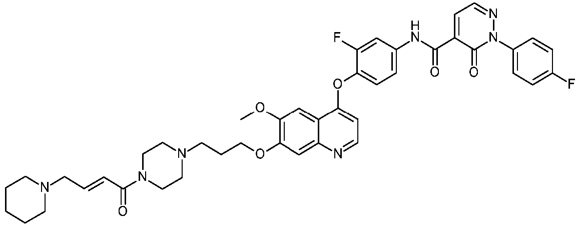 |
| Example 182 [AXL22108] | 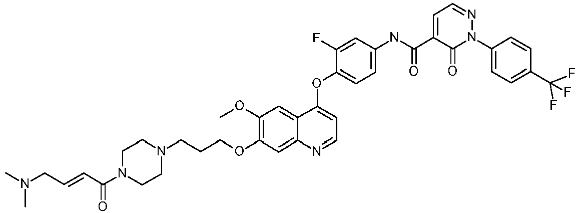 |

**14.** The compound according to any of points 1 to 12 and optionally according to point 13, or a pharmaceutically acceptable salt thereof, wherein the said compound is inhibitor of (capable of inhibiting) a TAM-family receptor tyrosine kinase, preferably a TAM-family receptor tyrosine kinase selected from the group consisting of AXL, MER, and Tyro3.
Highly preferably, said compound is an inhibitor of an AXL receptor tyrosine kinase (the AXL kinase), and more preferably is also an inhibitor of (capable of inhibiting) the MER receptor tyrosine kinase.
Preferably, the *in vitro* IC₅₀ value of the compound on the AXL kinase is lower than 100 nM, preferably lower than 30 nM, highly preferably lower than 10 nM, particularly highly preferably lower than 4 nM, and even more preferably lower than 2 nM.
Preferably, the *in vitro* IC₅₀ value of the compound on MER kinase is lower than 100 nM, preferably lower than 30 nM, highly preferably lower than 10 nM, and particularly highly preferably lower than 4 nM.
Very preferably, the *in vitro* IC₅₀ value of the compound both on the AXL kinase and on MER kinase is lower than 100 nM, preferably lower than 30 nM, highly preferably lower than 10 nM, and particularly highly preferably lower than 4 nM.
In a preferred embodiment, the *in vitro* IC₅₀ value of the compound on the AXL kinase is lower than 30 nM and the compound is selected from the compounds listed in Table "In vitro IC50 values of the compounds on the AXL kinase" wherein the average IC50 value is lower than 30 nM.
In a preferred embodiment, the *in vitro* IC₅₀ value of the compound on the AXL kinase is lower than 10 nM and the compound is selected from the compounds listed in Table "In vitro IC50 values of the compounds on the AXL kinase" wherein the average IC50 value is lower than 10 nM.
In a preferred embodiment, the *in vitro* IC₅₀ value of the compound on the AXL kinase is lower than 2 nM and the compound is selected from the compounds listed in Table "In vitro IC50 values of the compounds on the AXL kinase" wherein the average IC50 value is lower than 2 nM.
In a preferred embodiment, the *in vitro* IC₅₀ value of the compound on MER kinase is lower than 30 nM and the compound is selected from the compounds listed in Table *"In vitro* IC50 values of the compounds on the AXL kinase" wherein the average IC50 value is lower than 30 nM.
In a preferred embodiment, the *in vitro* IC₅₀ value of the compound on MER kinase is lower than 10 nM and the compound is selected from the compounds listed in Table *"In vitro* IC50 values of the compounds on the AXL kinase" wherein the average IC50 value is lower than 10 nM.
In a preferred embodiment, the *in vitro* IC₅₀ value of the compound on MER kinase is lower than 4nM and the compound is selected from the compounds listed in Table *"In vitro* IC50 values of the compounds on the AXL kinase" wherein the average IC50 value is lower than 4 nM.
In a further variant of the invention the compound is an inhibitor of an the AXL kinase wherein the *in vitro* AXL inhibition percentage value of the compound in 1.0 µM concentration is higher than 70%, preferably higher than 80%, highly preferably higher than 90%. Preferably, the compound is listed in Table: *"In vitro* AXL inhibition percentage values of the compounds in 7.8 µM, 1.0 µM and 0.1 1 µM concentrations" and MER inhibition value listed in the table is higher than 70%.
In a further variant of the invention the compound is an inhibitor of an MER kinase wherein the *in vitro* MER inhibition percentage value of the compound in 1.0 µM concentration is higher than 70% preferably higher than 80%, highly preferably higher than 90%. Preferably, the compound is listed in Table: *"In vitro* MER inhibition percentage values of the compounds in 7.8 µM, 1.0 µM and 0.1 1 µM concentrations" and MER inhibition value listed in the table is higher than 70%.
In a particularly preferred embodiment, the compound is non-toxic, preferably does not show acute toxicity after a single dose, and/or preferably does not show multiple toxicity after multiple doses; if examined in an animal model, preferably a murine model, by measuring body mass over at least 7 days, preferably at least 14 days; in particular, a mouse model wherein the compound is administered in at most 10 mg/kg, or at most 1 mg/kg, or at most 0,1 mg/kg dose.
**15.** The compound according to any of points 1 to 14, wherein said compound is a selective inhibitor of the AXL kinase and/or of the MER kinase, vis-à-vis an EGFR kinase.
**15.1** The compound according to any of points 1 to 14, wherein said compound is a selective inhibitor of the AXL kinase vis-à-vis an EGFR kinase.
Preferably, selective inhibition means that the level of inhibition of the AXL kinase is at least an order of magnitude higher than the level of inhibition of the EGFR kinase.
Preferably, the *in vitro* IC₅₀ value of the compound on the AXL kinase is lower than 100 nM, preferably lower than 30 nM, highly preferably lower than 10 nM, particularly highly preferably lower than 4 nM, and even more preferably lower than 2 nM.
In an embodiment the *in vitro* IC₅₀ value of the compound on EGFR kinase is higher than 100 nM in case the IC₅₀ value of the compound on the AXL kinase is lower than 10 nM,
or the *in vitro* IC₅₀ value of the compound on EGFR kinase is higher than 300 nM in case the IC₅₀ value of the compound on the AXL kinase is lower than 30 nM.
**15.2** The compound according to any of points 1 to 14 and optionally point 15.1, wherein said compound is a selective inhibitor of the MER kinase vis-à-vis an EGFR kinase.
Preferably, selective inhibition means that the level of inhibition of MER kinase is at least an order of magnitude higher than the level of inhibition of the EGFR kinase.
Preferably, the *in vitro* IC₅₀ value of the compound on MER kinase is lower than 100 nM, preferably lower than 30 nM, highly preferably lower than 10 nM, and particularly highly preferably lower than 4 nM,
In an embodiment the *in vitro* IC₅₀ value of the compound on EGFR kinase is higher than 100 nM in case the IC₅₀ value of the compound on the AXL kinase is lower than 10 nM,
or the *in vitro* IC₅₀ value of the compound on EGFR kinase is higher than 300 nM in case the IC₅₀ value of the compound on the AXL kinase is lower than 30 nM.
15.3 In a particular embodiment said compound is a selective inhibitor both of the the AXL kinase and of the MER kinase vis-à-vis an EGFR kinase.
In a preferred variant, the *in vitro* IC₅₀ value of the compound on the the AXL kinase and of the MER kinase is lower than 100 nM, preferably lower than 30 nM, highly preferably lower than 10 nM, and particularly highly preferably lower than 4 nM, whereas that of the EGFR kinase is as defined in any of points 15.1 or 15.2.
In a preferred variant, both of the inhibiton percentage of the the AXL kinase and of the MER kinae is higher than 70%.
Preferably, in case of selective inhibition over the EGFR kinase, the *in vitro* EGFR inhibition percentage values of the compounds in 7.8 µM concentration are lower than 90%, or preferably lower than 80% or lower than 70%.
In a particular embodiment the compound is selected from the compounds listed in the Table *"In vitro* EGFR inhibition percentage values of the compounds in 7.8 µM concentration" and having a selective inhibition over the EGFR kinase as defined in the previous paragraph.
Preferably, said compound is a compound according to point 13.
**16.** The compound according to any of points 1 to 14, wherein said compound is a broad kinase specificity inhibitor of an the AXL kinase, of a MER kinase and of an EGFR kinase.
Preferably, the *in vitro* IC₅₀ value of the compound the MER kinase is lower than 100 nM, preferably lower than 30 nM, highly preferably lower than 10 nM, particularly highly preferably lower than 4 nM, and the *in vitro* IC₅₀ value of the compound on the EGFR kinase is lower than 400 nM, preferably lower than 200 nM, highly preferably lower than 100 nM.
In a particularly preferred embodiment the compound is non toxic, preferably does not show acute toxicity after a single dose and/or preferably does not show multiple toxicity after multiple doses, if examiner in an animal model, preferably a murine model, by measuring body mass over at least 7 days, preferably at least 14 days. In particular a mouse model wherein the compound is administered in at most 10 mg/kg, or at most 1 mg/kg, or at most 0,1 mg/kg dose.
**17.** The compound according to any of points 1 to 14, wherein said compound is an inhibitor of an AXL-expressing cancer cell line.
Preferably the compound is an inhibitor of an AXL-expressing cancer cell line wherein the viability of the cell line is reduced by at least 70% wherein the compound is administered at 1 µM concentration.
Preferably the compound is an inhibitor of an AXL-expressing cancer cell line wherein the viability of the cell line is reduced by at least 50% wherein the compound is administered at 5 µM concentration.
In a preferred embodiment, the cell line is a glioblastoma cell line. In a particular embodiment the cell line is selected from an A172 cell line and an U87MG cell line.
In a particularly preferred embodiment the compound is non toxic, preferably does not show acute toxicity after a single dose and/or preferably does not show multiple toxicity after multiple doses, if examined in an animal model, preferably a murine model, by measuring body mass over at least 7 days, preferably at least 14 days, in particular a mouse model wherein the compound is administered in at most 10 mg/kg, or at most 1 mg/kg, or at most 0,1 mg/kg dose.
Preferably, said compound is a compound according to point 13.
Preferably, said compound is selected from the group consisting of
AXL22064, AXL22074, AXL22101, AXL22108, AXL22123, AXL22134, AXL22139, AXL22160, and AXL22176.
**18.** The compound wherein said compound is selected from a group as defined in any of claims 14 to 15, wherein said compound reduces tumor mass in an animal model, preferably in a mouse tumor model.
Preferably, said compound is selected from the group consisting of
AXL22064, AXL22074, AXL22101, AXL22108, AXL22123, AXL22134, AXL22139, AXL22160, and AXL22176,
preferably from the group consisting of AXL22064, AXL22108, and AXL22139.

**19.** A pharmaceutical composition comprising compound according to any of points 1 to 18 or a pharmaceutically acceptable salt thereof as a pharmaceutically active ingredient, as well as at least one pharmaceutically acceptable carrier, excipient and/or diluent.
**20.** The pharmaceutical composition according to point 19 formulated for oral administration.
**21.** The pharmaceutical composition according to point 19 formulated for parenteral application.
**22.** The pharmaceutical composition according to point 19 formulated for intravenous application. Preferably the intravenous formulation is an injection. Alternatively, the intravenous formulation is an infusion.
**23.** The pharmaceutical composition according to any of points 19 to 22, said composition further comprising another anti-cancer agent, preferably another anti-cancer kinase inhibitor, in particular another inhibitor of the AXL-dependent signaling pathway activator kinase, highly preferably another TAM kinase inhibitor.
**24.** The compound according to any of points 1 to 18 or the pharmaceutical composition according to points 19 to 23 for use in the treatment of a cell proliferative disease, in a subject; preferably cancer or malignant tumor in said subject; preferably in the treatment of a patient having a diagnosed cell proliferative disease, preferably cancer or malignant tumor.
   Preferably, the compound is any of the compounds as defined in claim 1.
   Preferably, the compound is any of the compounds as defined any of claims 1 to 13.
   Preferably, the compound is any of the compounds as defined any of claims 2, 3, 4, 5, 6, 7, 8, 9, 10, 11, 12 or 13.
   In a highly preferred embodiment the compound is a compound according to claim 13.
   Preferably, the compound is any of the compounds as defined any of claims 14, 15, 16, 17 or 18.
**25.** The compound according to points 1 to 18 or the pharmaceutical composition according to any of points 19 to 23 for use in the treatment of a TAM-receptor-tyrosine-kinase-induced disorder, i.e. a disorder induced by a member of the TAM (TYRO3-AXL-MER) family of receptor tyrosine kinases; wherein preferably the disorder, an AXL-receptor-tyrosine-kinase-induced disorder and/or the MER-receptor-tyrosine kinase-induced disorder.
**26.** The compound for use according to point 24 or 25 or the pharmaceutical composition according to any of points 19 to 23 for use in the treatment of a cancer or malignant tumor associated with overexpression of a TAM receptor kinase, preferably selected from AXL and MER, or a cancer or malignant tumor associated with a TAM receptor kinase hyperfunction, preferably an AXL and/or MER hyperfunction, in particular and highly preferably AXL hyperfunction.
   The invention also relates to the compound for use according to point 24 or 25 for use in the treatment of a hyperproliferative disorder associated with, accompanied by and/or caused by AXL hyperfunction, particularly AX- receptor-tyrosine-kinase-induced hyperproliferative disorders.
   The compound for use according to point 24 or 25 or the pharmaceutical composition according to any of points 19 to 23 for use in the treatment of a cancer or malignant tumor associated with overexpression of MER.
   Alternatively, the invention also relates to the compound for use according to point 24 or 25 for use in the treatment of a hyperproliferative disorder associated with, accompanied by and/or caused by MER hyperfunction, particularly the MER-receptor-tyrosinekinase-induced hyperproliferative disorders.
**27.** The compound for use according to any of points 1 to 18 or 24 to 26 for use or the pharmaceutical composition according to any of points 19 to 23 for use in the treatment according to any of points 19 to 23 for use or 24 to 26 for use in the prevention of metastasis in a subject having a disease as defined in points 24 to 26, respectively.
   Preferably, the compound is active in a migration assay, preferably in a TAS assay.
   In a particular embodiment the anti-migratory active compound shows an at least 50%, preferably at least 40%, at least 30%, at least 20% inhibition in a TAS assay.
   Preferably, the compound is a compound which shows an at least 50%, preferably at least 40%, at least 30%, at least 20% inhibition in the TAS assay as described in the Examples, preferably a compound listed in Table "TAS migration assay" or in Figure "TAS migration assay".
**28.** The compound for use according to any of points 24 to 27, for use in the treatment of an AXL-receptor-tyrosine-kinase-induced hyperproliferative disorder, wherein said disorder is selected from the group of hyperproliferative disorder consisting of: acute lymphoblastic leukemia, acute myeloid leukemia, adrenocortical carcinoma, aids-related cancers, aids-related lymphoma, anal cancer, appendix cancer, astrocytomas, atypical teratoid/rhabdoid tumor, basal cell carcinoma, bile duct cancer, bladder cancer, bone cancer, osteosarcoma and malignant fibrous histiocytoma, brain stem glioma, brain tumor, central nervous system atypical teratoid/rhabdoid tumor, astrocytomas, craniopharyngioma, ependymoblastoma, ependymoma, medulloblastoma, medulloepithelioma, pineal parenchymal tumors of intermediate differentiation, supratentorial primitive neuroectodermal tumors and pineoblastoma, brain and spinal cord tumors, breast cancer, bronchial tumors, Burkitt lymphoma, carcinoid tumor, gastrointestinal cancer, central nervous system (CNS) lymphoma, cervical cancer, chordoma, chronic lymphocytic leukemia, chronic myelogenous leukemia, chronic myeloproliferative disorders, colon cancer, colorectal cancer, craniopharyngioma, cutaneous t-cell lymphoma, mycosis fungoides, Sézary syndrome, endometrial cancer, ependymoblastoma, ependymoma, esophageal cancer, esthesioneuroblastoma, Ewing sarcoma family of tumors, extracranial germ cell tumor, extragonadal germ cell tumor, extrahepatic bile duct cancer, intraocular melanoma, retinoblastoma, gallbladder cancer, gastric (stomach) cancer, gastrointestinal carcinoid tumor, gastrointestinal stromal tumor (gist), gastrointestinal stromal cell tumor, extracranial germ cell tumor, extragonadal germ cell tumor, ovarian germ cell tumor, gestational trophoblastic tumor, glioma, hairy cell leukemia, head and neck cancer, heart cancer, hepatocellular (liver) cancer, histiocytosis, Hodgkin lymphoma, hypopharyngeal cancer, intraocular melanoma, islet cell tumors (endocrine pancreas), Kaposi sarcoma, renal cell cancer, kidney cancer, langerhans cell histiocytosis, laryngeal cancer, acute lymphoblastic leukemia, acute myeloid leukemia, chronic lymphocytic leukemia, chronic myelogenous leukemia, leukemia, lip and oral cavity cancer, liver cancer, lung cancer, non-small cell lung cancer, small cell lung cancer, aids-related lymphoma, Burkitt lymphoma, (cutaneous t-cell lymphoma, Hodgkin lymphoma, non-Hodgkin lymphoma, primary central nervous system lymphoma, macroglobulinemia, malignant fibrous histiocytoma of bone and osteosarcoma, medulloblastoma, medulloepithelioma, melanoma, melanoma intraocular (eye), merkel cell carcinoma, mesothelioma, metastatic squamous neck cancer with occult primary, mouth cancer, multiple endocrine neoplasia syndromes, multiple myeloma/plasma cell neoplasm, myelodysplastic syndromes, myelodysplastic/myeloproliferative neoplasms, myelogenous leukemia, myeloid leukemia, myeloma (multiple), myeloproliferative disorders, nasal cavity and paranasal sinus cancer, nasopharyngeal cancer, neuroblastoma, non-Hodgkin lymphoma, non-small cell lung cancer, oral cancer, oral cavity cancer, oropharyngeal cancer, osteosarcoma and malignant fibrous histiocytoma of bone, ovarian cancer, ovarian epithelial cancer, ovarian germ cell tumor, ovarian low malignant potential tumor, pancreatic cancer, papillomatosis, parathyroid cancer, penile cancer, pharyngeal cancer, pineoblastoma and supratentorial primitive neuroectodermal tumors, pituitary tumor, plasma cell neoplasm/multiple myeloma, pleuropulmonary blastoma, pregnancy and breast cancer, prostate cancer, rectal cancer, renal cell (kidney) cancer, transitional cell cancer, respiratory tract cancer, retinoblastoma, rhabdomyosarcoma, salivary gland cancer, sarcoma, Ewing sarcoma, Kaposi sarcoma, uterine sarcoma, nonmelanoma skin cancer, melanoma skin cancer, skin carcinoma, small cell lung cancer, small intestine cancer, soft tissue sarcoma, squamous cell carcinoma, squamous neck cancer, stomach (gastric) cancer, supratentorial primitive neuroectodermal tumors, t-cell lymphoma, testicular cancer, throat cancer, thymoma and thymic carcinoma, thyroid cancer, transitional cell cancer of the renal pelvis and ureter, trophoblastic tumor, gestational cancer, ureter and renal pelvis cancer, transitional cell cancer, urethral cancer, uterine cancer, endometrial cancer, uterine sarcoma, vaginal cancer, vulvar cancer, Waldenström macroglobulinemia and Wilms tumor.
**29.** Use of a compound according to any of points 1 to 12 or a pharmaceutically acceptable salt thereof *in vitro* as an inhibitor of a TAM-family receptor tyrosine kinase, preferably a TAM-family receptor tyrosine kinase selected from the group consisting of AXL, MER, and Tyro3.
**30.** The use of compound according to point 29 as an inhibitor of an AXL receptor tyrosine kinase (the AXL kinase).
**31.** The use of compound according to point 29 or 30 as an inhibitor of the MER receptor tyrosine kinase.
**32.** The use according to any of point 29 or 30, wherein said compound is a selective inhibitor of the AXL kinase which is selective for inhibition of the AXL kinase vis-à-vis an EGFR kinase.

### BRIEF DESCIPTION OF THE FIGURES

**Figure 1** shows result of the Western blot measurements for cell lines Hs-578T, MCF-7, A172, and U87MG cell lines, detected using the anti-AXL antibody (ProteinTech 68108-1-Ig).
**Figure 2** - Expression of GAS6-induced phospho-AXL studied in the Hs-578T cell line. The starved cells were stimulated with 250ng/µl and 400ng/µl GAS6
**Figure 3** **-** Expression of GAS6-induced phospho-AXL studied in the A172 and U87MG cell lines. The starved cells were stimulated with 250ng/µl and 400ng/µl GAS6
**Figure 4** - The effects of the inhibitors on GAS6-induced AXL phosphorylation in Hs-578T cell culture studied in 1000 nM, 100 nM, and 10 nM concentrations. The inhibition of AKT phosphorylation by the compounds was also measured, as well as total AXL and total AKT expression.
**Figure 5:** Results of the acute toxicity study with Example 179 [AXL22064]
**Figure 6****:** Results of the acute toxicity study with Example 56 [AXL22074]
**Figure 7****:** Results of the acute toxicity study with Example 78 [AXL22101]
**Figure 8****:** Results of the acute toxicity study with Example 182 [AXL22108]
**Figure 9****:** Results of the acute toxicity study with Example 97 [AXL22123]
**Figure 10****:** Results of the acute toxicity study with Example 151 [AXL22134]
**Figure 11****:** Results of the acute toxicity study with Example 154 [AXL22139]
**Figure 12****:** Results of the acute toxicity study with Example 174 [AXL22160]
**Figure 13****:** Results of the acute toxicity study with Example 188 [AXL22176]
**Figure 14****:** Results of the chronic toxicity study with Example 179 [AXL22064]
**Figure 15****:** Results of the chronic toxicity study with Example 56 [AXL22074]
**Figure 16****:** Results of the chronic toxicity study with Example 78 [AXL22101]
**Figure 17****:** Results of the chronic toxicity study with Example 182 [AXL22108]
**Figure 18****:** Results of the chronic toxicity study with Example 97 [AXL22123]
**Figure 19****:** Results of the chronic toxicity study with Example 151 [AXL22134]
**Figure 20****:** Results of the chronic toxicity study with Example 154 [AXL22139]
**Figure 21****:** Results of the chronic toxicity study with Example 174 [AXL22160]
**Figure 22****:** Results of the chronic toxicity study with Example 188 [AXL22176]
**Figure 23** shows the tumours excised from mice of the each experimental group.
**Figure 24** shows the transient agarose spot (TAS) migration assay with the Hs578t cells at 1 µM inhibitor concentration.
**Figure 25** shows the transient agarose spot (TAS) migration assays with the A172 cells at 1 µM inhibitor concentration.
**Figure 26** show the transient agarose spot (TAS) migration assays with the U87MG cells at 1 µM inhibitor concentration.

### DETAILED DESCRIPTION OF THE INVENTION

As used herein, the term "C₁-₃ alkyl" refers to -CH₃, -C₂H₅, -C₃H₇, -CH(CH₃)₂, wherein in the aforementioned groups one or more hydrogen atoms can be replaced by -OH, -OCH₃, -OC₂H₅, -SH, -SCH₃, - NO₂, -F, -Cl, -Br, -l, -N₃, -COCH₃, -COC₂H₅, -COOCH₃, -COOC₂H₅, -OOC-CH₃, -OOC-C₂H₅, -COOH, -CONH₂, -CON(CH₃)₂, -CON(C₂H₅)₂, -NH₂, -NHCH₃, -NHC₂H₅, -N(CH₃)₂, -N(C₂H₅)₂, -CF₃, -C≡CH (i.e. optionally substituted).

As used herein, the term "alkoxy" refers to the residue -O-C₁-C₃-alkyl, wherein C₁-C₃-alkyl has the meanings as defined above. The following C₁-C₃ alkoxy groups are preferred -O-CH₃, -O-C₂H₅, -O-C₃H₇, -O-CH(CH₃)₂, Most preferred are -O-CH₃ and -O-C₂H₅.

As used herein, the term "heteroaryl" or "heterocyclic ring" refers to heteroaromatic groups which have from 5 to 10 ring atoms, from 1 to 4 of which are selected from O, N, and/or S. Preferred groups have 1 or 2 heteroatoms in a 5- or 6-membered aromatic ring. Mono and bicyclic ring systems are included. Typical heteroaryl groups which are at least partially aromatic include pyridyl, furyl, thienyl, pyrrolyl, oxazolyl, thiazolyl, imidazolyl, isoxazolyl, isothiazolyl, oxadiazolyl, pyridazinyl, pyrimidyl, pyrazinyl, 1,3,5-triazinyl, 1,2,3-triazolyl, 1,3,4-thiadiazolyl, indolizinyl, indolyl, isoindolyl, benzo[b]furyl, thiophenyl, benzo[b]thienyl, indazolyl, benzimidazolyl, benzthiazolyl, thiazolyl, purinyl, quinolizinyl, quinolyl, isoquinolyl, quinazolinyl, quinoxalinyl, 1,8-naphthyridinyl, tetrahydroquinolyl, benzooxazolyl, chrom-2-onyl, indazolyl, indenyl, preferably thienyl and 1H-imidazolyl. Said heteroaryl groups may further be substituted by one, two, three, or more substituents selected from the group consisting of C₁-C₃-alkyl or halogen.

As used herein, the term " N-containing saturated heterocyclic ring" refers to heterocyclic groups which have 4 to 10 ring atoms, 1 to 3 of which are N, and 1 to 3 additional ring atoms may be selected from O, N, and/or S. Preferred groups have a nitrogen atom and optionally one other heteroatom in a 5- or 6-membered ring. More preferred heterocyclic rings are azetidinyl, pyrrolidinyl, piperidynyl, morpholinyl, thiomorpholinyl, or piperazinyl. Said groups may further be substituted by one, two, three, or more substituents selected from the group consisting of hidroxy, C₁-C₃-alkyl, allyl, -N(C₁-C₃-alkyl)₂, or halogen. The present invention also comprises pharmaceutically acceptable salts of the compounds according to the general formula (I), all stereoisomeric forms of the compounds according to the general formula (I) as well as solvates, especially hydrates or prodrugs thereof. A prodrug is commonly described as an inactive or protected derivative of an active ingredient or a drug, which is converted to the active ingredient or drug in the body.

In case the inventive compounds have basic and/or acidic substituents, they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in a conventional manner. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example, by treatment of a solution of the compound of the general formula (I) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Certain compounds of the general formula (I) may exist in the form of optical isomers, e.g. diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1 % w/w of the other isomers.

Another aspect of the present invention relates to the use of the inventive quinolinyloxyphenylsulfonamide derivatives as drugs, i.d. as pharmaceutically active agents applicable in medicine.

The preferred AXL-tyrosine-kinase-induced disorders are selected from adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary origin), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear tumors, nose tumors and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors of the gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, and tongue cancer.

Still another aspect of the present invention is directed to pharmaceutical compositions comprising at least one compound of the present invention as active ingredient, together with at least one pharmaceutically acceptable carrier, excipient, and/or diluents. The pharmaceutical compositions of the present invention can be prepared in a conventional solid or liquid carrier or diluent, and a conventional pharmaceutically-made adjuvant at suitable dosage level in a known way. The preferred preparations are adapted for oral application. These administration forms include, for example, pills, tablets, film tablets, coated tablets, capsules, powders, and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention, and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersible granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the compound of the invention and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate.

Moreover, the pharmaceutical compositions of the present invention may be formulated in sustained release form to provide a rate-controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol, and/or an emulsion and may be included in a transdermal patch of the matrix or reservoir type as is known in the art for this purpose.

Oral gels refer to the active ingredients dispersed or solubilised in a hydrophilic semi-solid matrix. Powders for constitution refers to powder blends containing the active ingredients and suitable diluents which can be suspended e.g. in water or in juice.

The compound of the present invention is for use as a medicament, preferably in a human subject or in an animal subject, in human medicine or in veterinary medicine, respectively. In particular, the compound is used in human medicine. In particular, the compound for use as a veterinary agent.

A "subject" as used herein is any animal or human (while animal in a broader sense involves humans and non-human animals, whereas the latter may be called animals, if limitation so requires) which may have a cancer or tumor. Preferably the subject is selected from a vertebrate animal, preferably selected from the group consisting of amphibians, reptiles, fishes, birds, and mammals, highly preferably mammals, particularly preferably humans.

A "patient" is a subject under medical care, preferably a subject having a disease or being endangered or susceptible to a disorder.

In this concept definition of treatment involves prevention.

A cancer is a disease, which is a malignant neoplasm, involving abnormal cell growth with the potential to invade or spread to other parts of the body (metastasis).

Preferably the compound is used as an anti¬ cancer agent in a patient having cancer or a patient endangered by development of cancer (e.g. recurrence of cancer) or a patient endangered by development of metastasis or having metastasis.

In particular, the compound for use is for use in the treatment of a neoplasm (or cancer). A cancer may be e.g. a carcinoma, a sarcoma, a hematologic cancer of blood-related cells, like a myeloma, a leukemia or a lymphoma or may be a mixed type.

In a particular embodiment the cancer is a blastoma.

As examples, the carcinoma is adenocarcinoma or squamous cell carcinoma. In particular, the sarcoma is selected from osteosarcoma or osteogenic sarcoma, chondrosarcoma, leiomyosarcoma, rhabdomyosarcoma, mesothelial sarcoma or mesothelioma, fibrosarcoma, angiosarcoma or hemangioendothelioma, liposarcoma, glioma or astrocytoma, myxosarcoma, and mesenchymous or mixed mesodermal tumors.

The neoplasm, without limitation may be e.g an ovarian cancer, a breast cancer, a skin cancer, a pancraetic cancer, a blastoma, like glioblastoma, etc.

The term hyperproliferative disorder relates to a disorder or disease which includes pathologies characterized by uncontrolled proliferation of skin cells. Typically a hyperproliferative disorder isa neoplasm. In an embodiment the neoplasm is any neoplasm listed herein or listed in are listed in (Kumar et al., 2017).

The term "hyperfunction", e.g. hyperfunction of an enzyme, involves increased expression (i.e. a protein expression level higher than the normal level or the level in healthy persons, and an increased activity e.g. by upregulation of the kinase activity).

A "normal level" or the "level in healthy persons" means a range of expression or activity levels which is typical of healthy person, if measured in a statistically reasonable number (sample) of healthy persons and a statistically reasonable range is calculated by using a distribution of data acceptable in the field.

The singular forms "a", "an" and "the", or at least "a", "an", include plural reference unless the context clearly dictates otherwise.

The term "comprises" or "comprising" or "including" are to be construed here as having a non-exhaustive meaning and allow the addition or involvement of further features or method steps or components to anything which comprises the listed features or method steps or components. "Comprising" can be substituted by "including" if the practice of a given language variant so requires or can be limited to "consisting essentially of" if other members or components are not essential to reduce the invention to practice. 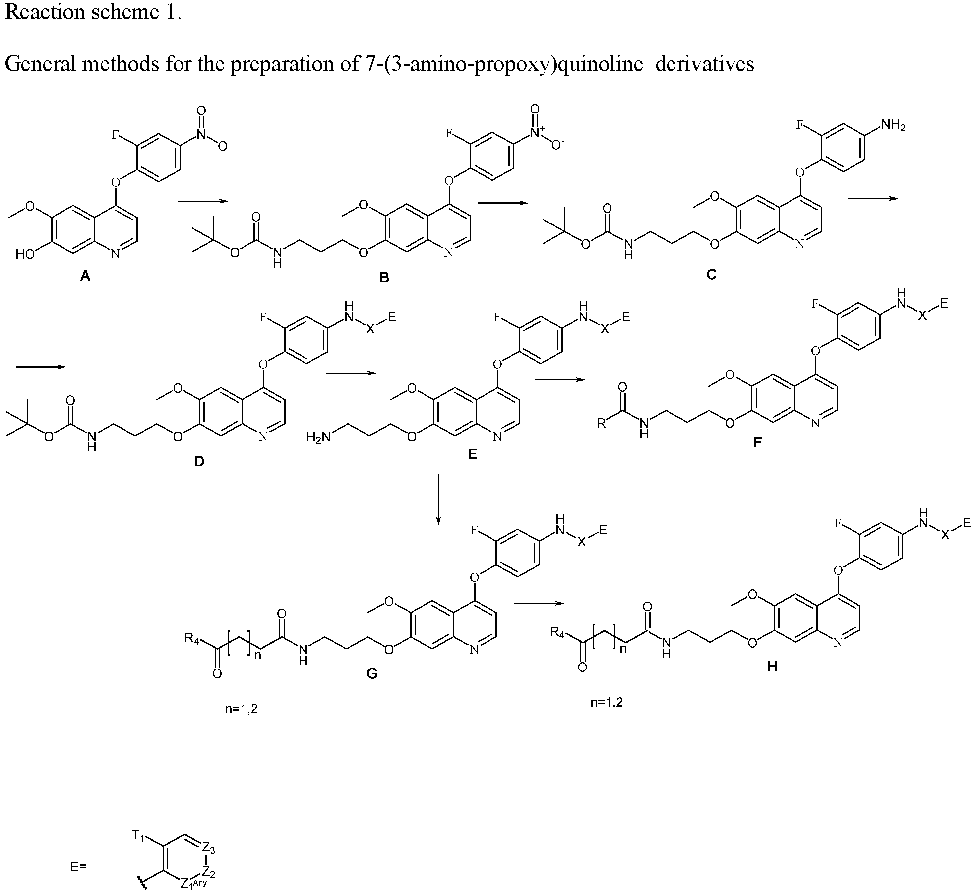 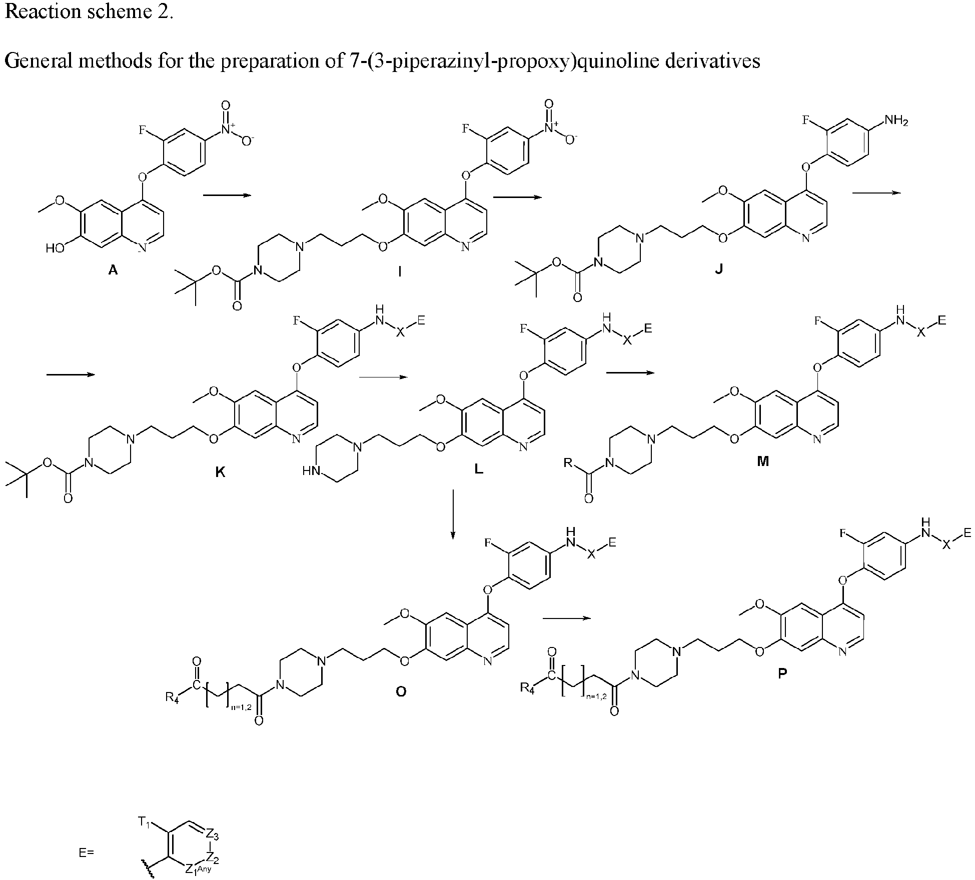 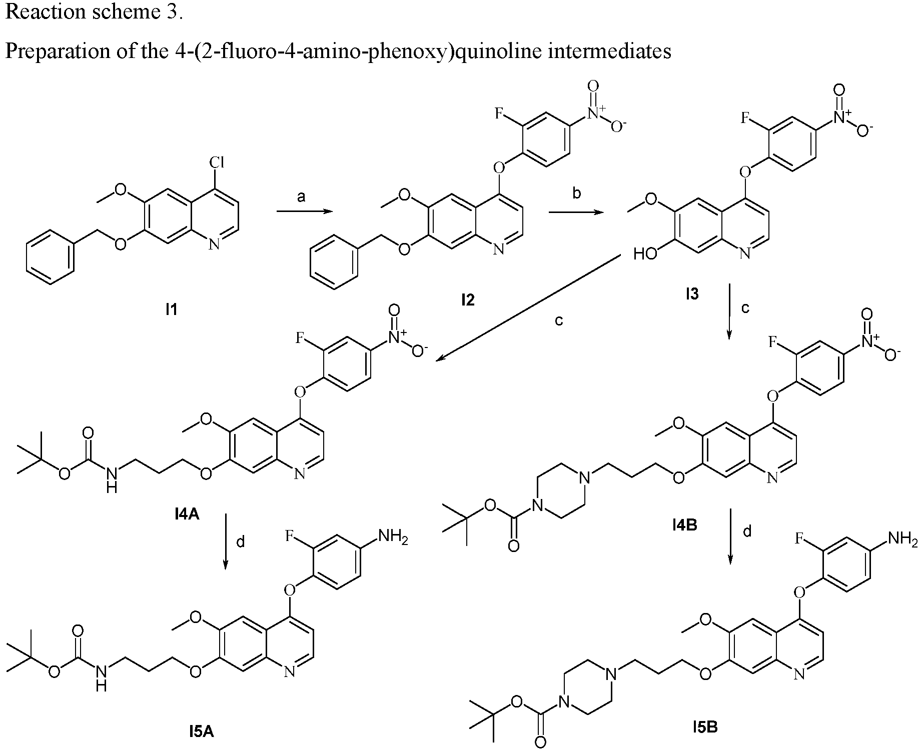 [J. Med. Chem. (2018), 61, 6277-6292]

Reagents and conditions: (a) 4-nitrophenols, chlorobenzene, reflux, 2 days; (b) 33% HBr solution, RT, 2 h; (c) alkyl halogenides, MeCN, Cs₂CO₃, KI, RT for 45 min, then reflux for 3 h; (d) Zn, NH₄Cl, EtOH/H₂O, reflux, 3 hours.

### 7-benzyloxy-4-(2-fluoro-4-nitro-phenoxy)-6-methoxy-quinoline (I2) SZI3052

A mixture of 7-benzyloxy-4-chloro-6-methoxy-quinoline (I1) (12.00 g, 40 mmol), 2-fluoro- 4-nitrophenol (7.00 g 44 mmol), N,N-diisopropylethylamine (10.32 g 80 mmol) and chlorobenzene (60 mL) was stirred at reflux temperature for 2 days. The reaction mixture was cooled down, diluted with aqueous sodium hydroxide solution (10 wt%, 80 mL) and stirred for 3 hours. The precipitate was filtered and the filtrate was separated. The aqueous layer was extracted with chloroform (3 x 60 mL). The organic layers were combined, washed with water and dried over sodium sulfate. The solvent was removed by evaporation under reduced pressure. The residue solidified under diisopropyl ether to give 11.74 g of the title compound. Yellow solid. Yield: 70%. Melting point: 170-171°C. LCMS Anal. calculated for C₂₃H₁₇FN₂O₅: 420.1, m/z: 421.2 [M+H]⁺, t_{R} = 3.42 min; peak area 98%. ¹H NMR (300 MHz, DMSO-d6): δ 8.56 (d, J = 5.1 Hz, 1H), 8.45 (dd, J = 10.5, 2.5 Hz, 1H), 8.20 (ddd, J = 8.8, 2.5, 1.5 Hz, 1H), 7.61 (dd, J = 8.8, 8.8 Hz, 1H), 7.56 (s, 1H), 7.53 (m, 2H), 7.48 (s, 1H), 7.32-7.47 (m, 3H), 6.78 (d, J = 5.1 Hz, 1H), 5.33 (s, 2H), 3.93 (s, 3H).

### 4-(2-fluoro-4-nitro-phenoxy)-6-methoxy-quinolin-7-ol (I3) SZI3102

7-Benzyloxy-4-(2-fluoro-4-nitro-phenoxy)-6-methoxy-quinoline (I2) (4.20 g, 10.00 mmol) was added slowly in portions into hydrobromic acid solution (33 % in acetic acid, 100 mL). The mixture was stirred at room temperature for 2 hours then poured into diethyl ether (600 mL). The precipitate was filtered and washed with diethyl ether. The solid hydrobromide salt was stirred in the mixture of sodium acetate aqueous solution (10 %, 120 mL) and ethyl acetate (30 mL) for 1 day. The precipitate was filtered, washed with water and dried to give 2.80 g of the title compound. Yellow solid. Yield: 85%. Melting point: 219-220 °C. LCMS Anal. calculated for C₁₆H₁₁FN₂O₅: 330.0, found m/z: 329.1 [M-H]⁻, 331.1 [M+H]⁺, t_{R} = 2.57 min; peak area 98%. ¹H NMR (300 MHz, DMSO-d6): δ 11.72 (br. s, 1H), 8.86 (d, J = 6.6 Hz, 1H), 8.56 (dd, J = 10.2, 2.5 Hz, 1H), 8.32 (ddd, J = 9.1, 2.5, 1.4 Hz, 1H), 7.90 (dd, J = 9.1, 7.8 Hz, 1H), 7.75 (s, 1H), 7.55 (s, 1H), 7.13 (d, J = 6.6 Hz, 1H), 4.04 (s, 3H).

### tert-butyl N-[3-[[4-(2-fluoro-4-nitro-phenoxy)-6-methoxy-7-quinolyl]oxy]propyl]carbamate (I4A) SZI3081

The mixture of 4-(2-fluoro-4-nitro-phenoxy)-6-methoxy-quinolin-7-ol (I3) (3.3 g, 10.0 mmol), cesium carbonate (9.77 g, 29.9 mmol), potassium iodide (150 mg, 0.90 mmol) and acetonitrile (150 mL) was stirred at room temperature for 30 minutes. Then (3-bromo-propyl)-carbamic acid tert-butyl ester (2.61 g, 11.0 mmol) was added to the reaction mixture. It was stirred at room temperature for 45 minutes, and then refluxed for 3 hours. The hot mixture was filtered, the filtrate was evaporated. The residue was taken up in the mixture of chloroform (50 mL) and water (50 mL). The two phases were separated then the aqueous layer was extracted with chloroform (2 x 20 mL). The organic layers were combined, washed with water and dried over sodium sulfate. The solvent was removed by evaporation under reduced pressure. The residue was solidified under diisopropyl ether. Thus, 4.33 g of the title compound was obtained. Yellow solid. Yield: 89%. Melting point: 114-115 °C. LCMS Anal. calculated for C₂₄H₂₆FN₃O₇: 487.1, found m/z: 488.3 [M+H]⁺, t_{R} = 3.45 min; peak area 97%. ¹H NMR (300 MHz, DMSO-d6): δ 8.57 (d, J = 5.1 Hz, 1H), 8.45 (dd, J = 10.4, 2.5 Hz, 1H), 8.20 (ddd, J = 9.0, 2.5, 1.3 Hz, 1H), 7.61 (dd, J = 9.0, 8.2 Hz, 1H), 7.46 (s, 1H), 7.44 (s, 1H), 6.89 (t, J ~ 5.0 Hz, 1H), 6.78 (d, J = 5.1 Hz, 1H), 4.19 (t, J = 6.1 Hz, 2H), 3.93 (s, 3H), 3.15 (q, J ~ 6.2 Hz, 2H), 1.94 (quint, J ~ 6.3 Hz, 2H), 1.14 (s, 9H).

### tert-butyl 4-[3-[[4-(2-fluoro-4-nitro-phenoxy)-6-methoxy-7-quinolyl]oxy]propyl]piperazine-1-carboxylate (I4B) SZI3117, SZI3008T2

The mixture of 4-(2-fluoro-4-nitro-phenoxy)-6-methoxy-quinolin-7-ol (I3) (8.72 g, 26.4 mmol), cesium carbonate (8.47 g, 32.2 mmol), potassium iodide (450 mg, 2.70 mmol) and acetonitrile (200 mL) was stirred at room temperature for 30 minutes. Then tert-butyl 4-(3-chloropropyl)piperazine-1-carboxylate (7.71 g, 29.3 mmol) was added to the reaction mixture. It was stirred at room temperature for 45 minutes, and then refluxed for 3 hours. The hot mixture was filtered, the filtrate was evaporated. The residue was taken up in the mixture of chloroform (50 mL) and water (50 mL). The two phases were separated then the aqueous layer was extracted with chloroform (2 x 20 mL). The organic layers were combined, washed with water and dried over sodium sulfate. The solvent was removed by evaporation under reduced pressure. The residue was purified by column chromatography (eluent: chloroform - methanol = 95:5). The pure product solidified under diisopropyl ether. Thus, 8.23 g of the title compound was obtained. Yellow solid. Yield: 56%. LCMS Anal. calculated for C28H33FN4O7: 556.2, found m/z: 557.3 [M+H]⁺, t_{R} = 2.73, 2.91 min; peak area 97%.

### tert-butyl N-[3-[[4-(4-amino-2-fluoro-phenoxy)-6-methoxy-7-quinolyl]oxy]propyl]carbamate (I5A) SZI3089A

A mixture of tert-butyl N-[3-[[4-(2-fluoro-4-nitro-phenoxy)-6-methoxy-7-quinolyl]oxy]propyl]carbamate (I4A) (2.31 mg, 4.73 mmol), ammonium chloride (2.31 mg, 43.1 mmol), zinc powder (14.0 g, 214.1 mmol) ethyl alcohol (230 mL) and water (23 mL) was stirred at reflux temperature for 3 hours. The hot reaction mixture was filtered through Celite, washed with hot ethyl alcohol then the filtrate was evaporated in vacuum. The residue was taken up in the mixture of ethyl acetate (100 mL) and aqueous sodium hydrogencarbonate solution (40 mL) and stirred for 1 hour. The two layers were separated. The aqueous layer was extracted with ethyl acetate (3 x 20 mL). The organic layers were combined, washed with brine and dried over sodium sulfate. The solvent was removed by evaporation under the reduced pressure. The residue solidified under diisopropyl ether to give 1.82 g of the title compound. Gray solid. Yield: 84%. LCMS Anal. calculated for C₂₄H₂₈FN₃O₅: 457.2, found m/z: 458.3 [M+H]⁺, t_{R} = 3.00 min; peak area 98%. ¹H NMR (300 MHz, DMSO-d6): δ 8.45 (d, J = 5.2 Hz, 1H), 7.51 (s, 1H), 7.36 (s, 1H), 7.07 (dd, J = 9.2, 8.8 Hz, 1H), 6.89 (t, J ~ 5.5 Hz, 1H), 6.55 (dd, J = 13.0, 2.5 Hz, 1H), 6.47 (ddd, J = 8.8, 2.5, 1.0 Hz, 1H), 6.39 (dd, J = 5.2, 0.9 Hz, 1H), 5.46 (s, 2H), 4.16 (t, J = 6.4 Hz, 2H), 3.95 (s, 3H), 3.15 (q, J ~ 5.9 Hz, 2H), 1.93 (quint, J ~ 6.4 Hz, 2H), 1.39 (s, 9H).

### tert-butyl 4-[3-[[4-(4-amino-2-fluoro-phenoxy)-6-methoxy-7-quinolyl]oxy]propyl]piperazine-1-carboxylate (I5B) SZI3082A

A mixture of tert-butyl 4-[3-[[4-(2-fluoro-4-nitro-phenoxy)-6-methoxy-7-quinolyl]oxy]propyl]piperazine-1-carboxylate (I4B) (3.31 g, 5.94 mmol), ammonium chloride (3.31 g, 6.18 mmol), zinc powder (19.8 g, 302.8 mmol) ethyl alcohol (320 mL) and water (32 mL) was stirred at reflux temperature for 3 hours. The hot reaction mixture was filtered through Celite, washed with hot ethyl alcohol then the filtrate was evaporated in vacuum. The residue was taken up in the mixture of ethyl acetate (100 mL) and aqueous sodium hydrogencarbonate solution (40 mL) and stirred for 1 hour. The two layers were separated. The aqueous layer was extracted with ethyl acetate (3 x 20 mL). The organic layers were combined, washed with brine and dried over sodium sulfate. The solvent was removed by evaporation under the reduced pressure. The residue solidified under diisopropyl ether to give 2.16 g of the title compound. Gray solid. Yield: 69%. LCMS Anal. calculated for C₂₈H₃₅FN₄O₅: 526.2, found m/z: 527.3 [M+H]⁺, t_{R} = 2.23 min; peak area 98%. ¹H NMR (300 MHz, DMSO-d6): δ 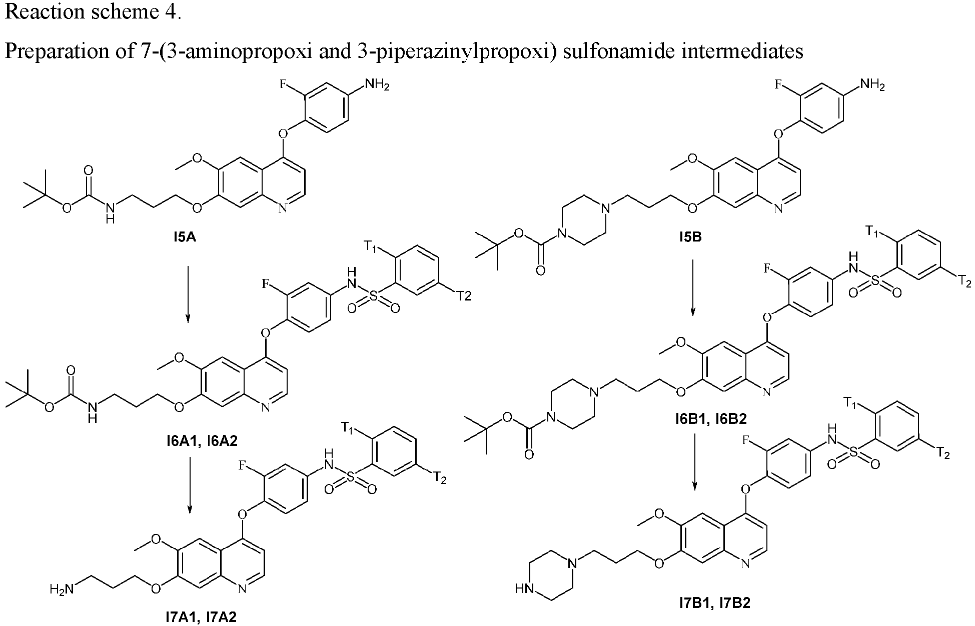 [J. Med. Chem. (2018), 61, 6277-6292]

### tert-butyl N-[3-[[4-[4-[(2,5-difluorophenyl)sulfonylamino]-2-fluoro-phenoxy]-6-methoxy-7-quinolyl]oxy]propyl]carbamate (I6A1) SZI3090

The mixture of 2,5-difluorobenzenesulfonyl chloride (678 mg, 3.09 mmol), pyridine (25 mL) and tert-butyl N-[3-[[4-(4-amino-2-fluoro-phenoxy)-6-methoxy-7-quinolyl]oxy]propyl]carbamate (I5a) (1374 mg 3.00 mmol) was stirred at room temperature for a week. The mixture was poured into the mixture of chloroform and saturated sodium hydrogencarbonate solution and stirred for 2 hours. The two layers were separated. The aqueous layer was extracted with chloroform (3 x 20 mL). The organic layers were combined, washed with water and dried over sodium sulfate. The solvent was removed by evaporation under reduced pressure. Ethyl alcohol was added to the residue and it was evaporated again in order to remove the remaining pyridine. The residue was dissolved in methanol, then Celite and activated charcoal was added. It was strirred at room temperature for one hours. Filtered, the filtrate was evaporated in vacuum. The residue was solidified under diisopropyl ether. Thus, 1.62 g of the title compound was obtained as a white solid. Yield: 85%. LCMS Anal. calculated for C₃₀H₃₀F₃N₃O₇S: 633.1, found m/z: 632.2 [M-H]⁻, 634.2 [M+H]⁺, t_{R} = 3.63 min.; peak area 96 %. ¹H NMR (300 MHz, DMSO-d6): δ 11.12 (br. s, 1H), 8.45 (d, J = 5.2 Hz, 1H), 7.72 (ddd, J = 8.2, 5.3, 2.9 Hz, 1H), 7.64 (dddd, J = 13.0, 9.0, 4.0, 2.9 Hz, 1H), 7.56 (ddd, J = 9.0, 9.0, 4.6 Hz, 1H), 7.48 (s, 1H), 7.39 (dd, J = 9.5, 8.8 Hz, 1H), 7.38 (s, 1H), 7.19 (dd, J = 13.5, 2.5 Hz, 1H), 7.05 (ddd, J = 8.8, 2.5, 1.1 Hz, 1H), 6.89 (t, J ~ 5.0 Hz, 1H), 6.35 (dd, J = 5.2, 0.9 Hz, 1H), 4.16 (t, J = 6.2 Hz, 2H), 3.92 (s, 3H), 3.14 (td, J ~ 7.0, 5.0 Hz, 2H), 1.93 (tt, J ~ 7.0, 6.2 Hz, 2H), 1.38 (s, 9H).

### tert-butyl N-[3-[[4-[2-fluoro-4-[[2-(trifluoromethyl)phenyl]sulfonylamino]phenoxy]-6-methoxy-7-quinolyl]oxy]propyl]carbamate ( I6A2). SZI3091

The mixture of 2-(trifluoromethyl)benzenesulfonyl chloride (780 mg, 3.09 mmol), pyridine (25 mL) and tert-butyl N-[3-[[4-(4-amino-2-fluoro-phenoxy)-6-methoxy-7-quinolyl]oxy] propyl]carbamate (I5a) (1374 mg 3.00 mmol) was stirred at room temperature for a week. The mixture was poured into the mixture of chloroform and saturated sodium hydrogencarbonate solution and stirred for 2 hours. The two layers were separated. The aqueous layer was extracted with chloroform (3 x 20 mL). The organic layers were combined, washed with water and dried over sodium sulfate. The solvent was removed by evaporation under reduced pressure. Ethyl alcohol was added to the residue and it was evaporated again in order to remove the remaining pyridine. The residue was dissolved in methanol, then Celite and activated charcoal was added. It was strirred at room temperature for one hours. Filtered, the filtrate was evaporated in vacuum. The residue was solidified under small amount of methanol. Thus, 1.33 g of the title compound was obtained as a white solid. Yield: 66%. LCMS Anal. calculated for C₃₁H₃₁F₄N₃O₇S: 665.18, found m/z: 664.2 [M-H]⁻, 666.2 [M+H]⁺, t_{R} = 3.75 min; peak area 100%. ¹H NMR (300 MHz, DMSO-d6): 11.03 (br. s, 1H), 8.45 (d, J = 5.2 Hz, 1H), 8.16 (dm, J = 7.8 Hz, 1H), 8.04 (dm, J = 6.8 Hz, 1H), 7.92 (m, 1H), 7.89 (m, 1H), 7.47 (s, 1H), 7.39 (dd, J = 8.9, 8.9 Hz, 1H), 7.37 (s, 1H), 7.17 (dd, J = 12.2, 2.4 Hz, 1H), 7.03 (ddd, J = 8.9, 2.4, 1.2 Hz, 1H), 6.89 (t, J ~ 5.3 Hz, 1H), 6.36 (dd, J = 5.2, 0.9 Hz, 1H), 4.16 (t, J = 6.2 Hz, 2H), 3.92 (s, 3H), 3.14 (q, J ~ 6.3 Hz, 2H), 1.92 (quint, J ~ 6.3 Hz, 2H), 1.38 (s, 9H).

### tert-butyl 4-[3-[[4-[4-[(2,5-difluorophenyl)sulfonylamino]-2-fluoro-phenoxy]-6-methoxy-7-quinolyl]oxy]propyl]piperazine-1-carboxylate (I6B1) SZI3084

The mixture of 2,5-difluorobenzenesulfonyl chloride (425 mg, 1.94 mmol), pyridine (25 mL) and tert-butyl 4-[3-[[4-(4-amino-2-fluoro-phenoxy)-6-methoxy-7-quinolyl]oxy]propyl]piperazine-1-carboxylate (I5b) (1053 mg 2.00 mmol) was stirred at room temperature for a week. The mixture was poured into the mixture of chloroform and saturated sodium hydrogencarbonate solution and stirred for 2 hours. The two layers were separated. The aqueous layer was extracted with chloroform (3 x 20 mL). The organic layers were combined, washed with water and dried over sodium sulfate. The solvent was removed by evaporation under reduced pressure. Ethyl alcohol was added to the residue and it was evaporated again in order to remove the remaining pyridine. The residue was dissolved in methanol, then Celite and activated charcoal was added. It was strirred at room temperature for one hours. Filtered, the filtrate was evaporated in vacuum. The residue was solidified under small amount of methanol. Thus, 0.82 g of the title compound was obtained as a white solid. Yield: 60%. LCMS Anal. calculated for C₃₄H₃₇F₃N₄O₇S: 702.2, found m/z: 701.1 [M-H]⁻, 703.1 [M+H]⁺, t_{R} = 2.86 min; peak area 96%. ¹H NMR (300 MHz, DMSO-d6): δ 10.8 (bs, 1H), 8.45 (d, J = 5.2 Hz, 1H), 7.72 (m, 1H), 7.64 (m, 1H), 7.56 (m, 1H), 7.47 (s, 1H), 7.38 (m, 2H), 7.15 (dd, J = 12.1, 1.7 Hz, 1H), 7.02 (d, J = 8.9 Hz, 1H), 6.35 (d, J = 5.1 Hz, 1H), 4.19 (m, 2H), 3.92 (s, 3H), 3.16 (bs, 4H), 2.49 (m, 2H), 2.38 (m, 4H), 1.98 (m, 2H), 1.39 (s, 9H).

### tert-butyl 4-[3-[[4-[2-fluoro-4-[[2-(trifluoromethyl)phenyl]sulfonylamino]phenoxy]-6-methoxy-7-quinolyl]oxy]propyl]piperazine-1-carboxylate (I6B2) SZI3085

The mixture of 2-(trifluoromethyl)benzenesulfonyl chloride (490 mg, 1.94 mmol), pyridine (25 mL) and tert-butyl 4-[3-[[4-(4-amino-2-fluoro-phenoxy)-6-methoxy-7-quinolyl]oxy] propyl]piperazine-1-carboxylate (I5b) (1053 mg 2.00 mmol) was stirred at room temperature for a week. The mixture was poured into the mixture of chloroform and saturated sodium hydrogencarbonate solution and stirred for 2 hours. The two layers were separated. The aqueous layer was extracted with chloroform (3 x 20 mL). The organic layers were combined, washed with water and dried over sodium sulfate. The solvent was removed by evaporation under reduced pressure. Ethyl alcohol was added to the residue and it was evaporated again in order to remove the remaining pyridine. The residue was dissolved in methanol, then Celite and activated charcoal was added. It was strirred at room temperature for one hours. Filtered, the filtrate was evaporated in vacuum. The residue was solidified under small amount of methanol. Thus, 0.85 g of the title compound was obtained as a white solid. Yield: 59%. LCMS Anal. calculated for C₃₅H₃₈F₄N₄O₇S: 734.2, found m/z: 733.3 [M-H]⁻, 735.3 [M+H]⁺, t_{R} = 3.01 min; peak area 95%. ¹H NMR (300 MHz, DMSO-d6): δ 11.0 (bs, 1H), 8.44 (d, J = 5.1 Hz, 1H), 8.16 (d, J = 7.2 Hz, 1H), 8.02 (d, J = 6.7 Hz, 1H), 7.89 (m, 2H), 7.47 (s, 1H), 7.37 (m, 2H), 7.17 (dd, J = 12.4, 2.2 Hz, 1H), 7.02 (d, J = 9.2 Hz, 1H), 6.36 (dd, J = 5.2, 0.9 Hz, 1H), 4.18 (m, 2H), 3.92 (s, 3H), 3.16 (bs, 4H), 2.50 (m, 2H), 2.38 (m, 4H), 1.97 (m, 2H), 1.39 (s, 9H).

### N-[4-[[7-(3-aminopropoxy)-6-methoxy-4-quinolyl]oxy]-3-fluoro-phenyl]-2,5-difluoro-benzenesulfonamide (I7A1). SZI3094

A mixture of 1.61 g (2.54 mmol) tert-butyl N-[3-[[4-[4-[(2,5-difluorophenyl)sulfonylamino]-2-fluoro-phenoxy]-6-methoxy-7-quinolyl]oxy]propyl]carbamate (I6A1), ethyl acetate (80 mL) and hydrogenchloride solution (6 mL, 4N in dioxane) was stirred at room temperature for a day. To the reaction mixture sodium hydrogencarbonate aqueous solution was added until its pH reached 9, then stirred for 1 hours. The two layers were separated. The aqueous layer was extracted with ethyl acetate (2 x 20 mL). The organic layers were combined, washed with brine and dried over sodium sulfate. The solvent was removed by evaporation under reduced pressure. The residue was solidified under diisopropyl ether. Thus, 1.316 g of the title compound was obtained as white solid. Yield: 96%. LCMS Anal. calculated for C₂₅H₂₂F₃N₃O₅S: 533.1, found m/z: 532.2 [M-H]⁻, 534.2 [M+H]⁺, t_{R} = 2.40, 2.25 and 0.47 min; peak area 97%. ¹H NMR (300 MHz, DMSO-d6): δ 11.37 (s, 1H), 8.43 (d, J = 5.2 Hz, 1H), 7.51 (s, 1H), 7.46 (ddd, J = 8.2, 5.3, 2.8 Hz, 1H), 7.39 (s, 1H), 7.20-7.28 (ovl. m, 2H), 6.98 (dd, J = 9.2, 8.8 Hz, 1H), 6.82 (dd, J = 14.3, 2.4 Hz, 1H), 6.67 (ddd, J = 8.8, 2.4, 0.8 Hz, 1H), 6.36 (dd, J = 5.2, 0.9 Hz, 1H), 4.24 (t, J = 6.2 Hz, 2H), 3.94 (s, 3H), 2.96 (t, J = 7.1 Hz, 2H), 2.06 (tt, J = 7.1, 6.2 Hz, 2H). (The sulfonamide NH signal obscured).

### N-[4-[[7-(3-aminopropoxy)-6-methoxy-4-quinolyl]oxy]-3-fluoro-phenyl]-2-(trifluoromethyl)benzenesulfonamide (I7A2). SZI3095

A mixture of tert-butyl N-[3-[[4-[2-fluoro-4-[[2-(trifluoromethyl)phenyl] sulfonylamino]phenoxy]-6-methoxy-7-quinolyl]oxy]propyl]carbamate ( I6A2, 1.32 g, 19.84 mmol), ethyl acetate (80 mL) and hydrogenchloride solution (10 mL, 4N in dioxane) was stirred at room temperature a day. To the reaction mixture sodium hydrogencarbonate aqueous solution was added until its pH reached 9, then stirred for 1 hours. The two layers were separated. The aqueous layer was extracted with ethyl acetate (2 x 20 mL). The organic layers were combined, washed with brine and dried over sodium sulfate. The solvent was removed by evaporation under reduced pressure. The residue was solidified under diisopropyl ether. Thus, 0.97 g of the title compound was obtained as white solid. Yield: 86%. LCMS Anal. calculated for C₂₆H₂₃F₄N₃O₅S: 565.1, found m/z: 564.2 [M-H]⁻, 566.2 [M+H]⁺, t_{R} = 2.47, 2.33 and 0.44 min; peak area 100%. ¹H NMR (300 MHz, DMSO-d6): δ 8.44 (d, J = 6.2 Hz, 1H), 8.10 (dd, J = 7.5, 1.0 Hz, 1H), 7.78 (dd, J = 7.0, 1.6 Hz, 1H), 7.65 (ddd, J = 7.4, 7.0, 1.0 Hz, 1H), 7.57 (ddd, J = 7.5, 7.4. 1.6 Hz, 1H), 7.51 (s, 1H), 7.40 (s, 1H), 6.98 (dd, J = 9.0, 8.8 Hz, 1H), 6.78 (dd, J = 12.0, 2.4 Hz, 1H), 6.66 (ddd, J = 8.8, 2.4, 1.2 Hz, 1H), 6.36 (dd, J = 6.2, 0.9 Hz, 1H), 4.25 (t, J = 6.0 Hz, 2H), 3.93 (s, 3H), 3.01 (t, J = 7.3 Hz, 2H), 2.09 (tt, J = 7.3, 6.0, 2H). (The sulfonamide NH signal obscured).

### 2,5-difluoro-N-[3-fluoro-4-[[6-methoxy-7-(3-piperazin-1-ylpropoxy)-4-quinolyl]oxy]phenyl]benzenesulfonamide (I7B1) SZI3092

A mixture of tert-butyl 4-[3-[[4-[4-[(2,5-difluorophenyl)sulfonylamino]-2-fluoro-phenoxy]-6-methoxy-7-quinolyl]oxy]propyl]piperazine-1-carboxylate (I6B1, 765 mg, 1.08 mmol), ethyl acetate (40 mL) and hydrogenchloride solution (5 mL, 4N in dioxane) was stirred at room temperature a day. To the reaction mixture sodium hydrogencarbonate aqueous solution was added until its pH reached 9, then stirred for 1 hours. The two layers were separated. The aqueous layer was extracted with ethyl acetate (2 x 20 mL). The organic layers were combined, washed with brine and dried over sodium sulfate. The solvent was removed by evaporation under reduced pressure. The residue was solidified under diisopropyl ether. Thus, 0.62 g of the title compound was obtained as white solid. Yield. 94%. LCMS Anal. calculated for C₂₉H₂₉F₃N₄O₅S: 602.18, found m/z: 601.3 [M-H]⁻, 603.3 [M+H]⁺, t_{R} = 2.36, 2.25 and 0.44 min; peak area 100%. ¹H NMR (300 MHz, DMSO-d6): 11.0 (bs, 1H), 8.42 (d, J = 5.2 Hz, 1H), 7.49 (bs, 2H), 7.37 (bs, 1H), 7.27 (bs, 2H), 7.01 (t, J = 8.9 Hz, 1H), 6.85 (d, J = 8.9 Hz, 1H), 6.70 (d, J = 7.9 Hz, 1H), 6.35 (d, J = 5.1 Hz, 1H), 4.19 (bs, 2H), 3.92 (s, 3H), 3.00 (bs, 4H), 2.49 (m, 2H), 2.38 (m, 4H), 1.96 (m, 2H).

### N-[3-fluoro-4-[[6-methoxy-7-(3-piperazin-1-ylpropoxy)-4-quinolyl]oxy]phenyl]-2-(trifluoromethyl)benzenesulfonamide (I7B2) SZI3093

A mixture of tert-butyl 4-[3-[[4-[2-fluoro-4-[[2-(trifluoromethyl)phenyl]sulfonylamino]phenoxy]-6-methoxy-7-quinolyl]oxy]propyl]piperazine-1-carboxylate (I6B2, 820 mg, 1.11 mmol), ethyl acetate (40 mL) and hydrogenchloride solution (5 mL, 4N in dioxane) was stirred at room temperature a day. To the reaction mixture sodium hydrogencarbonate aqueous solution was added until its pH reached 9, then stirred for 1 hours. The two layers were separated. The aqueous layer was extracted with ethyl acetate (2 x 20 mL). The organic layers were combined, washed with brine and dried over sodium sulfate. The solvent was removed by evaporation under reduced pressure. The residue was solidified under diisopropyl ether. Thus, 0.63 g of the title compound was obtained as white solid. Yield. 89%. LCMS Anal. calculated for C₃₀H₃₀F₄N₄O₅S: 634.18, found m/z: 633.3 [M-H]⁻, 634.3 [M+H]⁺, t_{R} = 2.48, 2.29 and 0.44 min; peak area 100%. ¹H NMR (300 MHz, DMSO-d6): 11.0 (bs, 1H), 8.44 (d, J = 5.1 Hz, 1H), 8.16 (d, J = 6.6 Hz, 1H), 7.89 (d, J = 6.7 Hz, 1H), 7.76 (m, 2H), 7.51 (s, 1H), 7.40 (s, 1H), 7.14 (m, 1H), 7.02 (d, J = 12.1 Hz, 1H), 6.89 (d, J = 8.8 Hz, 1H), 6.39 (d, J = 4.5 Hz, 1H), 4.22 (m, 2H), 3.93 (s, 3H), 2.97 (bs, 4H), 2.55 (m, 2H), 2.38 (m, 4H), 1.98 (m, 2H).

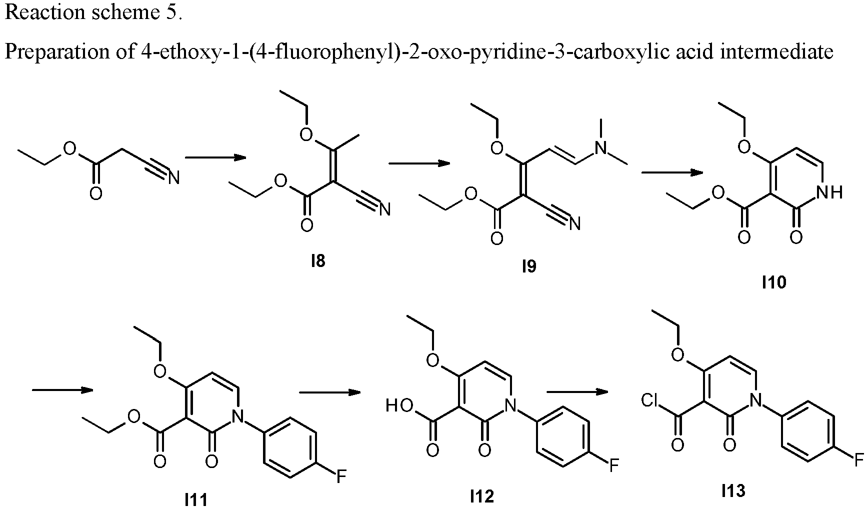

### [Organic Letters, (2015), 17(12) 2964 - 2967]

### Preparation of ethyl (2E/Z)-2-cyano-3-ethoxybut-2-enoate (I8)

20.0 g (177 mmol) ethyl cyanoacetate, 57.3 g triethyl orthoacetate and 3 ml glacial acetic acid were stirred in a 100 ml flask equipped with a Dean-Stark trap at 120-130 °C overnight. The residual volatiles were distilled off under vacuum and the oily crude product was treated with 25 ml DIPE. After cooling to 0 °C, the crystallized product was filtered off and dried. LCMS Anal. calculated for C₉H₁₃NO₃: 183.0, found m/z: 184.1 [M+H]⁺, t_{R} = 2.97, and 3.26 min; peak area 97%. ¹H NMR (300 MHz, DMSO-d6):

### Ethyl (2Z,4E)-2-cyano-5-(dimethylamino)-3-ethoxypenta-2,4-dienoate (I9)

15.0 g (81.9 mmol) ethyl (2E/Z)-2-cyano-3-ethoxybut-2-enoate and 19.5 g (163.7 mmol) N,N-dimethylformamide dimethyl acetal were stirred in a 100 ml flask equipped with a Dean-Stark trap and heated at 80 °C over 2 hours. The reaction was monitored by TLC. After the completion the volatiles were distilled off under vacuum and the crude product was used in the next step without purification. LCMS Anal. calculated for C₁₂H₁₈N₂O₃: 238.1, found m/z: 239.2 [M+H]⁺, t_{R} = 3.33 min; peak area 98%. ¹H NMR (300 MHz, DMSO-d6):

### Ethyl 4-ethoxy-2-oxo-1,2-dihydropyridine-3-carboxylate (I10)

20g (83.9mmol) ethyl (2Z,4*E*)-2-cyano-5-(dimethylamino)-3-ethoxypenta-2,4-dienoate was dissolved in 150 ml glacial acetic acid and refluxed over 48 hours. The reaction mixture was evaporated under reduced pressure and the oily residue was taken up with 50 ml aq.5% NaHCO₃ and 80 ml ethyl-acetate. The reaction mixture was stirred for 30-60 min and cooled down to 5-10 °C for an hour. The precipitated product was filtered off, washed with water and dried. LCMS Anal. calculated for C₁₀H₁₃NO₄: 211.0, found m/z: 210.1 [M-H]⁻, 212.1 [M+H]⁺, t_{R} = 3.33 min; peak area 98%. ¹H NMR (300 MHz, DMSO-d6): 11.60 (s, 1H); 7.46 (d, 1H, *J*=7.5 Hz); 6.22 (d, 1H, , *J*=7.5 Hz); 4.19-4.09 (m, 4H); 1.26-1.19 (m, 6H)

### Ethyl 4-ethoxy-1-(4-fluorophenyl)-2-oxo-1,2-dihydropyridine-3-carboxylate (I11)

4-ethoxy-2-oxo-1,2-dihydropyridine-3-carboxylic acid ethyl ester (1.50g, 7.1mmol), 4-fluorophenylboronic acid (2.0 g, 21.3 mmol), anhydrous copper acetate ( 2.85g, 14.2 mmol) and pyridine (2.2g, 28.5 mmol) in dichloromethane (20mL) exposed to air and stirred at room temperature for 6 hours, filtered, the filter residue was washed two times with 10 ml dichloromethane, and the organic phase was concentrated. The crude product was recrystallized from acetonitrile to obtain a white solid product:4-ethoxy-1-(4-fluorophenyl)-2-oxo-1,2-dihydropyridine-3-carboxylic acid ethyl ester. LCMS Anal. calculated for C₁₆H₁₆FNO₄: 305.1, found m/z: 306.2 [M+H]⁺, t_{R} = 3.33 min; peak area 98%. ¹H NMR (300 MHz, DMSO-d6): δ ; 7.81 (d, 1H, J=8.0 Hz); 7.46-7.43 (m, 2H); 7.36-7.30 (m, 2H); 6.43 (d, *J=* 8Hz, 1H), 4.17 (q, 2H), 4.23 (q, 2H), 1.28 (t, 3H, *J*=6.9 Hz), 1.22 (t, *J*=7.1 Hz, 3H).

### 4-ethoxy-1-(4-fluorophenyl)-2-oxo-1,2-dihydropyridine-3-carboxylic acid (I12)

3.05 g (10 mmol) ethyl 4-ethoxy-2-oxo-1,2-dihydropyridine-3-carboxylate was suspended in 25 ml 20% aq. HCl and stirred at reflux temperature for 16 hours. After cooling to room temperature the precipitated product was filtered off to give 1.14 g 4-ethoxy-1-(4-fluorophenyl)-2-oxo-1,2-dihydropyridine-3-carboxylic acid as a yellowish solid. LCMS Anal. calculated for C₁₄H₁₂FNO₄: 277.1, found m/z: 376.2 [M-H]⁻, 278.2 [M+H]⁺, t_{R} = 2.91 min; peak area 98%. ¹H NMR (300 MHz, DMSO-d6): δ 13.75 (brs, 1H); 7.95 (d, 1H, J=7.7 Hz); 7.51-7.47 (m, 2H); 7.39-7.33 (m, 2H); 6.60 (d, 1H, J=7.8 Hz); 4.30 (qt, 2H, J=6.9 Hz); 1.34 (t, 3H, J=6.9 Hz)

### 4-ethoxy-1-(4-fluorophenyl)-2-oxo-pyridine-3-carbonyl chloride (I13)

1 mmol carboxylic acid was dissolved in 5 ml SOCl₂ at room temperature, 1-2 droplets of DMF were added to the reaction mixture then stirred at 60 °C. After the completion, the SOCl₂ was evaporated under reduced pressure and 4-5 dry/anhydrous abs. toluene was distilled off from the residue then used in the next step without purification.

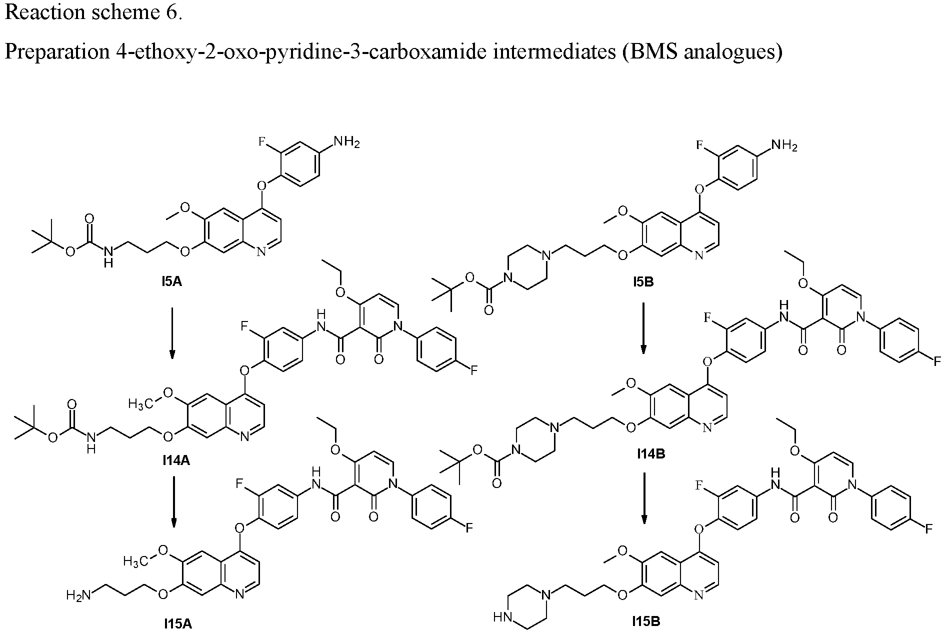

### tert-butyl [3-({4-[4-({[4-ethoxy-1-(4-fluorophenyl)-2-oxo-1,2-dihydropyridin-3-yl]carbonyl}amino)-2-fluorophenoxy]-6-methoxyquinolin-7-yl}oxy)propyl]carbamate (I14A)

0.46 g (1 mmol) *tert-*butyl (3-{[4-(4-amino-2-fluorophenoxy)-6-methoxyquinolin-7-yl]oxy}propyl)carbamate was dissolved in 15 ml anhydrous DCM and 0.18 ml TEA (1.3 mmol) was added to the solution and chilled to 0 °C. 0.37 g (1.25 mmol) 4-ethoxy-1-(4-fluorophenyl)-2-oxo-1,2-dihydropyridine-3-carbonyl chloride was dissolved in 3 ml anhydrous DCM and added to the reaction mixture under inert atmosphere then allowed to warm up to rt and stirred for 2 hours. The reaction mixture was evaporated under reduced pressure and the residue was partitioned between 10 ml 5 % aq. NaHCO₃ and 30 ml ethyl-acetate. The aqueous layer was extracted with 2x10 ml ethyl-acetate and the organic layers were combined, dried over NaSO₄ then the dessicant was filtered off. Evaporating the solvent under reduced pressure afforded the product as greyish solid. The crude product was purified by column chromatography (chloroform-methanol (sat. NH₃) 20:1). Yield: 0.31g. t_{R}: 3.40 min ¹H NMR (300 MHz, DMSO-d6): δ 10.58, (s, 1H); 8.47 (d, 1H, J=5.2 Hz); 7.85 (d, 1H, J=7.7 Hz); 7.53 (m, 1H); 7.48-7.44 (m, 3H); 7.42-7.33 (m, 3H); 6.89 (m, 1H); 6.59 (m, 1H); 6.47 (md, 1H, J=4.8 Hz); 4.26 (m, 2H); 4.17 (t, 2H, J=6.2 Hz); 3.95 (s, 3H); 3.18-3.11 (m, 4H); 1.93 (m, 2H); 1.38 (s, 9H); 1.31 (t, 3H, J=7.0 Hz).

*tert-*butyl 4-[3-({4-[4-({[4-ethoxy-1-(4-fluorophenyl)-2-oxo-1,2-dihydropyridin-3-yl]carbonyl}amino)-2-fluorophenoxy]-6-methoxyquinolin-7-yl}oxy)propyl]piperazine-1-carboxylate (14B)

0.52 g (1 mmol) *tert-*butyl 4-(3-{[4-(4-amino-2-fluorophenoxy)-6-methoxyquinolin-7-yl]oxy}propyl) piperazine-1- carboxylate was dissolved in 20 ml anhydrous DCM and 0.18 ml TEA (1.3 mmol) was added to the solution and chilled to 0 °C. 0.37 g (1.25 mmol) 4-ethoxy-1-(4-fluorophenyl)-2-oxo-1,2-dihydropyridine-3-carbonyl chloride (prepared by the above mentioned method) was dissolved in 3 ml anhydrous DCM and added to the reaction mixture under inert atmosphere then allowed to warm up to rt and stirred for 2 hours. The reaction mixture was evaporated under reduced pressure and the residue was partitioned between 10 ml 5 % aq. NaHCO₃ and 30 ml ethyl-acetate. The aqueous layer was extracted with 2x10 ml ethyl-acetate and the organic layers were combined, dried over NaSO₄ then the dessicant was filtered off. Evaporating the solvent under reduced pressure afforded the product as greyish solid. The crude product was purified by column chromatography (chloroform-methanol(sat. NH₃) 20: 1) Yield: 0.38g t_{R} : 2.84 min. ¹H NMR (300 MHz, DMSO-d6): 10.56 (s, 1H); 8.45 (d, 1H, J=5.2 Hz); 7.93 (d, 1H, J=5.6Hz);7.85 (d, 1H, J=5.2 Hz); 7.53-7.44 (m, 4H); 7.41-7.33 (m, 4H); 6.51 (d, 1H, 7.8 Hz); 6.46 (d, 1H, J=5.2); 4.28 (m, 2H); 4.19 (m, 2H); 3.91 (s, 3H); 2.35 (m, 4H); 1.78 (m, 2H); 1.39 (s, 9H), 1.31 (t, 3H).

### N-(4-{[7-(3-aminopropoxy)-6-methoxyquinolin-4-yl]oxy}-3-fluorophenyl)-4-ethoxy-1-(4-fluorophenyl)-2-oxo-1,2-dihydropyridine-3-carboxamide (I15A)

0.45 g (0.57 mmol) tert-butyl [3-({4-[4-({[4-ethoxy-1-(4-fluorophenyl)-2-oxo-1,2-dihydropyridin-3-yl]carbonyl}amino)-2-fluorophenoxy]-6-methoxyquinolin-7-yl}oxy)propyl]carbamate was dissolved in 20 ml ethyl-acetate and cooled to 0 °C. 3 ml saturated HCl (g) ethyl-acetate was added to the reaction mixture in one portion then allowed to warm up to rt and stirred for an hour. The solvent was removed under vacuum and the residue was taken up with 10 ml aq. saturated K₂CO₃ and extracted with 20 ml dichloromethane. The organic layer was separated and the aqueous layer was washed with 2x10 dichloromethane. The combined organic layer was dried over Na₂SO₄. The product was used in the next step without purification. t_{R}: 2.39 min ¹H NMR (300 MHz, DMSO-d6): δ 10.62 (s, 1H); 8.61 (d, 1H); 8.01 (d, 1H); 7.95 (d, 1H); 7.85 (bs, 2H);7.65 (s, 1H); 7.55-7.29 (ov.m. 7H); 6.70 (d, 1H); 6.52 (d, 1H); 4.00 (s, 3H); 3.91 (q, 4H); 3.04 (t, 3H); 2.51 (q, 2H); 1.31 (t, 3H)

### 4-ethoxy-N-(3-fluoro-4-{[6-methoxy-7-(3-piperazin-1-ylpropoxy)quinolin-4-yl]oxy}phenyl)-1-(4-fluorophenyl)-2-oxo-1,2-dihydropyridine-3-carboxamide (I15B)

0.392 g (0.5 mmol) *tert-*butyl 4-[3-({4-[4-({[4-ethoxy-1-(4-fluorophenyl)-2-oxo-1,2-dihydropyridin-3-yl]carbonyl}amino)-2-fluorophenoxy]-6-methoxyquinolin-7-yl}oxy)propyl]piperazine-1-carboxylate was dissolved in 20 ml ethyl-acetate and cooled to 0 °C. 3 ml saturated HCl (g) ethyl-acetate was added to the reaction mixture in one portion then allowed to warm up to rt and stirred for an hour. The solvent was removed under vacuum and the residue was taken up with 10 ml aq. saturated K₂CO₃ and extracted with 20 ml dichloromethane. The organic layer was separated and the aqueous layer was washed with 2x10 dichloromethane. The combined organic layer was dried over Na₂SO₄, and evaporated. t_{R}: 2.42 min. HPLC MS purity: 94% ¹H NMR (300 MHz, DMSO-d6): δ 10.58 (s, 1H); 8.46 (d, 1H); 7.95 (d, 1H); 7.85 (d, 1H); 7.54-7.03 (o m. 8H); 6.51 (d, 1H); 6.47 (d, 1H); 4.28 (t, 2H); 4.20 (t, 2H); 3.95 (s, 3H); 2.91 (m, 3H); 1.97 (q, 2H); 1.31 (t, 3H)

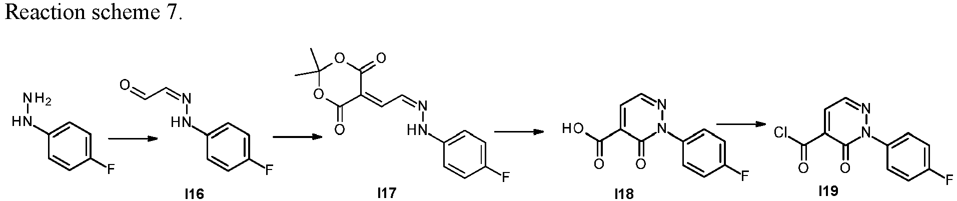

### (1E/Z)-ethanedial (4-fluorophenyl)hydrazone (I16)

US2007197537

A mixture of 1-(4-fluorophenyl)hydrazine hydrochloride (5.0 g, 30.75 mmol), water (20 mL), and acetic acid (20 mL) was added with stirring to a 40% aqueous solution of glyoxal (17.6 mL, 153.8 mmol) over 20 minutes. Stirring was continued for 2 hours and then the mixture was filtered. The precipitate was washed with water and dried to afford 4.94 g (96%) of the desired product. t_{R}: 3.44 min

### (1E)-(2,2-dimethyl-4,6-dioxo-1,3-dioxan-5-ylidene)acetaldehyde (4-fluorophenyl)hydrazone (I17)

(E)-2-(2-(4-fluorophenyl)hydrazono)acetaldehyde (4.8 g, 28.9 mmol) and 2, 2-dimethyl- 1, 3 -dioxane-4,6-di one were combined (4.7 g, 30.3 mmol) to a 100 mL round bottom flask and suspended in toluene (40 mL). Acetic acid (1.65 mL, 0.289 mmol) and piperidine (0.285 mL, 2.89 mmol) were added and mixture was stirred at room temperature overnight. The resulting precipitate was filtered, washed with hexane and dried in a vacuum exsiccator to afford (E)-5-(2-(2-(4-fluorophenyl)hydrazono)ethylidene)-2,2-dimethyl-l,3-dioxane-4,6-dione (8.48 g, 99%) as a bright red solid. t_{R}: 3.94 min

### 2-(4-fluorophenyl)-3-oxo-2,3-dihydropyridazine-4-carboxylic acid (I18)

2-(4-fluorophenyl)-3-oxo-2,3-dihydropyridazine-4-carboxylic acid; A mixture of (E)-5-(2-(2-(4-fluorophenyl)hydrazono)ethylidene)-2,2-dimethyl-1,3-dioxane-4,6-dione (0.60 g, 2.05 mmol) and NaOMe (0.133 g, 2.46 mmol) in MeOH (10 mL) was heated under reflux for 15 hours. The salt was treated with cold 1 N HCl solution, extrated with CH₂Cl₂, dried over MgSO₄, and concentrated to afford 0.42 g (87%) of the desired product. t_{R}: 2.42 min.

### 2-(4-fluorophenyl)-3-oxo-pyridazine-4-carbonyl chloride (I19)

1 mmol carboxylic acid was dissolved in 5 ml SOCl₂ at room temperature, 1-2 droplets of DMF were added were added to the reaction mixture then stirred at 60 °C. After the completion, the SOCl₂ was evaporated under reduced pressure and 4-5 anhydrous toluene was distilled off from the residue then used in the next step without purification.

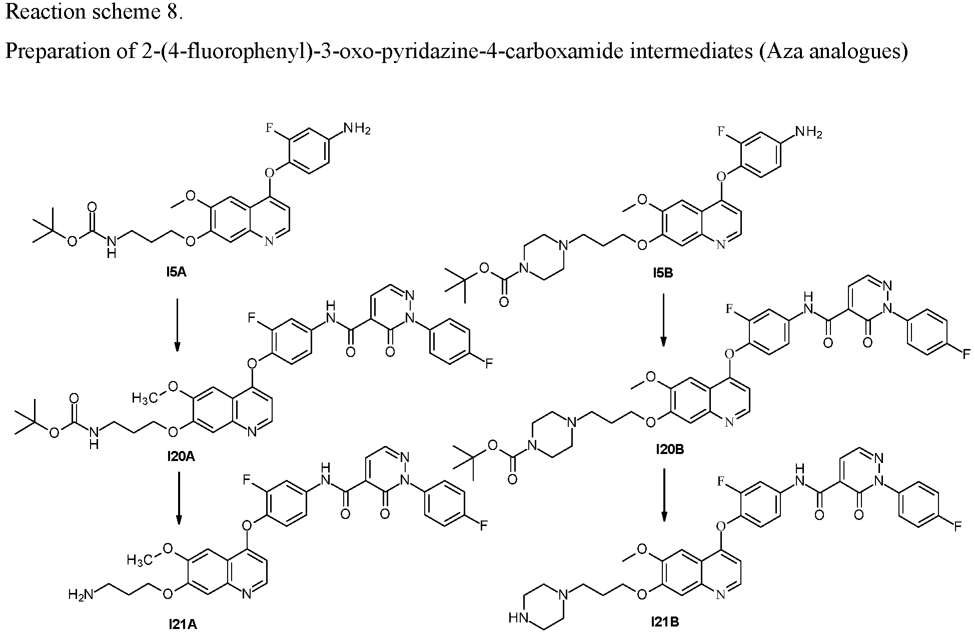

### tert-butyl[3-({4-[2-fluoro-4-({[2-(4-fluorophenyl)-3-oxo-2,3-dihydropyridazin-4-yl]carbonyl}amino) phenoxy]-6-methoxyquinolin-7-yloxy)propyl]carbamate (I20A)

The compound was prepared as described at the preparation of *tert*-butyl4-[3-({4-[2-fluoro-4-({[2-(4-fluorophenyl)-3-oxo-2,3-dihydropyridazin-4-yl]carbonyl}amino)phenoxy]-6-methoxyquinolin-7-yl}oxy)propyl]piperazine-1-carboxylate. t_{R}: 3.78 min ¹H NMR (300 MHz, DMSO-d6) δ 11.68 (s, 1H); 8.47 (d, 1H, J=5.2 Hz); 8.37 (d, 1H); J=4.2 Hz); 8.26 (d, 1H, J=4.2 Hz); 8.02 (m, 1H); 7.71-7.66 (m, 2H); 7.58 (m, 1H); 7.52 (m, 1H); 7.45-7.38 (m, 3H); 6.89 (m. 1H); 6.48 (d, 1H, J=5.2 Hz); 4.17 (t, 2H,J=6.2 Hz); 3.95(s, 3H); 3.19-3.11 (m, 4H); 1.94 (m, 2H); 1.38 (s, 9H)

### tert-butyl4-[3-({4-[2-fluoro-4-({[2-(4-fluorophenyl)-3-oxo-2,3-dihydropyridazin-4-yl]carbonyl}amino )phenoxy]-6-methoxyquinolin-7-yl}oxy)propyl]piperazine-1-carboxylate (I20B)

0.52 g (1 mmol) *tert-*butyl 4-(3-{[4-(4-amino-2-fluorophenoxy)-6-methoxyquinolin-7-yl]oxy}propyl) piperazine-1- carboxylate was dissolved in 20 ml anhydrous DCM and 0.18 ml TEA (1.3 mmol) was added to the solution and chilled to 0 °C. 0.31 g (1.25 mmol) 2-(4-fluorophenyl)-3-oxo-2,3-dihydropyridazine-4-carbonyl chloride was dissolved in 3 ml anhydrous DCM and added to the reaction mixture under inert atmosphere then allowed to warm up to RT and stirred for 2 hours. The reaction mixture was evaporated under reduced pressure and the residue was partitioned between 10 ml 5 % aq. NaHCO₃ and 30 ml ethyl-acetate. The aqueous layer was extracted with 2x10 ml ethyl-acetate and the organic layers were combined, dried over NaSO₄ then the dessicant was filtered off. Evaporating the solvent under reduced pressure afforded the product as greyish solid. The crude product was purified by column chromatography (chloroform-methanol (sat. NH₃) 20: 1) Yield: 0.38g t_{R}: 3.15 min ¹H NMR (300 MHz, DMSO-d6): δ 11.68 (s, 1H); 8.47 (d, 1H, J= 5.2 Hz); 8.37 (d, 1H); 8.26 (d, 1H); 8.02 (dd, 1H, J=8.0 Hz, J=1.0 Hz); 7.71-7.66 (m, 2H); 7.59 (d, 1H);7.55-7.41 (m, 2H); 7.44-7.38 (m, 3H);6.48 (d, 1H, J=5.2 Hz); 4.20 (t, 2H, J=6.0 Hz); 3.95 (s, 3H); 3.92 (m, 5H); 2.35 (m, 5H); 1.97 (m, 2H); 1.40 (s, 9H).

### N-(4-{[7-(3-aminopropoxy)-6-methoxyquinolin-4-yl]oxy}-3-fluorophenyl)-2-(4-fluorophenyl)-3-oxo-2,3-dihydropyridazine-4-carboxamide (I21A)

t_{R}: 2.76 min, ¹H NMR (300 MHz, DMSO-d6): δ 11.69 (s, 1H); 8.38 (m, 2H); 8.25 (m, 2H); 8.03 (dd, 1H, J=11.0 Hz, J=1.6 Hz); 7.71-7.66 (m, 2H); 7.61-7.58 (m, 1H); 7.54-7.38 (m, 4H); 6.49 (d, 1H, J=5.2 Hz); 4.25 (t, 2H, 6.2 Hz); 3.95 (s, 3H); 2.92 (t, 2H, J=7.0 Hz); 2.04 (m, 2H).

### N-(3-fluoro-4-{[6-methoxy-7-(3-piperazin-1-ylpropoxy)quinolin-4-yl]oxy}phenyl)-2-(4-fluorophenyl)-3-oxo-2,3-dihydropyridazine-4-carboxamide (I21B)

t_{R}: 2.67 min ¹H NMR (300 MHz, DMSO-d6): δ 11.68 (s, 1H); 8.62 (br.s, 1H); 8.52 (d, 1H, J=5.4 Hz); 8.38 (d, 1H, J=4.2 Hz); 8.25 (d, 1H, 4.2 Hz); 8.05 (dd, 1H, J=11.0 Hz, J=1.5 Hz); 7.71-7.57 (m, 4H); 7.49 (m, 1H); 7.44-7.34 (m, 3H), 6.56 (d, 1 H, J=5.0 Hz); 4.23 (t, 2H, J=5.4 Hz), 3.59(s, 3H); 3.15 (m, 6H), 2.73 (m, 4H); 2.03 (m, 2H)

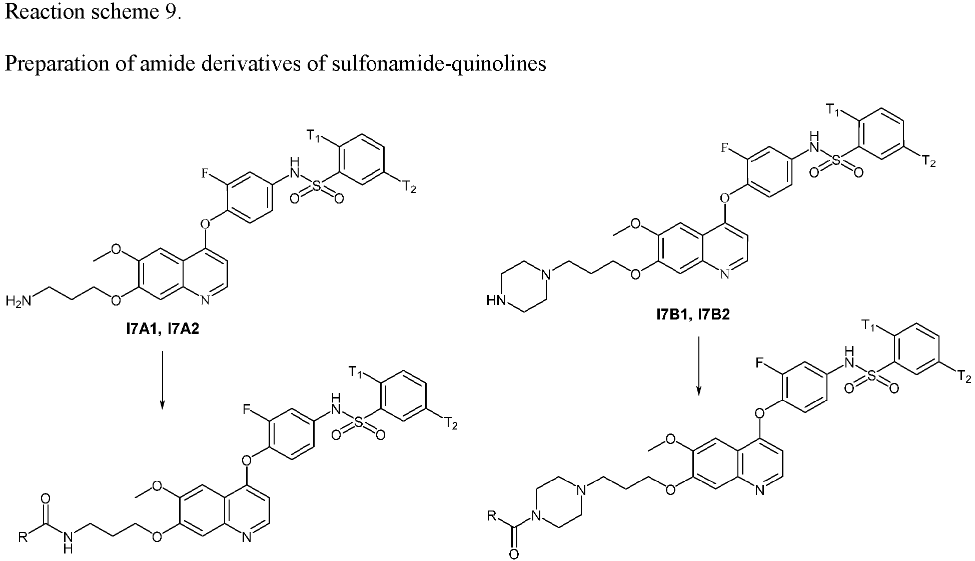

### General method

To the solution of the appriopriate carboxylic acid (0.038 mmol), the proper amine (I7A1, I7A2, I7B1, I7B2) (0.038 mmol), and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography (Sun Fire Prep C18 10um, 10x250mm, flow: 12 ml/min, injection 100-400 ul, 3-6 mg/ injection. Eluents: A/. MilliQ water +0.1% HCOOH, B/. acetonitrile; gradient from 10% B to 90%) and obtained the desired compound as a white solid after lyophilization.

### Example 1 [AXL22001]

### Synthesis of N-[4-[[7-[3-[4-[(E)-4-(dimethylamino)but-2-enoyl]piperazin-1-yl]propoxy]-6-methoxy-4-quinolyl]oxy]-3-fluoro-phenyl]-2,5-difluoro-benzenesulfonamide

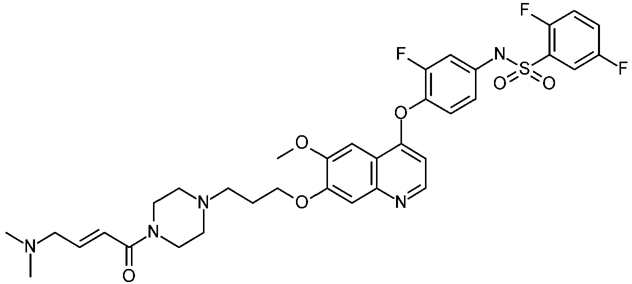

To the solution of trans-4-dimethylcrotonic acid hydrochloride (6.29 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (10.82 mg, 0.084 mmol) was added. The mixture was stirred for 3 hours at room temperature. The mixture was purified by reverse phase chromatography. Thus, 15.38 mg title compound was obtained as a white solid after lyophilization. Yield: 56%. LCMS Anal. calculated for C₃₅H₃₈F₃N₅O₆S: 713.25, found m/z: 712.4 [M-H]⁻, 714.4 [M+H]⁺, t_{R}=2.18 min; peak area 97%. ¹H NMR (300 MHz, DMSO-d6): δ 11.2 (bs, 1H), 8.43 (d, J=5.2Hz, 1H), 7.61 (m, 1H), 7.48 (m, 3H), 7.38 (s, 1H), 7.25 (t, J=9.0Hz, 1H), 7.09 (d, J=7.3Hz, 1H), 6.93 (d, J=8.5Hz, 1H), 6.60 (m, 2H), 6.35 (d, J=5.0Hz, 1H), 4.19 (t, J=6.1Hz, 2H), 3.92 (s, 3H), 3.53 (m, 4H), 3.13 (m, 2H), 2.54 (m, 2H), 2.45 (m, 4H), 2.23 (s, 6H), 1.98 (m, 2H).

### Example 2 [AXL22002]

### N-[4-[[7-[3-[4-[(E)-4-(dimethylamino)but-2-enoyl]piperazin-1-yl]propoxy]-6-methoxy-4-quinolyl]oxy]-3-fluoro-phenyl]-2-(trifluoromethyl)benzenesulfonamide

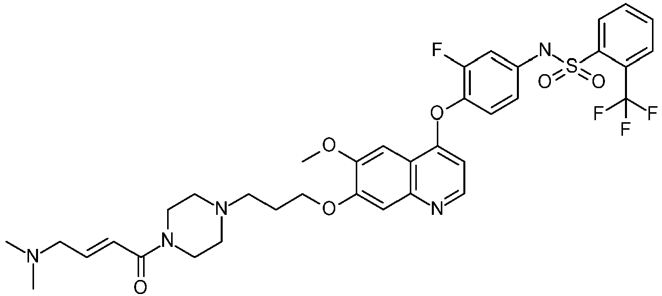

To the solution of trans-4-dimethylcrotonic acid hydrochloride (6.95 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (10.82 mg, 0.084 mmol) was added. The mixture was stirred for 3 hours at room temperature. The mixture was purified by reverse phase chromatography. Thus, 19.45 mg title compound was obtained as a white solid after lyophilization.. Yield: 68%. LCMS Anal. calculated for C₃₆H₃₉F₄N₅O₆S: 745.25, found m/z: 743.4 [M-H]⁻, 745.4 [M+H]⁺, t_{R} = 2.30 min; peak area 100%. ¹H NMR (300 MHz, DMSO-d6): δ 11.0 (bs, 1H), 8.44 (d, J=5.0Hz, 1H), 8.15 (d, J=7.2Hz, 1H), 7.99 (d, J=6.6Hz, 1H), 7.84 (m, 2H), 7.47 (s, 1H), 7.38 (s, 1H), 7.30 (t, J=9.0Hz, 1H), 7.09 (dd, J=12.4Hz, 2.1Hz, 1H), 6.96 (dd, J=8.6Hz, 1.6Hz, 1H), 6.60 (m, 2H), 6.35 (d, J=5.2Hz, 1H), 4.20 (t, J=6.0Hz, 2H), 3.91 (s, 3H), 3.53 (m, 4H), 3.12 (m, 2H), 2.40 (m, 6H), 2.22 (s, 6H), 1.98 (t, J=6.0Hz, 2H).

### Example 3 [AXL22005]

### 2,5-difluoro-N-[3-fluoro-4-[[6-methoxy-7-[3-(4-prop-2-enoylpiperazin-1-yl)propoxy]-4-quinolyl]oxy]phenyl]benzenesulfonamide

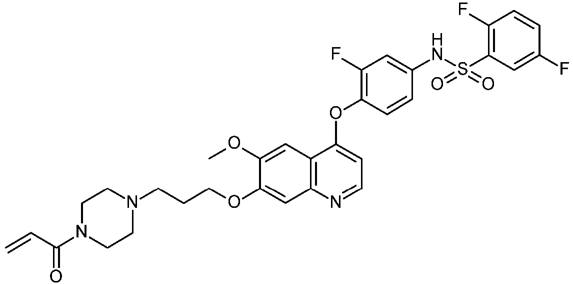

To the solution of acrylic acid (2.73 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 11.39 mg title compound was obtained as a white solid after lyophilization. Yield: 45%. LCMS Anal. calculated for C₃₂H₃₁F₃N₄O₆S: 656.2, found m/z: 655.4 [M-H]⁻, 657.4 [M+H]⁺, t_{R} = 3.00 min; peak area 98%. ¹H NMR (300 MHz, DMSO-d6): δ 8.44 (d, J=5.2Hz, 1H), 7.70 (m, 1H), 7.55 (m, 2H), 7.47 (s, 1H), 7.39 (s, 1H), 7.33 (t, J=9.0Hz, 1H), 7.14 (d, J=7.6Hz, 1H), 7.01 (d, J=8.5Hz, 1H), 6.85 (m, 1H), 6.35 (d, J=5.1Hz, 1H), 6.09 (dd, J=16.1Hz, 2.4Hz, 1H), 5.66 (dd, J=10.5Hz, 2.1Hz, 1H), 4.20 (t, J=5.7Hz, 2H), 3.92 (s, 3H), 3.53 (m, 4H), 2.41 (m, 6H), 1.99 (m, 2H).

### Example 4 [AXL22006]

### N-[4-[[7-[3-[4-(2-chloroacetyl)piperazin-1-yl]propoxy]-6-methoxy-4-quinolyl]oxy]-3-fluoro-phenyl]-2,5-difluoro-benzenesulfonamide

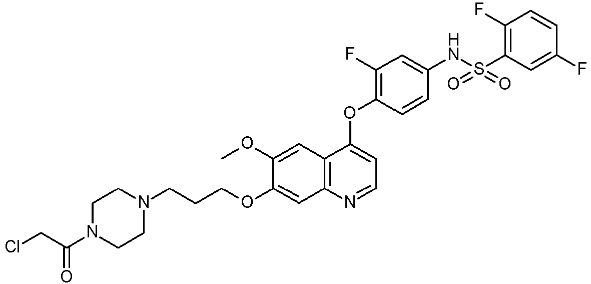

To the solution of chloroacetic acid (3.59 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 10.27 mg title compound was obtained as a white solid after lyophilization. Yield: 40%. LCMS Anal. calculated for C₃₁H₃₀ClF₃N₄O₆S: 678.15, found m/z: 677.2 [M-H]⁻, 679.2 [M+H]⁺, t_{R} = 2.59 min, peak area 99%. ¹H NMR (300 MHz, DMSO-d6): δ 10.8 (bs, 1H), 8.45 (d, J=5.1Hz, 1H), 7.72 (m, 1H), 7.63 (m, 1H), 7.58 (m, 1H), 7.48 (s, 1H), 7.39 (m, 2H), 7.20 (dd, J=12.1Hz, 2.0Hz, 1H), 7.06 (d, J=8.7Hz, 1H), 6.35 (d, J=5.1Hz, 1H), 4.34 (s, 2H), 4.20 (t, J=5.7Hz, 2H), 3.92 (s, 3H), 3.53 (m, 4H), 2.41 (m, 6H), 1.99 (m, 2H).

### Example 5 [AXL22007]

### N-[4-[[7-[3-[4-(2-chloroacetyl)piperazin-1-yl]propoxy]-6-methoxy-4-quinolyl]oxy]-3-fluoro-phenyl]-2-(trifluoromethyl)benzenesulfonamide

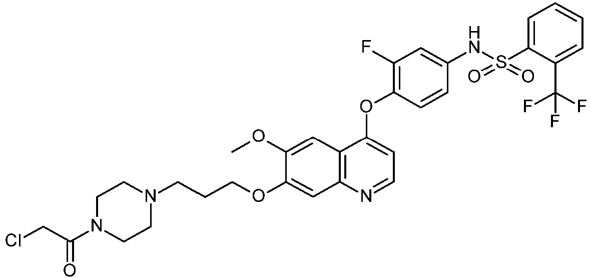

To the solution of chloroacetic acid (3.59 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 9.26 mg title compound was obtained as a white solid after lyophilization. Yield: 34%. LCMS Anal. calculated for C₃₂H₃₁ClF₄N₄O₆S: 710.0, found m/z: 708.8 [M-H]⁻, 710.8 [M+H]⁺, t_{R} = 2.58 min; peak area 100%. ¹H NMR (300 MHz, DMSO-d6): δ 10.7 (bs, 1H), 8.45 (d, J=4.9Hz, 1H), 8.16 (d, J=6.8Hz, 1H), 8.04 (d, J=6.8Hz, 1H), 7.91 (m, 2H), 7.49 (s, 1H), 7.39 (m, 2H), 7.19 (dd, J=11.6Hz, 2.1Hz, 1H), 7.03 (d, J=8.8Hz, 1H), 6.37 (d, J=4.8Hz, 1H), 4.37 (s, 2H), 4.22 (t, J=5.8Hz, 2H), 3.92 (s, 3H), 3.53 (m, 4H), 3.21 (m, 2H), 2.54 (m, 4H), 2.06 (m, 2H).

### Example 6 [AXL22008]

### N-[4-[[7-[3-(4-but-2-ynoylpiperazin-1-yl)propoxy]-6-methoxy-4-quinolyl]oxy]-3-fluoro-phenyl]-2,5-difluoro-benzenesulfonamide

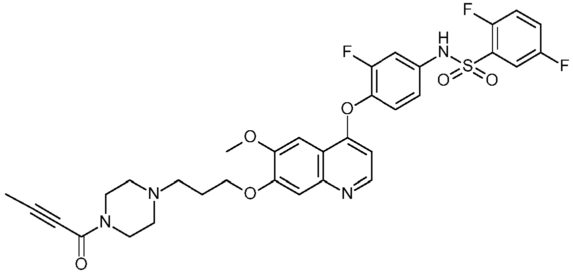

To the solution of 2-butynoic acid (3.20 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 12.35 title compound was obtained as a white solid after lyophilization. Yield: 47%. LCMS Anal. calculated for C₃₃H₃₁F₃N₄O₆S: 668.19, found m/z: 667.2 [M-H]⁻, 669.2 [M+H]⁺, t_{R} = 2.58 min; peak area 100%. ¹H NMR (300 MHz, DMSO-d6): δ 11.0 (bs, 1H), 8.45 (d, J=4.9Hz, 1H), 7.69 (m, 1H), 7.59 (m, 2H), 7.47 (s, 1H), 7.38 (m, 2H), 7.16 (d, J=12.5Hz, 1H), 7.03 (d, J=8.8Hz, 1H), 6.35 (d, J=5.2Hz, 1H), 4.20 (t, J=6.0Hz, 2H), 3.92 (s, 3H), 3.47 (m, 4H), 2.44 (m, 6H), 2.01 (s, 3H), 1.97 (m, 2H).

### Example 7 [AXL22009]

### 2,5-difluoro-N-[3-fluoro-4-[[6-methoxy-7-[3-[4-(3-methylbut-2-enoyl)piperazin-1-yl]propoxy]-4-quinolyl]oxy]phenyl]benzenesulfonamide

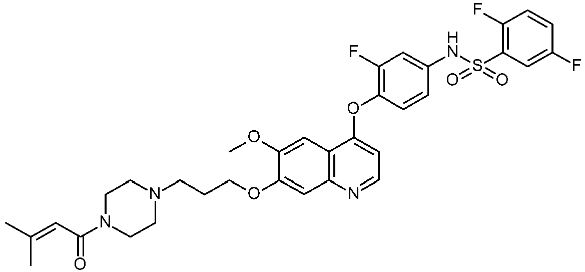

To the solution of 3,3-dimethylacrylic acid (3.80 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 8.91 mg title compound was obtained as a white solid after lyophilization. Yield: 34%. LCMS Anal. calculated for C₃₄H₃₅F₃N₄O₆S: 684.22, found m/z: 683.3 [M-H]⁻, 685.3 [M+H]⁺, t_{R} = 2.65 min; peak area 98%. ¹H NMR (300 MHz, DMSO-d6): δ 10.8 (bs, 1H), 8.44 (d, J=5.2Hz, 1H), 7.70 (m, 1H), 7.55 (m, 2H), 7.47 (s, 1H), 7.38 (m, 2H), 7,17 (dd, J=12.0Hz, 2.2Hz, 1H), 7.03 (d, J=8.3Hz, 1H), 6.34 (d, J=5.1Hz, 1H), 5.88 (s, 1H), 4.20 (t, J=6.3Hz, 2H), 3.92 (s, 3H), 3.47 (m, 4H), 2.50 (m, 6H), 1.98 (t, J=7.2Hz, 2H), 1.80 (s, 3H), 1.79 (s, 3H).

### Example 8 [AXL22010]

### 2,5-difluoro-N-[3-fluoro-4-[[6-methoxy-7-[3-[4-[(E)-3-(2-thienyl)prop-2-enoyl]piperazin-1-yl]propoxy]-4-quinolyl]oxy]phenyl]benzenesulfonamide

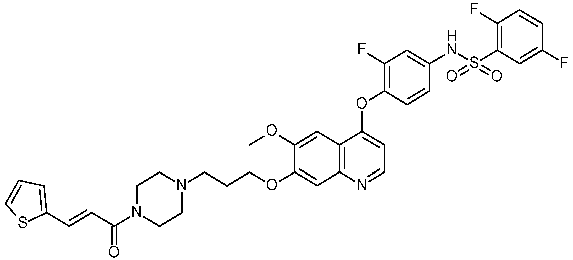

To the solution of 3-(2-thienyl)acrylic acid (5.86 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 14.61 mg title compound was obtained as a white solid after lyophilization. Yield: 52%. LCMS Anal. calculated for C₃₆H₃₃F₃N₄O₆S₂: 738.18, found m/z: 737.2 [M-H]⁻, 739.2 [M+H]⁺, t_{R} = 2.86 min; peak area 100%. ¹H NMR (300 MHz, DMSO-d6): δ 11.0 (bs, 1H), 8.45 (d, J=5.1Hz, 1H), 7.65 (m, 1H), 7.58 (m, 2H), 7.49 (s, 1H), 7.46 (m, 2H), 7.44 (m, 2H), 7.30 (t, J=8.8Hz, 1H), 7,17 (dd, J=12.4Hz, 2.1Hz, 1H), 7.08 (m, 2H), 6.85 (d, J=15.2Hz, 1H), 6.39 (d, J=5.4Hz, 1H), 4.25 (t, J=6.3Hz, 2H), 3.92 (s, 3H), 3.61 (m, 4H), 2.57 (m, 2H), 2.50(m, 4H), 1.98 (m, 2H).

### Example 9 [AXL22012]

### 2,5-difluoro-N-[3-fluoro-4-[[6-methoxy-7-[3-[4-[(E)-2-methylbut-2-enoyl]piperazin-1-yl]propoxy]-4-quinolyl]oxy]phenyl]benzenesulfonamide

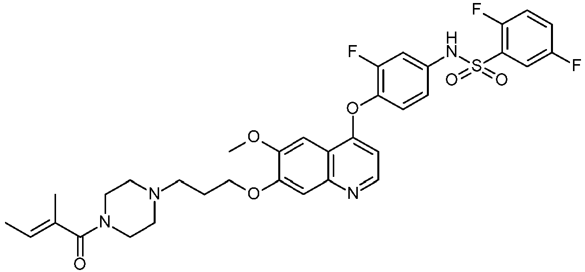

To the solution of 2,3-dimethylacrylic acid (3.80 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 8.65 mg title compound was obtained as a white solid after lyophilization. Yield: 33%. LCMS Anal. calculated for C₃₄H₃₅F₃N₄O₆S: 684.22, found m/z: 683.3 [M-H]⁻, 685.3 [M+H]⁺, t_{R} = 2.66 min; peak area 100%. ¹H NMR (300 MHz, DMSO-d6): δ 11.2 (bs, 1H), 8.44 (d, J=5.1Hz, 1H), 7.69 (m, 1H), 7.55 (m, 2H), 7.47 (s, 1H), 7.39 (s, 1H), 7,33 (t, J=9.0Hz, 1H), 7.14 (dd, J=12.5Hz, 2.0Hz, 1H), 7.01 (d, J=8.5Hz, 1H), 6.34 (d, J=5.1Hz, 1H), 5.52 (m, 1H), 4.19 (t, J=6.1Hz, 2H), 3.91 (s, 3H), 3.47 (m, 4H), 2.50 (m, 6H), 1.98 (m, 2H), 1.74 (s, 3H), 1.65 (d, J=6.0Hz, 3H).

### Example 10 [AXL22013]

### 2,5-difluoro-N-[3-fluoro-4-[[7-[3-[4-[(E)-3-(1H-imidazol-5-yl)prop-2-enoyl]piperazin-1-yl]propoxy]-6-methoxy-4-quinolyl]oxy]phenyl]benzenesulfonamide

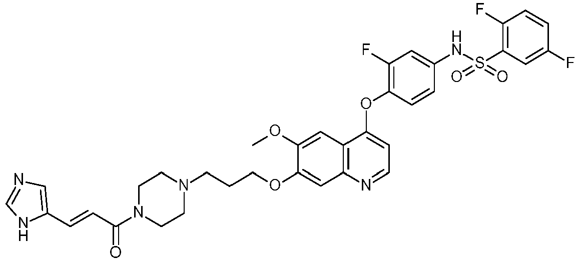

To the solution of urocanic acid (5.24 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 6.97 mg title compound was obtained as a white solid after lyophilization. Yield: 25%. LCMS Anal. calculated for C₃₅H₃₃F₃N₆O₆S: 722.10, found m/z: 721.3 [M-H]⁻, 723.3 [M+H]⁺, t_{R} = 2.38 min; peak area 100%. ¹H NMR (300 MHz, DMSO-d6): δ 11.8 (bs, 1H), 8.44 (d, J=5.2Hz, 1H), 7.70 (m, 2H), 7.53 (m, 2H), 7.48 (s, 1H), 7.35 (m, 4H), 7,14 (dd, J=14.7Hz, 3.1Hz, 1H), 7.03 (m, 2H), 6.35 (d, J=5.1Hz, 1H), 4.21 (t, J=6.3Hz, 2H), 3.92 (s, 3H), 3.61 (m, 4H), 2.50 (m, 6H), 2.00 (m, 2H).

### Example 11 [AXL22016]

### 2,5-difluoro-N-[3-fluoro-4-[[6-methoxy-7-[3-[4-[(E)-4,4,4-trifluoro-3-methyl-but-2-enoyl]piperazin-1-yl]propoxy]-4-quinolyl]oxy]phenyl]benzenesulfonamide

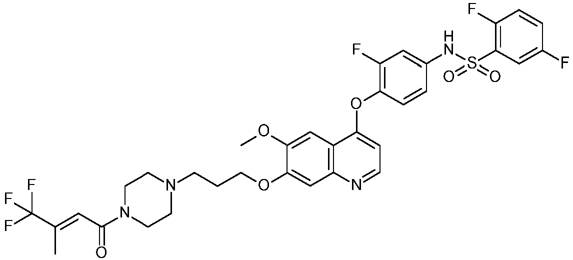

To the solution of 3-(trifluoromethyl)crotonic acid (5.85 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 4.53 mg title compound was obtained as a white solid after lyophilization. Yield: 16%. LCMS Anal. calculated for C₃₄H₃₂F₆N₄O₆S: 738.19, found m/z: 737.2 [M-H]⁻, 739.2 [M+H]⁺, t_{R} = 2.86 min; peak area 100%. ¹H NMR (300 MHz, DMSO-d6): δ 11.4 (bs, 1H), 8.44 (d, J=5.1Hz, 1H), 7.67 (m, 1H), 7.52 (m, 2H), 7.48 (s, 1H), 7.38 (s, 1H), 7.30 (t, J=9.2Hz, 1H), 7.12 (d, J=12.2Hz, 1H), 6.97 (d, J=8.3Hz, 1H), 6.79 (s, 1H), 6.35 (d, J=5.2Hz, 1H), 4.20 (t, J=6.0Hz, 2H), 3.92 (s, 3H), 3.54 (m, 4H), 2.44 (m, 6H), 1.98 (t, J=6.6Hz, 2H), 1.86 (s, 3H).

### Example 12 [AXL22017]

### 2,5-difluoro-N-[3-fluoro-4-[[6-methoxy-7-[3-[4-[(E)-3-phenylprop-2-enoyl]piperazin-1-yl]propoxy]-4-quinolyl]oxy]phenyl]benzenesulfonamide

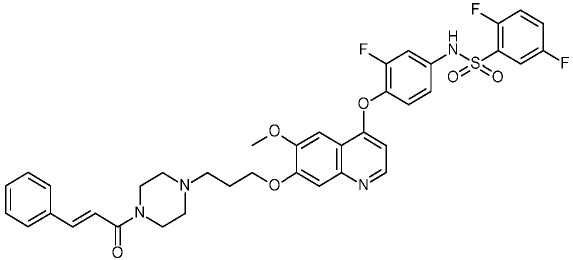

To the solution of trans-cinnamic acid (5.62 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 7.85 mg title compound was obtained as a white solid after lyophilization. Yield: 28%. LCMS Anal. calculated for C₃₈H₃₅F₃N₄O₆S: 732.00, found m/z: 730.9 [M-H]⁻, 732.9 [M+H]⁺, t_{R} = 2.99 min; peak area 100%. ¹H NMR (300 MHz, DMSO-d6): δ 10.8 (bs, 1H), 8.44 (d, J=5.1Hz, 1H), 7,71 (m, 2H), 7.59 (m, 2H), 7.54 (m, 1H), 7.51 (m, 1H), 7.48 (m, 2H), 7.37 (m, 6H), 7.25 (m, 1H), 7.21 (m, 1H), 7.17 (dd, J=12.2Hz, 2.1Hz, 1H), 7.05 (d, J=8.7Hz, 1H), 6.35 (d, J=5.2Hz, 1H), 4.20 (t, J=6.0Hz, 2H), 3.92 (s, 3H), 3.54 (m, 4H), 2.44 (m, 6H), 1.98 (t, J=6.6Hz, 2H).

### Example 13 [AXL22018]

### 2,5-difluoro-N-[3-fluoro-4-[[6-methoxy-7-[3-[4-[(E)-4-oxo-4-phenyl-but-2-enoyl]piperazin-1-yl]propoxy]-4-quinolyl]oxy]phenyl]benzenesulfonamide

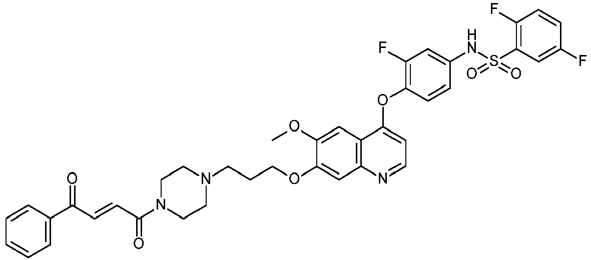

To the solution of trans-3-benzoylacrylic acid (6.68 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 15.03 mg title compound was obtained as a white solid after lyophilization. Yield: 55%. LCMS Anal. calculated for C₃₉H₃₅F₃N₄O₇S: 760.00, found m/z: 758.9 [M-H]⁻, 760.9 [M+H]⁺, t_{R} = 2.97 min; peak area 100%. ¹H NMR (300 MHz, DMSO-d6): δ 11.0 (bs, 1H), 8.44 (d, J=5.1Hz, 1H), 8.02 (d, J=5.2Hz, 2H), 7.68 (m, 3H), 7.58 (m, 3H), 7.47 (m, 3H), 7.38 (m, 1H), 7.32 (t, J=9.0Hz, 1H), 7.15 (dd, J=12.5Hz, 1.7Hz, 1H), 7.00 (d, J=8.8Hz, 1H), 6.35 (d, J=5.3Hz, 1H), 4.21 (t, J=6.0Hz, 2H), 3.92 (s, 3H), 3.60 (m, 4H), 2.45 (m, 6H), 2.00 (t, J=5.4Hz, 2H).

### Example 14 [AXL22020]

### 2,5-difluoro-N-[3-fluoro-4-[[6-methoxy-7-[3-[4-[(E)-4-morpholinobut-2-enoyl]piperazin-1-yl]propoxy]-4-quinolyl]oxy]phenyl]benzenesulfonamide

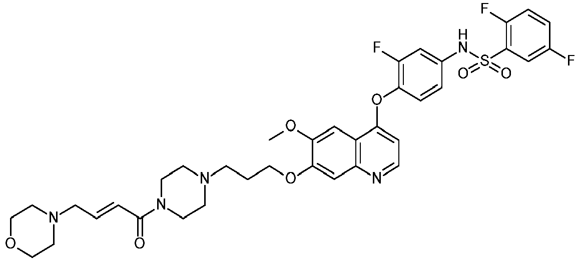

To the solution of 3-(4-morpholinyl)crotonic acid hydrochloride (7.89 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 11.08 mg title compound was obtained as a white solid after lyophilization. Yield: 37%. LCMS Anal. calculated for C₃₇H₄₀F₃N₅O₇S: 755.30, found m/z: 754.3 [M-H]⁻, 756.3 [M+H]⁺, t_{R} = 2.22 min; peak area 100%. ¹H NMR (300 MHz, DMSO-d6): δ 10.8 (bs, 1H), 8.44 (d, J=5.2Hz, 1H), 7.71 (m, 1H), 7.53 (m, 2H), 7.47 (s, 1H), 7.38 (m, 2H), 7.19 (dd, J=12.1Hz, 2.0Hz, 1H), 7.03 (d, J=8.3Hz, 1H), 6.60 (m, 2H), 6.35 (d, J=5.2Hz, 1H), 4.21 (t, J=6.0Hz, 2H), 3.92 (s, 3H), 3.57 (m, 8H), 3.10 (m, 2H), 2.43 (m, 6H), 2.36 (m, 4H), 2.00 (m, 2H).

### Example 15 [AXL22021]

### N-[3-fluoro-4-[[6-methoxy-7-[3-[4-(3-methylbut-2-enoyl)piperazin-1-yl]propoxy]-4-quinolyl]oxy]phenyl]-2-(trifluoromethyl)benzenesulfonamide

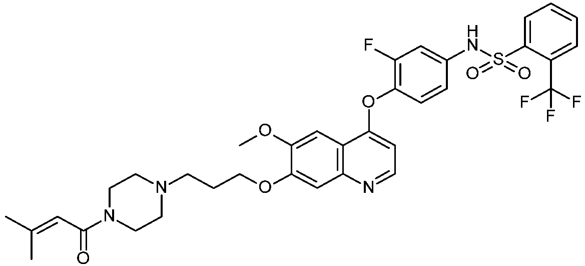

To the solution of 3,3-dimethylacrylic acid (3.80 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 16.52 mg title compound was obtained as a white solid after lyophilization. Yield: 60%. LCMS Anal. calculated for C₃₅H₃₆F₄N₄O₆S: 716.20, found m/z: 715.1 [M-H]⁻, 717.1 [M+H]⁺, t_{R} = 2.79 min; peak area 100%. ¹H NMR (300 MHz, DMSO-d6): δ 11.0 (bs, 1H), 8.44 (d, J=5.2Hz, 1H), 8.14 (d, J=7.6Hz, 1H), 8.02 (d, J=6.8Hz, 1H), 7.87 (m, 2H), 7.47 (s, 1H), 7.39 (s, 1H), 7.37 (t, J=8.7Hz, 1H), 7.15 (dd, J=12.1Hz, 2.1Hz, 1H), 7.02 (d, J=9.0Hz, 1H), 6.34 (d, J=5.1Hz, 1H), 5.88 (s, 1H), 4.19 (t, J=6.3Hz, 2H), 3.91 (s, 3H), 3.47 (m, 4H), 2.39 (m, 6H), 1.98 (t, J=7.2Hz, 2H), 1.80 (s, 3H), 1.79 (s, 3H).

### Example 16 [AXL22022]

### N-[3-fluoro-4-[[6-methoxy-7-[3-(4-prop-2-enoylpiperazin-1-yl)propoxy]-4-quinolyl]oxy] phenyl]-2-(trifluoromethyl)benzenesulfonamide

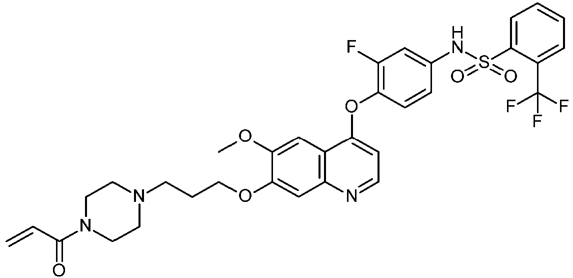

To the solution of acrylic acid (2.74 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 13.16 mg title compound was obtained as a white solid after lyophilization. Yield: 50%. LCMS Anal. calculated for C₃₃H₃₂F₄N₄O₆S: 688.20, found m/z: 687.2 [M-H]⁻, 689.2 [M+H]⁺, t_{R} = 3.10 min; peak area 97%. ¹H NMR (300 MHz, DMSO-d6): δ 11.2 (bs, 1H), 8.44 (d, J=5.2Hz, 1H), 8.15 (d, J=8.7Hz, 1H), 8.02 (d, J=7.0Hz, 1H), 7.87 (m, 2H), 7.47 (s, 1H), 7.38 (s, 1H), 7.35 (t, J=9.1Hz, 1H), 7.14 (d, J=12.3Hz, 1H), 7.01 (d, J=8.6Hz, 1H), 6.78 (m, 1H), 6.34 (d, J=5.4Hz, 1H), 6.09 (dd, J=16.8Hz, 2.1Hz, 1H), 5.66 (dd, J=10.5Hz, 2.7Hz, 1H), 4.20 (t, J=5.7Hz, 2H), 3.91 (s, 3H), 3.54 (m, 4H), 2.40 (m, 6H), 1.98 (t, J=7.2Hz, 2H).

### Example 17 [AXL22023]

### N-[3-fluoro-4-[[7-[3-[4-[(E)-3-(2-hydroxyphenyl)prop-2-enoyl]piperazin-1-yl]propoxy]-6-methoxy-4-quinolyl]oxy]phenyl]-2-(trifluoromethyl)benzenesulfonamide

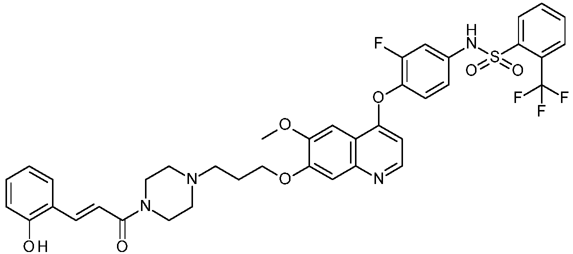

To the solution of trans-2-hydroxycinnamic acid (5.85 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 7.15 mg title compound was obtained as a white solid after lyophilization. Yield: 24%. LCMS Anal. calculated for C₃₉H₃₆F₄N₄O₇S: 780.20, found m/z: 779.2 [M-H]⁻, 781.2 [M+H]⁺, t_{R} = 3.10 min; peak area 100%. ¹H NMR (300 MHz, DMSO-d6): δ 10.15 (s, 1H), 8.65 (d, J=6.0Hz, 1H), 8.16 (m, 2H), 8.05 (d, J=6.3Hz, 1H), 7.92 (m, 2H), 7.62 (m, 2H), 7.48 (m, 3H), 7.18 (m, 2H), 7.07 (d, J=8.6Hz, 1H), 6.85 (m, 2H), 6.67 (m, 1H), 4.21 (t, J=6.3Hz, 2H), 3.92 (s, 3H), 3.47 (m, 4H), 2.39 (m, 6H), 2.00 (m, 2H).

### Example 18 [AXL22025]

### N-[3-fluoro-4-[[6-methoxy-7-[3-[4-[(E)-3-(2-methoxyphenyl)prop-2-enoyl]piperazin-1-yl]propoxy]-4-quinolyl]oxy]phenyl]-2-(trifluoromethyl)benzenesulfonamide

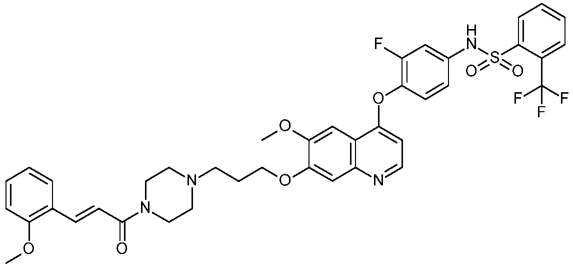

To the solution of trans-2-methoxycinnamic acid, 6.77 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 11.25 mg title compound was obtained as a white solid after lyophilization. Yield: 37%. LCMS Anal. calculated for C₄₀H₃₈F₄N₄O₇S: 794.20, found m/z: 793.2 [M-H]⁻, 795.2 [M+H]⁺, t_{R} = 3.07 min; peak area 100%. ¹H NMR (300 MHz, DMSO-d6): δ 10.8 (bs, 1H), 8.44 (d, J=5.0Hz, 1H), 8.15 (d, J=7.2Hz, 1H), 8.02 (d, J=7.2Hz, 1H), 7.91 (m, 2H), 7.79 (m, 2H), 7.47 (s, 1H), 7.38 (m, 3H), 7.15 (m, 2H), 7.01 (m, 3H), 6.36 (d, J=5.4Hz, 1H), 4.21 (t, J=6.3Hz, 2H), 3.92 (s, 3H), 3.82 (s, 3H), 3.47 (m, 4H), 2.39 (m, 6H), 2.00 (m, 2H).

### Example 19 [AXL22026]

### N-[4-[[7-[3-[4-[(E)-3-(2,3-dimethoxyphenyl)prop-2-enoyl]piperazin-1-yl]propoxy]-6-methoxy-4-quinolyl]oxy]-3-fluoro-phenyl]-2-(trifluoromethyl)benzenesulfonamide

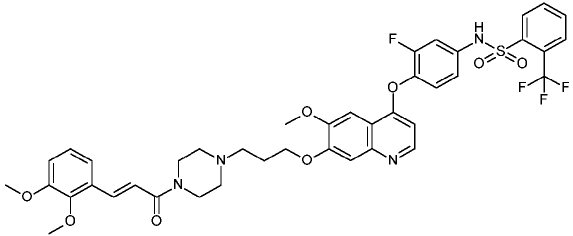

To the solution of trans-2,3-dimethoxycinnamic acid (7.90 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 5.21 mg title compound was obtained as a white solid after lyophilization. Yield: 16%. LCMS Anal. calculated for C₄₁H₄₀F₄N₄O₈S: 824.30, found m/z: 823.2 [M-H]⁻, 825.2 [M+H]⁺, t_{R} = 3.02 min; peak area 100%. ¹H NMR (300 MHz, DMSO-d6): δ 11.0 (bs, 1H), 8.44 (d, J=5.0Hz, 1H), 8.15 (d, J=7.8Hz, 1H), 8.02 (d, J=6.9Hz, 1H), 7.88 (m, 2H), 7.73 (d, J=15,6Hz, 1H), 7.47 (s, 1H), 7.42 (m, 2H), 7.34 (m, 1H), 7.23 (s, 1H), 7.16 (d, J=5.8Hz, 1H), 7.09 (m, 2H), 7.0 (d, J=9.0Hz, 1H), 6.36 (d, J=5.1Hz, 1H), 4.21 (t, J=6.0Hz, 2H), 3.92 (s, 3H), 3.81 (s, 3H), 3.74 (s, 3H), 3.47 (m, 4H), 2.39 (m, 6H), 2.00 (m, 2H).

### Example 20 [AXL22027]

### N-[3-fluoro-4-[[6-methoxy-7-[3-[4-[(E)-2-methylbut-2-enoyl]piperazin-1-yl]propoxy]-4-quinolyl]oxy]phenyl]-2-(trifluoromethyl)benzenesulfonamide

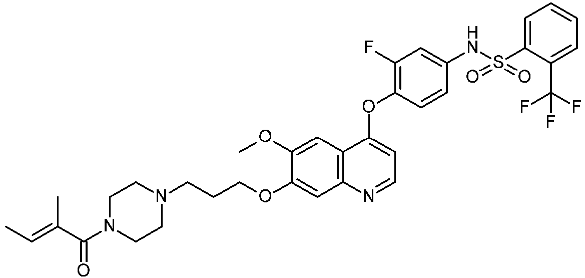

To the solution of trans-2,3-dimethylacrylic acid (3.80 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 7.56 mg title compound was obtained as a white solid after lyophilization. Yield: 27%. LCMS Anal. calculated for C₃₅H₃₆F₄N₄O₆S: 716.20, found m/z: 715.3 [M-H]⁻, 717.3 [M+H]⁺, t_{R} = 2.77 min; peak area 100%. ¹H NMR (300 MHz, DMSO-d6): δ 8.44 (d, J=5.2Hz, 1H), 8.15 (d, J=7.4Hz, 1H), 8.03 (d, J=6.8Hz, 1H), 7.89 (m, 2H), 7.47 (s, 1H), 7.39 (s, 1H), 7.36 (t, J=9.0Hz, 1H), 7.17 (dd, J=12.5Hz, 2.0Hz, 1H), 7.02 (d, J=9.0Hz, 1H), 6.35 (d, J=5.1Hz, 1H), 5.43 (t, J=7.8Hz, 1H), 4.19 (t, J=6.1Hz, 2H), 3.91 (s, 3H), 3.47 (m, 4H), 2.50 (m, 6H), 1.98 (m, 2H), 1.74 (s, 3H), 1.65 (d, J=6.0Hz, 3H).

### Example 21 [AXL22028]

### N-[3-fluoro-4-[[6-methoxy-7-[3-[4-[(E)-2-methylpent-2-enoyl]piperazin-1-yl]propoxy]-4-quinolyl]oxy]phenyl]-2-(trifluoromethyl)benzenesulfonamide

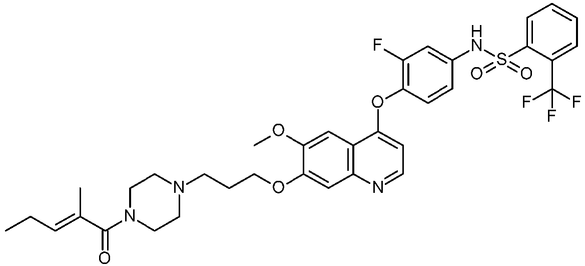

To the solution of trans-2-methyl-2-pentenoic acid (4.33 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 11.36 mg title compound was obtained as a white solid after lyophilization. Yield: 41%. LCMS Anal. calculated for C₃₆H₃₈F₄N₄O₆S: 730.20, found m/z: 729.3 [M-H]⁻, 731.3 [M+H]⁺, t_{R} = 2.89 min; peak area 100%. ¹H NMR (300 MHz, DMSO-d6): δ 10.8 (bs, 1H), 8.44 (d, J=5.2Hz, 1H), 8.16 (d, J=7.4Hz, 1H), 8.03 (d, J=6.8Hz, 1H), 7.89 (m, 2H), 7.47 (s, 1H), 7.39 (s, 1H), 7.36 (t, J=9.0Hz, 1H), 7.17 (dd, J=12.5Hz, 2.0Hz, 1H), 7.02 (d, J=9.0Hz, 1H), 6.35 (d, J=5.1Hz, 1H), 5.43 (t, J=7.8Hz, 1H), 4.19 (t, J=6.0Hz, 2H), 3.91 (s, 3H), 3.47 (m, 4H), 2.40 (m, 6H), 2.08 (m, 2H), 1.98 (m, 2H), 1.71 (s, 3H), 0.95 (t, J=7.5Hz, 3H).

### Example 22 [AXL22030]

### N-[3-fluoro-4-[[6-methoxy-7-[3-[4-[(E)-2-methyl-3-phenyl-prop-2-enoyl]piperazin-1-yl]propoxy]-4-quinolyl]oxy]phenyl]-2-(trifluoromethyl)benzenesulfonamide

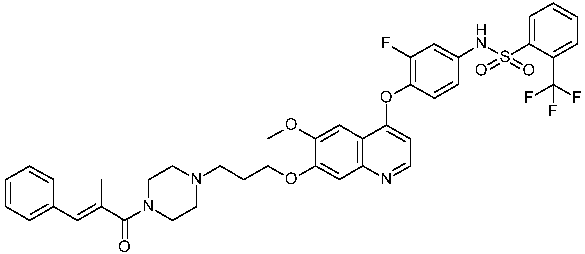

To the solution of α-methylcinnamic acid (6.15 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 14.62 mg title compound was obtained as a white solid after lyophilization. Yield: 49%. LCMS Anal. calculated for C₄₀H₃₈F₄N₄O₆S: 778.20, found m/z: 777.3 [M-H]⁻, 779.3 [M+H]⁺, t_{R} = 3.10 min; peak area 100%. ¹H NMR (300 MHz, DMSO-d6): δ 11.0 (bs, 1H) 8.44 (d, J=5.2Hz, 1H), 8.16 (d, J=7.5Hz, 1H), 8.03 (d, J=7.1Hz, 1H), 7.91 (m, 2H), 7.47 (s, 1H), 7.39 (m, 6H), 7.29 (m, 1H), 7.14 (dd, J=12.2Hz, 2.0Hz, 1H), 7.01 (d, J=9.0Hz, 1H), 6.48 (s, 1H), 6.36 (d, J=5.1Hz, 1H), 4.20 (t, J=6.0Hz, 2H), 3.91 (s, 3H), 3.54 (m, 4H), 2.40 (m, 6H), 2.00 (bs, 5H).

### Example 23 [AXL22032]

### N-[4-[[7-[3-[4-[(E)-but-2-enoyl]piperazin-1-yl]propoxy]-6-methoxy-4-quinolyl]oxy]-3-fluoro-phenyl]-2,5-difluoro-benzenesulfonamide

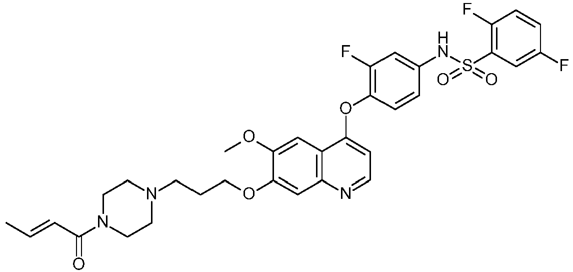

To the solution of crotonic acid (3.26 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 13.94 mg title compound was obtained as a white solid after lyophilization. Yield: 55%. LCMS Anal. calculated for C₃₃H₃₃F₃N₄O₆S: 670.2, found m/z: 669.2 [M-H]⁻, 671.2 [M+H]⁺, t_{R} = 2.55 min; peak area 98%. ¹H NMR (300 MHz, DMSO-d6): δ 10.8 (bs, 1H), 8.44 (d, J=5.1Hz, 1H), 7.67 (m, 1H), 7.54 (m, 2H), 7.48 (s, 1H), 7.39 (s, 1H), 7.31 (t, J=9.0Hz, 1H), 7.12 (dd, J=12.5Hz, 1.9Hz, 1H), 7.00 (d, J=8.1Hz, 1H), 6.65 (m, 1H), 6.43 (d, J=14.5Hz, 1H), 6.39 (d, J=5.1Hz, 1H), 4.20 (t, J=6.3Hz, 2H), 3.92 (s, 3H), 3.52 (m, 4H), 2.40 (m, 6H), 1.99 (m, 2H), 1.82 (d, J=6.6Hz, 3H).

### Example 24 [AXL22033]

### N-[4-[[7-[3-[4-[(E)-4-(benzotriazol-1-yloxy)but-2-enoyl]piperazin-1-yl]propoxy]-6-methoxy-4-quinolyl]oxy]-3-fluoro-phenyl]-2,5-difluoro-benzenesulfonamide

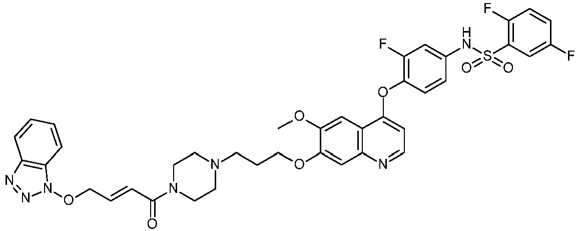

To the solution of trans-4-bromocrotonic acid (6.23 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 15.38 mg title compound was obtained as a white solid after lyophilization. Yield: 50%. LCMS Anal. calculated for C₃₉H₃₆F₃N₇O₇S: 803.2, found m/z: 802.2 [M-H]⁻, 804.2 [M+H]⁺, t_{R} = 3.02 min; peak area 100%. ¹H NMR (300 MHz, DMSO-d6): δ 10.6 (bs, 1H), 8.44 (d, J=5.2Hz, 1H), 8.06 (d, J=8.4Hz, 1H), 7.85 (d, J=8.6Hz, 1H), 7.72 (m, 1H), 7.58 (m, 3H), 7.47 (m, 2H), 7.40 (m, 2H), 7.19 (dd, J=11.9Hz, 2.0Hz, 1H), 7.05 (d, J=8.9Hz, 1H), 6.80 (m, 2H), 6.36 (d, J=5.1Hz, 1H), 5.25 (d, J=4.5Hz, 2H), 4.20 (t, J=6.0Hz, 2H), 3.92 (s, 3H), 3.50 (m, 4H), 2.53 (m, 2H), 2.41 (m, 4H), 1.99 (m, 2H).

### Example 25 [AXL22034]

### 2,5-difluoro-N-[3-fluoro-4-[[6-methoxy-7-[3-[4-[(E)-4,4,4-trifluorobut-2-enoyl]piperazin-1-yl]propoxy]-4-quinolyl]oxy]phenyl]benzenesulfonamide

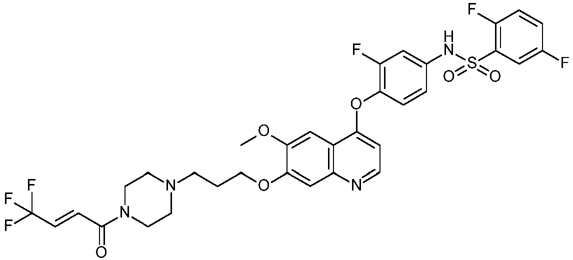

To the solution of 4,4,4-trifluorocrotonic acid (5.32 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 18.50 mg title compound was obtained as a white solid after lyophilization. Yield: 67%. LCMS Anal. calculated for C₃₃H₃₀F₆N₄O₆S: 724.2, found m/z: 723.2 [M-H]⁻, 725.2 [M+H]⁺, t_{R} = 3.04 min; peak area 100%. ¹H NMR (300 MHz, DMSO-d6): δ 10.6 8bs, 1H), 8.44 (d, J=5.2Hz, 1H), 7.70 (m, 1H), 7.62 (m, 1H), 7.55 (m, 2H), 7.48 (s, 1H), 7.37 (m, 2H), 7.19 (dd, J=12.0Hz, 2.2Hz, 1H), 7.05 (d, J=9.6Hz, 1H), 6.76 (m, 1H), 6.35 (d, J=5.2Hz, 1H), 4.21 (t, J=5.7Hz, 2H), 3.92 (s, 3H), 3.53 (m, 4H), 2.41 (m, 6H), 2.01 (m, 2H).

### Example 26 [AXL22035]

### 2,5-difluoro-N-[3-fluoro-4-[[6-methoxy-7-[3-[4-[(E)-3-[3-(trifluoromethyl)phenyl]prop-2-enoyl]piperazin-1-yl]propoxy]-4-quinolyl]oxy]phenyl]benzenesulfonamide

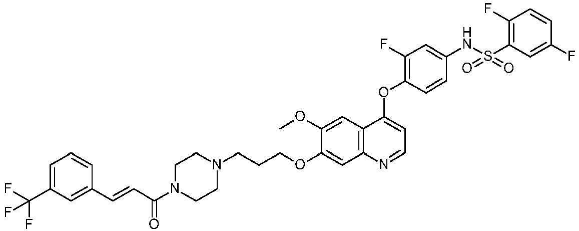

To the solution of 3-(trifluromethyl)cinnamic acid (8.20 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 9.7 mg title compound was obtained as a white solid after lyophilization. Yield: 32%. LCMS Anal. calculated for C₃₉H₃₄F₆N₄O₆S: 800.2, found m/z: 799.2 [M-H]⁻, 801.2 [M+H]⁺, t_{R} = 3.41 min; peak area 100%. ¹H NMR (300 MHz, DMSO-d6): δ 10.8 (bs, 1H), 8.44 (d, J=5.1Hz, 1H), 8.14 (d, J=4.5Hz, 1H), 8.00 (d, J=7.5Hz, 1H), 7.70 (m, 2H), 7.63 (m, 1H), 7.55 (m, 2H), 7.47 (s, 1H), 7.44 (s, 1H), 7.40 (m, 2H), 7.32 (t, J=9.2Hz, 1H), 7.14 (dd, J=12.8Hz, 1.8Hz, 1H), 7.00 (d, J=9.4Hz, 1H), 6.36 (d, J=9.4Hz, 1H), 4.22 (t, J=6.0Hz, 2H), 3.92 (s, 3H), 3.74 (bs, 2H), 3.60 (bs, 2H), 2.45 (s, 6H), 2.01 (m, 2H).

### Example 27 [AXL22036]

### N-[4-[[7-[3-[4-[(E)-3-(2,5-dimethoxyphenyl)prop-2-enoyl]piperazin-1-yl]propoxy]-6-methoxy-4-quinolyl]oxy]-3-fluoro-phenyl]-2,5-difluoro-benzenesulfonamide

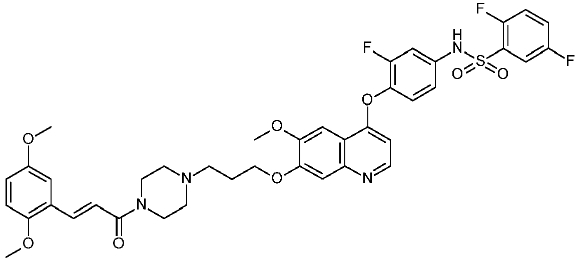

To the solution of 2,5-dimethoxycinnamic acid (7.90 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 17.95 mg title compound was obtained as a white solid after lyophilization. Yield: 59%. LCMS Anal. calculated for C₄₀H₃₉F₃N₄O₈S: 792.2, found m/z: 791.2 [M-H]⁻, 793.2 [M+H]⁺, t_{R} = 3.20 min; peak area 98%. ¹H NMR (300 MHz, DMSO-d6): δ 10.8 (bs, 1H), 8.44 (d, J=5.4Hz, 1H), 7.73 (m, 2H), 7.61 (m, 1H), 7.55 (m, 1H), 7.48 (s, 1H), 7.40 (s, 1H), 7.37 (m, 2H), 7.21 (m, 2H), 7.05 (d, J=9.2Hz, 1H), 6.95 (m, 2H), 6.35 (d, J=5.4Hz, 1H), 4.22 (t, J=6.0Hz, 2H), 3.92 (s, 3H), 3.79 (s, 3H), 3.75 (s, 3H), 3.61 (m, 4H), 2.60 (s, 6H), 2.03 (m, 2H).

### Example 28 [AXL22037]

### N-[4-[[7-[3-[4-[(E)-3-(2-chlorophenyl)prop-2-enoyl]piperazin-1-yl]propoxy]-6-methoxy-4-quinolyl]oxy]-3-fluoro-phenyl]-2,5-difluoro-benzenesulfonamide

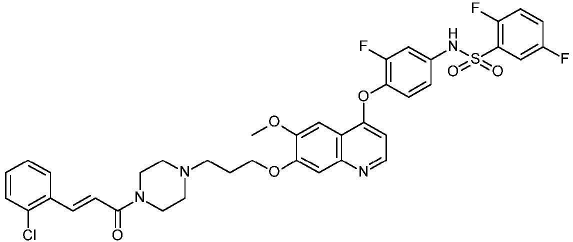

To the solution of 2-chlorocinnamic acid (6.91 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 16.51 mg title compound was obtained as a white solid after lyophilization. Yield: 57%. LCMS Anal. calculated for C₃₈H₃₄ClF₃N₄O₆S: 766.2, found m/z: 765.2 [M-H]⁻, 767.1 [M+H]⁺, t_{R} = 3.06 min; peak area 100%. ¹H NMR (300 MHz, DMSO-d6): δ 11.0 (bs, 1H), 8.44 (d, J=5.4Hz, 1H), 8.00 (m, 1H), 7.81 (d, J=15.0Hz, 1H), 7.71 (m, 1H), 7.62 (m, 1H), 7.55 (m, 2H), 7.48 (s, 1H), 7.38 (m, 5H), 7.19 (dd, J=12.1Hz, 2.2Hz, 1H), 7.05 (d, J=8.5Hz, 1H), 6.35 (d, J=5.1Hz, 1H), 4.22 (t, J=6.6Hz, 2H), 3.92 (s, 3H), 3.74 (bs, 2H), 3.61 (bs, 2H), 2.60 (m, 6H), 2.02 (m, 2H).

### Example 29 [AXL22038]

### N-[4-[[7-[3-[4-[(E)-but-2-enoyl]piperazin-1-yl]propoxy]-6-methoxy-4-quinolyl]oxy]-3-fluoro-phenyl]-2-(trifluoromethyl)benzenesulfonamide

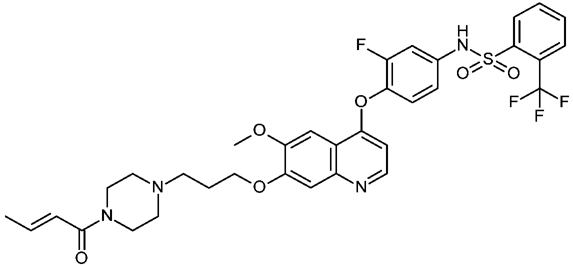

To the solution of crotonic acid (3.26 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 17.40 mg title compound was obtained as a white solid after lyophilization. Yield: 65%. LCMS Anal. calculated for C₃₄H₃₄F₄N₄O₆S: 702.2, found m/z: 701.2 [M-H]⁻, 703.2 [M+H]⁺, t_{R} = 2.95 min; peak area 98%. ¹H NMR (300 MHz, DMSO-d6): δ 10.8 (bs, 1H), 8.44 (d, J=5.2Hz, 1H), 8.15 (d, J=7.4Hz, 1H), 8.03 (d, J=6.5Hz, 1H), 7.90 (m, 2H), 7.47 (s, 1H), 7.40 (m, 2H), 7.18 (dd, J=12.5Hz, 2.0Hz, 1H), 7.02 (d, J=9.0Hz, 1H), 6.67 (m, 1H), 6.48 (d, J=15.0Hz, 1H), 6.36 (d, J=5.1Hz, 1H), 4.20 (t, J=5.7Hz, 2H), 3.92 (s, 3H), 3.56 (m, 4H), 2.53 (m, 2H), 2.41 (m, 4H), 2.01 (m, 2H), 1.82 (d, J=6.6Hz, 3H).

### Example 30 [AXL22039]

### N-[4-[[7-[3-[4-[(E)-4-(benzotriazol-1-yloxy)but-2-enoyl]piperazin-1-yl]propoxy]-6-methoxy-4-quinolyl]oxy]-3-fluoro-phenyl]-2-(trifluoromethyl)benzenesulfonamide

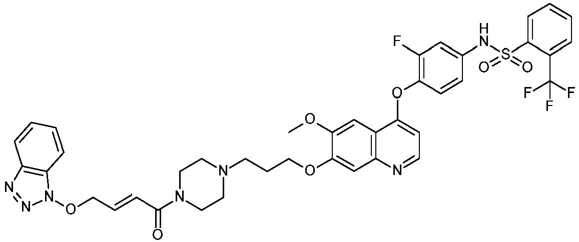

To the solution of 4-bromocrotonic acid (6.23 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 19.79 mg title compound was obtained as a white solid after lyophilization. Yield: 62%. LCMS Anal. calculated for C₄₀H₃₇F₄N₇O₇S: 835.2, found m/z: 834.2 [M-H]⁻, 836.2 [M+H]⁺, t_{R} = 3.15 min; peak area 100%. ¹H NMR (300 MHz, DMSO-d6): δ 10.8 (bs, 1H), 8.44 (d, J=5.2Hz, 1H), 8.15 (d, J=7.5Hz, 1H), 8.05 (m, 2H), 7.91 (m, 2H), 7.83 (d, J=8.3Hz, 1H), 7.63 (t, J=8.2Hz, 1H), 7.46 (m, 3H), 7.40 (m, 2H), 7.16 (dd, J=12.1Hz, 2.0Hz, 1H), 7.03 (d, J=9.0Hz, 1H), 6.80 (d, J=6.5Hz, 1H), 6.36 (d, J=5.4Hz, 1H), 5.25 (d, J=4.2Hz, 2H), 4.20 (t, J=6.0Hz, 2H), 3.92 (s, 3H), 3.51 (m, 4H), 2.53 (m, 2H), 2.41 (m, 4H), 2.00 (m, 2H).

### Example 31 [AXL22040]

### N-[3-fluoro-4-[[6-methoxy-7-[3-[4-[(E)-4,4,4-trifluorobut-2-enoyl]piperazin-1-yl]propoxy]-4-quinolyl]oxy]phenyl]-2-(trifluoromethyl)benzenesulfonamide

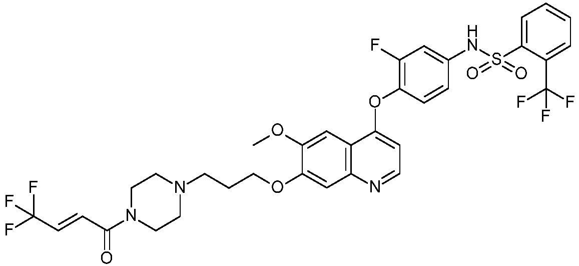

To the solution of 4,4,4-trifluorocrotonic acid (5.32 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 21.63 mg title compound was obtained as a white solid after lyophilization. Yield: 75%. LCMS Anal. calculated for C₃₄H₃₁F₇N₄O₆S: 756.2, found m/z: 755.2 [M-H]⁻, 757.2 [M+H]⁺, t_{R} = 3.11 min; peak area 100%. ¹H NMR (300 MHz, DMSO-d6): δ 11.0 (bs, 1H), 8.43 (d, J=5.2Hz, 1H), 8.13 (t, J=5.4Hz, 1H), 8.02 (d, J=7.0Hz, 1H), 7.92 (m, 2H), 7.47 (s, 1H), 7.38 (m, 3H), 7.16 (dd, J=11.5Hz, 2.0Hz, 1H), 7.02 (d, J=9.3Hz, 1H), 6.76 (m, 1H), 6.35 (d, J=5.4Hz, 1H), 4.20 (t, J=6.0Hz, 2H), 3.91 (s, 3H), 3.59 (m, 4H), 2.61 (m, 6H), 2.01 (m, 2H).

### Example 32 [AXL22041]

### (E)-N-[3-[[4-[4-[(2,5-difluorophenyl)sulfonylamino]-2-fluoro-phenoxy]-6-methoxy-7-quinolyl]oxy]propyl]but-2-enamide

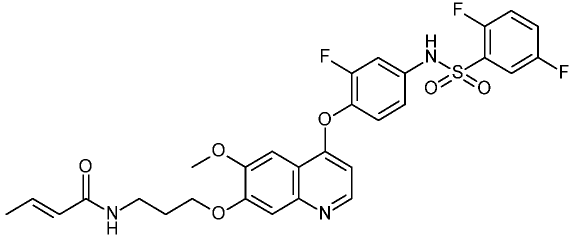

To the solution of crotonic acid (3.26 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 16.49 mg title compound was obtained as a white solid after lyophilization. Yield: 72%. LCMS Anal. calculated for C₂₉H₂₆F₃N₃O₆S: 601.1, found m/z: 600.2 [M-H]⁻, 602.2 [M+H]⁺, t_{R} = 3.32 min; peak area 100%. ¹H NMR (300 MHz, DMSO-d6): δ 11.2 (bs, 1H), 8.48 (d, J=5.1Hz, 1H), 7.96 (t, 1H), 7.73 (m, 1H), 7.64 (m, 1H), 7.56 (m, 1H), 7.47 (s, 1H), 7.39 (m, 2H), 7.18 (d, J=9.0Hz, 1H), 7.07 (d, J=6.9Hz, 1H), 6.61 (m, 1H), 6.41 (d, J=5.1Hz, 1H), 5.88 (d, J=15.3Hz, 1H), 4.18 (t, J=6.0Hz, 2H), 3.93 (s, 3H), 3.28 (m, 2H), 1.98 (m, 2H), 1.78 (d, J=6.9Hz, 3H).

### Example 33 [AXL22044]

### N-[3-[[4-[4-[(2,5-difluorophenyl)sulfonylamino]-2-fluoro-phenoxy]-6-methoxy-7-quinolyl]oxy]propyl]prop-2-enamide

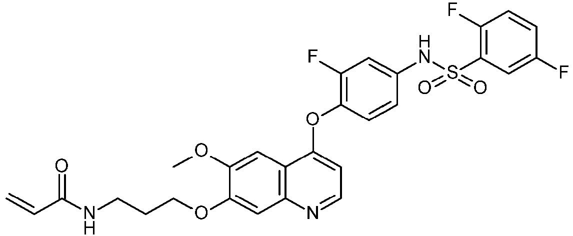

To the solution of acrylic acid (2.73 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 11.05 mg title compound was obtained as a white solid after lyophilization. Yield: 49%. LCMS Anal. calculated for C₂₈H₂₄F₃N₃O₆S: 587.1, found m/z: 586.2 [M-H]⁻, 588.2 [M+H]⁺, t_{R} = 3.44 min; peak area 99%. ¹H NMR (300 MHz, DMSO-d6): δ 11.2 (bs, 1H), 8.44 (d, J=5.1Hz, 1H), 8.18 (m, 1H), 7.72 (m, 1H), 7.64 (m, 1H), 7.56 (s, 1H), 7.47 (s, 1H), 7.39 (m, 2H), 7.18 (d, J=9.0Hz, 1H), 7.04 (d, J=8.9Hz, 1H), 6.35 (d, J=6.3Hz, 1H), 6.20 (m, 1H), 6.07 (m, 1H), 5.57 (dd, J=9.9Hz, 2.1Hz, 1H), 4.18 (t, J=6.0Hz, 2H), 3.92 (s, 3H), 3.26 (m, 2H), 2.02 (m, 2H).

### Example 34 [AXL22045]

### (E)-N-[3-[[4-[4-[(2,5-difluorophenyl)sulfonylamino]-2-fluoro-phenoxy]-6-methoxy-7-quinolyl]oxy]propyl]-2-methyl-but-2-enamide

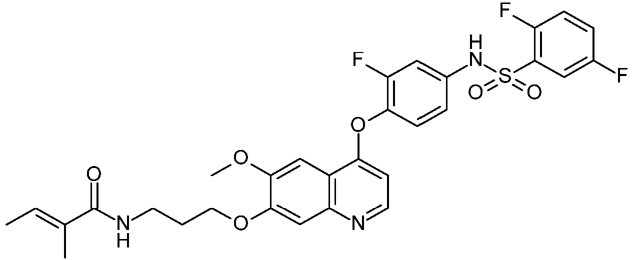

To the solution of trans-2,3-dimethylacrylic acid (3.80 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 16.49 mg title compound was obtained as a white solid after lyophilization. Yield: 70%. LCMS Anal. calculated for C₃₀H₂₈F₃N₃O₆S: 615.2, found m/z: 614.2 [M-H]⁻, 616.2 [M+H]⁺, t_{R} = 3.44 min; peak area 99%. ¹H NMR (300 MHz, DMSO-d6): δ 11.2 (bs, 1H), 8.47 (d, J=5.3Hz, 1H), 7.80 (m, 1H), 7.73 (m, 1H), 7.55 (m, 2H), 7.49 (s, 1H), 7.39 (m, 2H), 7.20 (dd, J=9.6Hz, 2.4Hz, 1H), 7.07 (d, J=9.2Hz, 1H), 6.40 (d, J=4.9Hz, 1H), 6.30 (m, 1H), 4.18 (t, J=6.0Hz, 2H), 3.92 (s, 3H), 3.35 (m, 2H), 2.02 (m, 2H), 1.76 (s, 3H), 1.71 (d, J=6.3Hz, 3H).

### Example 35 [AXL22046]

### (E)-N-[3-[[4-[4-[(2,5-difluorophenyl)sulfonylamino]-2-fluoro-phenoxy]-6-methoxy-7-quinolyl]oxy]propyl]-4,4,4-trifluoro-but-2-enamide

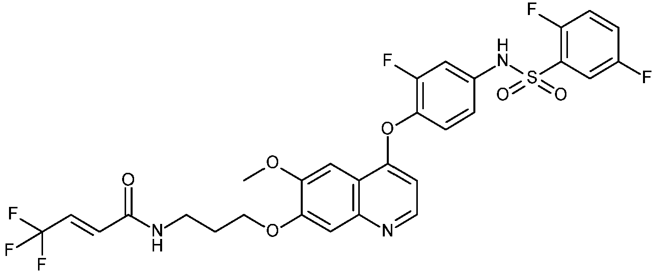

To the solution of trans-4,4,4-trifluorocrotonic acid (5.32 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 11.53 mg title compound was obtained as a white solid after lyophilization. Yield: 46%. LCMS Anal. calculated for C₂₉H₂₃F₆N₃O₆S: 655.1, found m/z: 654.1 [M-H]⁻, 656.1 [M+H]⁺, t_{R} = 3.72 min; peak area 100%. ¹H NMR (300 MHz, DMSO-d6): δ 11.1 (bs, 1H), 8.63 (t, J=5.1Hz, 1H), 8.48 (d, J=5.3Hz, 1H), 7.72 (m, 1H), 7.63 (m, 1H), 7.54 (m, 1H), 7.48 (s, 1H), 7.39 (m, 2H), 7.19 (dd, J=11.8z, 2.0Hz, 1H), 7.10 (d, J=8.9Hz, 1H), 6.77 (m, 2H), 6.41 (d, J=5.2Hz, 1H), 4.21 (t, J=6.0Hz, 2H), 3.92 (s, 3H), 3.26 (m, 2H), 2.05 (m, 2H).

### Example 36 [AXL22047]

### (E)-N-[3-[[4-[4-[(2,5-difluorophenyl)sulfonylamino]-2-fluoro-phenoxy]-6-methoxy-7-quinolyl]oxy]propyl]-2-methyl-pent-2-enamide

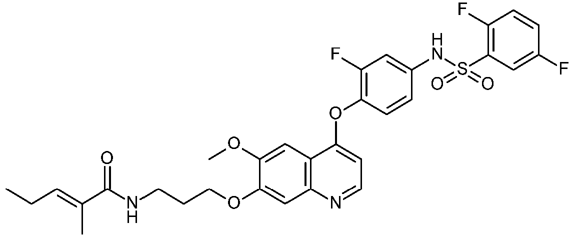

To the solution of 2-methyl-2-pentanoic acid (4.33 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 15.40 mg title compound was obtained as a white solid after lyophilization. Yield: 64%. LCMS Anal. calculated for C₃₁H₃₀F₃N₃O₆S: 629.2, found m/z: 628.2 [M-H]⁻, 630.2 [M+H]⁺, t_{R} = 3.59 min; peak area 99%. ¹H NMR (300 MHz, DMSO-d6): δ 11.0 (bs, 1H), 8.44 (d, J=5.2Hz, 1H), 7.80 (m, 1H), 7.72 (m, 1H), 7.59 (m, 1H), 7.52 (m, 1H), 7.48 (s, 1H), 7.35 (m, 2H), 7.18 (dd, J=12.0z, 2.0Hz, 1H), 7.07 (d, J=8.9Hz, 1H), 6.35 (d, J=5.2Hz, 1H), 6.22 (t, J=7.2Hz, 1H), 4.21 (t, J=6.0Hz, 2H), 3.91 (s, 3H), 3.25 (m, 2H), 2.12 (m, 2H), 2.00 (m, 2H), 1.76 (s, 3H), 0.99 (t, J=7.5Hz, 3H).

### Example 37 [AXL22048]

### N-[3-[[4-[4-[(2,5-difluorophenyl)sulfonylamino]-2-fluoro-phenoxy]-6-methoxy-7-quinolyl]oxy]propyl]but-2-ynamide

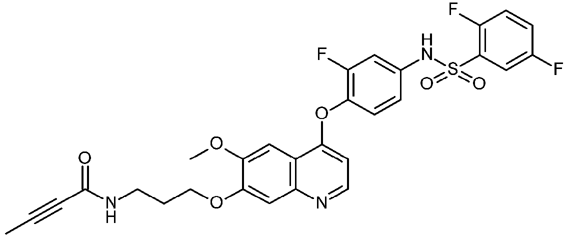

To the solution of 2-butynoic acid (3.19 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 14.70 mg title compound was obtained as a white solid after lyophilization. Yield: 64%. LCMS Anal. calculated for C₂₉H₂₄F₃N₃O₆S: 599.1, found m/z: 598.2 [M-H]⁻, 600.2 [M+H]⁺, t_{R} = 3.31 min; peak area 99%. ¹H NMR (300 MHz, DMSO-d6): δ 11.1 (bs, 1H), 8.51 (t, 1H), 8.46 (d, J=5.5Hz, 1H), 7.72 (m, 1H), 7.62 (m, 1H), 7.57 (m, 1H), 7.48 (s, 1H), 7.38 (m, 2H), 7.19 (dd, J=11.9Hz, 2.1Hz, 1H), 7.06 (d, J=9.3Hz, 1H), 6.38 (d, J=4.8Hz, 1H), 4.21 (t, J=6.0Hz, 2H), 3.92 (s, 3H), 3.26 (m, 2H), 1.95 (bs, 5H).

### Example 38 [AXL22049]

### 2-chloro-N-[3-[[4-[4-[(2,5-difluorophenyl)sulfonylamino]-2-fluoro-phenoxy]-6-methoxy-7-quinolyl]oxy]propyl]acetamide

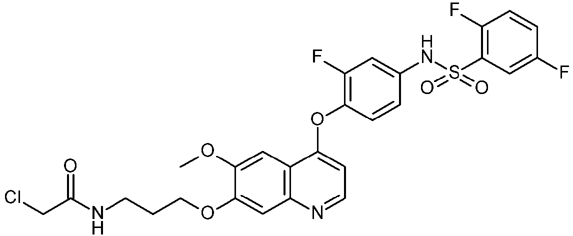

To the solution of chloroacetic acid (3.57 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 16.43 mg title compound was obtained as a white solid after lyophilization. Yield: 71%. LCMS Anal. calculated for C₂₇H₂₃ClF₃N₃O₆S: 609.1, found m/z: 608.1 [M-H]⁻, 610.1 [M+H]⁺, t_{R} = 3.30 min; peak area 100%. ¹H NMR (300 MHz, DMSO-d6): δ 11.1 (bs, 1H), 8.45 (d, J=5.2Hz, 1H), 8.31 (t, 1H), 7.72 (m, 1H), 7.63 (m, 1H), 7.54 (m, 1H), 7.48 (s, 1H), 7.38 (m, 2H), 7.20 (dd, J=12.0z, 2.0Hz, 1H), 7.07 (d, J=7.8Hz, 1H), 6.36 (d, J=5.1Hz, 1H), 4.18 (t, J=6.3Hz, 2H), 4.06 (s, 2H), 3.92 (s, 3H), 3.21 (m, 2H), 1.98 (m, 2H).

### Example 39 [AXL22050]

### (E)-N-[3-[[4-[4-[(2,5-difluorophenyl)sulfonylamino]-2-fluoro-phenoxy]-6-methoxy-7-quinolyl]oxy]propyl]-4-(dimethylamino)but-2-enamide

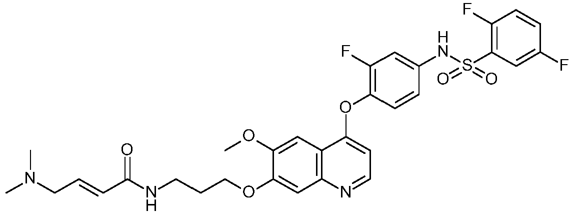

To the solution trans-4-dimethylcrotonic acid hydrochloride (6.95 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (10.82 mg, 0.084 mmol) was added. The mixture was stirred for 3 hours at room temperature. The mixture was purified by reverse phase chromatography. Thus, 13.40 mg title compound was obtained as a white solid after lyophilization. Yield: 55%. LCMS Anal. calculated for C₃₁H₃₁F₃N₄O₆S: 644.2, found m/z: 643.2 [M-H]⁻, 645.2 [M+H]⁺, t_{R} = 2.72 min; peak area 98%. ¹H NMR (300 MHz, DMSO-d6): δ 11.2 (bs, 1H), 8.45 (d, J=5.2Hz, 1H), 8.14 (bs, 1H), 7.65 (m, 1H), 7.50 (m, 3H), 7.37 (s, 1H), 7.25 (t, J=9.0Hz, 1H), 7.10 (d, J=12.4Hz, 1H), 6.96 (d, J=9.3Hz, 1H), 6.55 (m, 1H), 6.35 (d, J=9.3Hz, 1H), 6.05 (d, 15.3Hz, 1H), 4.18 (t, J=6.0Hz, 2H), 3.93 (s, 3H), 3.15 (m, 4H), 2.26 (s, 6H), 1.98 (m, 2H).

### Example 40 [AXL22051]

### (E)-N-[3-[[4-[4-[(2,5-difluorophenyl)sulfonylamino]-2-fluoro-phenoxy]-6-methoxy-7-quinolyl]oxy]propyl]-3-(2-hydroxyphenyl)prop-2-enamide

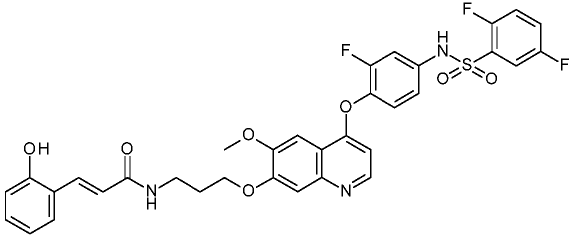

To the solution 2-hydroxycinnamic acid (6.23 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 11.53 mg title compound was obtained as a white solid after lyophilization. Yield: 44%. LCMS Anal. calculated for C₃₄H₂₈F₃N₃O₇S: 679.2, found m/z: 678.2 [M-H]⁻, 680.2 [M+H]⁺, t_{R} = 3.47 min; peak area 98%. ¹H NMR (300 MHz, DMSO-d6): δ 11.2 (bs, 1H), 10.00 (s, 1H), 8.48 (d, J=5.3Hz, 1H), 8.17 (s, 1H), 7.74 (m, 1H), 7.61 (m, 3H), 7.51 (s, 1H), 7.41 (m, 3H), 7.19 (m, 2H), 7.07 (d, J=9.4Hz, 1H), 6.88 (d, J=8.0Hz, 1H), 6.81 (t, J=7.2Hz, 1H), 6.65 (d, J=15.8Hz, 1H), 6.41 (d, J=5.8Hz, 1H), 4.22 (t, J=6.0Hz, 2H), 3.94 (s, 3H), 3.22 (m, 2H), 2.02 (m, 2H).

### Example 41 [AXL22052]

### N-[3-[[4-[4-[(2,5-difluorophenyl)sulfonylamino]-2-fluoro-phenoxy]-6-methoxy-7-quinolyl]oxy]propyl]-3-methyl-but-2-enamide

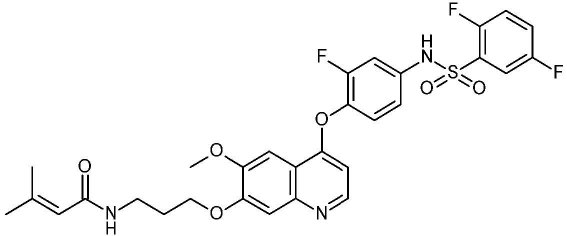

To the solution 2,3-dimethylacrylic acid (3.80 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 14.03 mg title compound was obtained as a white solid after lyophilization. Yield: 60%. LCMS Anal. calculated for C₃₀H₂₈F₃N₃O₆S: 615.2, found m/z: 614.2 [M-H]⁻, 616.2 [M+H]⁺, t_{R} = 3.50 min; peak area 99%. ¹H NMR (300 MHz, DMSO-d6): δ 11.1 (bs, 1H), 8.44 (d, J=5.2Hz, 1H), 7.82 (t, J=4.6Hz, 1H), 7.72 (m, 1H), 7.63 (m, 1H), 7.54 (m, 1H), 7.48 (s, 1H), 7.38 (m, 2H), 7.20 (dd, J=12.1z, 2.0Hz, 1H), 7.06 (d, J=9.0Hz, 1H), 6.48 (d, J=5.2Hz, 1H), 5.64 (s, 1H), 4.17 (t, J=6.0Hz, 2H), 3.93 (s, 3H), 3.27 (m, 2H), 2.07 (s, 3H), 1.94 (m, 2H), 1.77 (s, 3H).

### Example 42 [AXL22053]

### (E)-N-[3-[[4-[4-[(2,5-difluorophenyl)sulfonylamino]-2-fluoro-phenoxy]-6-methoxy-7-quinolyl]oxy]propyl]-3-(2-thienyl)prop-2-enamide

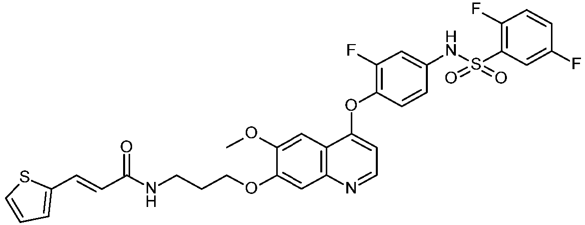

To the solution 3-(2-thienyl)acrylic acid (5.85 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 18.56 mg title compound was obtained as a white solid after lyophilization. Yield: 70%. LCMS Anal. calculated for C₃₂H₂₆F₃N₃O₆S₂: 669.1, found m/z: 668.1 [M-H]-, 670.1 [M+H]+, t_{R} = 3.63 min; peak area 98%. 1H NMR (300 MHz, DMSO-d6): δ 11.1 (bs, 1H), 8.44 (d, J=5.2Hz, 1H), 8.22 (m, 1H), 7.72 (m, 1H), 7.58 (m, 4H), 7.48 (s, 1H), 7.39 (m, 3H), 7.18 (dd, J=12.0Hz, 2.0Hz, 1H), 7.10 (m, 2H), 6.49 (s, 1H), 6.36 (d, J=5.7Hz, 1H), 4.20 (t, J=6.0Hz, 2H), 3.93 (s, 3H), 3.22 (m, 2H), 2.00 (m, 2H).

### Example 43 [AXL22054]

### (E)-N-[3-[[4-[4-[(2,5-difluorophenyl)sulfonylamino]-2-fluoro-phenoxy]-6-methoxy-7-quinolyl]oxy]propyl]-3-(1H-imidazol-5-yl)prop-2-enamide

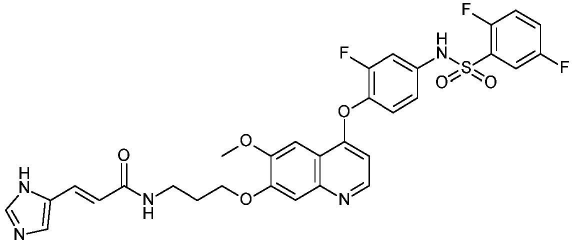

To the solution urocanic acid (4-Imidazoleacrylic acid, 5.24 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 10.20 mg title compound was obtained as a white solid after lyophilization. Yield: 41%. LCMS Anal. calculated for C₃₁H₂₆F₃N₅O₆S: 653.2, found m/z: 652.2 [M-H]⁻, 654.2 [M+H]⁺, t_{R} = 2.80 min; peak area 98%. ¹H NMR (300 MHz, DMSO-d6): δ 11.8 (bs, 1H), 8.44 (d, J=5.2Hz, 1H), 8.08 (t, J=5.3Hz, 1H), 7.70 (m, 2H), 7.63 (m, 1H), 7.54 (m, 1H), 7.48 (s, 1H), 7.38 (m, 3H), 7.20 (dd, J=12.0Hz, 2.0Hz, 1H), 7.05 (t, J=11.9Hz, 1H), 6.49 (d, J=15.6Hz, 1H), 6.36 (d, J=5.1Hz, 1H), 4.20 (t, J=6.0Hz, 2H), 3.93 (s, 3H), 3.29 (m, 2H), 2.00 (m, 2H).

### Example 44 [AXL22055]

### (E)-N-[3-[[4-[4-[(2,5-difluorophenyl)sulfonylamino]-2-fluoro-phenoxy]-6-methoxy-7-quinolyl]ox y]propyl]-4,4,4-trifluoro-3-methyl-but-2-enamide

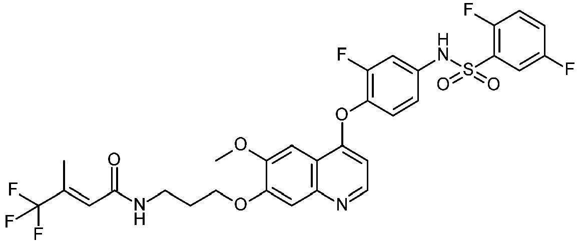

To the solution 3-(trifluoromethyl)crotonic acid (5.85 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 15.62 mg title compound was obtained as a white solid after lyophilization. Yield: 61%. LCMS Anal. calculated for C₃₀H₂₅F₆N₃O₆S: 669.1, found m/z: 668.2 [M-H]⁻, 670.2 [M+H]⁺, t_{R} = 3.70 min; peak area 100%. ¹H NMR (300 MHz, DMSO-d6): δ 11.1 (bs, 1H), 8.44 (m, 2H), 7.73 (m, 1H), 7.63 (m, 1H), 7.54 (m, 1H), 7.48 (s, 1H), 7.38 (m, 2H), 7.20 (dd, J=12.0Hz, 2.0Hz, 1H), 7.05 (t, J=11.9Hz, 1H), 6.47 (s, 1H), 6.41 (d, J=5.4Hz, 1H), 4.20 (t, J=6.0Hz, 2H), 3.93 (s, 3H), 3.29 (m, 2H), 2.15 (s, 3H), 2.00 (m, 2H).

### Example 45 [AXL22058]

### N-[3-[[4-[2-fluoro-4-[[2-(trifluoromethyl)phenyl]sulfonylamino]phenoxy]-6-methoxy-7-quinolyl]oxy]propyl]prop-2-enamide

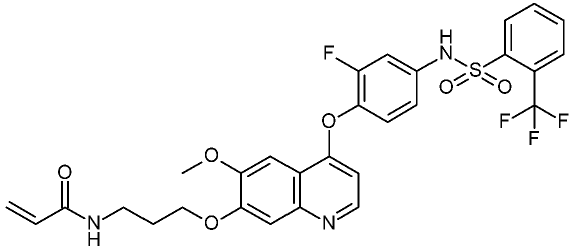

To the solution acrylic acid (2.73 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 10.44 mg title compound was obtained as a white solid after lyophilization. Yield: 44%. LCMS Anal. calculated for C₂₉H₂₅F₄N₃O₆S: 619.1, found m/z: 618.2 [M-H]⁻, 620.2 [M+H]⁺, t_{R} = 3.57 min; peak area 98%. ¹H NMR (300 MHz, DMSO-d6): δ 11.1 (bs, 1H), 8.44 (d, J=5.2Hz, 1H), 8.15 (m, 2H), 8.02 (d, J=6.7Hz, 1H), 7.89 (m, 2H), 7.47 (s, 1H), 7.37 (m, 2H), 7.14 (dd, J=12.0Hz, 2.0Hz, 1H), 7.01 (d, J=8.4Hz, 1H), 6.35 (d, J=5.1Hz, 1H), 6.20 (m, 1H), 6.10 (m, 1H), 5.58 (dd, J=9.9Hz, 2.1Hz, 1H), 4.18 (t, J=6.0Hz, 2H), 3.92 (s, 3H), 3.27 (m, 2H), 2.02 (m, 2H).

### Example 46 [AXL22059]

### (E)-N-[3-[[4-[2-fluoro-4-[[2-(trifluoromethyl)phenyl]sulfonylamino]phenoxy]-6-methoxy-7-quinolyl]oxy]propyl]but-2-enamide

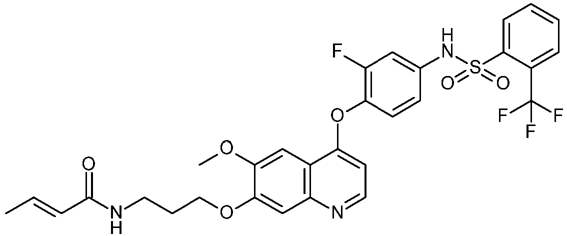

To the solution crotonic acid (3.27 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 7.49 mg title compound was obtained as a white solid after lyophilization. Yield: 31%. LCMS Anal. calculated for C₃₀H₂₇F₄N₃O₆S: 633.2, found m/z: 632.2 [M-H]⁻, 634.2 [M+H]⁺, t_{R} = 3.44; peak area 99%. ¹H NMR (300 MHz, DMSO-d6): δ 11.0 (bs, 1H), 8.44 (d, J=5.2Hz, 1H), 8.14 (m, 1H), 8.03 (d, J=6.9Hz, 1H), 7.90 (m, 3H), 7.47 (s, 1H), 7.37 (m, 2H), 7.16 (dd, J=12.0Hz, 2.0Hz, 1H), 7.02 (d, J=8.4Hz, 1H), 6.61 (m, 1H), 6.36 (d, J=5.2Hz, 1H), 5.89 (d, J=15.3Hz, 1H), 4.18 (t, J=6.0Hz, 2H), 3.92 (s, 3H), 3.27 (m, 2H), 1.95 (m, 2H), 1.79 (d, J=6.9Hz, 3H).

### Example 47 [AXL22060]

### (E)-N-[3-[[4-[2-fluoro-4-[[2-(trifluoromethyl)phenyl]sulfonylamino]phenoxy]-6-methoxy-7-quinolyl]oxy]propyl]-2-methyl-but-2-enamide

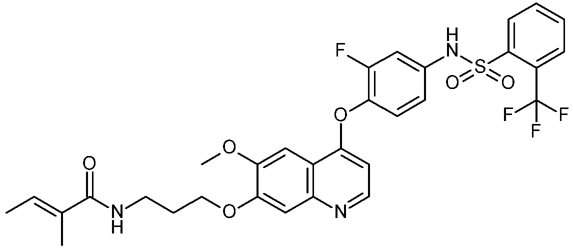

To the solution 2,3-dimethylacrylic acid (3.80 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 11.02 mg title compound was obtained as a white solid after lyophilization. Yield: 43 %. LCMS Anal. calculated for C₃₁H₂₉F₄N₃O₆S: 647.2, found m/z: 646.2 [M-H]⁻, 648.2 [M+H]⁺, t_{R} = 3.55 min; peak area 98%. ¹H NMR (300 MHz, DMSO-d6): δ 11.1 (bs, 1H), 8.62 (d, J=5.4Hz, 1H), 8.17 (d, J=7.2Hz, 1H), 8.03 (d, J=7.2Hz, 1H), 7.91 (m, 2H), 7.82 (m, 1H), 7.57 (s, 1H), 7.45 (d, J=9.1Hz, 1H), 7.42 (s, 1H), 7.22 (dd, J=12.0Hz, 2.0Hz, 1H), 7.06 (d, J=6.8Hz, 1H), 6.60 (d, J=5.8Hz, 1H), 6.32 (dd, J=12.6Hz, 5.7Hz, 1H), 4.20 (t, J=6.0Hz, 2H), 3.96 (s, 3H), 3.27 (m, 2H), 2.02 (m, 2H), 1.74 (s, 3H), 1.69 (d, J=6.9Hz, 3H).

### Example 48 [AXL22061]

### (E)-4,4,4-trifluoro-N-[3-[[4-[2-fluoro-4-[[2-(trifluoromethyl)phenyl]sulfonylamino]phenoxy]-6-methoxy-7-quinolyl]oxy]propyl]but-2-enamide

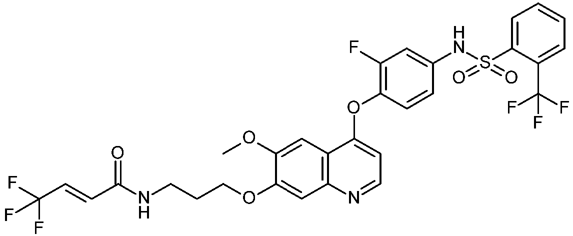

To the solution 4,4,4-trifluorocrotonic acid (5.32 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 13.40 mg title compound was obtained as a white solid after lyophilization. Yield: 51%. LCMS Anal. calculated for C₃₀H₂₄F₇N₃O₆S: 687.1, found m/z: 686.2 [M-H]⁻, 688.1 [M+H]⁺, t_{R} = 3.68 min; peak area 98%. ¹H NMR (300 MHz, DMSO-d6): δ 11.1 (bs, 1H), 8.62 (m, 2H), 8.17 (d, J=7.4Hz, 1H), 8.05 (d, J=7.2Hz, 1H), 7.91 (m, 2H), 7.58 (s, 1H), 7.44 (m, 2H), 7.21 (dd, J=12.0Hz, 2.0Hz, 1H), 7.06 (d, J=6.2Hz, 1H), 6.75 (bs, 2H), 6.60 (d, J=5.3Hz, 1H), 4.20 (t, J=6.0Hz, 2H), 3.92 (s, 3H), 3.27 (m, 2H), 2.02 (m, 2H).

### Example 49 [AXL22066]

### 2-chloro-N-[3-[[4-[2-fluoro-4-[[2-(trifluoromethyl)phenyl]sulfonylamino]phenoxy]-6-methoxy-7-quinolyl]oxy]propyl]acetamide

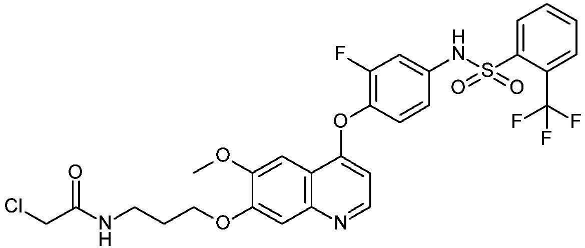

To the solution chloroacetic acid (3.59 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 14.25 mg title compound was obtained as a white solid after lyophilization. Yield: 58 %. LCMS Anal. calculated for C₂₈H₂₄ClF₄N₃O₆S: 641.1, found m/z: 640.1 [M-H]⁻, 642.1 [M+H]⁺, t_{R} = 3.43 min; peak area 97%. ¹H NMR (300 MHz, DMSO-d6): δ 11.0 (bs, 1H), 8.46 (d, J=5.0Hz, 1H), 8.30 (t, 1H), 8.16 (d, J=7.4Hz, 1H), 8.04 (d, J=6.5Hz, 1H), 7.91 (m, 2H), 7.48 (s, 1H), 7.38 (m, 2H), 7.17 (d, J=11.8Hz, 1H), 7.04 (d, J=8.5Hz, 1H), 6.38 (bs, 1H), 4.18 (t, J=6.0Hz, 2H), 4.06 (s, 2H), 3.92 (s, 3H), 3.27 (m, 2H), 1.98 (m, 2H).

### Example 50 [AXL22067]

### N-[3-[[4-[2-fluoro-4-[[2-(trifluoromethyl)phenyl]sulfonylamino]phenoxy]-6-methoxy-7-quinolyl]oxy]propyl]but-2-ynamide

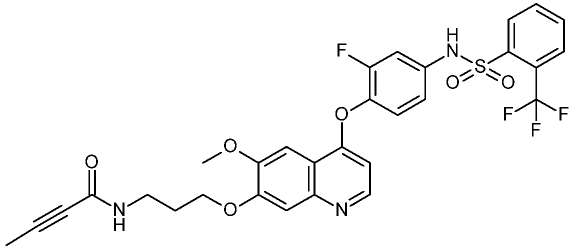

To the solution 2-butynoic acid (3.19 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 10.18 mg title compound was obtained as a white solid after lyophilization. Yield: 42%. LCMS Anal. calculated for C₃₀H₂₅F₄N₃O₆S: 631.1, found m/z: 630.2 [M-H]⁻, 632.2 [M+H]⁺, t_{R} = 3.41 min; peak area 100%. ¹H NMR (300 MHz, DMSO-d6): δ 11.0 (bs, 1H), 8.51 (t, J=5.1Hz, 1H), 8.47 (d, J=5.2Hz, 1H), 8.15 (d, J=7.7Hz, 1H), 8.03 (d, J=7.0Hz, 1H), 7.89 (m, 2H), 7.47 (s, 1H), 7.37 (m, 2H), 7.15 (dd, J=11.2Hz, 2.2Hz, 1H), 7.01 (d, J=8.9Hz, 1H), 6.36 (bs, 1H), 4.18 (t, J=6.0Hz, 2H), 3.92 (s, 3H), 3.27 (m, 2H), 1.94 (m, 5H).

### Example 51 [AXL22068]

### (E)-N-[3-[[4-[2-fluoro-4-[[2-(trifluoromethyl)phenyl]sulfonylamino]phenoxy]-6-methoxy-7-quinolyl]oxy]propyl]-2-methyl-pent-2-enamide

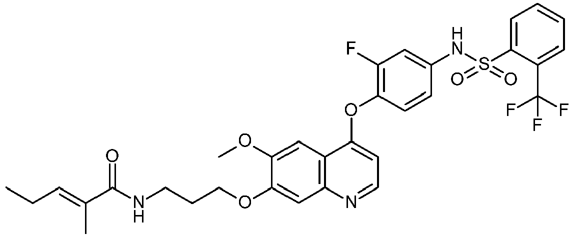

To the solution trans-3-methyl-2-pentenoic acid (4.33 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 13.38 mg title compound was obtained as a white solid after lyophilization. Yield: 53%. LCMS Anal. calculated for C₃₂H₃₁F₄N₃O₆S: 661.2, found m/z: 660.2 [M-H]⁻, 662.2 [M+H]⁺, t_{R} = 3.64 min; peak area 100%. ¹H NMR (300 MHz, DMSO-d6): δ 11.0 (bs, 1H), 8.53 (d, J=5.6Hz, 1H), 8.16 (d, J=7.0Hz, 1H), 8.04 (d, J=6.8Hz, 1H), 7.92 (m, 2H), 7.83 (m, 1H), 7.53 (s, 1H), 7.42 (m, 2H), 7.18 (dd, J=12.0Hz, 2.2Hz, 1H), 7.05 (d, J=9.3Hz, 1H), 6.50 (d, J=5.1Hz, 1H), 6.23 (t, J=6.8Hz, 1H), 4.18 (t, J=6.0Hz, 2H), 3.92 (s, 3H), 3.27 (m, 2H), 2.02 (m, 2H), 1.74 (s, 3H), 0.97 (t, J=7.5Hz, 3H).

### Example 52 [AXL22069]

### (E)-4-(dimethylamino)-N-[3-[[4-[2-fluoro-4-[[2-(trifluoromethyl)phenyl]sulfonylamino]phenoxy]-6-methoxy-7-quinolyl]oxy]propyl]but-2-enamide

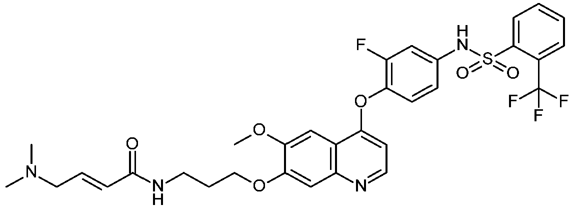

To the solution trans-4-dimethylaminocrotonic acid hydrochloride (6.29 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (10.82 mg, 0.084 mmol) was added. The mixture was stirred for 3 hours at room temperature. The mixture was purified by reverse phase chromatography. Thus, 8.75 mg title compound was obtained as a white solid after lyophilization. Yield: 34%. LCMS Anal. calculated for C₃₂H₃₂F₄N₄O₆S: 676.2, found m/z: 675.3 [M-H]⁻, 677.3 [M+H]⁺, t_{R} = 3.19 min; peak area 100%. ¹H NMR (300 MHz, DMSO-d6): δ 11.2 (bs, 1H), 8.45 (d, J=5.2Hz, 1H), 8.13 (m, 2H), 8.00 (d, J=7.2Hz, 1H), 7.86 (m, 2H), 7.48 (s, 1H), 7.38 (s, 1H), 7.32 (t, J=8.9Hz, 1H), 7.11 (dd, J=12.4Hz, 2.2Hz, 1H), 6.97 (d, J=8.8Hz, 1H), 6.56 (m, 1H), 6.36 (d, J=5.2Hz, 1H), 6.06 (d, J=5.5Hz, 1H), 4.18 (t, J=6.0Hz, 2H), 3.92 (s, 3H), 3.35 (m, 2H), 3.11 (m, 2H), 2.23 (s, 6H), 2.02 (m, 2H).

### Example 53 [AXL22070]

### N-[3-[[4-[2-fluoro-4-[[2-(trifluoromethyl)phenyl]sulfonylamino]phenoxy]-6-methoxy-7-quinolyl]oxy]propyl]-3-methyl-but-2-enamide

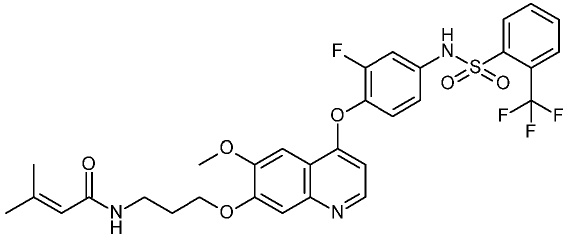

To the solution 3,3-dimethylacrylic acid (3.80 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 17.72 mg title compound was obtained as a white solid after lyophilization. Yield: 72%. LCMS Anal. calculated for C₃₁H₂₉F₄N₃O₆S: 647.2, found m/z: 646.2 [M-H]⁻, 648.2 [M+H]⁺, t_{R} = 3.91 min; peak area 99%. ¹H NMR (300 MHz, DMSO-d6): δ 11.1 (bs, 1H), 8.51 (d, J=5.5Hz, 1H), 8.16 (d, J=7.1Hz, 1H), 8.04 (d, J=6.8Hz, 1H), 7.93 (m, 2H), 7.80 (m, 1H), 7.51 (s, 1H), 7.41 (m, 2H), 7.18 (dd, J=12.2Hz, 2.3Hz, 1H), 7.05 (d, J=9.5Hz, 1H), 6.46 (d, J=5.2Hz, 1H), 5.63 (s, 1H), 4.18 (t, J=6.0Hz, 2H), 3.92 (s, 3H), 3.37 (m, 2H), 2.06 (s, 3H), 1.96 (m, 2H), 1.77 (s, 3H).

### Example 54 [AXL22071]

### (E)-N-[3-[[4-[2-fluoro-4-[[2-(trifluoromethyl)phenyl]sulfonylamino]phenoxy]-6-methoxy-7-quinolyl]oxy]propyl]-3-(2-thienyl)prop-2-enamide

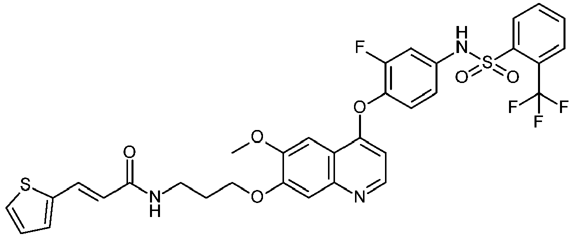

To the solution 3-(2-thienyl)acrylic acid (5.85 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 15.03 mg title compound was obtained as a white solid after lyophilization. Yield: 56%. LCMS Anal. calculated for C₃₃H₂₇F₄N₃O₆S₂: 701.2, found m/z: 700.2 [M-H]⁻, 702.2 [M+H]⁺, t_{R} = 4.05 min; peak area 100%. ¹H NMR (300 MHz, DMSO-d6): δ 11.0 (bs, 1H), 8.49 (d, J=5.3Hz, 1H), 8.15 (m, 2H), 8.04 (d, J=6.8Hz, 1H), 7.91 (m, 2H), 7.55 (m, 2H), 7.51 (s, 1H), 7.38 (m, 3H), 7.18 (dd, J=12.4Hz, 2.4Hz, 1H), 7.07 (m, 2H), 6.43 (d, J=5.1Hz, 1H), 6.37 (d, J=15.6Hz, 1H), 4.21 (t, J=6.0Hz, 2H), 3.92 (s, 3H), 3.39 (m, 2H), 2.01 (m, 2H).

### Example 55 [AXL22072]

### (E)-N-[3-[[4-[2-fluoro-4-[[2-(trifluoromethyl)phenyl]sulfonylamino]phenoxy]-6-methoxy-7-quinolyl]oxy]propyl]-3-(2-hydroxyphenyl)prop-2-enamide

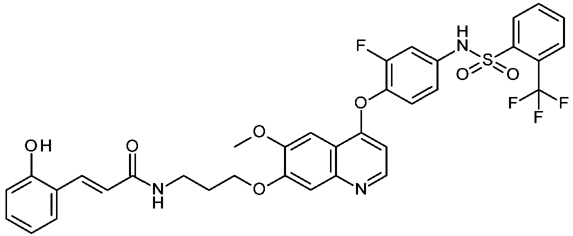

To the solution 2-hydroxycinnamic acid (6.23 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 6.53 mg title compound was obtained as a white solid after lyophilization. Yield: 24%. LCMS Anal. calculated for C₃₅H₂₉F₄N₃O₇S: 711.2, found m/z: 710.2 [M-H]⁻, 712.2 [M+H]⁺, t_{R} = 3.87 min; peak area 100%. ¹H NMR (300 MHz, DMSO-d6): δ 11.1 (bs, 1H), 10.15 (s, 1H), 8.65 (d, J=6.0Hz, 1H), 8.16 (m, 2H), 8.05 (d, J=6.3Hz, 1H), 7.92 (m, 2H), 7.62 (m, 2H), 7.48 (m, 3H), 7.18 (m, 2H), 7.07 (d, J=8.6Hz, 1H), 6.88 (m, 1H), 6.82 (m, 1H), 6.67 (m, 1H), 4.21 (t, J=6.0Hz, 2H), 3.98 (s, 3H), 3.37 (m, 2H), 2.04 (t, J=6.0Hz, 2H).

### Example 56 [AXL22074]

### 2,5-difluoro-N-[3-fluoro-4-[[6-methoxy-7-[3-[4-[(E)-2-methylpent-2-enoyl]piperazin-1-yl]propoxy]-4-quinolyl]oxy]phenyl]benzenesulfonamide

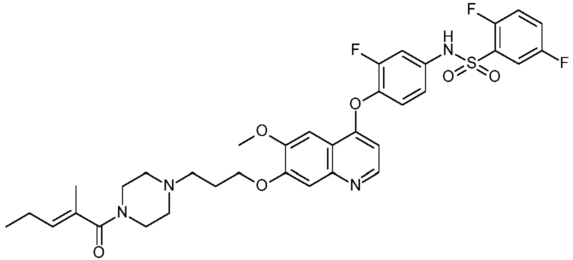

To the solution of trans-2-methyl-2-pentenoic acid (4.33 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 12.11 mg title compound was obtained as a white solid after lyophilization. Yield: 45%. LCMS Anal. calculated for C₃₅H₃₇F₃N₄O₆S: 698.2, found m/z: 697.3 [M-H]⁻, 699.3 [M+H]⁺, t_{R} = 2.93 min; peak area 98%. ¹H NMR (300 MHz, DMSO-d6): δ 10.8 (bs, 1H), 8.44 (d, J=5.1Hz, 1H), 7.73 (m, 1H), 7.60 (m, 2H), 7.47 (s, 1H), 7.38 (m, 2H), 7.18 (dd, J=12.1Hz, 2.3Hz, 1H), 7.05 (d, J=8.0Hz, 1H), 6.35 (d, J=5.1Hz, 1H), 5.45 (t, J=7.2Hz, 1H), 4.20 (t, J=6.1Hz, 2H), 3.92 (s, 3H), 3.46 (m, 4H), 2.41 (m, 6H), 2.04 (m, 4H), 1.72 (s, 3H), 0.95 (t, J=7.2Hz, 3H).

### Example 57 [AXL22075]

### N-[4-[[7-[3-(4-but-2-ynoylpiperazin-1-yl)propoxy]-6-methoxy-4-quinolyl]oxy]-3-fluoro-phenyl]-2-(trifluoromethyl)benzenesulfonamide

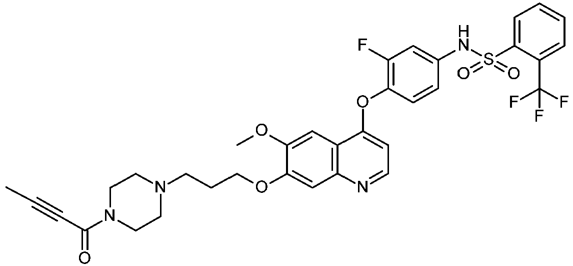

To the solution of 2-butynoic acid (3.19 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 12.51 mg title compound was obtained as a white solid after lyophilization.. Yield: 47%. LCMS Anal. calculated for C₃₄H₃₂F₄N₄O₆S: 700.2, found m/z: 699.3 [M-H]⁻, 701.3 [M+H]⁺, t_{R} = 3.17 min; peak area 98%. ¹H NMR (300 MHz, DMSO-d6): δ 10.8 (bs, 1H), 8.45 (d, J=5.2Hz, 1H), 8.15 (d, J=7.6Hz, 1H), 8.04 (d, J=7.2Hz, 1H), 7.91 (m, 2H), 7.47 (s, 1H), 7.39 (m, 2H), 7.17 (dd, J=12.1Hz, 2.1Hz, 1H), 7.03 (d, J=8.7Hz, 1H), 6.36 (d, J=5.1Hz, 1H), 4.20 (t, J=6.0Hz, 2H), 3.91 (s, 3H), 3.50 (m, 4H), 2.54 (m, 4H), 2.39 (m, 2H), 2.06 (m, 5H).

### Example 58 [AXL22076]

### (E)-N-[3-[[4-[4-[(2,5-difluorophenyl)sulfonylamino]-2-fluoro-phenoxy]-6-methoxy-7-quinolyl]oxy]propyl]-3-phenyl-prop-2-enamide

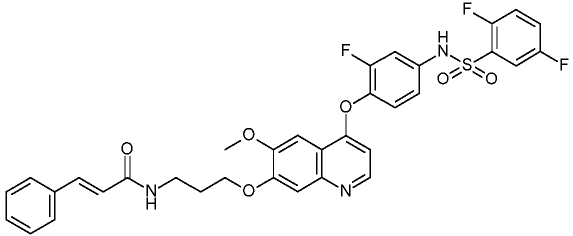

To the solution trans-cinnamic acid (5.63 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 13.82 mg title compound was obtained as a white solid after lyophilization. Yield: 54%. LCMS Anal. calculated for C₃₄H₂₈F₃N₃O₆S: 663.2, found m/z: 662.2 [M-H]⁻, 664.2 [M+H]⁺, t_{R} = 3.96 min; peak area 100%. ¹H NMR (300 MHz, DMSO-d6): δ 11.2 (bs, 1H), 8.48 (d, J=5.0Hz, 1H), 8.23 (t, J=5.2Hz, 1H), 7.74 (m, 1H), 7.62 (m, 1H), 7.54 (m, 3H), 7.51 (s, 1H), 7.38 (m, 6H), 7.20 (dd, J=12.1Hz, 2.1Hz, 1H), 7.07 (d, J=8.9Hz, 1H), 6.62 (d, J=12.6Hz, 1H), 6.41 (d, J=4.8Hz, 1H), 4.22 (t, J=6.0Hz, 2H), 3.93 (s, 3H), 3.39 (m, 2H), 2.02 (m, 2H).

### Example 59 [AXL22077]

### (E)-N-[3-[[4-[4-[(2,5-difluorophenyl)sulfonylamino]-2-fluoro-phenoxy]-6-methoxy-7-quinolyl]oxy]propyl]-2-methyl-3-phenyl-prop-2-enamide

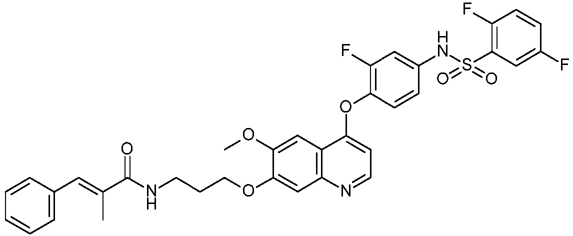

To the solution α-methylcinnamic acid (6.15 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 17.83 mg title compound was obtained as a white solid after lyophilization. Yield: 69%. LCMS Anal. calculated for C₃₅H₃₀F₃N₃O₆S: 677.2, found m/z: 676.2 [M-H]⁻, 678.2 [M+H]⁺, t_{R} = 3.77 min; peak area 98%. ¹H NMR (300 MHz, DMSO-d6): δ 11.2 (bs, 1H), 8.55 (d, J=5.0Hz, 1H), 8.12 (t, J=4.8Hz, 1H), 7.73 (m, 1H), 7.64 (m, 1H), 7.58 (m, 1H), 7.56 (s, 1H), 7.42 (m, 2H), 7.39 (m, 4H), 7.31 (m, 1H), 7.20 (m, 2H), 7.08 (d, J=9.0Hz, 1H), 6.52 (d, J=6.0Hz, 1H), 4.22 (t, J=6.0Hz, 2H), 3.94 (s, 3H), 3.35 (m, 2H), 2.04 (m, 5H).

### Example 60 [AXL22078]

### N-[4-[[7-[3-[4-[(E)-3-(2,5-dimethoxyphenyl)prop-2-enoyl]piperazin-1-yl]propoxy]-6-methoxy-4-quinolyl]oxy]-3-fluoro-phenyl]-2-(trifluoromethyl)benzenesulfonamide

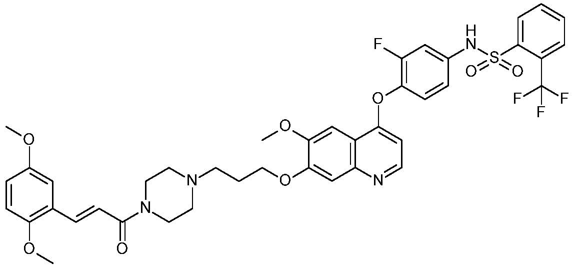

To the solution 3-(2,5-dimethoxy)cinnamic acid (7.90 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 17.35 mg title compound was obtained as a white solid after lyophilization.. Yield: 55%. LCMS Anal. calculated for C₄₁H₄₀F₄N₄O₈S: 824.3, found m/z: 823.2 [M-H]⁻, 825.3 [M+H]⁺, t_{R} = 3.30 min; peak area 100%. ¹H NMR (300 MHz, DMSO-d6): δ 10.8 (bs, 1H), 8.45 (d, J=5.2Hz, 1H), 8.15 (t, J=6.4Hz, 1H), 8.03 (m, 1H), 7.91 (m, 2H), 7.78 (d, J=15.4Hz, 1H), 7.48 (s, 1H), 7.38 (m, 3H), 7.19 (m, 2H), 7.03 (d, J=10.6Hz, 1H), 6.94 (m, 2H), 6.37 (d, J=5.1Hz, 1H), 4.23 (t, J=6.0Hz, 2H), 3.92 (s, 3H), 3.79 (s, 3H), 3.76 (s, 3H), 3.53 (m, 4H), 2.40 (m, 6H), 2.06 (m, 2H).

### Example 61 [AXL22079]

### (E)-N-[3-[[4-[4-[(2,5-difluorophenyl)sulfonylamino]-2-fluoro-phenoxy]-6-methoxy-7-quinolyl]oxy]propyl]-3-(2,5-dimethoxyphenyl)prop-2-enamide

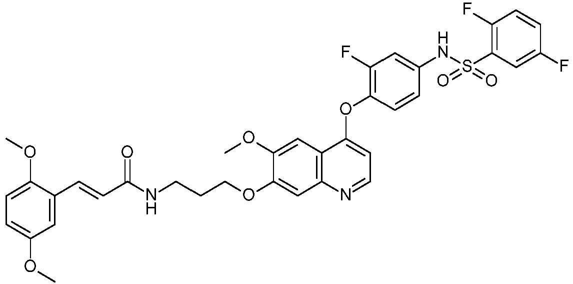

To the solution 3-(2,5-dimethoxy)cinnamic acid (7.91 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 15.90 mg title compound was obtained as a white solid after lyophilization. Yield: 58%. LCMS Anal. calculated for C₃₆H₃₂F₃N₃O₈S: 723.2, found m/z: 722.3 [M-H]⁻, 724.2 [M+H]⁺, t_{R} = 3.72 min; peak area 98%. ¹H NMR (300 MHz, DMSO-d6): δ 11.2 (bs, 1H), 8.51 (d, J=5.2Hz, 1H), 8.15 (m, 1H), 7.77 (m, 1H), 7.60 (m, 3H), 7.52 (s, 1H), 7.42 (m, 2H), 7.21 (m, 1H), 7.07 (m, 2H), 6.96 (m, 2H), 6.65 (d, J=15.9Hz, 1H), 6.45 (d, J=5.4Hz, 1H), 4.23 (t, J=6.0Hz, 2H), 3.94 (s, 3H), 3.79 (s, 3H), 3.73 (s, 3H), 3.40 (m, 2H), 2.02 (m, 2H).

### Example 62 [AXL22080]

### (E)-3-(2-chlorophenyl)-N-[3-[[4-[4-[(2,5-difluorophenyl)sulfonylamino]-2-fluoro-phenoxy]-6-methoxy-7-quinolyl]oxy]propyl]prop-2-enamide

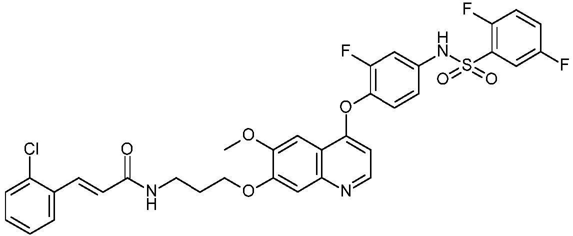

To the solution 2-chlorocinnamic acid (6.93 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 18.67 mg title compound was obtained as a white solid after lyophilization. Yield: 70%. LCMS Anal. calculated for C₃₄H₂₇ClF₃N₃O₆S: 697.1, found m/z: 696.2 [M-H]⁻, 698.2 [M+H]⁺, t_{R} = 3.77 min; peak area 100%. ¹H NMR (300 MHz, DMSO-d6): δ 11.2 (bs, 1H), 8.51 (d, J=5.2Hz, 1H), 8.34 (t, J=5.4Hz, 1H), 7.72 (m, 3H), 7.60 (m, 1H), 7.51 (m, 3H), 7.42 (m, 4H), 7.21 (dd, J=12.2Hz, 2.1Hz, 1H), 7.07 (d, J=7.8Hz, 1H), 6.67 (d, J=15.9Hz, 1H), 6.45 (d, J=5.1Hz, 1H), 4.23 (t, J=6.0Hz, 2H), 3.94 (s, 3H), 3.41 (m, 2H), 2.04 (m, 2H).

### Example 63 [AXL22081]

### (E)-N-[3-[[4-[4-[(2,5-difluorophenyl)sulfonylamino]-2-fluoro-phenoxy]-6-methoxy-7-quinolyl]oxy]propyl]-3-[4-(trifluoromethyl)phenyl]prop-2-enamide

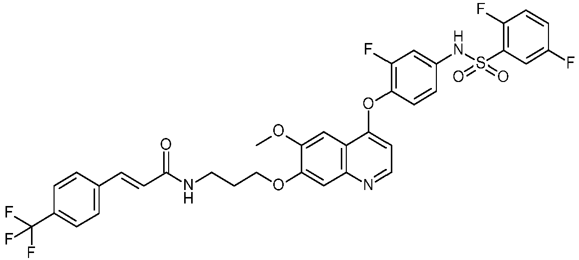

To the solution trans-4-(trifluoromethyl)cinnamic acid (8.20 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 18.49 mg title compound was obtained as a white solid after lyophilization. Yield: 66%. LCMS Anal. calculated for C₃₅H₂₇F₆N₃O₆S: 731.2, found m/z: 730.2 [M-H]⁻, 732.2 [M+H]⁺, t_{R} = 3.90 min; peak area 100%. ¹H NMR (300 MHz, DMSO-d6): δ 11.2 (bs, 1H), 8.50 (d, J=5.2Hz, 1H), 8.26 (t, J=5.5Hz, 1H), 7.89 (m, 2H), 7.73 (m, 2H), 7.62 (m, 2H), 7.54 (m, 3H), 7.41 (m, 2H), 7.20 (dd, J=12.0Hz, 2.0Hz, 1H), 7.07 (d, J=8.8Hz, 1H), 6.75 (d, J=15.6Hz, 1H), 6.43 (d, J=5.1Hz, 1H), 4.23 (t, J=6.0Hz, 2H), 3.94 (s, 3H), 3.41 (m, 2H), 2.04 (m, 2H).

### Example 64 [AXL22082]

### (E)-N-[3-[[4-[4-[(2,5-difluorophenyl)sulfonylamino]-2-fluoro-phenoxy]-6-methoxy-7-quinolyl]oxy]propyl]-4-oxo-4-phenyl-but-2-enamide

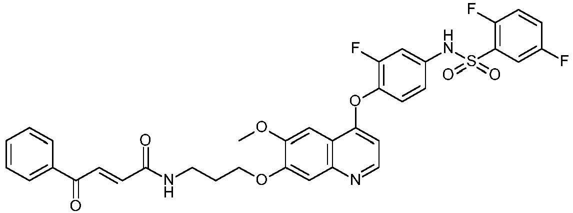

To the solution trans-3-benzoylacrylic acid (6.69 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 14.10 mg title compound was obtained as a white solid after lyophilization. Yield: 53%. LCMS Anal. calculated for C₃₅H₂₈F₃N₃O₇S: 691.2, found m/z: 690.2 [M-H]⁻, 692.2 [M+H]⁺, t_{R} = 3.61 min; peak area 100%. ¹H NMR (300 MHz, DMSO-d6): δ 11.2 (bs, 1H), 8.68 (t, J=5.6Hz, 1H), 8.48 (t, J=5.5Hz, 1H), 8.00 (d, J=8.2Hz, 2H), 7.73 (m, 2H), 7.62 (m, 2H), 7.54 (m, 3H), 7.49 (s, 1H), 7.40 (m, 2H), 7.20 (dd, J=12.2Hz, 2.2Hz, 1H), 7.07 (d, J=6.9Hz, 1H), 6.98 (d, J=15.3Hz, 1H), 6.42 (d, J=5.4Hz, 1H), 4.23 (t, J=6.0Hz, 2H), 3.93 (s, 3H), 3.43 (m, 2H), 2.02 (m, 2H).

### Example 65 [AXL22083]

### (E)-4,4,4-trifluoro-N-[3-[[4-[2-fluoro-4-[[2-(trifluoromethyl)phenyl]sulfonylamino]phenoxy]-6-methoxy-7-quinolyl]oxy]propyl]-3-methyl-but-2-enamide

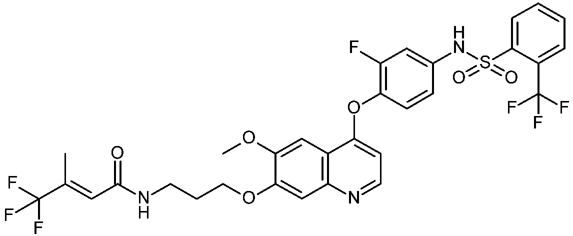

To the solution 3-(trifluoromethyl)crotonic acid (5.85 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 11.12 mg title compound was obtained as a white solid after lyophilization. Yield: 41%. LCMS Anal. calculated for C₃₁H₂₆F₇N₃O₆S: 701.1, found m/z: 700.2 [M-H]⁻, 702.2 [M+H]⁺, t_{R} = 4.18 min; peak area 98%. ¹H NMR (300 MHz, DMSO-d6): δ 11.0 (bs, 1H), 8.51 (s, 1H), 8.49 (s, 1H), 8.16 (m, 1H), 8.04 (d, J=6.9Hz, 1H), 7.89 (m, 2H), 7.51 (s, 1H), 7.40 (m, 2H), 7.17 (dd, J=12.1Hz, 2.3Hz, 1H), 7.04 (d, J=8.8Hz, 1H), 6.45 (m, 2H), 4.20 (t, J=6.0Hz, 2H), 3.93 (s, 3H), 3.50 (m, 2H), 2.16 (s, 3H), 2.01 (m, 2H).

### Example 66 [AXL22088]

### N-[4-[[7-[3-[4-(2-cyanoacetyl)piperazin-1-yl]propoxy]-6-methoxy-4-quinolyl]oxy]-3-fluoro-phenyl]-2,5-difluoro-benzenesulfonamide

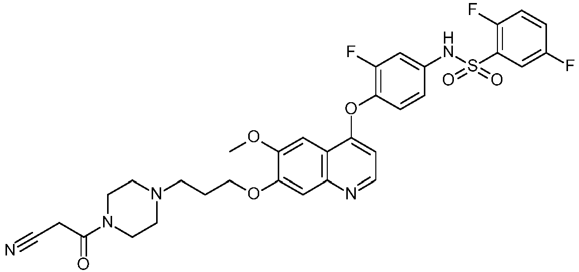

To the solution of cyanoacetic acid (3.23 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 8.46 mg title compound was obtained as a white solid after lyophilization. Yield: 33%. LCMS Anal. calculated for C₃₂H₃₀F₃N₅O₆S: 669.2, found m/z: 668.3 [M-H]⁻, 670.3 [M+H]⁺, t_{R} = 2.99 min; peak area 98%. ¹H NMR (300 MHz, DMSO-d6): δ 11.0 (bs, 1H), 8.42 (d, J=5.2Hz, 1H), 7.56 (m, 1H), 7.48 (s, 1H), 7.38 (m, 3H), 7.15 (t, J=9.1Hz, 1H), 6.98 (d, J=8.8Hz, 1H), 6.82 (d, J=8.0Hz, 1H), 6.35 (d, J=5.2Hz, 1H), 4.20 (t, J=6.0Hz, 2H), 4.02 (s, 2H), 3.93 (s, 3H), 3.53 (bs, 4H), 2.40 (m, 6H), 1.98 (m, 2H).

### Example 67 [AXL22089]

### 2-cyano-N-[3-[[4-[2-fluoro-4-[[2-(trifluoromethyl)phenyl]sulfonylamino]phenoxy]-6-methoxy-7-quinolyl]oxy]propyl]acetamide

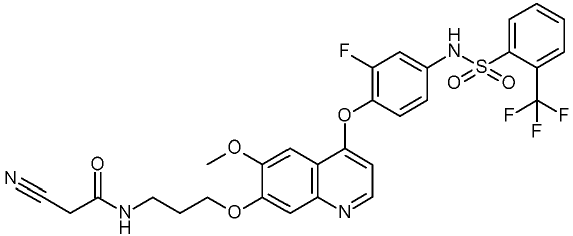

To the solution cyanoacetic acid (3.23 mg 0.038 mmol N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 6.78 mg title compound was obtained as a white solid after lyophilization. Yield: 28%. LCMS Anal. calculated for C₂₉H₂₄F₄N₄O₆S: 632.1, found m/z: 631.2 [M-H]⁻, 633.2 [M+H]⁺, t_{R} = 3.59 min; peak area 98%. ¹H NMR (300 MHz, DMSO-d6): δ 11.0 (bs, 1H), 8.44 (d, J=5.2Hz, 1H), 8.15 (d, J=7.5Hz, 1H), 8.02 (d, J=6.9Hz, 1H), 7.89 (m, 2H), 7.47 (s, 1H), 7.37 (m, 2H), 7.17 (dd, J=12.1Hz, 2.3Hz, 1H), 7.04 (d, J=8.8Hz, 1H), 6.38 (d, J=5.1Hz, 1H), 4.20 (t, J=6.0Hz, 2H), 4.02 (s, 2H), 3.92 (s, 3H), 3.37 (m, 2H), 2.01 (m, 2H).

### Example 68 [AXL22091]

### 2,5-difluoro-N-[3-fluoro-4-[[6-methoxy-7-[3-[4-[(E)-4-(1-piperidyl)but-2-enoyl]piperazin-1-yl]propoxy]-4-quinolyl]oxy]phenyl]benzenesulfonamide

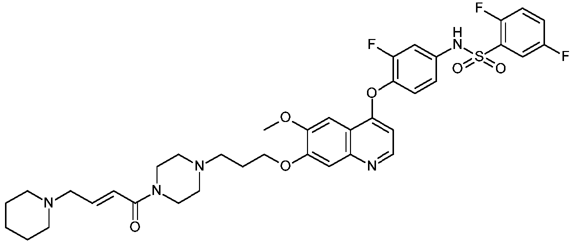

To the solution of (2E)-4-(1-piperidinyl)-2-butenoic acid hydrochloride (7.81 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 6.86 mg title compound was obtained as a white solid after lyophilization. Yield: 24%. LCMS Anal. calculated for C₃₈H₄₂F₃N₅O₆S: 753.3, found m/z: 752.3 [M-H]⁻, 377.7 [M/2+H]⁺, t_{R} = 2.74 min; peak area 98%. ¹H NMR (300 MHz, DMSO-d6): δ 11.2 (bs, 1H), 8.43 (d, J=5.2Hz, 1H), 7.61 (m, 1H), 7.48 (m, 3H), 7.38 (s, 1H), 7.25 (t, J=9.0Hz, 1H), 7.09 (d, J=7.3Hz, 1H), 6.93 (d, J=8.5Hz, 1H), 6.60 (m, 2H), 6.35 (d, J=5.0Hz, 1H), 4.17 (t, J=6.0Hz, 2H), 3.92 (s, 3H), 3.51 (m, 4H), 3.09 (m, 2H), 2.40 (m, 10H), 2.00 (m, 2H), 1.38 (m, 4H), 1.28 (m, 2H).

### Example 69 [AXL22092]

### N-[3-fluoro-4-[[6-methoxy-7-[3-[4-[(E)-4-(1-piperidyl)but-2-enoyl]piperazin-1-yl]propoxy]-4-quinolyl]oxy]phenyl]-2-(trifluoromethyl)benzenesulfonamide

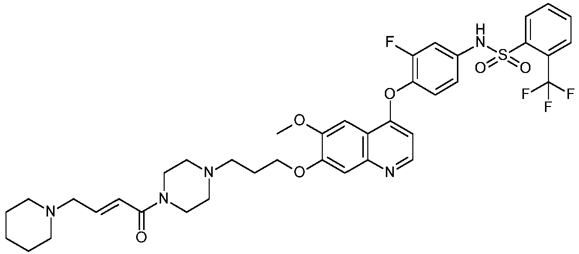

To the solution (2E)-4-(1-piperidinyl)-2-butenoic acid hydrochloride (7.81 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 5.41 mg title compound was obtained as a white solid after lyophilization.. Yield: 18%. LCMS Anal. calculated for C₃₉H₄₃F₄N₅O₆S: 785.3, found m/z: 784.4 [M-H]⁻, 786.5 [M+H]⁺, t_{R} = 2.66 min; peak area 100%. ¹H NMR (300 MHz, DMSO-d6): δ 11.0 (bs, 1H), 8.44 (d, J=5.2Hz, 1H), 8.16 (m, 2H), 7.89 (m, 1H), 7.74 (m, 2H), 7.51 (s, 1H), 7.40 (s, 1H), 7.15 (m, 1H), 7.02 (m, 1H), 6.91 (m, 1H), 6.58 (m, 1H), 6.39 (d, J=5.1Hz, 1H), 4.23 (t, J=6.0Hz, 2H), 3.93 (s, 3H), 3.51 (m, 4H), 3.16 (m, 2H), 2.40 (m, 10H), 2.00 (m, 2H), 1.51 (m, 4H), 1.40 (m, 2H).

### Example 70 [AXL22093]

### (E)-N-[3-[[4-[4-[(2,5-difluorophenyl)sulfonylamino]-2-fluoro-phenoxy]-6-methoxy-7-quinolyl]oxy]propyl]-4-(1-piperidyl)but-2-enamide

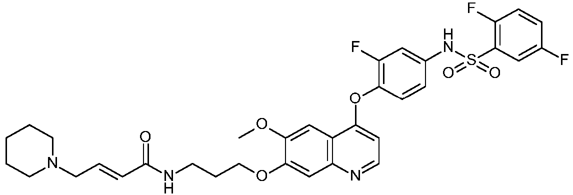

To the solution (2E)-4-(1-piperidinyl)-2-butenoic acid hydrochloride (7.81 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 10.44 mg title compound was obtained as a white solid after lyophilization. Yield: 40%. LCMS Anal. calculated for C₃₄H₃₅F₃N₄O₆S: 684.2 found m/z: 683.3 [M-H]⁻, 685.3 [M+H]⁺, t_{R} = 3.09 min; peak area 99%. ¹H NMR (300 MHz, DMSO-d6): δ 10.8 (bs, 1H), 8.43 (d, J=5.2Hz, 1H), 8.16 (s, 1H), 7.65 (m, 1H), 7.54 (m, 2H), 7.41 (s, 1H), 7.36 (m, 3H), 7.15 (m, 1H), 6.94 (m, 1H), 6.80 (m, 1H), 6.36 (m, 1H), 4.20 (t, J=6.0Hz, 2H), 3.93 (s, 3H), 3.43 (m, 2H), 3.16 (m, 2H), 2.36 (m, 4H), 1.94 (m, 2H), 1.48 (m, 4H), 1.38 (m, 2H).

### Example 71 [AXL22094]

### (E)-N-[3-[[4-[2-fluoro-4-[[2-(trifluoromethyl)phenyl]sulfonylamino]phenoxy]-6-methoxy-7-quinolyl]oxy]propyl]-4-(1-piperidyl)but-2-enamide

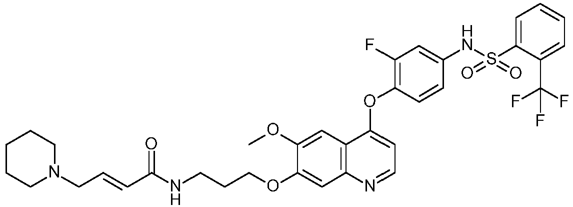

To the solution (2E)-4-(1-piperidinyl)-2-butenoic acid hydrochloride (7.81 mg 0.038 mmol), N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 7.59 mg title compound was obtained as a white solid after lyophilization. Yield: 27%. LCMS Anal. calculated for C₃₅H₃₆F₄N₄O₆S: 716.2, found m/z: 715.3 [M-H]⁻, 717.3 [M+H]⁺, t_{R} = 3.22 min; peak area 99%. ¹H NMR (300 MHz, DMSO-d6): δ 10.8 (bs, 1H), 8.46 (d, J=5.0Hz, 1H), 8.16 (m, 2H), 8.04 (d, J=6.5Hz, 1H), 7.91 (m, 2H), 7.48 (s, 1H), 7.38 (m, 2H), 7.17 (d, J=11.8Hz, 1H), 7.04 (d, J=8.5Hz, 1H), 6.38 (bs, 1H), 4.19 (t, J=6.0Hz, 2H), 3.92 (s, 3H), 3.35 (m, 2H), 3.16 (m, 2H), 2.23 (m, 4H), 1.98 (m, 2H), 1.48 (m, 4H), 1.38 (m, 2H).

### Example 72 [AXL22095]

### N-[4-[[7-[3-[4-(2-cyanoacetyl)piperazin-1-yl]propoxy]-6-methoxy-4-quinolyl]oxy]-3-fluoro-phenyl]-2-(trifluoromethyl)benzenesulfonamide

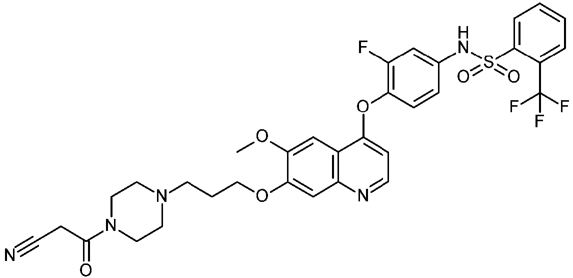

To the solution cyanoacetic acid (3.23 mg 0.038 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 5.33 mg title compound was obtained as a white solid after lyophilization.. Yield: 20%. LCMS Anal. calculated for C₃₃H₃₁F₄N₅O₆S: 701.2, found m/z: 700.3 [M-H]⁻, 702.3 [M+H]⁺, t_{R} = 3.10 min; peak area 100%. ¹H NMR (300 MHz, DMSO-d6): δ 10.6 (bs, 1H), 8.42 (d, J=5.2Hz, 1H), 8.15 (d, J=7.5Hz, 1H), 8.02 (d, J=6.8Hz, 1H), 7.89 (m, 2H), 7.47 (s, 1H), 7.38 (s, 1H), 7.34 (d, J=9.0Hz, 1H), 7.13 (dd, J=12.4Hz, 2.2Hz, 1H), 7.00 (d, J=6.7Hz, 1H), 6.35 (d, J=5.1Hz, 1H), 4.19 (t, J=6.3Hz, 2H), 4.04 (s, 2H), 3.93 (s, 3H), 3.55 (bs, 4H), 2.40 (m, 6H), 1.97 (m, 2H).

### Example 73 [AXL22096]

### 2-cyano-N-[3-[[4-[4-[(2,5-difluorophenyl)sulfonylamino]-2-fluoro-phenoxy]-6-methoxy-7-quinolyl]oxy]propyl]acetamide

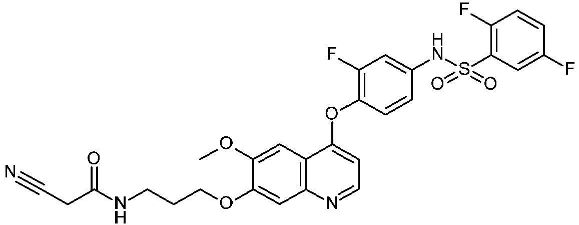

To the solution cyanoacetic acid (3.23 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 6.78 mg title compound was obtained as a white solid after lyophilization.. Yield: 29%. LCMS Anal. calculated for C₂₈H₂₃F₃N₄O₆S: 600.1, found m/z: 599.2 [M-H]⁻, 601.3 [M+H]⁺, t_{R} = 3.26 min; peak area 100%. ¹H NMR (300 MHz, DMSO-d6): δ 10.6 (bs, 1H), 8.43 (d, J=5.2Hz, 1H), 8.16 (s, 1H), 7.65 (m, 1H), 7.54 (m, 2H), 7.41 (s, 1H), 7.36 (m, 3H), 7.15 (m, 1H), 6.94 (m, 1H), 6.80 (m, 1H), 6.36 (m, 1H), ), 4.04 (s, 2H), 3.93 (s, 3H), 3.55 (bs, 4H), 2.40 (m, 6H), 1.97 (m, 2H).

### Example 74 [AXL22097]

### 2,5-difluoro-N-[3-fluoro-4-[[6-methoxy-7-[3-[4-[(E)-4-(4-methylpiperazin-1-yl)but-2-enoyl]piperazin-1-yl]propoxy]-4-quinolyl]oxy]phenyl]benzenesulfonamide

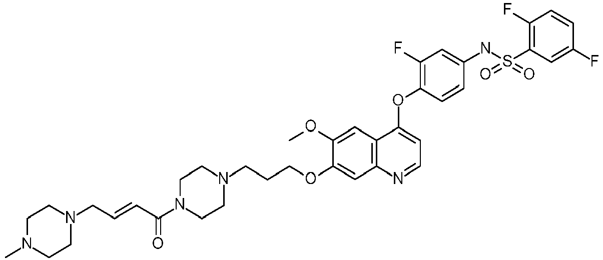

To the solution of (2E)-4-(4-methyl-1-piperazinyl)-2-butenoic acid dihydrochloride (9.75 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 12.62 mg title compound was obtained as a white solid after lyophilization. Yield: 43%. LCMS Anal. calculated for C₃₈H₄₃F₃N₆O₆S: 768.3, found m/z: 767.3 [M-H]⁻, t_{R} = 2.63 min; peak area 100%. ¹H NMR (300 MHz, DMSO-d6): δ 10.8 (bs, 1H), 8.43 (d, J=5.2Hz, 1H), 7.65 (m, 1H), 7.54 (m, 2H), 7.50 (s, 1H), 7.38 (s, 1H), 7.28 (t, J=9.1Hz, 1H), 7.10 (dd, J=12.5Hz, 2.0Hz, 1H), 6.96 (d, J=8.5Hz, 1H), 6.58 (m, 2H), 6.35 (d, J=5.4Hz, 1H), 4.18 (t, J=6.3Hz, 2H), 3.92 (s, 3H), 3.52 (m, 4H), 3.10 (m, 2H), 2.43 (m, 14H), 2.26 (s, 3H), 2.02 (m, 2H).

### Example 75 [AXL22098]

### N-[3-fluoro-4-[[6-methoxy-7-[3-[4-[(E)-4-(4-methylpiperazin-1-yl)but-2-enoyl]piperazin-1-yl]propoxy]-4-quinolyl]oxy]phenyl]-2-(trifluoromethyl)benzenesulfonamide

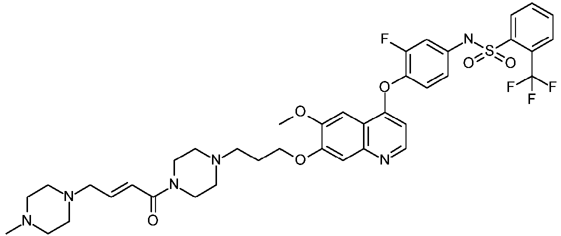

To the solution (2E)-4-(4-methyl-1-piperazinyl)-2-butenoic acid dihydrochloride (9.75 mg 0.038 mmol), (N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 15.01 mg title compound was obtained as a white solid after lyophilization. Yield: 49%. LCMS Anal. calculated for C₃₉H₄₄F₄N₆O₆S: 800.3, found m/z: 799.3 [M-H]⁻, t_{R} = 2.86 min; peak area 100%. ¹H NMR (300 MHz, DMSO-d6): δ 10.8 (bs, 1H), 8.44 (d, J=5.2Hz, 1H), 8.15 (d, J=6.6Hz, 1H), 7.98 (d, J=7.4Hz, 1H), 7.84 (m, 2H), 7.47 (s, 1H), 7.38 (s, 1H), 7.30 (t, J=8.8Hz, 1H), 7.08 (dd, J=12.6Hz, 2.1Hz, 1H), 6.94 (d, J=8.2Hz, 1H), 6.57 (m, 2H), 6.35 (d, J=5.1Hz, 1H), 4.19 (t, J=6.0Hz, 2H), 3.91 (s, 3H), 3.52 (m, 4H), 3.09 (m, 2H), 2.45 (m, 14H), 2.55 (s, 3H), 1.98 (m, 2H).

### Example 76 [AXL22099]

### (E)-N-[3-[[4-[4-[(2,5-difluorophenyl)sulfonylamino]-2-fluoro-phenoxy]-6-methoxy-7-quinolyl]oxy]propyl]-4-(4-methylpiperazin-1-yl)but-2-enamide

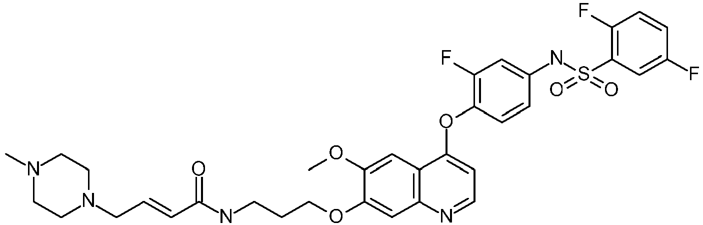

To the solution (2E)-4-(4-methyl-1-piperazinyl)-2-butenoic acid dihydrochloride (9.75 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 8.29 mg title compound was obtained as a white solid after lyophilization. Yield: 31%. LCMS Anal. calculated for C₃₄H₃₆F₃N₅O₆S: 699.2, found m/z: 698.4 [M-H]⁻, 700.3 [M+H]⁺, t_{R} = 2.72 min; peak area 98%. ¹H NMR (300 MHz, DMSO-d6): δ 10.6 (bs, 1H), 8.44 (d, J=5.2Hz, 1H), 8.14 (m, 1H), 7.62 (m, 1H), 7.45 (m, 3H), 7.37 (s, 1H), 7.26 (t, J=9.0Hz, 1H), 7.08 (dd, J=12.3Hz, 2.0Hz, 1H), 6.94 (d, J=8.6Hz, 1H), 6.54 (m, 1H), 6.35 (d, J=4.5Hz, 1H), 6.04 (d, J=7.5Hz, 1H), 4.18 (t, J=6.3Hz, 2H), 3.92 (s, 3H), 3.07 (d, J=4.8Hz, 2H), 2.45 (m, 10H), 2.28 (s, 3H), 1.98 (m, 2H).

### Example 77 [AXL22100]

### (E)-N-[3-[[4-[2-fluoro-4-[[2-(trifluoromethyl)phenyl]sulfonylamino]phenoxy]-6-methoxy-7-quinolyl]oxy]propyl]-4-(4-methylpiperazin-1-yl)but-2-enamide

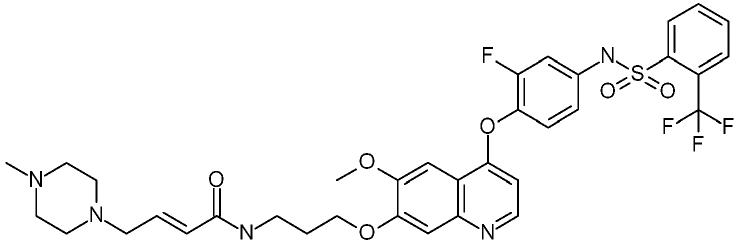

To the solution (2E)-4-(4-methyl-1-piperazinyl)-2-butenoic acid dihydrochloride (9.75 mg 0.038 mmol), N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 8.17 mg title compound was obtained as a white solid after lyophilization. Yield: 29%. LCMS Anal. calculated for C₃₅H₃₇F₄N₅O₆S: 731.2, found m/z: 730.3 [M-H]⁻, 732.3 [M+H]⁺, t_{R} = 2.65 min; peak area 100%. ¹H NMR (300 MHz, DMSO-d6): δ 10.8 (bs, 1H), 8.44 (d, J=5.2Hz, 1H), 8.15 (m, 2H), 7.97 (d, J=7.2Hz, 1H), 7.83 (m, 2H), 7.48 (s, 1H), 7.37 (s, 1H), 7.28 (t, J=9.1Hz, 1H), 7.07 (d, J=8.8Hz, 1H), 6.93 (d, J=8.8Hz, 1H), 6.54 (m, 1H), 6.36 (d, J=5.1Hz, 1H), 6.03 (m, 1H), 4.17 (t, J=6.3Hz, 2H), 3.92 (s, 3H), 3.35 (m, 2H), 3.04 (d, J=6.0Hz, 2H), 2.40 (m, 8H), 2.28 (s, 3H), 1.98 (m, 2H).

### Example 78 [AXL22101]

### N-[4-[[7-[3-[4-[(E)-4-(diethylamino)but-2-enoyl]piperazin-1-yl]propoxy]-6-methoxy-4-quinolyl]oxy]-3-fluoro-phenyl]-2,5-difluoro-benzenesulfonamide

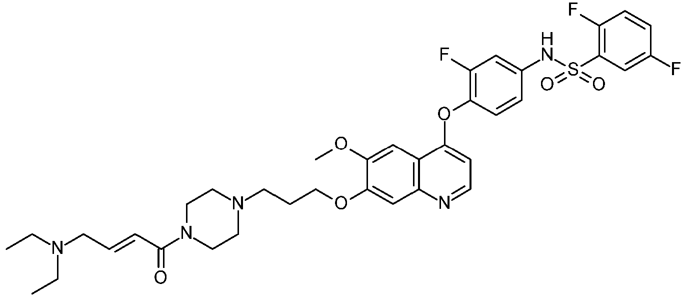

To the solution of trans-4-(diethylamino)crotonic acid hydrochloride (7.35 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 13.04 mg title compound was obtained as a white solid after lyophilization. Yield: 46%. LCMS Anal. calculated for C₃₇H₄₂F₃N₅O₆S: 741.3, found m/z: 740.4 [M-H]⁻, 742.3 [M+H]⁺, t_{R} = 2.23 min; peak area 100%. ¹H NMR (300 MHz, DMSO-d6): δ 10.8 (bs, 1H), 8.43 (d, J=5.2Hz, 1H), 7.61 (m, 1H), 7.48 (m, 3H), 7.38 (s, 1H), 7.27 (t, J=9.1Hz, 1H), 7.08 (dd, J=12.7Hz, 1.9Hz, 1H), 6.94 (d, J=8.4Hz, 1H), 6.69 (m, 1H), 6.61 (m, 1H), 6.35 (d, J=5.2Hz, 1H), 4.19 (t, J=6.1Hz, 2H), 3.92 (s, 3H), 3.53 (m, 4H), 3.14 (m, 2H), 2.65 (q, 4H), 2.40 (m, 6H), 1.98 (t, J=6.0Hz, 2H), 1.02 (t, J=7.2Hz, 6H).

### Example 79 [AXL22102]

### N-[4-[[7-[3-[4-[(E)-4-(diethylamino)but-2-enoyl]piperazin-1-yl]propoxy]-6-methoxy-4-quinolyl]oxy]-3-fluoro-phenyl]-2-(trifluoromethyl)benzenesulfonamide

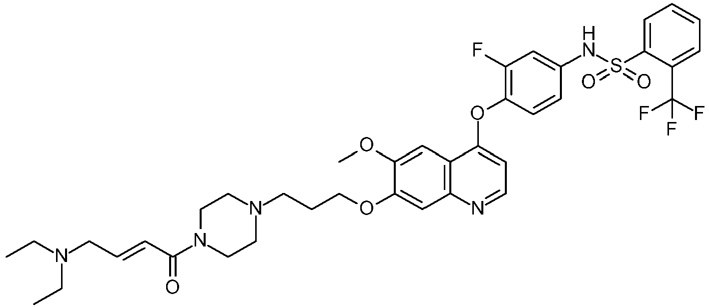

To the solution of trans-4-(diethylamino)crotonic acid hydrochloride (7.35 mg 0.038 mmol), (N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 16.31 mg title compound was obtained as a white solid after lyophilization. Yield: 55%. LCMS Anal. calculated for C₃₈H₄₃F₄N₅O₆S: 773.3, found m/z: 772.4 [M-H]⁻, 774.3 [M+H]⁺, t_{R} = 2.37 min; peak area 100%. ¹H NMR (300 MHz, DMSO-d6): δ 10.6 (bs, 1H), 8.43 (d, J=5.2Hz, 1H), 8.14 (d, J=7.3Hz, 1H), 7.98 (d, J=7.2Hz, 1H), 7.84 (m, 2H), 7.47 (s, 1H), 7.38 (s, 1H), 7.29 (t, J=9.0Hz, 1H), 7.08 (dd, J=12.8Hz, 2.1Hz, 1H), 6.94 (d, J=8.7Hz, 1H), 6.62 (m, 2H), 6.35 (d, J=5.2Hz, 1H), 4.19 (t, J=6.1Hz, 2H), 3.92 (s, 3H), 3.53 (m, 4H), 3.14 (m, 2H), 2.65 (m, 4H), 2.40 (m, 6H), 1.98 (m, 2H), 0.99 (t, J=6.9Hz, 6H).

### Example 80 [AXL22103]

### (E)-4-(diethylamino)-N-[3-[[4-[4-[(2,5-difluorophenyl)sulfonylamino]-2-fluoro-phenoxy]-6-methoxy-7-quinolyl]oxy]propyl]but-2-enamide

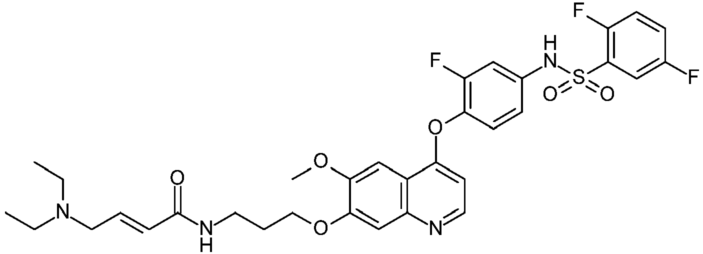

To the solution of trans-4-(diethylamino)crotonic acid hydrochloride (7.35 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 15.98 mg title compound was obtained as a white solid after lyophilization. Yield: 62%. LCMS Anal. calculated for C₃₃H₃₅F₃N₄O₆S: 672.2, found m/z: 671.3 [M-H]⁻, 673.3 [M+H]⁺, t_{R} = 2.74 min; peak area 98%. ¹H NMR (300 MHz, DMSO-d6): δ 10.6 (bs, 1H), 8.43 (d, J=5.2Hz, 1H), 8.12 (m, 1H), 7.61 (m, 1H), 7.48 (m, 3H), 7.38 (s, 1H), 7.27 (t, J=9.1Hz, 1H), 7.08 (dd, J=12.7Hz, 1.9Hz, 1H), 6.94 (d, J=8.4Hz, 1H), 6.60 (m, 1H), 6.35 (d, J=4.9Hz, 1H), 6.10 (d, J=15.3Hz, 1H), 4.19 (t, J=6.1Hz, 2H), 3.92 (s, 3H), 3.23 (m, 2H), 2.90 (m, 2H), 2.58 (q, 4H), 1.98 (t, J=6.0Hz, 2H), 1.00 (t, J=6.9Hz, 6H).

### Example 81 [AXL22104]

### (E)-4-(diethylamino)-N-[3-[[4-[2-fluoro-4-[[2-(trifluoromethyl)phenyl]sulfonylamino]phenoxy]-6-methoxy-7-quinolyl]oxy]propyl]but-2-enamide

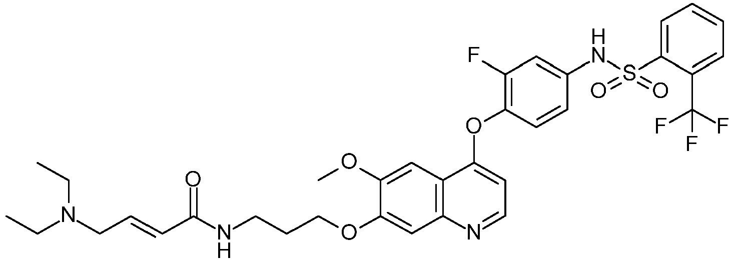

To the solution of trans-4-(diethylamino)crotonic acid hydrochloride (7.35 mg 0.038 mmol), N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 14.35 mg title compound was obtained as a white solid after lyophilization. Yield: 53%. LCMS Anal. calculated for C₃₄H₃₆F₄N₄O₆S: 704.2, found m/z: 703.3 [M-H]⁻, 705.3 [M+H]⁺, t_{R} = 2.64 min; peak area 100%. ¹H NMR (300 MHz, DMSO-d6): δ 10.8 (bs, 1H), 8.44 (d, J=5.2Hz, 1H), 8.13 (m, 2H), 7.99 (d, J=7.6Hz, 1H), 7.85 (m, 2H), 7.48 (s, 1H), 7.38 (s, 1H), 7.30 (t, J=9.0Hz, 1H), 7.10 (dd, J=12.5Hz, 2.0Hz, 1H), 6.96 (d, J=8.3Hz, 1H), 6.58 (m, 1H), 6.36 (d, J=5.1Hz, 1H), 6.08 (d, J=15.5Hz, 1H), 4.19 (t, J=5.7Hz, 2H), 3.92 (s, 3H), 3.28 (m, 2H), 3.25 (m, 2H), 2.58 (q, 4H), 1.98 (t, J=6.0Hz, 2H), 0.99 (t, J=6.9Hz, 6H).

### Example 82 [AXL22105]

### N-[3-fluoro-4-[[6-methoxy-7-[3-[4-[(E)-4-morpholinobut-2-enoyl]piperazin-1-yl]propoxy]-4-quinolyl]oxy]phenyl]-2-(trifluoromethyl)benzenesulfonamide

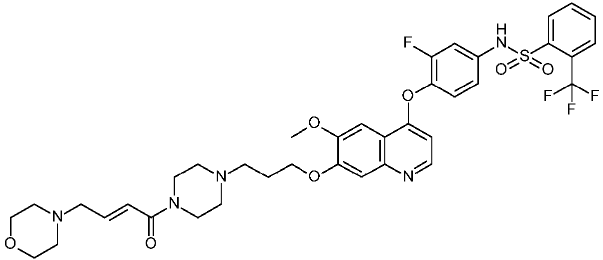

To the solution of (E)-4-(morpholin-4-yl)but-2-enoic acid hydrochloride (7.91 mg 0.038 mmol), (N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 16.49 mg title compound was obtained as a white solid after lyophilization. Yield: 55%. LCMS Anal. calculated for C₃₈H₄₁F₄N₅O₇S: 787.3, found m/z: 786.3 [M-H]⁻, 788.4 [M+H]⁺, t_{R} = 2.25 min; peak area 99%. ¹H NMR (300 MHz, DMSO-d6): δ 10.8 (bs, 1H), 8.44 (d, J=5.2Hz, 1H), 8.15 (d, J=6.6Hz, 1H), 8.01 (d, J=7.1Hz, 1H), 7.88 (m, 2H), 7.47 (s, 1H), 7.38 (s, 1H), 7.36 (m, 1H), 7.15 (dd, J=12.0Hz, 2.1z, 1H), 7.02 (d, J=8.7Hz, 1H), 6.58 (m, 2H), 6.35 (d, J=5.2Hz, 1H), 4.19 (t, J=6.0Hz, 2H), 3.91 (s, 3H), 3.57 (m, 8H), 3.10 (m, 2H), 2.35 (m, 10H), 1.98 (m, 2H).

### Example 83 [AXL22106]

### (E)-N-[3-[[4-[4-[(2,5-difluorophenyl)sulfonylamino]-2-fluoro-phenoxy]-6-methoxy-7-quinolyl]oxy]propyl]-4-morpholino-but-2-enamide

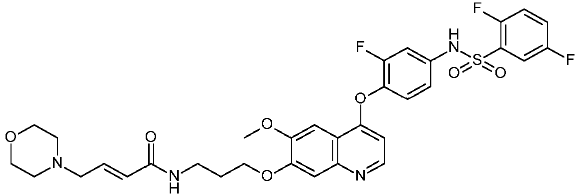

To the solution of (E)-4-(morpholin-4-yl)but-2-enoic acid hydrochloride (7.91 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 10.79 mg title compound was obtained as a white solid after lyophilization. Yield: 41%. LCMS Anal. calculated for C₃₃H₃₃F₃N₄O₇S: 686.2, found m/z: 685.3 [M-H]⁻, 687.3 [M+H]⁺, t_{R} = 2.74 min; peak area 100%. ¹H NMR (300 MHz, DMSO-d6): δ 11.0 (bs, 1H), 8.44 (d, J=5.2Hz, 1H), 8.10 (m, 1H), 7.72 (m, 1H), 7.58 (m, 2H), 7.48 (s, 1H), 7.37 (m, 2H), 7.17 (dd, J=12.0Hz, 2.0Hz, 1H), 7.04 (d, J=8.4Hz, 1H), 6.54 (m, 1H), 6.35 (d, J=5.1Hz, 1H), 6.04 (d, J=15.3Hz, 1H), 4.18 (t, J=6.0Hz, 2H), 3.92 (s, 3H), 3.56 (m, 6H), 3.05 (m, 2H), 2.36 (m, 4H), 1.98 (m, 2H).

### Example 84 [AXL22107]

### (E)-N-[3-[[4-[2-fluoro-4-[[2-(trifluoromethyl)phenyl]sulfonylamino]phenoxy]-6-methoxy-7-quinolyl]oxy]propyl]-4-morpholino-but-2-enamide

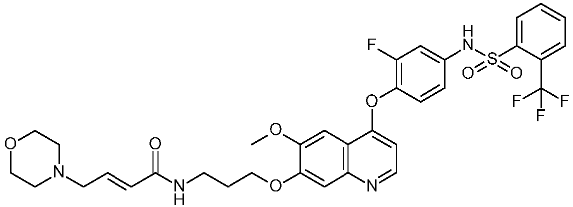

To the solution of (E)-4-(morpholin-4-yl)but-2-enoic acid hydrochloride (7.91 mg 0.038 mmol), N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 13.76 mg title compound was obtained as a white solid after lyophilization. Yield: LCMS Anal. calculated for C₃₄H₃₄F₄N₄O₇S: 718.2, found m/z: 717.3 [M-H]⁻, 719.3 [M+H]⁺, t_{R} = 3.25 min; peak area 100%. ¹H NMR (300 MHz, DMSO-d6): δ 11.0 (bs, 1H), 8.44 (d, J=5.2Hz, 1H), 8.15 (d, J=7.6Hz, 1H), 8.09 (t, J=5.2Hz, 1H), 8.02 (d, J=7.0Hz, 1H), 7.89 (m, 2H), 7.48 (s, 1H), 7.37 (m, 2H), 7.14 (dd, J=12.2Hz, 2.0Hz, 1H), 7.01 (d, J=9.3Hz, 1H), 6.55 (m, 1H), 6.36 (d, J=5.2Hz, 1H), 6.04 (d, J=15.7Hz, 1H), 4.18 (t, J=5.7Hz, 2H), 3.92 (s, 3H), 3.56 (m, 6H), 3.04 (m, 2H), 2.35 (m, 4H), 1.98 (m, 2H).

### Example 85 [AXL22111]

### (Z)-4-[4-[3-[[4-[4-[(2,5-difluorophenyl)sulfonylamino]-2-fluoro-phenoxy]-6-methoxy-7-quinolyl]oxy]propyl]piperazin-1-yl]-4-oxo-but-2-enoic acid

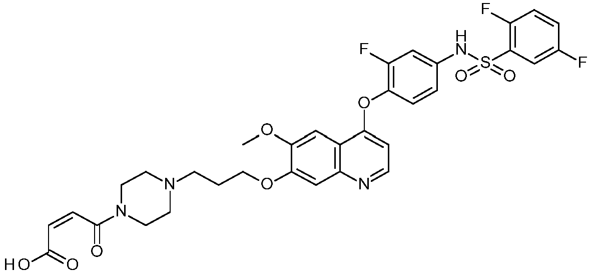

To the solution of maleic anhydride (3.72 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 15.67 mg title compound was obtained as a white solid after lyophilization. Yield: 59%. LCMS Anal. calculated for C₃₃H₃₁F₃N₄O₈S: 700.2, found m/z: 699.3 [M-H]⁻, 701.3 [M+H]⁺, t_{R} = 2.49 min; peak area 100%. ¹H NMR (300 MHz, DMSO-d6): δ 11.0 (bs, 1H), 8.44 (d, J=5.2Hz, 1H), 7.67 (m, 1H), 7.56 (m, 2H), 7.48 (s, 1H), 7.38 (m, 2H), 7.16 (dd, J=12.0Hz, 2.0Hz, 1H), 7.03 (d, J=9.0Hz, 1H), 6.66 (d, J=12.0Hz, 1H), 6.35 (d, J=5.1Hz, 1H), 5.98 (t, J=12.0Hz, 1H), 4.20 (t, J=6.0Hz, 2H), 3.92 (s, 3H), 3.48 (m, 4H), 2.65 (m, 2H), 2.42 (m, 4H), 1.98 (m, 2H).

### Example 86 [AXL22112]

### (Z)-4-[4-[3-[[4-[2-fluoro-4-[[2-(trifluoromethyl)phenyl]sulfonylamino]phenoxy]-6-methoxy-7-quinolyl]oxy]propyl]piperazin-1-yl]-4-oxo-but-2-enoic acid

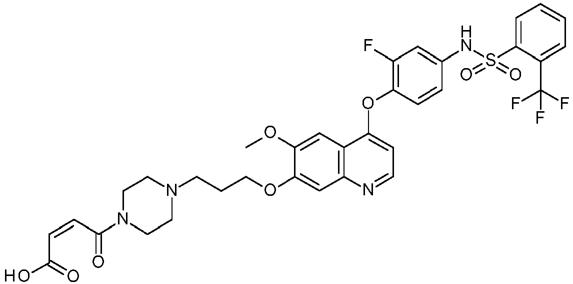

To the solution of maleic anhydride (3.72 mg 0.038 mmol) and (N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 12.65 mg title compound was obtained as a white solid after lyophilization. Yield: 45%. LCMS Anal. calculated for C₃₄H₃₂F₄N₄O₈S: 732.2, found m/z: 731.3 [M-H]⁻, 733.3 [M+H]⁺, t_{R} = 2.80 min; peak area 100%. ¹H NMR (300 MHz, DMSO-d6): δ 11.0 (bs, 1H), 8.44 (d, J=5.2Hz, 1H), 8.15 (d, J=7.8Hz, 1H), 8.02 (d, J=7.8Hz, 1H), 7.89 (m, 2H), 7.46 (s, 1H), 7.37 (m, 2H), 7.15 (d, J=15.4Hz, 1H), 7.01 (d, J=8.5Hz, 1H), 6.66 (d, J=11.9Hz, 1H), 6.36 (d, J=5.1Hz, 1H), 5.97 (t, J=12.0Hz, 1H), 4.20 (t, J=6.0Hz, 2H), 3.92 (s, 3H), 3.48 (m, 4H), 2.60 (m, 2H), 2.42 (m, 4H), 1.98 (m, 2H).

### Example 87 [AXL22113]

### (Z)-4-[3-[[4-[4-[(2,5-difluorophenyl)sulfonylamino]-2-fluoro-phenoxy]-6-methoxy-7-quinolyl]oxy]propylamino]-4-oxo-but-2-enoic acid

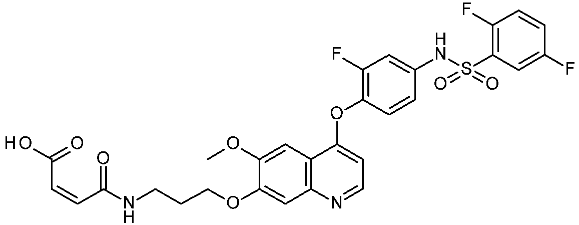

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 9.26 mg title compound was obtained as a white solid after lyophilization. Yield: 38%. LCMS Anal. calculated for C₂₉H₂₄F₃N₃O₈S: 631.2, found m/z: 630.3 [M-H]⁻, 632.3 [M+H]⁺, t_{R} = 3.21 min; peak area 100%. ¹H NMR (300 MHz, DMSO-d6): δ 10.8 (bs, 1H), 9.09 (s, 1H), 8.45 (d, J=5.1Hz, 1H), 7.67 (m, 1H), 7.56 (m, 2H), 7.48 (s, 1H), 7.38 (m, 2H), 7.18 (d, J=12.0Hz, 1H), 7.05 (d, J=8.1Hz, 1H), 6.37 (m, 2H), 6.22 (d, J=12.0Hz, 1H), 4.21 (t, J=6.0Hz, 2H), 3.92 (s, 3H), 3.12 (m, 2H), 2.03 (m, 2H).

### Example 88 [AXL22114]

### (Z)-4-[3-[[4-[2-fluoro-4-[[2-(trifluoromethyl)phenyl]sulfonylamino]phenoxy]-6-methoxy-7-quinolyl]oxy]propylamino]-4-oxo-but-2-enoic acid

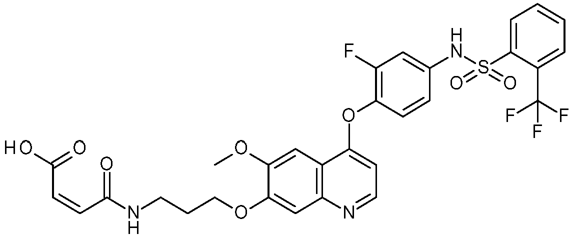

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 11.38 mg title compound was obtained as a white solid after lyophilization. Yield: 45%. LCMS Anal. calculated for C₃₀H₂₅F₄N₃O₈S: 663.2, found m/z: 662.3 [M-H]⁻, 664.3 [M+H]⁺, t_{R} = 3.32 min; peak area 100%. ¹H NMR (300 MHz, DMSO-d6): δ 11.0 (bs, 1H), 9.09 (s, 1H), 8.45 (d, J=5.2Hz, 1H), 8.16 (d, J=7.8Hz, 1H), 8.03 (d, J=6.6Hz, 1H), 7.89 (m, 2H), 7.50 (s, 1H), 7.38 (m, 2H), 7.16 (dd, J=12.1Hz, 2.3Hz, 1H), 7.02 (d, J=8.9Hz, 1H), 6.38 (m, 2H), 6.22 (d, J=12.0Hz, 1H), 4.21 (t, J=6.0Hz, 2H), 3.92 (s, 3H), 3.12 (m, 2H), 2.01 (m, 2H).

### Example 89 [AXL22115]

### 4-[4-[3-[[4-[4-[(2,5-difluorophenyl)sulfonylamino]-2-fluoro-phenoxy]-6-methoxy-7-quinolyl]oxy]propyl]piperazin-1-yl]-4-oxo-butanoic acid

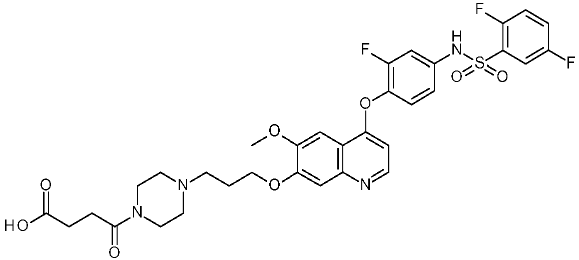

To the solution of succinic anhydride (3.80 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 16.01 mg title compound was obtained as a white solid after lyophilization. Yield: 60%. LCMS Anal. calculated for C₃₃H₃₃F₃N₄O₈S: 702.2, found m/z: 701.3 [M-H]⁻, 703.3 [M+H]⁺, t_{R} = 2.41 min; peak area 100%. ¹H NMR (300 MHz, DMSO-d6): δ 11.6 (bs, 1H), 8.44 (d, J=4.8Hz, 1H), 7.66 (m, 1H), 7.57 (m, 1H), 7.52 (m, 1H), 7.49 (s, 1H), 7.47 (s, 1H), 7.33 (t, J=9.1Hz, 1H), 7.15 (dd, J=12.5Hz, 2.1Hz, 1H), 7.01 (d, J=8.0Hz, 1H), 6.35 (d, J=5.1Hz, 1H), 4.20 (t, J=6.0Hz, 2H), 3.92 (s, 3H), 3.56 (m, 4H), 2.56 (m, 4H), 2.41 (m, 6H), 1.99 (m, 2H).

### Example 90 [AXL22116]

### 4-[4-[3-[[4-[2-fluoro-4-[[2-(trifluoromethyl)phenyl]sulfonylamino]phenoxy]-6-methoxy-7-quinolyl]oxy]propyl]piperazin-1-yl]-4-oxo-butanoic acid

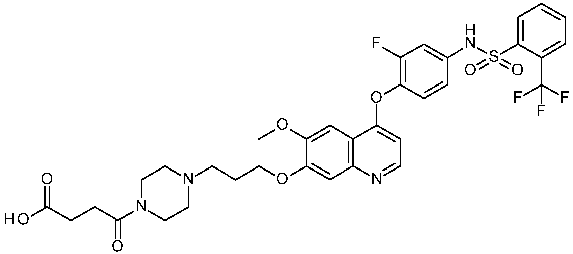

To the solution of succinic anhydride (3.80 mg 0.038 mmol) and (N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 12.53 mg title compound was obtained as a white solid after lyophilization. Yield: 45%. LCMS Anal. calculated for C₃₄H₃₄F₄N₄O₈S: 734.2, found m/z: 733.3 [M-H]⁻, 735.3 [M+H]⁺, t_{R} = 2.77 min; peak area 100%. ¹H NMR (300 MHz, DMSO-d6): δ 11.0 (bs, 1H), 8.44 (d, J=4.7Hz, 1H), 8.15 (d, J=6.0Hz, 1H), 7.99 (d, J=7.3Hz, 1H), 7.86 (m, 2H), 7.47 (s, 1H), 7.38 (s, 1H), 7.32 (t, J=8.0Hz, 1H), 7.10 (d, J=12.3Hz, 1H), 6.97 (d, J=9.2Hz, 1H), 6.35 (d, J=5.1Hz, 1H), 4.20 (t, J=6.0Hz, 2H), 3.92 (s, 3H), 3.56 (m, 4H), 2.56 (m, 4H), 2.40 (m, 6H), 1.98 (m, 2H).

### Example 91 [AXL22117]

### 4-[3-[[4-[4-[(2,5-difluorophenyl)sulfonylamino]-2-fluoro-phenoxy]-6-methoxy-7-quinolyl]oxy]propylamino]-4-oxo-butanoic acid

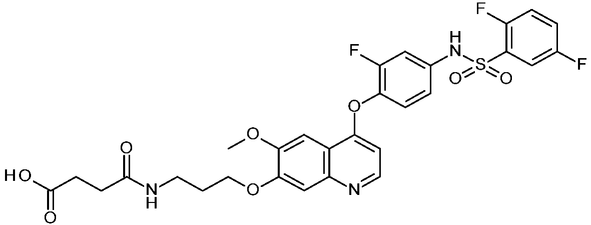

To the solution of succinic anhydride (3.80 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 14.26 mg title compound was obtained as a white solid after lyophilization. Yield: 59%. LCMS Anal. calculated for C₂₉H₂₆F₃N₃O₈S: 633.1, found m/z: 632.3 [M-H]⁻, 634.3 [M+H]⁺, t_{R} = 3.09 min; peak area 100%. ¹H NMR (300 MHz, DMSO-d6): δ 11.1 (bs, 1H), 8.44 (d, J=5.2Hz, 1H), 7.92 (m, 1H), 7.72 (m, 1H), 7.63 (m, 1H), 7.57 (m, 1H), 7.48 (s, 1H), 7.38 (m, 2H), 7.18 (dd, J=12.1Hz, 2.0Hz, 1H), 7.05 (d, J=9.0Hz, 1H), 6.35 (d, J=5.1Hz, 1H), 4.20 (t, J=6.0Hz, 2H), 3.92 (s, 3H), 3.26 (m, 2H), 2.32 (m, 4H), 1.94 (m, 2H).

### Example 92 [AXL22118]

### 4-[3-[[4-[2-fluoro-4-[[2-(trifluoromethyl)phenyl]sulfonylamino]phenoxy]-6-methoxy-7-quinolyl]oxy]propylamino]-4-oxo-butanoic acid

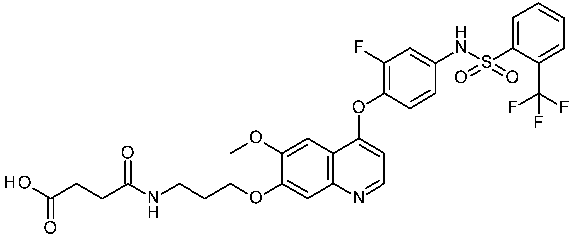

To the solution of succinic anhydride (3.80 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 13.57 mg title compound was obtained as a white solid after lyophilization. Yield: 53%. LCMS Anal. calculated for C₃₀H₂₇F₄N₃O₈S: 665.1, found m/z: 664.3 [M-H]⁻, 666.3 [M+H]⁺, t_{R} = 3.17 min; peak area 100%. ¹H NMR (300 MHz, DMSO-d6): δ 11.4 (bs, 1H), 8.44 (d, J=5.1Hz, 1H), 8.16 (d, J=7.6 Hz, 1H), 8.03 (d, J=6.9Hz, 1H), 7.90 (m, 2H), 7.47 (s, 1H), 7.37 (m, 2H), 7.15 (dd, J=12.1Hz, 2.1Hz, 1H), 7.02 (d, J=8.7Hz, 1H), 6.36 (d, J=5.3Hz, 1H), 4.20 (t, J=6.0Hz, 2H), 3.92 (s, 3H), 3.44 (m, 2H), 2.32 (m, 4H), 1.94 (m, 2H).

### Example 93 [AXL22119]

### 5-[4-[3-[[4-[4-[(2,5-difluorophenyl)sulfonylamino]-2-fluoro-phenoxy]-6-methoxy-7-quinolyl]oxy]propyl]piperazin-1-yl]-5-oxo-pentanoic acid

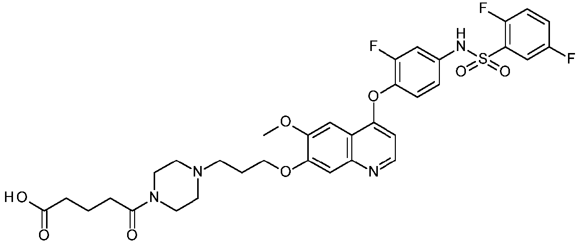

To the solution of glutaric anhydride (4.33 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 12.86 mg title compound was obtained as a white solid after lyophilization. Yield: 47%. LCMS Anal. calculated for C₃₄H₃₅F₃N₄O₈S: 716.2, found m/z: 715.3 [M-H]⁻, 717.3 [M+H]⁺, t_{R} = 2.46 min; peak area 100%. ¹H NMR (300 MHz, DMSO-d6): δ 11.9 (bs, 1H), 8.44 (d, j=5.2Hz, 1H), 7.70 (m, 1H), 7.58 (m, 1H), 7.52 (m, 1H), 7.48 (s, 1H), 7.39 (s, 1H), 7.33 (d, J=9.0Hz, 1H), 7.16 (dd, J=12.2Hz, 2.2Hz, 1H), 7.02 (d, J=8.8Hz, 1H), 6.35 (d, J=5.4Hz, 1H), 4.19 (t, J=6.0Hz, 2H), 3.92 (s, 3H), 3.44 (m, 4H), 3.32 (m, 4H), 2.40 (m, 6H), 2.03 (m, 2H), 1.71 (m, 2H).

### Example 94 [AXL22120]

### 5-[4-[3-[[4-[2-fluoro-4-[[2-(trifluoromethyl)phenyl]sulfonylamino]phenoxy]-6-methoxy-7-quinolyl]oxy]propyl]piperazin-1-yl]-5-oxo-pentanoic acid

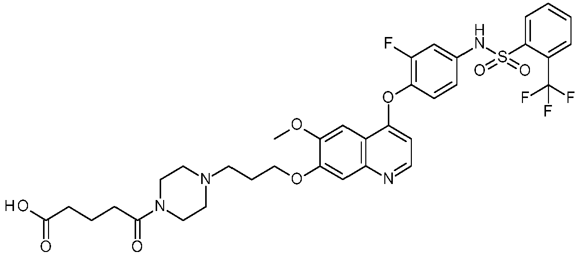

To the solution of glutaric anhydride (4.33 mg 0.038 mmol) and (N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 14.68 mg title compound was obtained as a white solid after lyophilization. Yield: 36%. LCMS Anal. calculated for C₃₅H₃₆F₄N₄O₈S: 748.2, found m/z: 747.3 [M-H]⁻, 749.3 [M+H]⁺, t_{R} = 2.78 min; peak area 100%. ¹H NMR (300 MHz, DMSO-d6): δ 11.6 (bs, 1H), 8.44 (d, J=4.7Hz, 1H), 8.15 (d, J=6.0Hz, 1H), 7.99 (d, J=7.3Hz, 1H), 7.86 (m, 2H), 7.47 (s, 1H), 7.38 (s, 1H), 7.30 (t, J=8.0Hz, 1H), 7.08 (d, J=14.0Hz, 1H), 6.95 (d, J=8.3Hz, 1H), 6.35 (d, J=5.1Hz, 1H), 4.20 (t, J=6.0Hz, 2H), 3.92 (s, 3H), 3.56 (m, 4H), 2.54 (m, 2H), 2.26 (m, 8H), 1.98 (m, 2H), 1.71 (m, 2H).

### Example 95 [AXL22121]

### 5-[3-[[4-[4-[(2,5-difluorophenyl)sulfonylamino]-2-fluoro-phenoxy]-6-methoxy-7-quinolyl]oxy]propylamino]-5-oxo-pentanoic acid

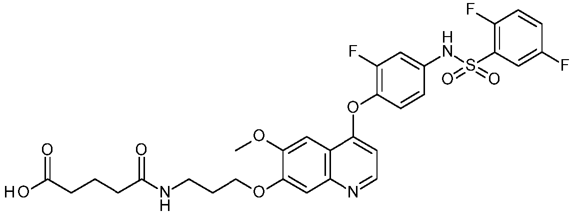

To the solution of glutaric anhydride (4.33 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 12.46 mg title compound was obtained as a white solid after lyophilization. Yield: 50%. LCMS Anal. calculated for C₃₀H₂₈F₃N₃O₈S: 647.1, found m/z: 646.3 [M-H]⁻, 648.3 [M+H]⁺, t_{R} = 3.07 min; peak area 100%. ¹H NMR (300 MHz, DMSO-d6): δ 11.1 (bs, 1H), 8.44 (d, J=5.2Hz, 1H), 7.92 (m, 1H), 7.72 (m, 1H), 7.63 (m, 1H), 7.57 (m, 1H), 7.48 (s, 1H), 7.38 (m, 2H), 7.18 (dd, J=12.1Hz, 2.0Hz, 1H), 7.06 (d, J=9.0Hz, 1H), 6.35 (d, J=5.1Hz, 1H), 4.20 (t, J=6.0Hz, 2H), 3.92 (s, 3H), 3.22 (m, 2H), 2.21 (t, J=7.2Hz, 2H), 2.12 (t, J=7.5Hz, 2H), 1.98 (m, 2H), 1.74 (m, 2H).

### Example 96 [AXL22122]

### 5-[3-[[4-[2-fluoro-4-[[2-(trifluoromethyl)phenyl]sulfonylamino]phenoxy]-6-methoxy-7-quinolyl]oxy]propylamino]-5-oxo-pentanoic acid

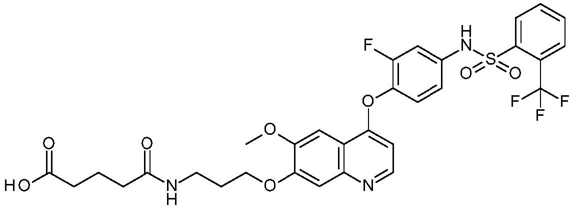

To the solution of glutaric anhydride (4.33 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMSO (1.6 mL) DIPEA (5.41 mg, 0.042 mmol) was added. The mixture was stirred for 3 hours at room temperature. The mixture was purified by reverse phase chromatography. Thus, 12.46 mg title compound was obtained as a white solid after lyophilization. Yield: 48%. LCMS Anal. calculated for C₃₁H₂₉F₄N₃O₈S: 679.1, found m/z: 678.3 [M-H]⁻, 680.3 [M+H]⁺, t_{R} = 3.21 min; peak area 99%. ¹H NMR (300 MHz, DMSO-d6): δ 12.0 (bs, 1H), 8.44 (d, J=5.1Hz, 1H), 8.15 d, J=7.1Hz, 1H), 8.03 (d, J=6.9Hz, 1H), 7.90 (m, 3H), 7.47 (s, 1H), 7.37 (m, 2H), 7.15 (dd, J=12.3Hz, 2.0Hz, 1H), 7.02 (d, J=8.7Hz, 1H), 6.36 (d, J=5.4Hz, 1H), 4.16 (t, J=6.0Hz, 2H), 3.92 (s, 3H), 3.23 (m, 2H), 2.20 (t, J=7.2Hz, 2H), 2.11 (t, J=7.5Hz, 2H), 1.94 (m, 2H), 1,72 (m, 2H).

### Example 97 [AXL22123]

### 2,5-difluoro-N-[3-fluoro-4-[[6-methoxy-7-[3-[4-[(E)-4-(3-methyl-1-piperidyl)but-2-enoyl]piperazin-1-yl]propoxy]-4-quinolyl]oxy]phenyl]benzenesulfonamide

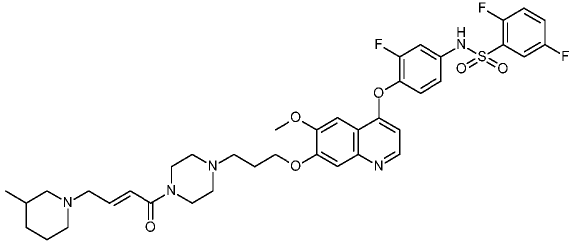

To the solution of (E)-4-(3-methyl-1-piperidyl)but-2-enoic acid hydrochloride (8.35 mg 0.038 mmol), 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 14.59 mg title compound was obtained as a white solid after lyophilization. Yield: 50%. LCMS Anal. calculated for C₃₉H₄₄F₃N₅O₆S: 767.3, found m/z: 766.3 [M-H]⁻, 768.3 [M+H]⁺, t_{R} = 2.49 min; peak area 100%. ¹H NMR (300 MHz, DMSO-d6): δ 11.6 (bs, 1H), 8.43 (d, J=5.2Hz, 1H), 7.66 (m, 1H), 7.58 (m, 1H), 7.52 (m, 1H), 7.48 (s, 1H), 7.39 (s, 1H), 7.30 (t, J=9.0Hz, 1H), 7.12 (dd, J=12.5Hz, 2.0Hz, 1H), 6.98 (d, j=8.7Hz, 1H), 6.60 (m, 2H), 6.35 (d, J=5.1Hz, 1H), 4.20 (t, J=6.0Hz, 2H), 3.92 (s, 3H), 3.53 (m, 4H), 3.19 (m, 2H), 2.80 (m, 2H), 2.40 (m, 6H), 1.98 (m, 3H), 1.73 (m, 2H), 1.61 (m, 2H), 1.47 (m, 2H), 0.83 (d, J=6.0Hz, 3H).

### Example 98 [AXL22124]

### N-[3-fluoro-4-[[6-methoxy-7-[3-[4-[(E)-4-(3-methyl-1-piperidyl)but-2-enoyl]piperazin-1-yl]propoxy]-4-quinolyl]oxy]phenyl]-2-(trifluoromethyl)benzenesulfonamide

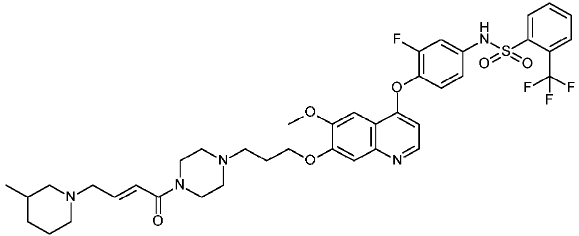

To the solution of (E)-4-(3-methyl-1-piperidyl)but-2-enoic acid hydrochloride (8.35 mg 0.038 mmol), (N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 8.25 mg title compound was obtained as a white solid after lyophilization. Yield: 27%. LCMS Anal. calculated for C₄₀H₄₅F₄N₅O₆S: 799.3, found m/z: 798.4 [M-H]⁻, 800.4 [M+H]⁺, t_{R} = 2.56 min; peak area 100%. ¹H NMR (300 MHz, DMSO-d6): δ 11.0 (bs, 1H), 8.44 (d, J=5.1Hz, 1H), 8.16 (d, J=7.2Hz, 1H), 7.99 (d, J=6.6Hz, 1H), 7.84 (m, 2H), 7.47 (s, 1H), 7.38 (s, 1H), 7.30 (t, J=9.0Hz, 1H), 7.09 (dd, J=12.4Hz, 2.1Hz, 1H), 6.96 (d, J=8.6Hz, 1H), 6.60 (m, 2H), 6.35 (d, J=5.2Hz, 1H), 4.20 (t, J=6.0Hz, 2H), 3.92 (s, 3H), 3.53 (m, 4H), 3.19 (m, 2H), 2.80 (m, 2H), 2.40 (m, 6H), 1.98 (m, 3H), 1.73 (m, 2H), 1.61 (m, 2H), 1.47 (m, 2H), 0.83 (d, J=6.0Hz, 3H).

### Example 99 [AXL22125]

### (E)-N-[3-[[4-[4-[(2,5-difluorophenyl)sulfonylamino]-2-fluoro-phenoxy]-6-methoxy-7-quinolyl]oxy]propyl]-4-(3-methyl-1-piperidyl)but-2-enamide

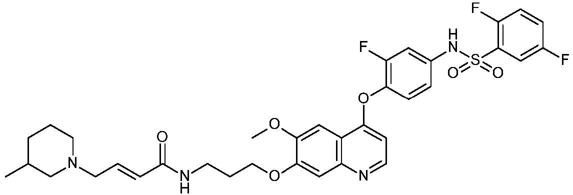

To the solution of (E)-4-(3-methyl-1-piperidyl)but-2-enoic acid hydrochloride (8.35 mg 0.038 mmol), N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 13.57 mg title compound was obtained as a white solid after lyophilization. Yield: 51%. LCMS Anal. calculated for C₃₅H₃₇F₃N₄O₆S: 698.2, found m/z: 697.3 [M-H]⁻, 699.4 [M+H]⁺, t_{R} = 2.83 min; peak area 99%. ¹H NMR (300 MHz, DMSO-d6): δ 10.6 (bs, 1H), 8.42 (d, J=5.1Hz, 1H), 8.11 (m, 1H), 7.65 (m, 1H), 7.48 (m, 3H), 7.37 (s, 1H), 7.28 (t, J=8.9Hz, 1H), 7.10 (dd, J=12.2Hz, 2.0Hz, 1H), 6.95 (d, j=8.9Hz, 1H), 6.75 (m, 1H), 6.35 (d, J=6.2Hz, 1H), 6.04 (d, J=15.0Hz, 1H), 4.18 (t, J=6.0Hz, 2H), 3.92 (s, 3H), 3.11 (m, 4H), 2.78 (m, 2H), 1.96 (m, 3H), 1.62 (m, 4H), 1.48 (m, 2H), 0.82 (m, 3H).

### Example 100 [AXL22126]

### (E)-N-[3-[[4-[2-fluoro-4-[[2-(trifluoromethyl)phenyl]sulfonylamino]phenoxy]-6-methoxy-7-quinolyl]oxy]propyl]-4-(3-methyl-1-piperidyl)but-2-enamide

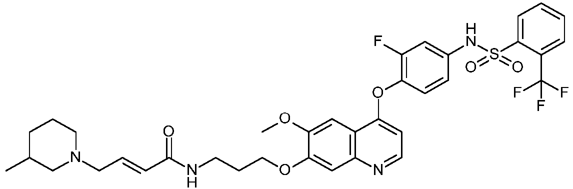

To the solution of (E)-4-(3-methyl-1-piperidyl)but-2-enoic acid hydrochloride (8.35 mg 0.038 mmol), N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) and HBTU (15.96 mg, 0.042 mmol) in DMSO (1.6 mL) DIPEA (5.41 mg, 0.042 mmol) was added. The mixture was stirred for 3 hours at room temperature. The mixture was purified by reverse phase chromatography. Thus, 11.34 mg title compound was obtained as a white solid after lyophilization. Yield: 41%. LCMS Anal. calculated for C₃₆H₃₈F₄N₄O₆S: 730.2, found m/z: 729.4 [M-H]⁻, 731.4 [M+H]⁺, t_{R} = 2.94 min; peak area 100%. ¹H NMR (300 MHz, DMSO-d6): δ 10.8 (bs, 1H), 8.44 (d, J=5.1Hz, 1H), 8.16 (m, 2H), 7.99 (d, J=7.1Hz, 1H), 7.86 (m, 2H), 7.47 (s, 1H), 7.37 (s, 1H), 7.32 (t, J=9.0Hz, 1H), 7.11 (dd, J=12.3Hz, 2.2Hz, 1H), 6.97 (d, J=8.5Hz, 1H), 6.55 (m, 1H), 6.36 (d, J=5.1Hz, 1H), 6.03 (d, J=15.3Hz, 1H), 4.18 (t, J=6.0Hz, 2H), 3.92 (s, 3H), 3.10 (m, 4H), 2.77 (m, 2H), 1.96 (m, 3H), 1.58 (m, 4H), 1.44 (m, 2H), 0.81 (m, 3H).

### Example 101 [AXL22163]

### (Z)-N-[3-[[4-[2-fluoro-4-[[2-(trifluoromethyl)phenyl]sulfonylamino]phenoxy]-6-methoxy-7-quinolyl]oxy]propyl]-N',N'-dimethyl-but-2-enediamide

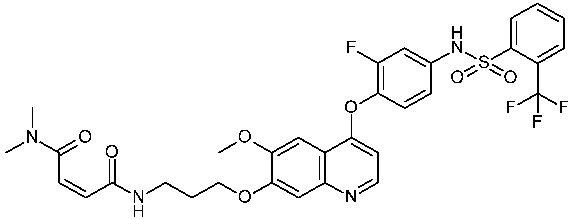

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and dimethylamine hydrochloride (3.76 mg, 0.042 mmol) was added, and the mixture was stirred for further 3 hours at room temperature. The mixture was purified by reverse phase chromatography. Thus, 10.13 mg title compound was obtained as a white solid after lyophilization. Yield: 38%. LCMS Anal. calculated for C₃₂H₃₀F₄N₄O₇S: 690.2, found m/z: 689.4 [M-H]⁻, 691.4 [M+H]⁺, t_{R} = 3.20 min; peak area 100%. ¹H NMR (300 MHz, DMSO-d6): δ 11.0 (bs, 1H), 8.44 (d, J=5.1Hz, 1H), 8.26 (m, 1H), 8.14 (m, 1H), 8.04 (d, J=7.3Hz, 1H), 7.90 (m, 2H), 7.47 (s, 1H), 7.38 (m, 2H), 7.16 (dd, J=12.1Hz, 2.3Hz, 1H), 7.02 (d, J=8.0Hz, 1H), 6.35 (m, 2H), 6.00 (d, J=11.7Hz, 1H), 4.18 (t, J=6.0Hz, 2H), 3.92 (s, 3H), 3.21 (m, 2H), 2.83 (s, 3H), 2.81 (s, 3H), 1.96 (m, 2H).

### Example 102 [AXL22164]

### (Z)-N',N'-diethyl-N-[3-[[4-[2-fluoro-4-[[2-(trifluoromethyl)phenyl]sulfonylamino]phenoxy]-6-methoxy-7-quinolyl]oxy]propyl]but-2-enediamide

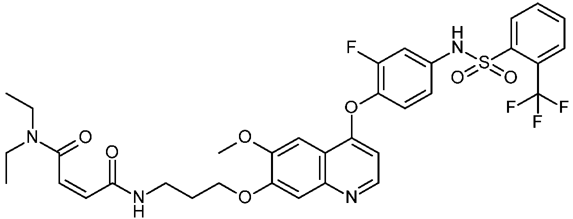

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and diethylamine (3.07 mg, 0.042 mmol) was added, and the mixture was stirred for further 3 hours at room temperature. The mixture was purified by reverse phase chromatography. Thus, 8.33 mg title compound was obtained as a white solid after lyophilization. Yield: 30%. LCMS Anal. calculated for C₃₄H₃₄F₄N₄O₇S: 718.3, found m/z: 717.5 [M-H]⁻, 719.5 [M+H]⁺, t_{R} = 3.34 min; peak area 100%. ¹H NMR (300 MHz, DMSO-d6): δ 11.0 (bs, 1H), 8.47 (d, J=5.1Hz, 1H), 8.22 (t, J=5.4Hz, 1H), 8.14 (d, J=5.1Hz, 1H), 8.04 (d, J=6.7Hz, 1H), 7.91 (m, 2H), 7.47 (s, 1H), 7.38 (m, 2H), 7.18 (dd, J=12.2Hz, 2.1Hz, 1H), 7.03 (d, J=8.7Hz, 1H), 6.35 (m, 2H), 5.97 (d, J=12.0Hz, 1H), 4.18 (t, J=6.0Hz, 2H), 3.92 (s, 3H), 3.20 (m, 6H), 1.98 (m, 2H), 1.07 (m, 6H).

### Example 103 [AXL22165]

### (Z)-N-[3-[[4-[2-fluoro-4-[[2-(trifluoromethyl)phenyl]sulfonylamino]phenoxy]-6-methoxy-7-quinolyl]oxy]propyl]-4-morpholino-4-oxo-but-2-enamide

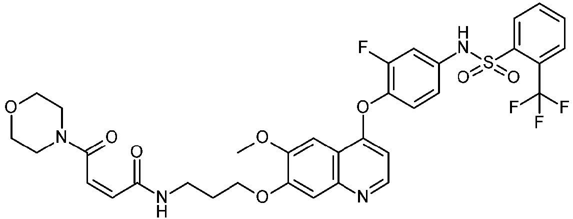

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and morpholine (3.63 mg, 0.042 mmol) was added, and the mixture was stirred for further 3 hours at room temperature. The mixture was purified by reverse phase chromatography. Thus, 5.67 mg title compound was obtained as a white solid after lyophilization. Yield: 20%. LCMS Anal. calculated for C₃₄H₃₂F₄N₄O₈S: 732.2, found m/z: 731.4 [M-H]⁻, 733.4 [M+H]⁺, t_{R} = 3.08 min; peak area 100%. ¹H NMR (300 MHz, DMSO-d6): δ

### Example 104 [AXL22166]

### (Z)-N-[3-[[4-[2-fluoro-4-[[2-(trifluoromethyl)phenyl]sulfonylamino]phenoxy]-6-methoxy-7-quinolyl]oxy]propyl]-4-oxo-4-pyrrolidin-1-yl-but-2-enamide

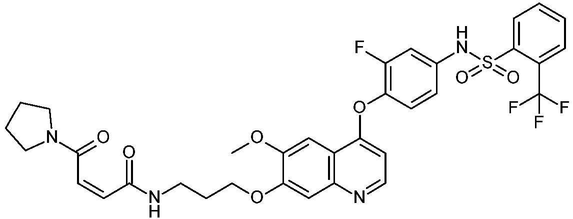

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and pyrrolidine (2.98 mg, 0.042 mmol) was added, and the mixture was stirred for further 3 hours at room temperature. The mixture was purified by reverse phase chromatography. Thus, 6.29 mg title compound was obtained as a white solid after lyophilization. Yield: 23%. LCMS Anal. calculated for C₃₄H₃₂F₄N₄O₇S: 716.2, found m/z: 715.4 [M-H]⁻, 717.4 [M+H]⁺, t_{R} = 3.20 min; peak area 100%. ¹H NMR (300 MHz, DMSO-d6): δ

### Example 105 [AXL22167]

### (Z)-N-[3-[[4-[2-fluoro-4-[[2-(trifluoromethyl)phenyl]sulfonylamino]phenoxy]-6-methoxy-7-quinolyl]oxy]propyl]-4-oxo-4-(1-piperidyl)but-2-enamide

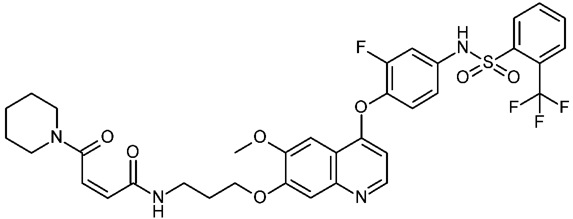

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and piperidine (3.57 mg, 0.042 mmol) was added, and the mixture was stirred for further 3 hours at room temperature. The mixture was purified by reverse phase chromatography. Thus, 6.29 mg title compound was obtained as a white solid after lyophilization. Yield: 22%. LCMS Anal. calculated for C₃₅H₃₄F₄N₄O₇S: 730.3, found m/z: 729.5 [M-H]⁻, 731.5 [M+H]⁺, t_{R} = 3.32 min; peak area 100%. ¹H NMR (300 MHz, DMSO-d6): δ

### Example 106 [AXL22168]

### (Z)-N-[3-[[4-[2-fluoro-4-[[2-(trifluoromethyl)phenyl]sulfonylamino]phenoxy]-6-methoxy-7-quinolyl]oxy]propyl]-4-(3-methyl-1-piperidyl)-4-oxo-but-2-enamide

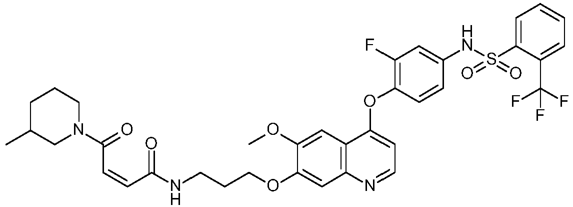

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and 3-methylpiperidine (4.17 mg, 0.042 mmol) was added, and the mixture was stirred for further 3 hours at room temperature. The mixture was purified by reverse phase chromatography. Thus, 7.81 mg title compound was obtained as a white solid after lyophilization. Yield: 27%. LCMS Anal. calculated for C₃₆H₃₆F₄N₄O₇S: 744.2, found m/z: 743.5 [M-H]⁻, 745.5 [M+H]⁺, t_{R} = 3.54 min; peak area 100%. ¹H NMR (300 MHz, DMSO-d6): δ 11.0 (bs, 1H), 8.47 (d, J=5.1Hz, 1H), 8.14 (m, 2H), 8.02 (d, J=8.2Hz, 1H), 7.87 (m, 2H), 7.47 (s, 1H), 7.36 (m, 2H), 7.14 (dd, J=12.0Hz, 2.0Hz, 1H), 7.01 (d, J=9.0Hz, 1H), 6.42 (m, 1H), 6.32 (d, J=5.7Hz, 1H), 5.93 (dd, J=12.0Hz, 2.1Hz, 1H), 4.18 (t, J=6.0Hz, 2H), 3.92 (s, 3H), 3.52 (m, 2H), 3.19 (m, 4H), 1.96 (m, 3H), 1.58 (m, 2H), 1.44 (m, 2H), 0.81 (m, 3H).

### Example 107 [AXL22169]

### (Z)-N-[3-[[4-[2-fluoro-4-[[2-(trifluoromethyl)phenyl]sulfonylamino]phenoxy]-6-methoxy-7-quinolyl]oxy]propyl]-4-(4-methylpiperazin-1-yl)-4-oxo-but-2-enamide

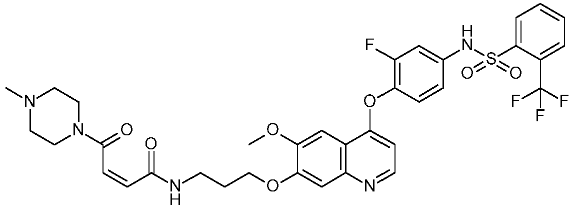

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and 1-methylpiperazine (4.20 mg, 0.042 mmol) was added, and the mixture was stirred for further 3 hours at room temperature. The mixture was purified by reverse phase chromatography. Thus, 6.23 mg title compound was obtained as a white solid after lyophilization. Yield: 22%. LCMS Anal. calculated for C₃₅H₃₅F₄N₅O₇S: 745.2, found m/z: 744.4 [M-H]⁻, 746.4 [M+H]⁺, t_{R} = 2.71 min; peak area 100%. ¹H NMR (300 MHz, DMSO-d6): δ

### Example 108 [AXL22170]

### (Z)-N-[3-[[4-[2-fluoro-4-[[2-(trifluoromethyl)phenyl]sulfonylamino]phenoxy]-6-methoxy-7-quinolyl]oxy]propyl]-4-oxo-4-thiomorpholino-but-2-enamide

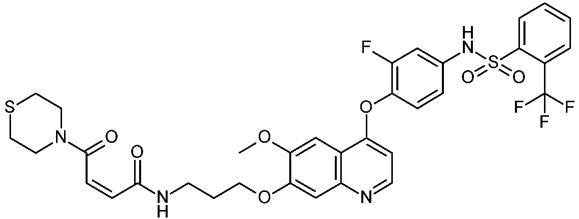

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and thiomorpholine (4.32 mg, 0.042 mmol) was added, and the mixture was stirred for further 3 hours at room temperature. The mixture was purified by reverse phase chromatography. Thus, 10.12 mg title compound was obtained as a white solid after lyophilization. Yield: 35%. LCMS Anal. calculated for C₃₄H₃₂F₄N₄O₇S₂: 748.2, found m/z: 747.4 [M-H]⁻, 749.5 [M+H]⁺, t_{R} = 3.32 min; peak area 100%. ¹H NMR (300 MHz, DMSO-d6): δ 11.1 (bs, 1H), 8.45 (d, J=5.2Hz, 1H), 8.26 (t, J=5.4Hz, 1H), 8.14 (d, J=7.2Hz, 1H), 8.04 (d, J=6.8Hz, 1H), 7.90 (m, 2H), 7.47 (s, 1H), 7.38 (m, 2H), 7.15 (dd, J=11.9Hz, 2.1Hz, 1H), 7.03 (d, J=8.6Hz, 1H), 6.37 (m, 2H), 6.02 (d, J=12.0Hz, 1H), 4.17 (t, J=6.0Hz, 2H), 3.92 (s, 3H), 3.78 (m, 2H), 3.71 (m, 2H), 3.52 (m, 2H), 2.55 (m, 4H), 1.98 (m, 2H).

### Example 109 [AXL22173]

### (Z)-4-[4-(diethylamino)-1-piperidyl]-N-[3-[[4-[2-fluoro-4-[[2-(trifluoromethyl)phenyl]sulfonylamino]phenoxy]-6-methoxy-7-quinolyl]oxy]propyl]-4-oxo-but-2-enamide

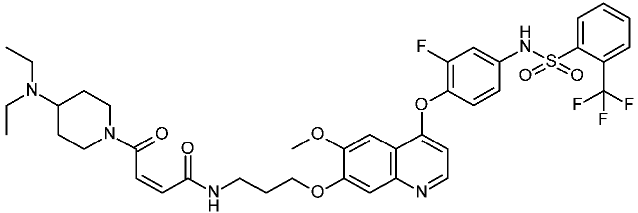

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and 4-(dimethylamino)piperidine (5.38 mg, 0.042 mmol) was added, and the mixture was stirred for further 3 hours at room temperature. The mixture was purified by reverse phase chromatography. Thus, 9.86 mg title compound was obtained as a white solid after lyophilization. Yield: 32%. LCMS Anal. calculated for C₃₉H₄₃F₄N₅O₇S: 801.3, found m/z: 800.5 [M-H]⁻, 802.5 [M+H]⁺, t_{R} = 2.88 min; peak area 100%. ¹H NMR (300 MHz, DMSO-d6): δ 11.1 (bs, 1H), 8.45 (d, J=5.2Hz, 1H), 8.27 (t, J=5.4Hz, 1H), 8.14 (d, J=6.8Hz, 1H), 7.95 (d, J=7.2Hz, 1H), 7.80 (m, 2H), 7.47 (s, 1H), 7.38 (s, 1H), 7.23 (t, J=9.0Hz, 1H), 7.02 (dd, J=12.3Hz, 2.1Hz, 1H), 6.89 (d, J=8.6Hz, 1H), 6.39 (m, 2H), 6.02 (d, J=12.0Hz, 1H), 4.17 (t, J=6.0Hz, 2H), 3.92 (s, 3H), 3.68 (m, 4H), 3.12 (m, 2H), 2.92 (m, 1H), 2.70 (m, 4H), 1.98 (m, 2H), 1.74 (m, 1H), 1.61 (m, 1H), 1.45 (m, 2H), 1.02 (m, 6H).

### Example 110 [AXL22174]

### (Z)-N',N'-diallyl-N-[3-[[4-[2-fluoro-4-[[2-(trifluoromethyl)phenyl]sulfonylamino]phenoxy]-6-methoxy-7-quinolyl]oxy]propyl]but-2-enediamide

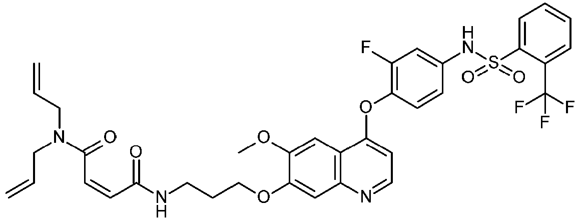

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and diallylamine (4.08 mg, 0.042 mmol) was added, and the mixture was stirred for further 3 hours at room temperature. The mixture was purified by reverse phase chromatography. Thus, 12.67 mg title compound was obtained as a white solid after lyophilization. Yield: 45%. LCMS Anal. calculated for C₃₆H₃₄F₄N₄O₇S: 742.3, found m/z: 741.4 [M-H]⁻, 743.5 [M+H]⁺, t_{R} = 3.38 min; peak area 100%. ¹H NMR (300 MHz, DMSO-d6): δ 11.0 (bs, 1H), 8.44 (d, J=5.2Hz, 1H), 8.26 (t, J=5.1Hz, 1H), 8.14 (d, J=6.8Hz, 1H), 8.04 (d, J=7.0Hz, 1H), 7.91 (m, 2H), 7.49 (s, 1H), 7.39 (m, 2H), 7.17 (dd, J=12.0Hz, 2Hz, 1H), 7.04 (d, J=8.4Hz, 1H), 6.38 (m, 2H), 6.02 (d, J=12.0Hz, 1H), 5.74 (m, 2H), 5.18 (m, 4H), 4.17 (t, J=6.0Hz, 2H), 3.92 (m, 5H), 3.77 (m, 2H), 3.36 (m, 2H), 1.98 (m, 2H).

### Example 111 [AXL22178]

### (Z)-N-[3-[[4-[4-[(2,5-difluorophenyl)sulfonylamino]-2-fluoro-phenoxy]-6-methoxy-7-quinolyl]oxy]propyl]-N',N'-dimethyl-but-2-enediamide

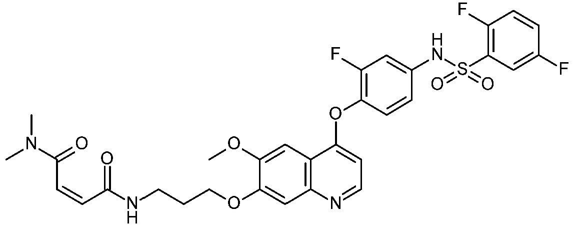

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and dimethylamine hydrochloride (3.76 mg, 0.042 mmol) was added, and the mixture was stirred for further 3 hours at room temperature. The mixture was purified by reverse phase chromatography. Thus, 8.25 mg title compound was obtained as a white solid after lyophilization. Yield: 33%. LCMS Anal. calculated for C₃₁H₂₉F₃N₄O₇S: 658.2, found m/z: 657.4 [M-H]⁻, 659.4 [M+H]⁺, t_{R} = 2.97 min; peak area 100%. ¹H NMR (300 MHz, DMSO-d6): δ

### Example 112 [AXL22179]

### (Z)-N-[3-[[4-[4-[(2,5-difluorophenyl)sulfonylamino]-2-fluoro-phenoxy]-6-methoxy-7-quinolyl]oxy]propyl]-N',N'-diethyl-but-2-enediamide

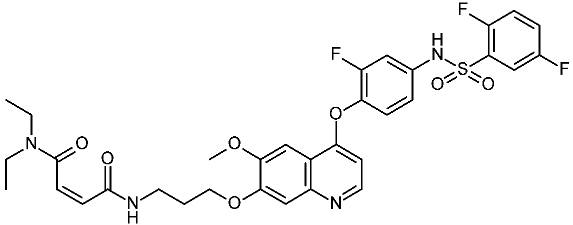

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and diethylamine (3.07 mg, 0.042 mmol) was added, and the mixture was stirred for further 3 hours at room temperature. The mixture was purified by reverse phase chromatography. Thus, 10.53 mg title compound was obtained as a white solid after lyophilization. Yield: 40%. LCMS Anal. calculated for C₃₃H₃₃F₃N₄O₇S: 686.3, found m/z: 685.5 [M-H]⁻, 687.5 [M+H]⁺ , t_{R} = 3.16 min; peak area 98%. ¹H NMR (300 MHz, DMSO-d6): δ 11.0 (bs, 1H), 8.43 (d, J=5.1Hz, 1H), 8.22 (m, 1H), 7.72 (m, 1H), 7.56 (m, 2H), 7.47 (s, 1H), 7.38 (s, 1H), 7.34 (m, 1H), 7.14 (dd, J=12.0Hz, 2.0Hz, 1H), 7.02 (d, J=9.0Hz, 1H), 6.36 (m, 2H), 5.97 (d, J=12.0Hz, 1H), 4.17 (t, J=6.0Hz, 2H), 3.92 (s, 3H), 3.20 (m, 6H), 2.00 (m, 2H), 1.07 (m, 6H).

### Example 113 [AXL22180]

### (Z)-N',N'-diallyl-N-[3-[[4-[4-[(2,5-difluorophenyl)sulfonylamino]-2-fluoro-phenoxy]-6-methoxy-7-quinolyl]oxy]propyl]but-2-enediamide

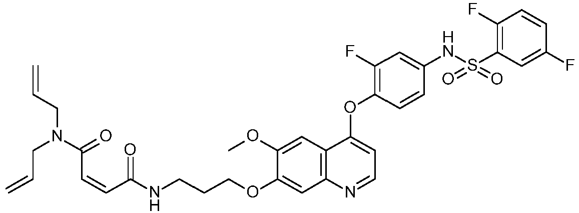

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and diallylamine (4.08 mg, 0.042 mmol) was added, and the mixture was stirred for further 3 hours at room temperature. The mixture was purified by reverse phase chromatography. Thus, 5.87 mg title compound was obtained as a white solid after lyophilization. Yield: 21%. LCMS Anal. calculated for C₃₅H₃₃F₃N₄O₇S: 710.2, found m/z: 709.4 [M-H]⁻, 711.4 [M+H]⁺, t_{R} = 3.32 min; peak area 100%. ¹H NMR (300 MHz, DMSO-d6): δ

### Example 114 [AXL22181]

### (Z)-4-(azetidin-1-yl)-N-[3-[[4-[4-[(2,5-difluorophenyl)sulfonylamino]-2-fluoro-phenoxy]-6-methoxy-7-quinolyl]oxy]propyl]-4-oxo-but-2-enamide

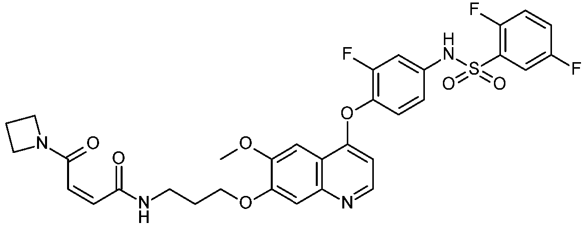

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and azetidine (2.40 mg, 0.042 mmol) was added, and the mixture was stirred for further 3 hours at room temperature. The mixture was purified by reverse phase chromatography. Thus, 5.73 mg title compound was obtained as a white solid after lyophilization. Yield: 22%. LCMS Anal. calculated for C₃₂H₂₉F₃N₄O₇S: 670.2, found m/z: 669.4 [M-H]⁻, 671.4 [M+H]⁺, t_{R} = 3.05 min; peak area 100%. ¹H NMR (300 MHz, DMSO-d6): δ

### Example 115 [AXL22182]

### (Z)-N-[3-[[4-[4-[(2,5-difluorophenyl)sulfonylamino]-2-fluoro-phenoxy]-6-methoxy-7-quinolyl]oxy]propyl]-4-oxo-4-pyrrolidin-1-yl-but-2-enamide

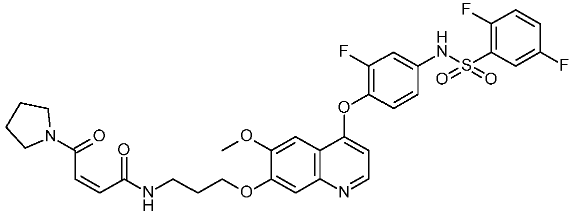

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and pyrrolidine (2.98 mg, 0.042 mmol) was added, and the mixture was stirred for further 3 hours at room temperature. The mixture was purified by reverse phase chromatography. Thus, 5.73 mg title compound was obtained as a white solid after lyophilization. Yield: 22%. LCMS Anal. calculated for C₃₃H₃₁F₃N₄O₇S: 684.2, found m/z: 683.4 [M-H]⁻, 685.4 [M+H]⁺, t_{R} = 3.07 min; peak area 99%. ¹H NMR (300 MHz, DMSO-d6): δ

### Example 116 [AXL22183]

### (Z)-N-[3-[[4-[4-[(2,5-difluorophenyl)sulfonylamino]-2-fluoro-phenoxy]-6-methoxy-7-quinolyl]oxy]propyl]-4-oxo-4-(1-piperidyl)but-2-enamide

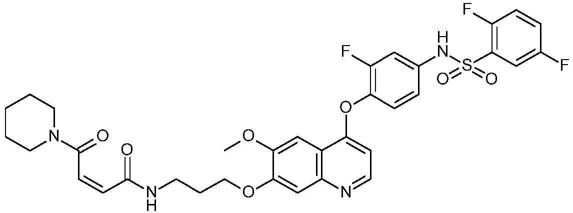

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and piperidine (2.98 mg, 0.042 mmol) was added, and the mixture was stirred for further 3 hours at room temperature. The mixture was purified by reverse phase chromatography. Thus, 5.92 mg title compound was obtained as a white solid after lyophilization. Yield: 22%. LCMS Anal. calculated for C₃₄H₃₃F₃N₄O₇S: 698.2, found m/z: 697.5 [M-H]⁻, 699.5 [M+H]⁺, t_{R} = 3.14 min; peak area 100%. ¹H NMR (300 MHz, DMSO-d6): δ

### Example 117 [AXL22184]

### (Z)-N-[3-[[4-[4-[(2,5-difluorophenyl)sulfonylamino]-2-fluoro-phenoxy]-6-methoxy-7-quinolyl]oxy]propyl]-4-morpholino-4-oxo-but-2-enamide

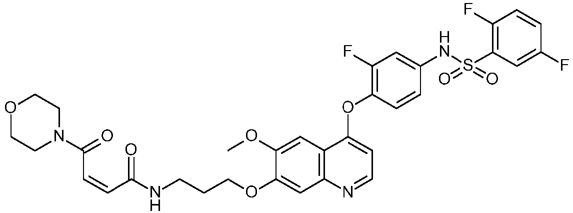

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and morpholine (3.65 mg, 0.042 mmol) was added, and the mixture was stirred for further 3 hours at room temperature. The mixture was purified by reverse phase chromatography. Thus, 6.80 mg title compound was obtained as a white solid after lyophilization. Yield: 25%. LCMS Anal. calculated for C₃₃H₃₁F₃N₄O₈S: 700.2, found m/z: 699.4 [M-H]⁻, 701.4 [M+H]⁺, t_{R} = 3.01 min; peak area 100%. ¹H NMR (300 MHz, DMSO-d6): δ 11.1 (bs, 1H), 8.43 (d, J=5.1Hz, 1H), 8.27 (m, 1H), 7.68 (m, 1H), 7.50 (m, 2H), 7.47 (s, 1H), 7.38 (s, 1H), 7.34 (m, 1H), 7.14 (dd, J=12.0Hz, 2.0Hz, 1H), 7.02 (d, J=9.0Hz, 1H), 6.36 (m, 2H), 5.97 (d, J=12.0Hz, 1H), ), 4.19 (t, J=6.0Hz, 2H), 3.93 (s, 3H), 3.54 (m, 8H), 3.50 (m, 2H), 2.00 (m, 2H).

### Example 118 [AXL22185]

### (Z)-N-[3-[[4-[4-[(2,5-difluorophenyl)sulfonylamino]-2-fluoro-phenoxy]-6-methoxy-7-quinolyl]oxy]propyl]-4-(4-methylpiperazin-1-yl)-4-oxo-but-2-enamide

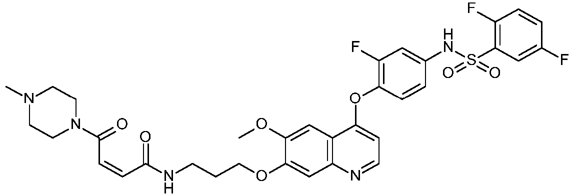

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and 1-methylpiperazine (4.20 mg, 0.042 mmol) was added, and the mixture was stirred for further 3 hours at room temperature. The mixture was purified by reverse phase chromatography. Thus, 8.89 mg title compound was obtained as a white solid after lyophilization. Yield: 33%. LCMS Anal. calculated for C₃₄H₃₄F₃N₅O₇S: 713.2, found m/z: 712.3 [M-H]⁻, 714.3 [M+H]⁺, t_{R} = 2.56 min; peak area 100%. ¹H NMR (300 MHz, DMSO-d6): δ

### Example 119 [AXL22186]

### (Z)-4-(azetidin-1-yl)-N-[3-[[4-[2-fluoro-4-[[2-(trifluoromethyl)phenyl]sulfonylamino]phenoxy]-6-methoxy-7-quinolyl]oxy]propyl]-4-oxo-but-2-enamide

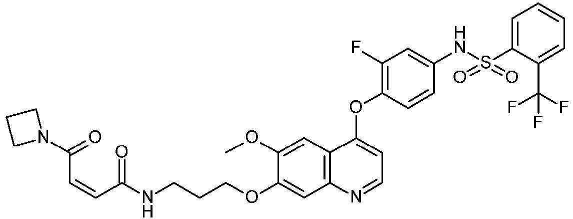

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and azetidin (2.39 mg, 0.042 mmol) was added, and the mixture was stirred for further 3 hours at room temperature. The mixture was purified by reverse phase chromatography. Thus, 1.15 mg title compound was obtained as a white solid after lyophilization. Yield: 4%. LCMS Anal. calculated for C₃₃H₃₀F₄N₄O₇S: 702.2, found m/z: 701.4 [M-H]⁻, 703.4 [M+H]⁺, t_{R} = 3.13 min; peak area 100%. ¹H NMR (300 MHz, DMSO-d6): δ

### Example 120 [AXL22187]

### (Z)-4-(4-allylpiperazin-1-yl)-N-[3-[[4-[2-fluoro-4-[[2-(trifluoromethyl)phenyl]sulfonylamino]phenoxy]-6-methoxy-7-quinolyl]oxy]propyl]-4-oxo-but-2-enamide

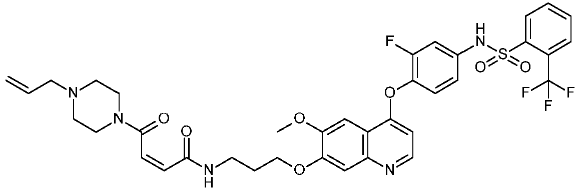

To the solution of maleic anhydride (3.72 mg 0.038 mmol) and N-[4-[[7-(3-aminopropoxy)-6-methoxy-4-quinolyl]oxy]-3-fluoro-phenyl]-2-(trifluoromethyl)benzenesulfonamide (I7A2, 21.49 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and 1-allylpiperazine (5.29 mg, 0.042 mmol) was added, and the mixture was stirred for further 3 hours at room temperature. The mixture was purified by reverse phase chromatography. Thus, 14.73 mg title compound was obtained as a white solid after lyophilization. Yield: 50%. LCMS Anal. calculated for C₃₇H₃₇F₄N₅O₇S: 771.2, found m/z: 770.5 [M-H]⁻, 772.5 [M+H]⁺, t_{R} = 2.75 min; peak area 100%. ¹H NMR (300 MHz, DMSO-d6): δ 11.0 (bs, 1H), 8.44 (d, J=5.0Hz, 1H), 8.23 (m, 1H), 8.15 (m, 1H), 8.01 (d, J=8.9Hz, 1H), 7.90 (m, 2H), 7.48 (s, 1H), 7.38 (s, 1H), 7.32 (m, 1H), 7.13 (d, J=12.3Hz, 1H), 7.00 (d, J=8.9Hz, 1H), 6.33 (m, 2H), 6.01 (d, J=12.0Hz, 1H), 5.76 (m, 1H), 5.17 (m, 1H), 5.08 (m, 1H), 4.18 (t, J=6.0Hz, 2H), 3.92 (s, 3H), 3.52 (m, 4H), 3.10 (m, 2H), 2.93 (m, 2H), 2.29 (m, 4H), 1.98 (m, 2H).

### Example 121 [AXL22202]

### 4-[4-[3-[[4-[4-[(2,5-difluorophenyl)sulfonylamino]-2-fluoro-phenoxy]-6-methoxy-7-quinolyl]oxy]propyl]piperazin-1-yl]-N,N-diethyl-4-oxo-butanamide

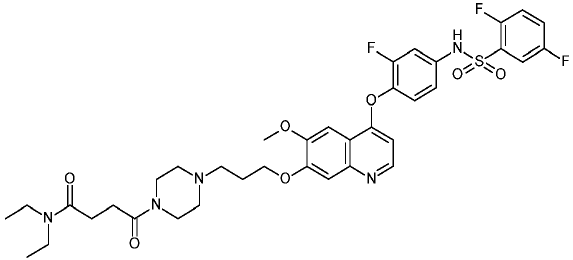

To the solution of succinic anhydride (3.80 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and diethylamine (3.07 mg, 0.042 mmol) was added, and the mixture was stirred for further 3 hours at room temperature. The mixture was purified by reverse phase chromatography. Thus 15.64 mg title compound was obtained as a white solid after lyophilization. Yield: 54%. LCMS Anal. calculated for C₃₇H₄₂F₃N₅O₇S: 757.3, found m/z: 756.6 [M-H]⁻, 758.5 [M+H]⁺, t_{R} = 2.72 min; peak area 100%. ¹H NMR (300 MHz, DMSO-d6): δ 10.8 (bs, 1H), 8.44 (d, J=5.2Hz, 1H), 7.72 (m, 1H), 7.62 (m, 1H), 7.52 (m, 1H), 7.49 (s, 1H), 7.38 (m, 2H), 7.18 (dd, J=12.1Hz, 2.1Hz, 1H), 7.05 (d, J=9.0Hz, 1H), 6.35 (d, J=5.1Hz, 1H), 4.20 (t, J=6.0Hz, 2H), 3.92 (s, 3H), 3.56 (m, 4H), 3.32 (m, 4H), 2.56 (m, 6H), 2.42 (m, 4H), 2.03 (m, 2H), 1.12 (t, J=6.9Hz, 3H), 0.98 (t, J=6.9Hz, 3H).

### Example 122 [AXL22203]

### N-[4-[[7-[3-[4-[5-(azetidin-1-yl)-5-oxo-pentanoyl]piperazin-1-yl]propoxy]-6-methoxy-4-quinolyl]oxy]-3-fluoro-phenyl]-2,5-difluoro-benzenesulfonamide

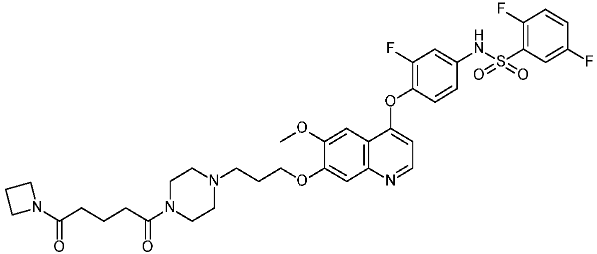

To the solution of glutaric anhydride (4.33 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and azetidin (2.39 mg, 0.042 mmol) was added, and the mixture was stirred for further 3 hours at room temperature. The mixture was purified by reverse phase chromatography. Thus, 6.55 mg title compound was obtained as a white solid after lyophilization. Yield: 22%. LCMS Anal. calculated for C₃₇H₄₀F₃N₅O₇S: 755.3, found m/z: 754.5 [M-H]⁻, 756.5 [M+H]⁺, t_{R} = 2.58 min; peak area 100%. ¹H NMR (300 MHz, DMSO-d6): δ 10.7 (bs, 1H), 8.44 (d, J=5.2Hz, 1H), 7.72 (m, 1H), 7.62 (m, 1H), 7.52 (m, 1H), 7.49 (s, 1H), 7.38 (m, 2H), 7.18 (dd, J=12.1Hz, 2.1Hz, 1H), 7.05 (d, J=9.0Hz, 1H), 6.35 (d, J=5.1Hz, 1H), 4.20 (t, J=6.0Hz, 2H), 4.05 (t, J=7.8Hz, 2H), 3.92 (s, 3H), 3.81 (t, J=7.5m, 2H), 3.46 (m, 4H), 2.54 (m, 8H), 2.30 (m, 2H), 2.16 (m, 2H), 2.00 (m, 2H), 1.69 (m, 2H).

### Example 123 [AXL22204]

### 2,5-difluoro-N-[3-fluoro-4-[[6-methoxy-7-[3-[4-(5-morpholino-5-oxo-pentanoyl)piperazin-1-yl]propoxy]-4-quinolyl]oxy]phenyl]benzenesulfonamide

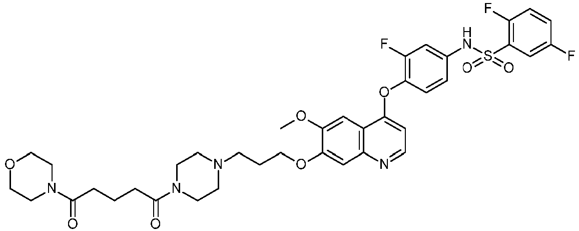

To the solution of glutaric anhydride (4.33 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and morpholine (3.65 mg, 0.042 mmol) was added, and the mixture was stirred for further 3 hours at room temperature. The mixture was purified by reverse phase chromatography. Thus, 9.78 mg title compound was obtained as a white solid after lyophilization. Yield: 32%. LCMS Anal. calculated for C₃₈H₄₂F₃N₅O₈S: 785.3, found m/z: 784.6 [M-H]⁻, 786.6 [M+H]⁺, t_{R} = 2.62 min; peak area 100%. ¹H NMR (300 MHz, DMSO-d6): δ 10.7 (bs, 1H), 8.44 (d, J=5.2Hz, 1H), 7.72 (m, 1H), 7.62 (m, 1H), 7.52 (m, 1H), 7.48 (s, 1H), 7.38 (m, 2H), 7.18 (dd, J=12.1Hz, 2.1Hz, 1H), 7.05 (d, J=9.0Hz, 1H), 6.35 (d, J=5.1Hz, 1H), 4.20 (t, J=6.0Hz, 2H), 3.92 (s, 3H), 3.43 (m, 12H), 2.60 (m, 2H), 2.40 (m, 8H), 2.00 (m, 2H), 1.75 (m, 2H).

### Example 124 [AXL22205]

### 2,5-difluoro-N-[3-fluoro-4-[[6-methoxy-7-[3-[4-[5-(4-methylpiperazin-1-yl)-5-oxo-pentanoyl]piperazin-1-yl]propoxy]-4-quinolyl]oxy]phenyl]benzenesulfonamide

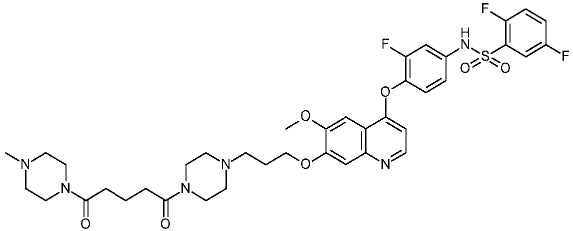

To the solution of glutaric anhydride (4.33 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and 1-methylpiperazine (4.20 mg, 0.042 mmol) was added, and the mixture was stirred for further 3 hours at room temperature. The mixture was purified by reverse phase chromatography. Thus, 11.64 mg title compound was obtained as a white solid after lyophilization. Yield: 38%. LCMS Anal. calculated for C₃₉H₄₅F₃N₆O₇S: 798.4, found m/z: 797.6 [M-H]⁻, 799.6 [M+H]⁺, t_{R} = 2.32 min; peak area 100%. ¹H NMR (300 MHz, DMSO-d6): δ 10.7 (bs, 1H), 8.44 (d, J=5.2Hz, 1H), 7.72 (m, 1H), 7.62 (m, 1H), 7.52 (m, 1H), 7.48 (s, 1H), 7.38 (m, 2H), 7.18 (dd, J=12.1Hz, 2.1Hz, 1H), 7.05 (d, J=9.0Hz, 1H), 6.35 (d, J=5.1Hz, 1H), 4.20 (t, J=6.0Hz, 2H), 3.92 (s, 3H), 3.44 (m, 8H), 2.32 (m, 14H), 2.24 (s, 3H), 1.99 (m, 2H), 1.69 (m, 2H).

### Example 125 [AXL22206]

### 2,5-difluoro-N-[3-fluoro-4-[[6-methoxy-7-[3-[4-[5-(3-methyl-1-piperidyl)-5-oxo-pentanoyl]piperazin-1-yl]propoxy]-4-quinolyl]oxy]phenyl]benzenesulfonamide

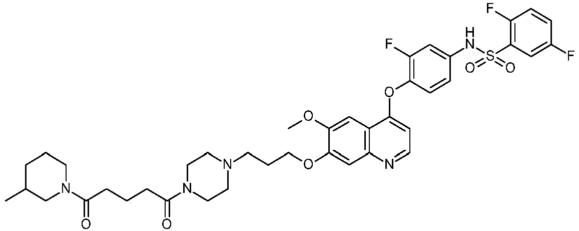

To the solution of glutaric anhydride (4.33 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMF (1.6 mL) DIPEA (10.82 mg, 0.084 mmol) was added. The mixture was stirred for 3 hours at room temperature. To the reaction mixture HBTU (15.96 mg, 0.042 mmol) and 3-methylpiperidine (4.16 mg, 0.042 mmol) was added, and the mixture was stirred for further 3 hours at room temperature. The mixture was purified by reverse phase chromatography. Thus, 9.13 mg title compound was obtained as a white solid after lyophilization. Yield: 30%. LCMS Anal. calculated for C₄₀H₄₆F₃N₅O₇S: 797.4, found m/z: 796.6 [M-H]⁻, 798.6 [M+H]+, t_{R} = 2.88 min; peak area 100%. ¹H NMR (300 MHz, DMSO-d6): δ 10.8 (bs, 1H), 8.44 (d, J=5.2Hz, 1H), 7.72 (m, 1H), 7.62 (m, 1H), 7.52 (m, 1H), 7.48 (s, 1H), 7.38 (m, 2H), 7.18 (dd, J=12.1Hz, 2.1Hz, 1H), 7.05 (d, J=9.0Hz, 1H), 6.35 (d, J=5.1Hz, 1H), 4.20 (t, J=6.0Hz, 2H), 3.92 (s, 3H), 3.71 (m, 2H), 3.44 (m, 6H), 2.32 (m, 10H), 2.00 (m, 2H), 1.71 (m, 3H), 1.38 (m, 2H), 1.14 (m, 2H), 0.88 (m, 3H).

### Example 126 [AXL22207]

### 4-[4-[3-[[4-[4-[(2,5-difluorophenyl)sulfonylamino]-2-fluoro-phenoxy]-6-methoxy-7-quinolyl]oxy]propyl]piperazin-1-yl]-N,N-dimethyl-4-oxo-butanamide

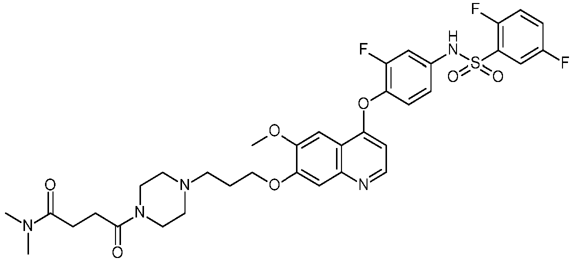

To the solution of succinic anhydride (3.80 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and dimethylamine hydrochloride (3.42 mg, 0.042 mmol) was added, and the mixture was stirred for further 3 hours at room temperature. The mixture was purified by reverse phase chromatography. Thus, 12.32 mg title compound was obtained as a white solid after lyophilization. Yield: 44%. LCMS Anal. calculated for C₃₅H₃₈F₃N₅O₇S: 729.4, found m/z: 728.4 [M-H]⁻, 730.4 [M+H]⁺, t_{R} = 2.59 min; peak area 100%. ¹H NMR (300 MHz, DMSO-d6): δ 10.8 (bs, 1H), 8.44 (d, J=5.2Hz, 1H), 7.72 (m, 1H), 7.62 (m, 1H), 7.52 (m, 1H), 7.48 (s, 1H), 7.38 (m, 2H), 7.18 (dd, J=12.1Hz, 2.1Hz, 1H), 7.05 (d, J=9.0Hz, 1H), 6.35 (d, J=5.1Hz, 1H), 4.20 (t, J=6.0Hz, 2H), 3.92 (s, 3H), 3.45 (m, 4H), 2.98 (s, 3H), 2.80 (s, 3H), 2.40 (m, 10H), 2.00 (m, 2H).

### Example 127 [AXL22208]

### 2,5-difluoro-N-[3-fluoro-4-[[6-methoxy-7-[3-[4-(4-oxo-4-pyrrolidin-1-yl-butanoyl)piperazin-1-yl]propoxy]-4-quinolyl]oxy]phenyl]benzenesulfonamide

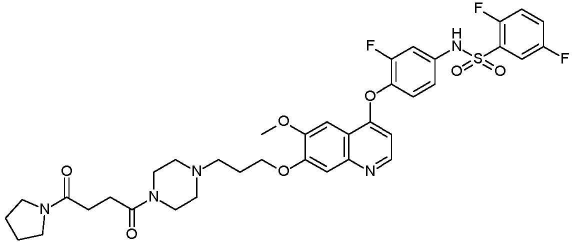

To the solution of succinic anhydride (3.80 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and pyrrolidine (2.98 mg, 0.042 mmol) was added, and the mixture was stirred for further 3 hours at room temperature. The mixture was purified by reverse phase chromatography. Thus, 14.05 mg title compound was obtained as a white solid after lyophilization. Yield: 49%. LCMS Anal. calculated for C₃₇H₄₀F₃N₅O₇S: 755.3, found m/z: 754.6 [M-H]⁻, 756.6 [M+H]⁺, t_{R} = 2.65 min; peak area 100%. ¹H NMR (300 MHz, DMSO-d6): δ 10.7 (bs, 1H), 8.44 (d, J=5.2Hz, 1H), 7.72 (m, 1H), 7.62 (m, 1H), 7.52 (m, 1H), 7.48 (s, 1H), 7.38 (m, 2H), 7.18 (dd, J=12.1Hz, 2.1Hz, 1H), 7.05 (d, J=9.0Hz, 1H), 6.35 (d, J=5.1Hz, 1H), 4.20 (t, J=6.0Hz, 2H), 3.92 (s, 3H), 3.44 (m, 8H), 2.40 (m, 10H), 2.00 (m, 2H), 1.87 (m, 2H), 1.76 (m, 2H).

### Example 128 [AXL22209]

### N-[4-[[7-[3-[4-[4-(4-allylpiperazin-1-yl)-4-oxo-butanoyl]piperazin-1-yl]propoxy]-6-methoxy-4-quinolyl]oxy]-3-fluoro-phenyl]-2,5-difluoro-benzenesulfonamide

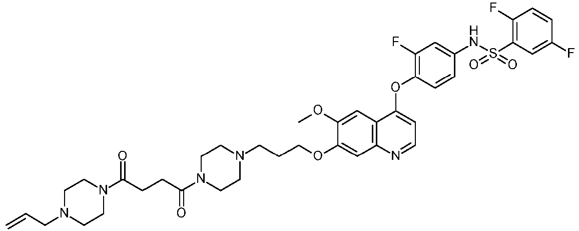

To the solution of succinic anhydride (3.80 mg 0.038 mmol) and 2,5-difluoro-N-[3-fluoro-4-[[6-methoxy-7-(3-piperazin-1-ylpropoxy)-4-quinolyl]oxy]phenyl]benzenesulfonamide (I7B1, 22.88 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and 1-allylpiperazine (5.29 mg, 0.042 mmol) was added, and the mixture was stirred for further 3 hours at room temperature. The mixture was purified by reverse phase chromatography. Thus, 13.78 mg title compound was obtained as a white solid after lyophilization. Yield: 45%. LCMS Anal. calculated for C₄₀H₄₅F₃N₆O₇S: 810.3, found m/z: 809.5 [M-H]⁻, 811.5 [M+H]⁺, t_{R} = 2.33 min; peak area 100%. ¹H NMR (300 MHz, DMSO-d6): δ 10.7 (bs, 1H), 8.44 (d, J=5.2Hz, 1H), 7.72 (m, 1H), 7.62 (m, 1H), 7.52 (m, 1H), 7.48 (s, 1H), 7.38 (m, 2H), 7.18 (dd, J=12.1Hz, 2.1Hz, 1H), 7.05 (d, J=9.0Hz, 1H), 6.35 (d, J=5.1Hz, 1H), 5.80 (m, 1H), 5.16 (m, 2H), 4.20 (t, J=6.0Hz, 2H), 3.92 (s, 3H), 3.44 (m, 8H), 2.96 (d, J=6.3Hz, 2H), 2.45 (m, 10H), 2.30 (m, 4H), 2.00 (m, 2H).

### Example 129 [AXL22210]

### 4-[4-[3-[[4-[2-fluoro-4-[[2-(trifluoromethyl)phenyl]sulfonylamino]phenoxy]-6-methoxy-7-quinolyl]oxy]propyl]piperazin-1-yl]-N,N-dimethyl-4-oxo-butanamide

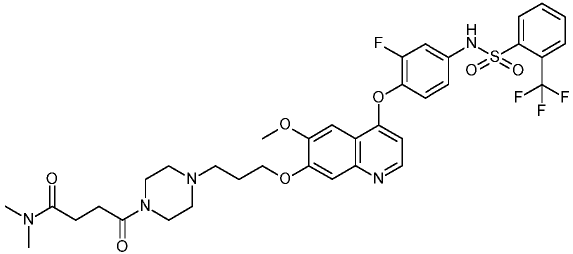

To the solution of succinic anhydride (3.80 mg 0.038 mmol) and N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and dimethylamine hydrochloride (3.42 mg, 0.042 mmol) was added, and the mixture was stirred for further 3 hours at room temperature. The mixture was purified by reverse phase chromatography. Thus, 15.40 mg title compound was obtained as a white solid after lyophilization. Yield: 53%. LCMS Anal. calculated for C₃₆H₃₉F₄N₅O₇S: 761.3, found m/z: 760.5 [M-H]⁻, 762.5 [M+H]⁺, t_{R} = 2.65 min; peak area 100%. ¹H NMR (300 MHz, DMSO-d6): δ 10.7 (bs, 1H), 8.44 (d, J=4.7Hz, 1H), 8.15 (d, J=5.1Hz, 1H), 8.04 (d, J=7.2Hz, 1H), 7.90 (m, 2H), 7.47 (s, 1H), 7.38 (m, 2H), 7.16 (dd, J=12.2Hz, 2.2Hz, 1H), 7.02 (d, J=9.0Hz, 1H), 6.36 (d, J=5.1Hz, 1H), 4.20 (t, J=6.0Hz, 2H), 3.92 (s, 3H), 3.45 (m, 4H), 2.98 (s, 3H), 2.80 (s, 3H), 2.45 (m, 10H), 1.98 (m, 2H).

### Example 130 [AXL22211]

### N-[4-[[7-[3-[4-[4-(azetidin-1-yl)-4-oxo-butanoyl]piperazin-1-yl]propoxy]-6-methoxy-4-quinolyl]oxy]-3-fluoro-phenyl]-2-(trifluoromethyl)benzenesulfonamide

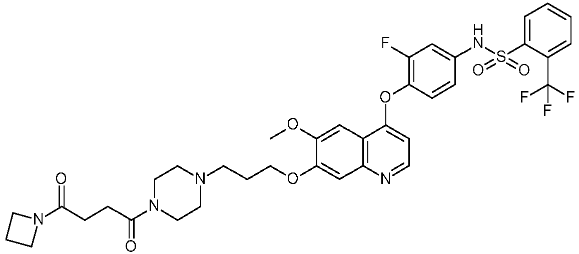

To the solution of succinic anhydride (3.80 mg 0.038 mmol) and N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and azetidine (2.39 mg, 0.042 mmol) was added, and the mixture was stirred for further 3 hours at room temperature. The mixture was purified by reverse phase chromatography. Thus, 8.09 mg title compound was obtained as a white solid after lyophilization. Yield: 27%. LCMS Anal. calculated for C₃₇H₃₉F₄N₅O₇S: 773.3, found m/z: 772.5 [M-H]⁻, 774.5 [M+H]⁺, t_{R} = 2.68 min; peak area 100%. ¹H NMR (300 MHz, DMSO-d6): δ 10.9 (bs, 1H), 8.44 (d, J=4.7Hz, 1H), 8.15 (d, J=5.1Hz, 1H), 8.04 (d, J=7.2Hz, 1H), 7.90 (m, 2H), 7.47 (s, 1H), 7.38 (m, 2H), 7.16 (dd, J=12.2Hz, 2.2Hz, 1H), 7.02 (d, J=9.0Hz, 1H), 6.36 (d, J=5.1Hz, 1H), 4.20 (t, J=6.0Hz, 2H), 4.13 (t, J=7.5Hz, 2H), 3.92 (s, 3H), 3.80 (t, J=7.5Hz, 2H), 3.48 (m, 4H), 2.40 (m, 8H), 2.15 (m, 4H), 1.98 (m, 2H).

### Example 131 [AXL22212]

### N-[3-fluoro-4-[[6-methoxy-7-[3-[4-(4-morpholino-4-oxo-butanoyl)piperazin-1-yl]propoxy]-4-quinolyl]oxy]phenyl]-2-(trifluoromethyl)benzenesulfonamide

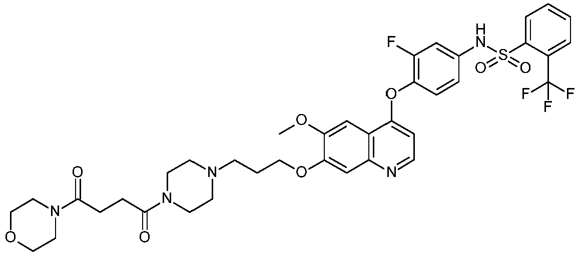

To the solution of succinic anhydride (3.80 mg 0.038 mmol) and N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and morpholine (3.65 mg, 0.042 mmol) was added, and the mixture was stirred for further 3 hours at room temperature. The mixture was purified by reverse phase chromatography. Thus, 19.24 mg title compound was obtained as a white solid after lyophilization. Yield: 63%. LCMS Anal. calculated for C₃₈H₄₁F₄N₅O₈S: 803.3, found m/z: 802.5 [M-H]⁻, 804.5 [M+H]⁺, t_{R} = 2.68 min; peak area 100%. ¹H NMR (300 MHz, DMSO-d6): δ 10.9 (bs, 1H), 8.44 (d, J=4.7Hz, 1H), 8.15 (d, J=5.1Hz, 1H), 8.04 (d, J=7.2Hz, 1H), 7.90 (m, 2H), 7.47 (s, 1H), 7.38 (m, 2H), 7.16 (dd, J=12.2Hz, 2.2Hz, 1H), 7.02 (d, J=9.0Hz, 1H), 6.36 (d, J=5.1Hz, 1H), 4.20 (t, J=6.0Hz, 2H), 3.92 (s, 3H), 3.50 (m, 12H), 2.53 (m, 6H), 2.40 (m, 4H), 1.98 (m, 2H).

### Example 132 [AXL22213]

### N-[3-fluoro-4-[[6-methoxy-7-[3-[4-[4-(4-methylpiperazin-1-yl)-4-oxo-butanoyl]piperazin-1-yl]propoxy]-4-quinolyl]oxy]phenyl]-2-(trifluoromethyl)benzenesulfonamide

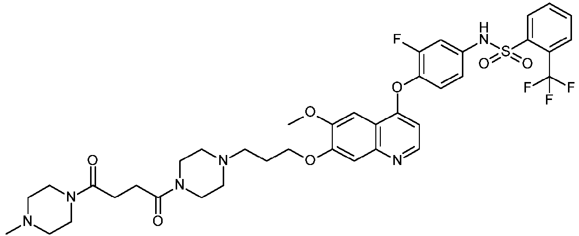

To the solution of succinic anhydride (3.80 mg 0.038 mmol) and N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and 1-methylpiperazine (4.20 mg, 0.042 mmol) was added, and the mixture was stirred for further 3 hours at room temperature. The mixture was purified by reverse phase chromatography. Thus, 14.76 mg title compound was obtained as a white solid after lyophilization. Yield: 47%. LCMS Anal. calculated for C₃₉H₄₄F₄N₆O₇S: 816.4, found m/z: 815.5 [M-H]⁻, 817.5 [M+H]⁺, t_{R} = 2.40 min; peak area 100%. ¹H NMR (300 MHz, DMSO-d6): δ 11.1 (bs, 1H), 8.46 (d, J=5.2Hz, 1H), 8.17 (d, J=7.2Hz, 1H), 7.98 (d, J=5.4Hz, 1H), 7.86 (m, 2H), 7.50 (s, 1H), 7.38 (s, 1H), 7.28 (t, J=9.1Hz, 1H), 7.14 (dd, J=12.3Hz, 2.3Hz, 1H), 7.01 (d, J=8.9Hz, 1H), 6.39 (d, J=5.1Hz, 1H), 4.20 (t, J=6.0Hz, 2H), 3.92 (s, 3H), 3.47 (m, 8H), 2.54 (m, 6H), 2.42 (m, 4H), 2.30 (m, 4H), 2.21 (s, 3H), 1.98 (m, 2H).

### Example 133 [AXL22214]

### N,N-diethyl-5-[4-[3-[[4-[2-fluoro-4-[[2-(trifluoromethyl)phenyl]sulfonylamino]phenoxy]-6-methoxy-7-quinolyl]oxy]propyl]piperazin-1-yl]-5-oxo-pentanamide

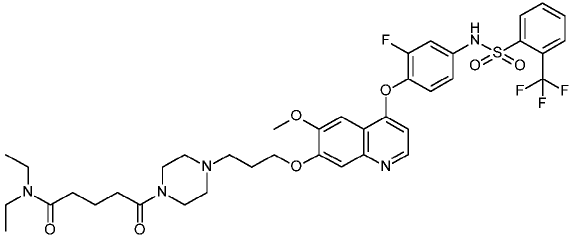

To the solution of glutaric anhydride (4.33 mg 0.038 mmol) and N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and diethylamine (3.06 mg, 0.042 mmol) was added, and the mixture was stirred for further 3 hours at room temperature. The mixture was purified by reverse phase chromatography. Thus, 16.73 mg title compound was obtained as a white solid after lyophilization. Yield: 55%. LCMS Anal. calculated for C₃₉H₄₅F₄N₅O₇S: 803.3, found m/z: 802.5 [M-H]⁻, 804.5 [M+H]⁺, t_{R} = 2.83 min; peak area 100%. ¹H NMR (300 MHz, DMSO-d6): δ 10.9 (bs, 1H), 8.44 (d, J=5.2Hz, 1H), 8.15 (d, J=7.0Hz, 1H), 8.03 (d, J=5.4Hz, 1H), 7.90 (m, 2H), 7.47 (s, 1H), 7.39 (m, 2H), 7.16 (dd, J=12.2Hz, 2.3Hz, 1H), 7.03 (d, J=8.6Hz, 1H), 6.36 (d, J=5.1Hz, 1H), 4.20 (t, J=6.0Hz, 2H), 3.91 (s, 3H), 3.46 (m, 4H), 3.25 (m, 4H), 2.54 (m, 2H), 2.42 (m, 4H), 2.30 (m, 4H), 2.00 (m, 2H), 1.70 (m, 2H), 1.08 (t, J=7.2Hz, 3H), 0.99 (t, J=7.2Hz, 3H).

### Example 134 [AXL22215]

### N-[3-fluoro-4-[[6-methoxy-7-[3-[4-(5-oxo-5-pyrrolidin-1-yl-pentanoyl)piperazin-1-yl]propoxy]-4-quinolyl]oxy]phenyl]-2-(trifluoromethyl)benzenesulfonamide

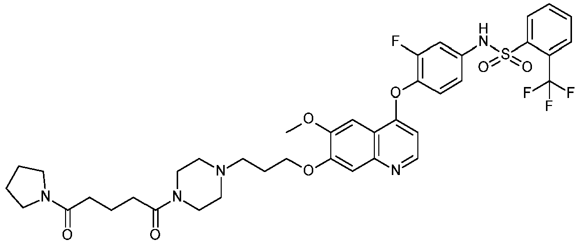

To the solution of glutaric anhydride (4.33 mg 0.038 mmol) and N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and pyrrolidine (2.98 mg, 0.042 mmol) was added, and the mixture was stirred for further 3 hours at room temperature. The mixture was purified by reverse phase chromatography. Thus, 15.55 mg title compound was obtained as a white solid after lyophilization. Yield: 51%. LCMS Anal. calculated for C₃₉H₄₃F₄N₅O₇S: 801.3, found m/z: 800.5 [M-H]⁻, 802.5 [M+H]⁺, t_{R} = 2.78 min; peak area 100%. ¹H NMR (300 MHz, DMSO-d6): δ 10.9 (bs, 1H), 8.44 (d, J=5.2Hz, 1H), 8.15 (d, J=7.0Hz, 1H), 8.03 (d, J=5.4Hz, 1H), 7.90 (m, 2H), 7.47 (s, 1H), 7.39 (m, 2H), 7.16 (dd, J=12.2Hz, 2.3Hz, 1H), 7.03 (d, J=8.6Hz, 1H), 6.36 (d, J=5.1Hz, 1H), 4.20 (t, J=6.0Hz, 2H), 3.91 (s, 3H), 3.46 (m, 4H), 3.23 (m, 4H), 2.54 (m, 2H), 2.42 (m, 4H), 2.24 (m, 4H), 1.99 (m, 2H), 1.85 (m, 6H).

### Example 135 [AXL22216]

### N-[3-fluoro-4-[[6-methoxy-7-[3-[4-[5-oxo-5-(1-piperidyl)pentanoyl]piperazin-1-yl]propoxy]-4-quinolyl]oxy]phenyl]-2-(trifluoromethyl)benzenesulfonamide

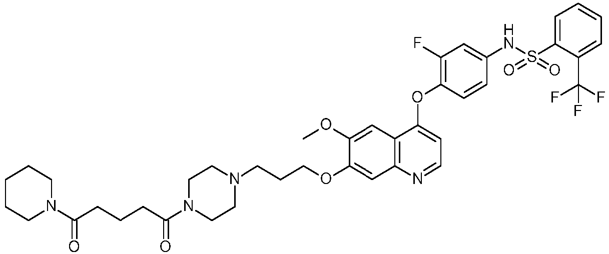

To the solution of glutaric anhydride (4.33 mg 0.038 mmol) and N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and piperidine (3.57 mg, 0.042 mmol) was added, and the mixture was stirred for further 3 hours at room temperature. The mixture was purified by reverse phase chromatography. Thus, 17.19 mg title compound was obtained as a white solid after lyophilization. Yield: 55%. LCMS Anal. calculated for C₄₀H₄₅F₄N₅O₇S: 815.3, found m/z: 814.5 [M-H]⁻, 816.5 [M+H]⁺, t_{R} = 2.86 min; peak area 100%. ¹H NMR (300 MHz, DMSO-d6): δ 10.9 (bs, 1H), 8.44 (d, J=4.7Hz, 1H), 8.15 (d, J=6.0Hz, 1H), 8.02 (d, J=7.2Hz, 1H), 7.89 (m, 2H), 7.47 (s, 1H), 7.38 (m, 2H), 7.15 (dd, J=12.2Hz, 1.9Hz, 1H), 7.02 (d, J=8.8Hz, 1H), 6.35 (d, J=5.1Hz, 1H), 4.20 (t, J=6.0Hz, 2H), 3.91 (s, 3H), 3.45 (m, 8H), 2.66 (m, 2H), 2.42 (m, 4H), 2.32 (m, 4H), 1.99 (m, 2H), 1.71 (m, 2H), 1.58 (m, 2H), 1.46 (m, 4H).

### Example 136 [AXL22217]

### N-[4-[[7-[3-[4-[5-(4-allylpiperazin-1-yl)-5-oxo-pentanoyl]piperazin-1-yl]propoxy]-6-methoxy-4-quinolyl]oxy]-3-fluoro-phenyl]-2-(trifluoromethyl)benzenesulfonamide

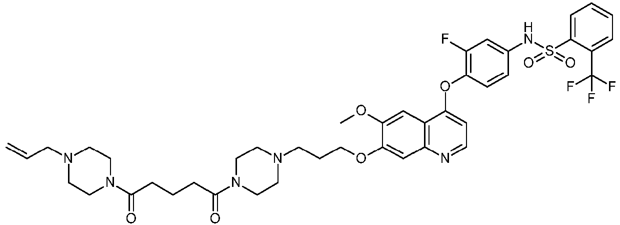

To the solution of glutaric anhydride (4.33 mg 0.038 mmol) and N-[3-fluoro-4-[[6-methoxy-7-(3-piperazin-1-ylpropoxy)-4-quinolyl]oxy]phenyl]-2-(trifluoromethyl)benzenesulfonamide (I7B2, 24.09 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and 1-allylpiperazine (5.29 mg, 0.042 mmol) was added, and the mixture was stirred for further 3 hours at room temperature. The mixture was purified by reverse phase chromatography. Thus, 14.18 mg title compound was obtained as a white solid after lyophilization. Yield: 43%. LCMS Anal. calculated for C₄₂H₄₈F₄N₆O₇S: 856.4, found m/z: 855.6 [M-H]⁻, 857.6 [M+H]⁺, t_{R} = 2.43 min; peak area 100%. ¹H NMR (300 MHz, DMSO-d6): δ 11.0 (bs, 1H), 8.44 (d, J=5.2z, 1H), 8.15 (d, J=7.0Hz, 1H), 8.01 (d, J=7.6Hz, 1H), 7.88 (m, 2H), 7.47 (s, 1H), 7.35 (m, 2H), 7.12 (dd, J=12.5Hz, 2.2Hz, 1H), 7.00 (d, J=8.6Hz, 1H), 6.35 (d, J=5.1Hz, 1H), 5.86 (m, 1H), 5.14 (m, 2H), 4.19 (t, J=5.7Hz, 2H), 3.91 (s, 3H), 3.45 (m, 8H), 2.94 (m, 2H), 2.40 (m, 6H), 2.32 (m, 8H), 1.98 (m, 2H), 1.68 (m, 2H).

### Example 137 [AXL22218]

### (Z)-4-(4-allylpiperazin-1-yl)-N-[3-[[4-[4-[(2,5-difluorophenyl)sulfonylamino]-2-fluoro-phenoxy]-6-methoxy-7-quinolyl]oxy]propyl]-4-oxo-but-2-enamide

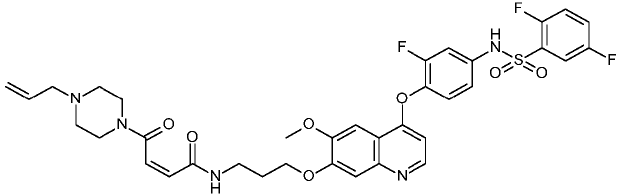

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and 1-allylpiperazine (5.29 mg, 0.042 mmol) was added, and the mixture was stirred for further 3 hours at room temperature. The mixture was purified by reverse phase chromatography. Thus, 10.14 mg title compound was obtained as a white solid after lyophilization. Yield: 36%. LCMS Anal. calculated for C₃₆H₃₆F₃N₅O₇S: 739.2, found m/z: 738.4 [M-H]⁻, 740.4 [M+H]⁺, t_{R} = 2.64 min; peak area 100%.

### Example 138 [AXL22219]

### (Z)-N-[3-[[4-[4-[(2,5-difluorophenyl)sulfonylamino]-2-fluoro-phenoxy]-6-methoxy-7-quinolyl]oxy]propyl]-4-(3-methyl-1-piperidyl)-4-oxo-but-2-enamide

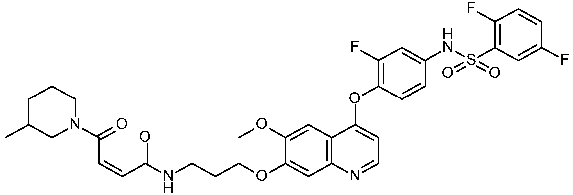

To the solution of maleic anhydride (3.72 mg 0.038 mmol) and N-[4-[[7-(3-aminopropoxy)-6-methoxy-4-quinolyl]oxy]-3-fluoro-phenyl]-2,5-difluoro-benzenesulfonamide (I7A1, 20.25 mg, 0.038 mmol) in DMF (1.6 mL) DIPEA (16.23 mg, 0.126 mmol) was added. The mixture was stirred for 3 hours at room temperature. To the reaction mixture HBTU (15.96 mg, 0.042 mmol) and 3-methylpiperidine (4.16 mg, 0.042 mmol) was added, and the mixture was stirred for further 3 hours at room temperature. The mixture was purified by reverse phase chromatography. Thus, 15.94 mg title compound was obtained as a white solid after lyophilization. Yield: 58%. LCMS Anal. calculated for C₃₅H₃₅F₃N₄O₇S: 712.2, found m/z: 711.5 [M-H]⁻, 713.5 [M+H]⁺, t_{R} = 3.28 min; peak area 100%.

### Example 139 [AXL22031]

### N-[4-[[7-[3-[4-[(E)-4-(dimethylamino)but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide

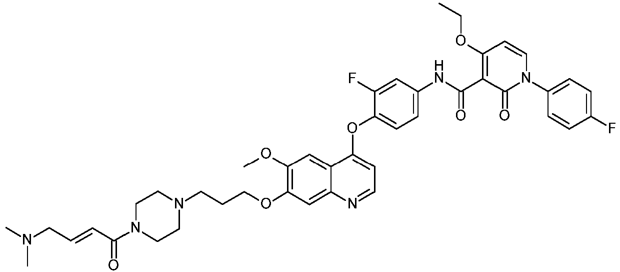

LCMS Anal. calculated for C₄₃H₄₆F₂N₆O₇: 796.3, found m/z: 795.4 [M-H]⁻, 797.4 [M+H]⁺, t_{R} = 2.07 min; peak area 97%.

### Example 140 [AXL22042]

### 4-ethoxy-N-[3-fluoro-4-[[6-methoxy-7-[3-[4-[(E)-4-morpholinobut-2-enoyl]piperazin-1-yl]propoxy]-4-quinolyl]oxy]phenyl]-1-(4-fluorophenyl)-2-oxo-pyridine-3-carboxamide

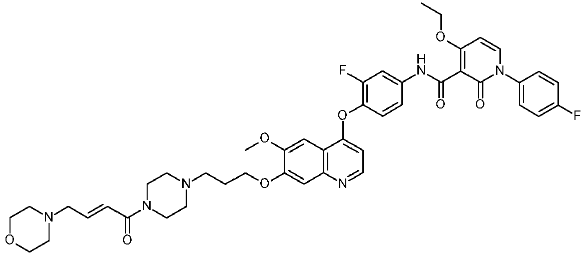

LCMS Anal. calculated for C₄₅H₄₈F₂N₆O₈: 838.4, found m/z: 837.4 [M-H]⁻, 839.4 [M+H]⁺, t_{R} = 2.08 min; peak area 99%.

### Example 141 [AXL22043]

### 4-ethoxy-N-[3-fluoro-4-[[6-methoxy-7-[3-(4-prop-2-enoylpiperazin-1-yl)propoxy]-4-quinolyl]oxy]phenyl]-1-(4-fluorophenyl)-2-oxo-pyridine-3-carboxamide

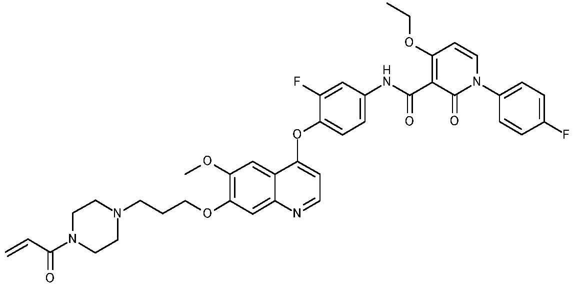

LCMS Anal. calculated for C₄₀H₃₉F₂N₅O₇: 739.3, found m/z: 738.2 [M-H]⁻, 740.3 [M+H]⁺, t_{R} = 2.67 min; peak area 98%.

### Example 142 [AXL22056]

### 4-ethoxy-N-[3-fluoro-4-[[6-methoxy-7-[3-[4-[(E)-4-(1-piperidyl)but-2-enoyl]piperazin-1-yl]propoxy]-4-quinolyl]oxy]phenyl]-1-(4-fluorophenyl)-2-oxo-pyridine-3-carboxamide

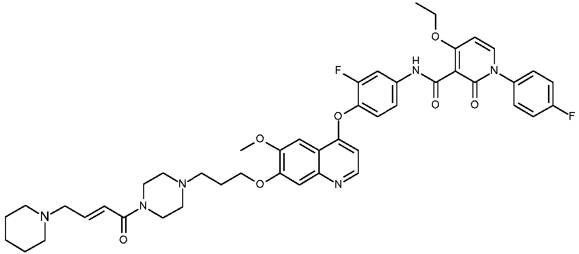

LCMS Anal. calculated for C₄₆H₅₀F₂N₆O₇: 836.4, found m/z: 835.3 [M-H]⁻, 837.3 [M+H]⁺, t_{R} = 2.42 min; peak area 98%.

### Example 143 [AXL22057]

### 4-ethoxy-N-[3-fluoro-4-[[6-methoxy-7-[3-[4-[(E)-4-(4-methylpiperazin-1-yl)but-2-enoyl]piperazin-1-yl]propoxy]-4-quinolyl]oxy]phenyl]-1-(4-fluorophenyl)-2-oxo-pyridine-3-carboxamide

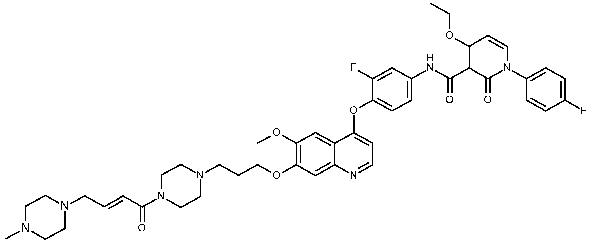

LCMS Anal. calculated for C₄₆H₅₁F₂N₇O₇: 851.4, found m/z: 850.5 [M-H]⁻, 852.5 [M+H]⁺, t_{R} = 2.37 min; peak area 97%.

### Example 144 [AXL22090]

### (Z)-4-[3-[[4-[4-[[4-ethoxy-1-(4-fluorophenyl)-2-oxo-pyridine-3-carbonyl]amino]-2-fluoro-phenoxy]-6-methoxy-7-quinolyl]oxy]propylamino]-4-oxo-but-2-enoic acid

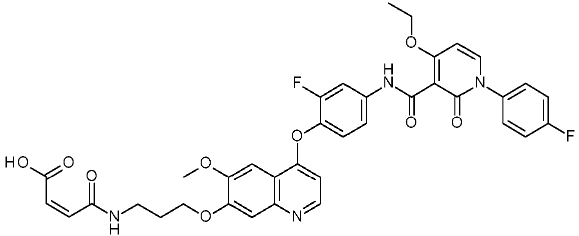

LCMS Anal. calculated for C₃₇H₃₂F₂N₄O₉: 714.2, found m/z: 713.3 [M-H]⁻, 715.3 [M+H]⁺, t_{R} = 3.38 min; peak area 98%.
¹H NMR (300 MHz, DMSO-d6): δ 10.57 (s, 1H); 8.47 (d, 1H, J=5.1 Hz); 7.94-7.83 (m, 2H); 7.54-7.33 (m, 8H); 6.52 -6.46 (m, 3H); 6.08 (m, 1H); 4.24-4.19 (m, 2H); 3.96 (s, 3H); 2.72-2.60 (m, 4H); 2.40-2.27 (m, 2H); 2.00 (m, 2H); 1.31 (t, 3H, J=6.9 Hz)

### Example 145 [AXL22127]

### (Z)-4-[4-[3-[[4-[4-[[4-ethoxy-1-(4-fluorophenyl)-2-oxo-pyridine-3-carbonyl]amino]-2-fluoro-phenoxy]-6-methoxy-7-quinolyl]oxy]propyl]piperazin-1-yl]-4-oxo-but-2-enoic acid

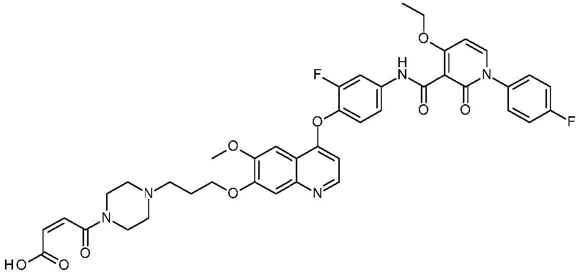

685 mg (1 mmol) 4-ethoxy-N-(3-fluoro-4-{[6-methoxy-7-(3-piperazin-1-ylpropoxy)quinolin-4-yl]oxy}phenyl)-1-(4-fluorophenyl)-2-oxo-1,2-dihydropyridine-3-carboxamide was dissolved in 20 ml anhydrous acetonitrile at 50-60 °C and 98 mg (1 mmol) maleic anhydride was added to the solution and refluxed for 5 mins. The solvent was removed under reduced pressure and the crude product was used in the next step without purification. LCMS Anal. calculated for C₄₁H₃₉F₂N₅O₉: 783.3, found m/z: 782.4 [M-H]⁻, 784.4 [M+H]⁺, t_{R} = 2.60 min; peak area 99%.
¹H NMR (300 MHz, DMSO-d6): δ 10.58 (s, 1H); 8.45 (d, 1H, J=5.2 Hz); 8.29 (brs, 1H); 7.92 (dd, 1H, J=11.0 Hz, J=1.9Hz); 7.86 (d, 1H, J=7.8Hz); 7.53-7.48 (m, 2H); 7.47-7.34 (m, 6H); 6.52 (d, 1H, J=7.9 Hz); 6.47-6.43 (m, 1H); 6.00-5.90 (m, 1H); 4.30-4.19 (m, 5H); 3.95 (s, 3H); 3.47-3.42 (m, 4H); 2.40-2.36 (m, 6H); 1.98 (m, 2H); 1.31 (t, 3H, J=7.0 Hz)

### Example 146 [AXL22128]

### 4-ethoxy-N-[3-fluoro-4-[[6-methoxy-7-[3-[4-[(E)-4-(3-methyl-1-piperidyl)but-2-enoyl]piperazin-1-yl]propoxy]-4-quinolyl]oxy]phenyl]-1-(4-fluorophenyl)-2-oxo-pyridine-3-carboxamide

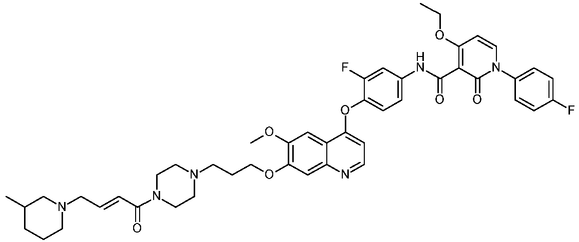

LCMS Anal. calculated for C₄₇H₅₂F₂N₆O₇: 850.4, found m/z: 849.5 [M-H]⁻, 851.5 [M+H]⁺, t_{R} = 2.44 min; peak area 100%.
¹H NMR (300 MHz, DMSO-d6): δ 10.57 (s, 1H); 8.45 (d, 1H, J=5.2 Hz); 7.92 (m, 1H); 7.85 (m, 1H); 7.53-7.51 (m, 2H); 7.48-7.33 (m, 7H); 6.59 (m, 1H); 6.53-6.46 (m, 2H); 4.28-4.19 (m, 4H); 3.96 (s, 3H); 3.53-3.48 (m, 4H); 3.20-3.07 (m, 10H); 2.72 (m, 2H); 2.40-2.27 (m, 6H); 1.98 (m, 2H); 1.65-1.48 (m, 2H); 1.31 (t, 3H, J=6.8 Hz)

### Example 147 [AXL22129]

### 4-ethoxy-N-[3-fluoro-4-[[6-methoxy-7-[3-[4-[(E)-3-(2-thienyl)prop-2-enoyl]piperazin-1-yl]propoxy]-4-quinolyl]oxy]phenyl]-1-(4-fluorophenyl)-2-oxo-pyridine-3-carboxamide

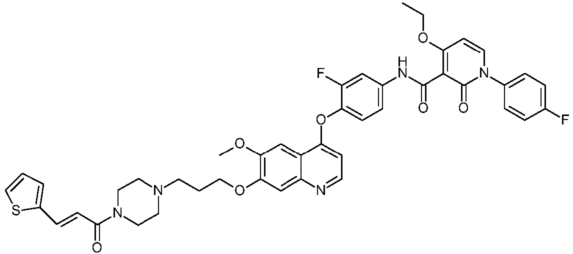

LCMS Anal. calculated for C₄₄H₄₁F₂N₅O₇S: 821.3, found m/z: 820.4 [M-H]⁻, 822.4 [M+H]⁺, t_{R} = 2.97 min; peak area 97%.
¹H NMR (300 MHz, DMSO-d6): δ 10.58 (s, 1H); 8.45 (d, 1H, J=5.3 Hz); 7.93 (m, 1H); 7.84 (d, 1H, J=7.9 Hz); 7.81-7.70 (m, 2H); 7.54-7.52 (m, 1H); 7.48-7.44(m, 3H); 7.39-7.33 (m, 4H); 7.21 (m, 1H); 7.05 (d, 1H, J=8.0 Hz); 6.97 (t, 1H, J=8.1 Hz); 6.52 (d, 1H, J=7.8 Hz); 6.47 (d, 1H, J=5.1 Hz); 4.30-4.16 (m, 4H);3.96 (s, 3H); 3.85 (m, 3H); 3.68-3.59 (m, 2H);3.20-3.03 (m, 2H);2.43-2.27 (m, 3H); 2.01 (m, 2H); 1.31 (t, 3H, J=6.9 Hz)

### Example 148 [AXL22130]

### N-[4-[[7-[3-[4-[(E)-4-(diethylamino)but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide

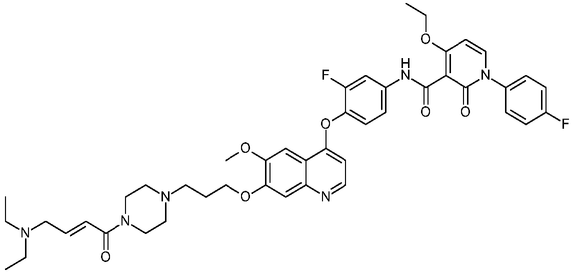

LCMS Anal. calculated for C₄₅H₅₀F₂N₆O₇: 824.4, found m/z: 823.5 [M-H]⁻, 825.5 [M+H]⁺, t_{R} = 2.36 min; peak area 98%.

### Example 149 [AXL22131]

### 4-ethoxy-N-[3-fluoro-4-[[6-methoxy-7-[3-[4-[(E)-2-methylbut-2-enoyl]piperazin-1-yl]propoxy]-4-quinolyl]oxy]phenyl]-1-(4-fluorophenyl)-2-oxo-pyridine-3-carboxamide

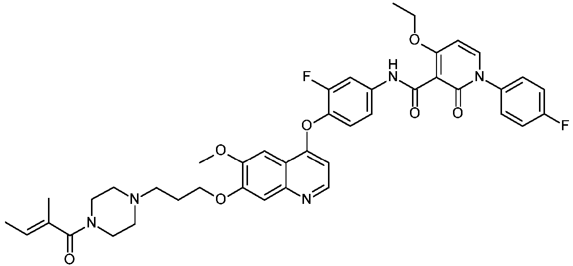

LCMS Anal. calculated for C₄₂H₄₃F₂N₅O₇: 767.3, found m/z: 766.5 [M-H]⁻, 768.5 [M+H]⁺, t_{R} = 2.76 min; peak area 100%.
¹H NMR (300 MHz, DMSO-d6): δ 10.58 (s, 1H); 8.45 (d, 1H, J=5.2 Hz); 7.92 (dd, 1H, J=7.7 Hz); 7.54-7.52 (m, 2H); 7.49-7.44 (m, 4H); 7.41-7.34 (m, 3H); 6.51 (d, 1H, J=8.0 Hz); 6.46 (d, 1H, J=4.9 Hz); 5.51 (m, 1H); 4.30-4.19 (m, 4H); 3.95 (s, 3H); 3.46-3.44 (m, 6H); 3.18-3.13 (m, 4H); 2.39-2.37 (m, 2H); 1.99 (m, 2H); 1.71 (s, 2H); 1.64 (d, 2H, J=6.8 Hz); 1.31 (t, 3H, J=6.9 Hz)

### Example 150 [AXL22133]

### N-[4-[[7-[3-(4-but-2-ynoylpiperazin-1-yl)propoxy]-6-methoxy-4-quinolyl]oxy]-3-fluoro-phenyl]-4-ethoxy-1-(4-fluorophenyl)-2-oxo-pyridine-3-carboxamide

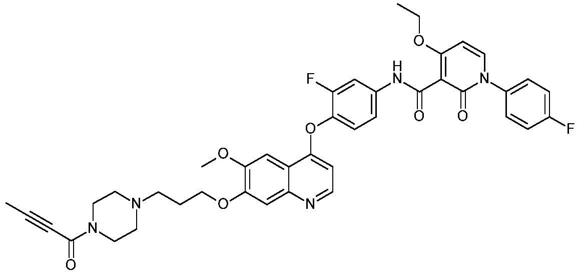

LCMS Anal. calculated for C₄₁H₃₉F₂N₅O₇: 751.3, found m/z: 750.4 [M-H]⁻, 752.4 [M+H]⁺, t_{R} = 2.69 min; peak area 100%.
¹H NMR (300 MHz, DMSO-d6): δ 10.58 (s, 1H); 8.45 (d, 1H, J=5.3 Hz); 7.93 (dd, 1H, J=11.0 Hz, J=1.9 Hz); 7.53 (m, 1H); 7.49-7.46 (m, 2H); 7.44-7.37 (m, 4H); 7.36-7.33 (m, 1H); 6.51 (d, 1H, J=7.8 Hz); 6.47 (d, 1H, J=5.3 Hz); 6.47 (d, 1H, J=5.3 Hz); 4.30-4.08 (m, 4H); 3.67 (m, 2H); 3.58-3.47 (m, 5H); 2.45-2.37 (m, 6H); 2.01-1.97 (m, 5H); 1.31 (t, 3H, J=7.0 Hz)

### Example 151 [AXL22134]

### 4-ethoxy-N-[3-fluoro-4-[[6-methoxy-7-[3-[4-[(E)-2-methylpent-2-enoyl]piperazin-1-yl]propoxy]-4-quinolyl]oxy]phenyl]-1-(4-fluorophenyl)-2-oxo-pyridine-3-carboxamide

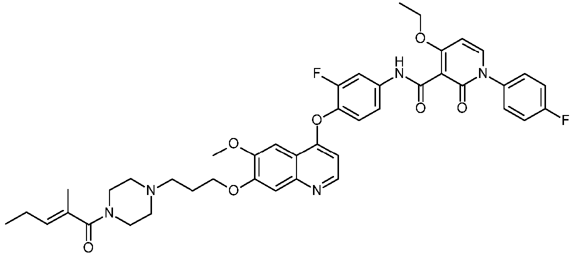

LCMS Anal. calculated for C₄₃H₄₅F₂N₅O₇: 781.3, found m/z: 780.5 [M-H]⁻, 782.2 [M+H]⁺, t_{R} = 2.84 min; peak area 100%.

### Example 152 [AXL22136]

### N-[4-[[7-[3-[4-[(E)-3-(2,5-dimethoxyphenyl)prop-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide

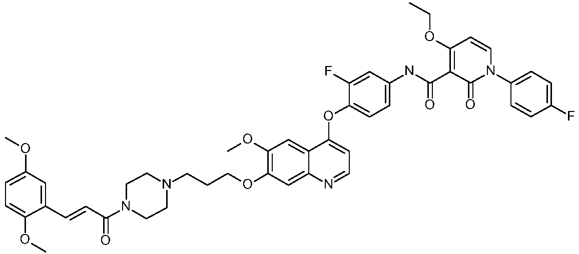

LCMS Anal. calculated for C₄₈H₄₇F₂N₅O₉: 875.3, found m/z: 874.6 [M-H]⁻, 876.6 [M+H]⁺, t_{R} = 3.03 min; peak area 100%.
¹H NMR (300 MHz, DMSO-d6): δ 10.58 (s, 1H); 8.46 (d, 1H, J=5.2 Hz); 7.92 (dd, 1H, J=11.1 Hz, 1.8 Hz); 7.85 (d, 1H, J=7.7 Hz); 7.75 (d, 1H, J=15.1 Hz); 7.54-7.52 (m, 1H); 7.49-7.44 (m, 3H); 7.41-7.33 (m, 5H); 7.20 (d, 1H, J=15.1 Hz); 7.00-6.92 (m, 2H); 6.51 (d, 1H, J=7.9 Hz); 6.47 (d, 1H, J=5.3 Hz); 4.28-4.23 (m, 4H); 3.96 (s, 3H); 3.80 (s, 3H); 3.76 (s, 3H); 3.66-3.59 (m, 4H); 3.21-3.13 (m, 2H); 2.44-2.42 (m, 4H); 2.01 (m, 2H); 1.31 (t, 3H, J=6.9 Hz)

### Example 153 [AXL22137]

### N-[4-[[7-[3-[4-[(E)-but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide

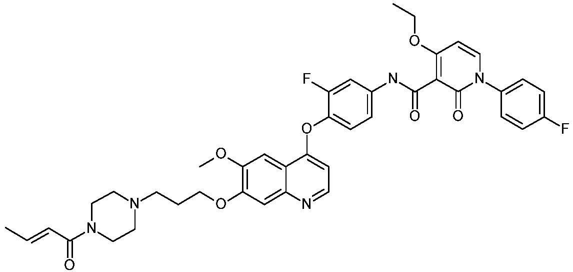

LCMS Anal. calculated for C₄₁H₄₁F₂N₅O₇: 753.3, found m/z: 752.5 [M-H]⁻, 754.5 [M+H]⁺, t_{R} = 2.66 min; peak area 100%.
10.58 (s, 1H); 8.45 (d, 1H, J=5.3 Hz); 7.91 (dd, 1H, J=11.1, J=1.7 Hz); 7.84 (d, 1H, J=7.7 Hz); 7.53 (s, 1H); 7.50-5.44 (m, 3H); 7.41-7.31 (m, 4H); 6.64 (m, 1H); 6.53-6.46 (m, 3H); 4.27-4.21 (m, 4H); 3.95 (s, 3H); 3.53-3.45 (m, 4H); 3.28-3.23 (m, 4H); 2.38 (m, 2H); 1.99 (m, 2H); 1.83 (d, 3H, J=6.3 Hz); 1.13 (t, 3H, J=6.8 Hz)

### Example 154 [AXL22139]

### 4-ethoxy-N-[3-fluoro-4-[[6-methoxy-7-[3-[4-[(E)-4,4,4-trifluorobut-2-enoyl]piperazin-1-yl]propoxy]-4-quinolyl]oxy]phenyl]-1-(4-fluorophenyl)-2-oxo-pyridine-3-carboxamide

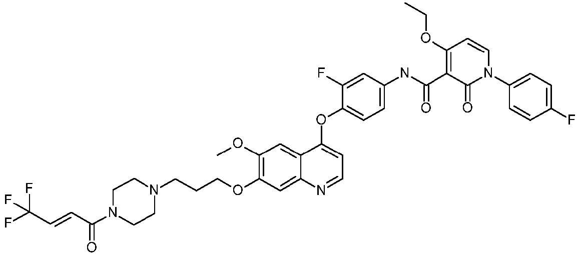

LCMS Anal. calculated for C₄₁H₃₈F₅N₅O₇: 807.4, found m/z: 806.6 [M-H]⁻, 808.6 [M+H]⁺, t_{R} = 2.87 min; peak area 100%.
¹H NMR (300 MHz, DMSO-d6): δ 10.58 (s; 1H); 8.45 (d, 1H, J=5.2Hz); 7.94-7.91 (m, 1H); 7.86-7.84 (1H, J=7.8Hz);7.54-7.44 (m, 4H); 7.41-7.30 (m, 5H); 6.81-6.68 (m, 1H); 6.53-6.50 (d, 1H, J=7.8Hz); 6.48-6.38 (m, 1H); 4.30-4.19 (m, 4H); 3.95(s, 3H); 3.59-3.57(m, 5H); 2.57-2.55 (m, 4H); 2.01 (t, 2H, J=6.3 Hz); 1.33-1.27 (m, 4H)

### Example 155 [AXL22140]

### 4-ethoxy-N-[3-fluoro-4-[[6-methoxy-7-[3-[4-(3-methylbut-2-enoyl)piperazin-1-yl]propoxy]-4-quinolyl]oxy]phenyl]-1-(4-fluorophenyl)-2-oxo-pyridine-3-carboxamide

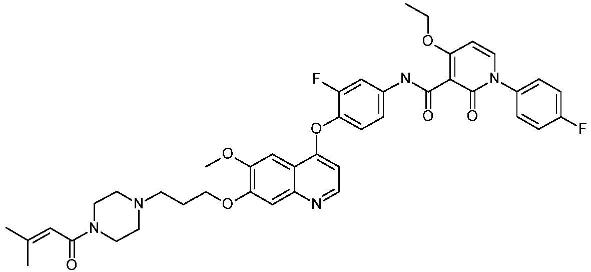

LCMS Anal. calculated for C₄₂H₄₃F₂N₅O₇: 767.4, found m/z: 766.7 [M-H]⁻, 768.6 [M+H]⁺, t_{R} = 3.85 min; peak area 99%. ¹H NMR (300 MHz, DMSO-d6): δ 10.58 (s, 1H); 8.45 (d, 1H, J=5.0 Hz); 7.93 (dd, 1H, J= 11.0 Hz, J=2.0 Hz); 7.85 (d, 1H, J=7.8 Hz); 7.53-7.44 (m, 4H); 7.41-7.33 (m, 4H); 6.52 (d, 1H, J=7.9 Hz); 6.46 (d, 1H, J=5.0 Hz); 5.89 (s, 1H); 4.30-4.19 (m, 4H); 3.95 (s, 3H); 3.47 (m, 3H); 3.21 (m, 3H); 2.39 (m, 4H); 1.99 (m,2H); 1.81-1.79 (m, 6H); 1.31 (t, 3H, J=7.0 Hz)

### Example 156 [AXL22141]

### 4-ethoxy-N-[3-fluoro-4-[[7-[3-[4-[(E)-3-(1H-imidazol-4-yl)prop-2-enoyl]piperazin-1-yl]propoxy]-6-methoxy-4-quinolyl]oxy]phenyl]-1-(4-fluorophenyl)-2-oxo-pyridine-3-carboxamide

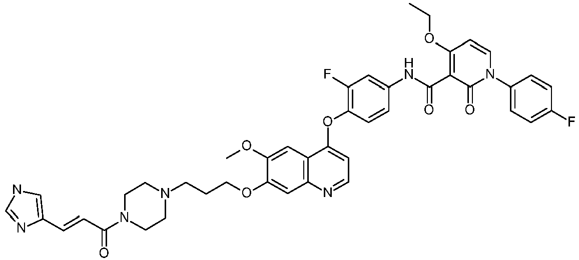

LCMS Anal. calculated for C₄₃H₄₁F₂N₇O₇: 805.4, found m/z: 804.6 [M-H]⁻, 806.6 [M+H]⁺, t_{R} = 3.55 min; peak area 98%.

### Example 157 [AXL22143]

### 4-ethoxy-N-[3-fluoro-4-[[6-methoxy-7-[3-[4-[(E)-3-phenylprop-2-enoyl]piperazin-1-yl]propoxy]-4-quinolyl]oxy]phenyl]-1-(4-fluorophenyl)-2-oxo-pyridine-3-carboxamide

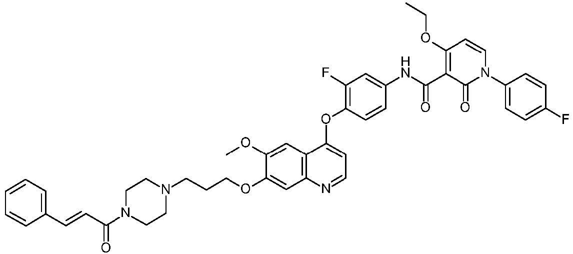

LCMS Anal. calculated for C₄₆H₄₃F₂N₅O₇: 815.4, found m/z: 814.6 [M-H]⁻, 816.6 [M+H]⁺, t_{R} = 3.04 min; peak area 100%.

### Example 158 [AXL22144]

### 4-ethoxy-N-[3-fluoro-4-[[6-methoxy-7-[3-[4-[(E)-2-methyl-3-phenyl-prop-2-enoyl]piperazin-1-yl]propoxy]-4-quinolyl]oxy]phenyl]-1-(4-fluorophenyl)-2-oxo-pyridine-3-carboxamide

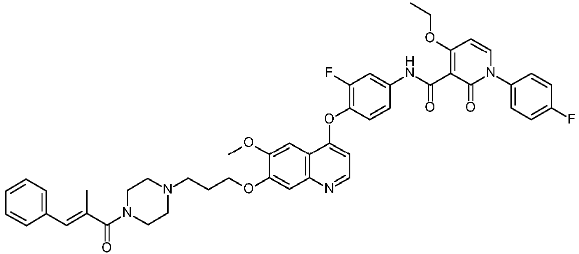

LCMS Anal. calculated for C₄₇H₄₅F₂N₅O₇: 829.3, found m/z: 828.6 [M-H]⁻, 830.6 [M+H]⁺, t_{R} = 4.24 min; peak area 98%.

### Example 159 [AXL22145]

### N-[4-[[7-[3-[4-[(E)-3-(2,3-dimethoxyphenyl)prop-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide

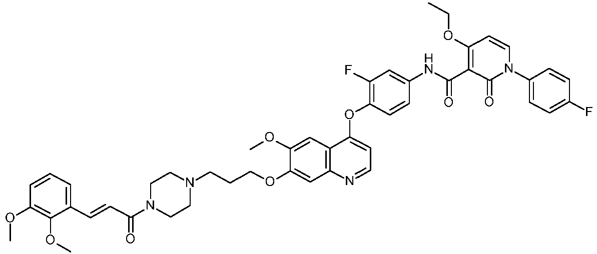

LCMS Anal. calculated for C₄₈H₄₇F₂N₅O₉: 875.4, found m/z: 874.6 [M-H]⁻, 876.6 [M+H]⁺, t_{R} = 2.97 min; peak area 100%.

### Example 160 [AXL22146]

### 4-ethoxy-N-[3-fluoro-4-[[6-methoxy-7-[3-[4-[(E)-4-oxo-4-phenyl-but-2-enoyl]piperazin-1-yl]propoxy]-4-quinolyl]oxy]phenyl]-1-(4-fluorophenyl)-2-oxo-pyridine-3-carboxamide

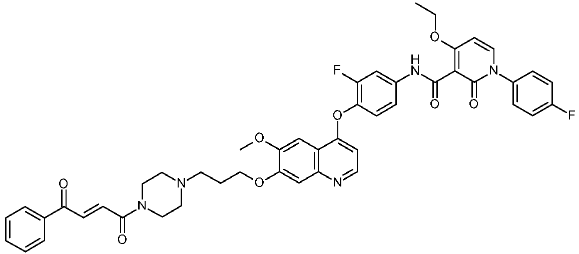

LCMS Anal. calculated for C₄₇H₄₃F₂N₅O₈: 843.3, found m/z: 842.5 [M-H]⁻, 844.5 [M+H]⁺, t_{R} = 2.94 min; peak area 100%. ¹H NMR (300 MHz, DMSO-d6): δ 10.58 (s, 1H); 8.45 (d, 1H, J=5.0 Hz); 8.45 (d, 1H, J=5.1 Hz); 8.02 (m, 1H); 7.95-7.84 (m, 2H); 7.78-7.67 (m, 2H); 7.60-7.52 (m, 3H); 7.48-7.44 (m, 4H); 7.41-7.33 (m, 4H); 6.52 (d, 1H, J=8.0 Hz); 6.46 (d, 1H, J=5.2 Hz); 4.30-4.22 (m, 5H); 3.95 (s, 3H); 3.61 (m, 4H); 3.21-3.11 (m, 4H); 2.30 (m, 2H); 1.38-1.23 (m, 4H).

Example 161 [AXL22147]

### 4-ethoxy-N-[3-fluoro-4-[[6-methoxy-7-[3-[4-[(E)-3-(2-methoxyphenyl)prop-2-enoyl]piperazin-1-yl]propoxy]-4-quinolyl]oxy]phenyl]-1-(4-fluorophenyl)-2-oxo-pyridine-3-carboxamide

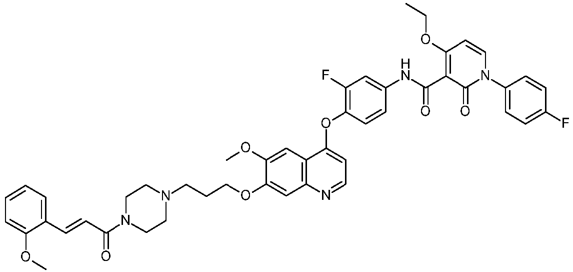

LCMS Anal. calculated for C₄₇H₄₅F₂N₅O₈: 845.3, found m/z: 844.6 [M-H]⁻, 846.6 [M+H]⁺, t_{R} = 3.02 min; peak area 100%. ¹H NMR (300 MHz, DMSO-d6): δ 10.58 (s, 1H); 8.45 (d, 1H, J=5.2 Hz); 7.95-7.70 (m, 4H); 7.54-7.33 (m, 9H); 7.18 (m, 1H); 7.05 (d, 1H, J=8.3 Hz); 6.79 (t, 2H, J=7.7 Hz); 6.52 (d, 1H, J=7.8 Hz); 6.46 (d, 1H, J=5.1 Hz); 4.30-4.16 (m, 3H); 3.95 (s, 3H); 3.85 (s, 3H); 3.68-3.59 (m, 4H); 3.20-3.03 (m, 2H); 2.43-2.27 (m, 4H); 2.01 (m, 2H); 1.31 (t, 3H, J=6.9 Hz)

### Example 162 [AXL22148]

### 4-ethoxy-N-[3-fluoro-4-[[7-[3-[4-[(E)-3-(2-hydroxyphenyl)prop-2-enoyl]piperazin-1-yl]propoxy]-6-methoxy-4-quinolyl]oxy]phenyl]-1-(4-fluorophenyl)-2-oxo-pyridine-3-carboxamide

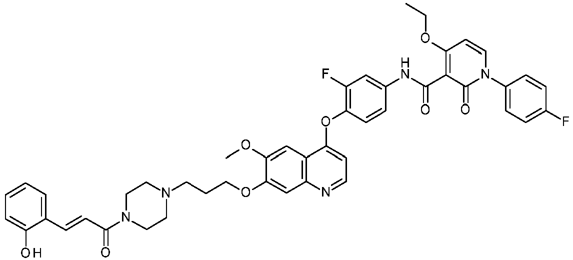

LCMS Anal. calculated for C₄₆H₄₃F₂N₅O₈: 831.4, found m/z: 830.5 [M-H]⁻, 832.6 [M+H]⁺, t_{R} = 3.92 min; peak area 98%.

### Example 163 [AXL22149]

### 4-ethoxy-N-[3-fluoro-4-[[6-methoxy-7-[3-[4-[(E)-3-[3-(trifluoromethyl)phenyl]prop-2-enoyl]piperazin-1-yl]propoxy]-4-quinolyl]oxy]phenyl]-1-(4-fluorophenyl)-2-oxo-pyridine-3-carboxamide

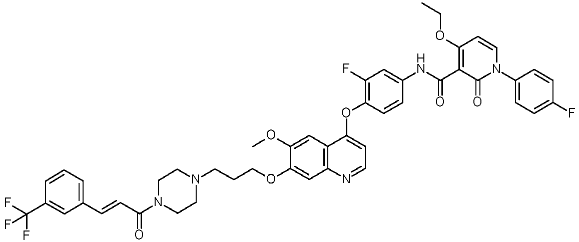

LCMS Anal. calculated for C₄₇H₄₂F₅N₅O₇: 883.4, found m/z: 882.6 [M-H]⁻, 884.6 [M+H]⁺, t_{R} = 3.33 min; peak area 100%. ¹H NMR (300 MHz, DMSO-d6): δ 10.57 (s, 1H); 8.45 (d, 1H, J=5.0 Hz); 8.15 (m, 1H); 8.07-7.84 (m, 3H); 7.74-7.59 (m, 3H); 7.54-7.31 (m, 7H); 6.52 (d, 1H, J=7.8 Hz); 6.46 (d, 1H, J=5.1 Hz); 4.30-4.23 (m, 4H); 4.16 (m, 2H); 3.95 (s, 3H); 3.75-3.51 (m, 4H); 3.20 (m, 2H); 2.45-2.27 (m, 4H); 2.02 (m, 2H); 1.30 (t, 3H, J=7.0 Hz)

### Example 164 [AXL22150]

### 4-ethoxy-N-[3-fluoro-4-[[6-methoxy-7-[3-[4-[(Z)-4-morpholino-4-oxo-but-2-enoyl]piperazin-1-yl]propoxy]-4-quinolyl]oxy]phenyl]-1-(4-fluorophenyl)-2-oxo-pyridine-3-carboxamide

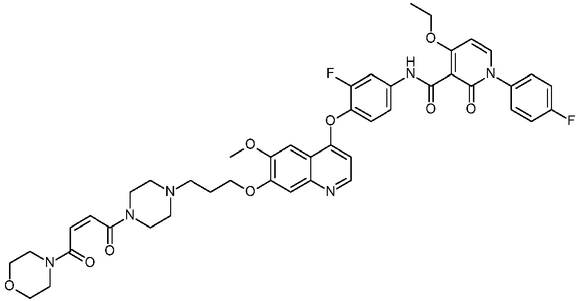

LCMS Anal. calculated for C₄₅H₄₆F₂N₆O₉: 852.3, found m/z: 851.6 [M-H]⁻, 853.5 [M+H]⁺, t_{R} = 3.37 min; peak area 98%. ¹H NMR (300 MHz, DMSO-d6): δ 10.57 (s, 1H); 8.45 (d, 1H, J=5.0 Hz); 7.93 (m, 1H); 7.85 (d, 1H, J= 7.9 Hz); 7.53-7.44 (m, 4H); 7.41-7.33 (m, 3H); 6.52 (d, 1H, J=8.0 Hz); 6.47-6.38 (m, 2H); 4.30-4.19 (m, 4H); 3.95 (s, 3H); 3.54 (m, 8H); 3.26-3.23 (m,6H); 2.40 (m, 6H); 1.98 (m, 2H); 1.31 (t, 3H, J=7.0 Hz)

### Example 165 [AXL22151]

### N-[4-[[7-[3-[4-[(Z)-4-(dimethylamino)-4-oxo-but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide

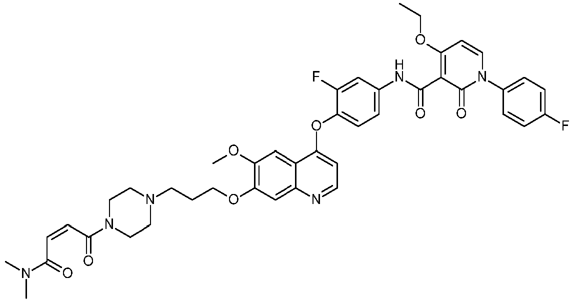

LCMS Anal. calculated for C₄₃H₄₄F₂N₆O₈: 810.3, found m/z: 809.5 [M-H]⁻, 811.5 [M+H]⁺, t_{R} = 3.36 min; peak area 97%. ¹H NMR (300 MHz, DMSO-d6): δ 10.58 (s, 1H); 8.45 (d, 1H, J=5.1 Hz); 7.93 (m, 1H); 7.85 (d, 1H, J= 7.9 Hz); 7.53-7.44 (m, 4H); 7.41-7.33 (m, 4H); 6.52 (d, 1H, J=8.0 Hz); 6.47-6.38 (m, 2H); 4.30-4.19 (m, 4H); 3.95 (s, 3H); 3.45 (m, 2H); 3.26-3.23 (m,3H); 2.94 (m, 3H); 2.81 (m, 3H); 2.40 (m, 6H); 1.98 (m, 2H); 1.31 (t, 3H, J=7.0 Hz)

### Example 166 [AXL22152]

### N-[4-[[7-[3-[4-[(Z)-4-(diethylamino)-4-oxo-but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide

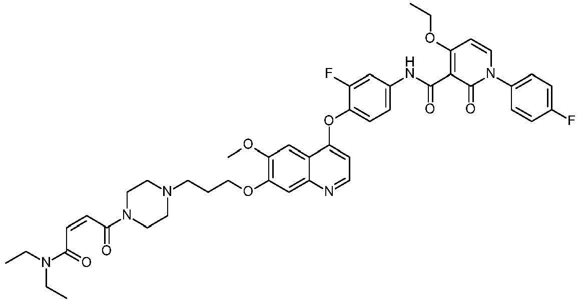

LCMS Anal. calculated for C₄₅H₄₈F₂N₆O₈: 838.4, found m/z: 837.5 [M-H]⁻, 839.6 [M+H]⁺, t_{R} = 3.62 min; peak area 97%. ¹H NMR (300 MHz, DMSO-d6): δ 10.59 (s, 1H); 8.45 (d, 1H, J=5.0 Hz); 7.91 (m, 1H); 7.85 (d, 1H, J= 7.9 Hz); 7.53-7.44 (m, 4H); 7.42-7.33 (m, 4H); 6.52 (d, 1H, J=8.0 Hz); 6.47-6.38 (m, 2H); 4.30-4.19 (m, 4H); 3.95 (s, 3H); 3.44 (m, 5H); 3.26-3.23 (m,6H); 2.40 (m, 4H); 1.98 (m, 2H); 1.31 (t, 3H, J=7.0 Hz); 1.06 (m, 6H)

### Example 167 [AXL22153]

### 4-ethoxy-N-[3-fluoro-4-[[6-methoxy-7-[3-[4-[(Z)-4-oxo-4-pyrrolidin-1-yl-but-2-enoyl]piperazin-1-yl]propoxy]-4-quinolyl]oxy]phenyl]-1-(4-fluorophenyl)-2-oxo-pyridine-3-carboxamide

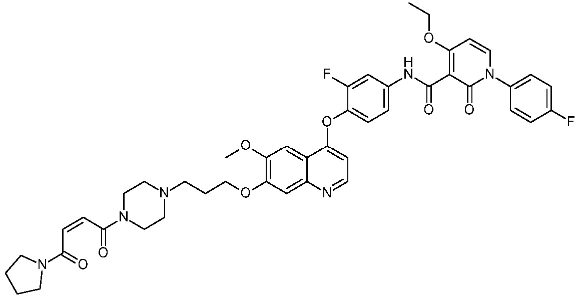

LCMS Anal. calculated for C₄₅H₄₆F₂N₆O₈: 836.4, found m/z: 835.6 [M-H]⁻, 837.6 [M+H]⁺, t_{R} = 3.61 min; peak area 98%.

### Example 168 [AXL22154]

### 4-ethoxy-N-[3-fluoro-4-[[6-methoxy-7-[3-[4-[(Z)-4-(4-methylpiperazin-1-yl)-4-oxo-but-2-enoyl]piperazin-1-yl]propoxy]-4-quinolyl]oxy]phenyl]-1-(4-fluorophenyl)-2-oxo-pyridine-3-carboxamide

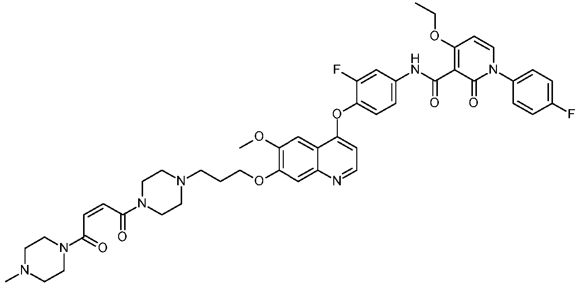

LCMS Anal. calculated for C₄₆H₄₉F₂N₇O₈: 865.5, found m/z: 864.7 [M-H]⁻, 866.7 [M+H]⁺, t_{R} = 2.39 min; peak area 97%.

### Example 169 [AXL22155]

### 4-ethoxy-N-[3-fluoro-4-[[6-methoxy-7-[3-[4-[(Z)-4-oxo-4-(1-piperidyl)but-2-enoyl]piperazin-1-yl]propoxy]-4-quinolyl]oxy]phenyl]-1-(4-fluorophenyl)-2-oxo-pyridine-3-carboxamide

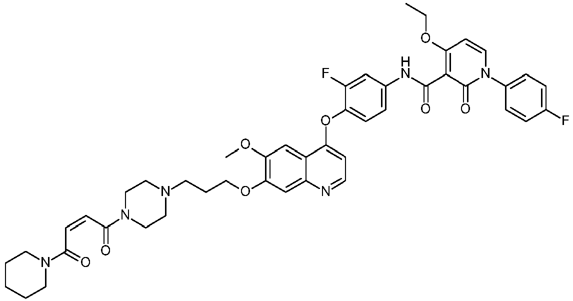

LCMS Anal. calculated for C₄₆H₄₈F₂N₆O₈: 850.4, found m/z: 849.6 [M-H]⁻, 851.6 [M+H]⁺, t_{R} = 2.77 min; peak area 100%. ¹H NMR (300 MHz, DMSO-d6): δ 10.58 (s, 1H); 8.45 (d, 1H, J=5.3 Hz); 7.91 (m, 1H); 7.85 (d, 1H, J= 7.9 Hz); 7.53-7.33 (m, 7H); 7.21 (d, 1H, J=1.5 Hz); 6.52 (d, 1H, J=7.9 Hz); 6.46 (d, 1H, J= 5.2 Hz); 4.30-4.19 (m, 4H); 3.95 (s, 3H); 3.64-3.48 (m, 7H); 3.20 (m, 4H); 2.43 (m, 5H); 1.99 (m, 2H); 1.59-1.48 (m, 6H); 1.31 (t, 3H, J=7.0 Hz)

### Example 170 [AXL22156]

### 4-ethoxy-N-[3-fluoro-4-[[7-[3-[4-[(Z)-4-[(3R)-3-hydroxypyrrolidin-1-yl]-4-oxo-but-2-enoyl]piperazin-1-yl]propoxy]-6-methoxy-4-quinolyl]oxy]phenyl]-1-(4-fluorophenyl)-2-oxo-pyridine-3-carboxamide

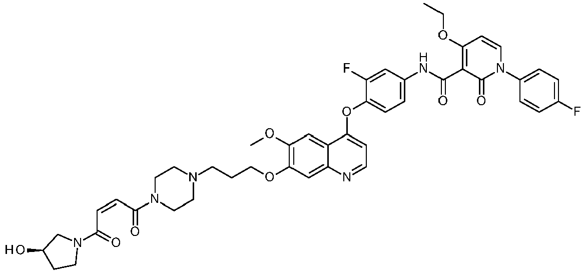

LCMS Anal. calculated for C₄₅H₄₆F₂N₆O₉: 852.4, found m/z: 851.5 [M-H]⁻, 853.6 [M+H]⁺, t_{R} = 2.48 min; peak area 100%.

### Example 171 [AXL22157]

### N-[4-[[7-[3-[4-[(Z)-4-[(3R)-3-(dimethylamino)pyrrolidin-1-yl]-4-oxo-but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide

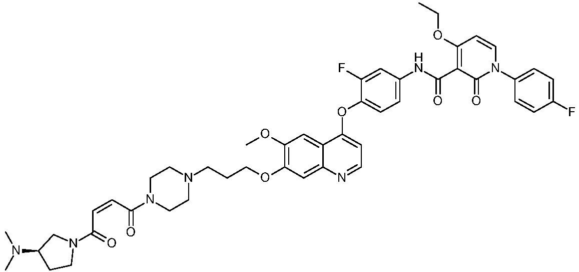

LCMS Anal. calculated for C₄₇H₅₁F₂N₇O₈: 879.5, found m/z: 878.7 [M-H]⁻, 880.7 [M+H]⁺, t_{R} = 2.28 min; peak area 100%.

### Example 172 [AXL22158]

### 4-ethoxy-N-[3-fluoro-4-[[7-[3-[4-[(Z)-4-(hydroxyamino)-4-oxo-but-2-enoyl]piperazin-1-yl]propoxy]-6-methoxy-4-quinolyl]oxy]phenyl]-1-(4-fluorophenyl)-2-oxo-pyridine-3-carboxamide

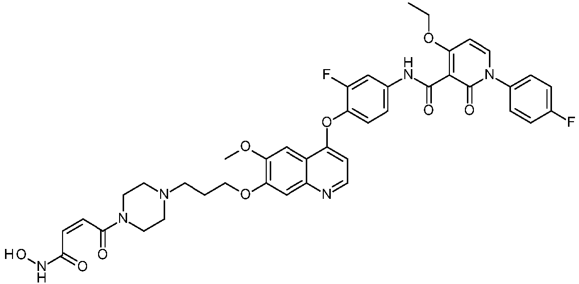

LCMS Anal. calculated for C₄₁H₄₀F₂N₆O₉: 798.3, found m/z: 797.5 [M-H]⁻, 799.5 [M+H]⁺, t_{R} = 2.42 min; peak area 100%.

### Example 173 [AXL22159]

### 4-ethoxy-N-[3-fluoro-4-[[6-methoxy-7-[3-[4-[(Z)-4-[(3R)-3-methylmorpholin-4-yl]-4-oxo-but-2-enoyl]piperazin-1-yl]propoxy]-4-quinolyl]oxy]phenyl]-1-(4-fluorophenyl)-2-oxo-pyridine-3-carboxamide

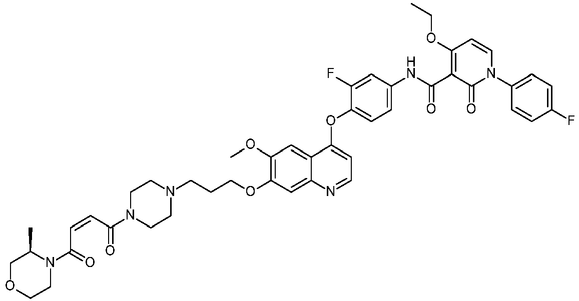

LCMS Anal. calculated for C₄₆H₄₈F₂N₆O₉: 866.3, found m/z: 865.7 [M-H]⁻, 867.7 [M+H]⁺, t_{R} = 2.50 min; peak area 100%. ¹H NMR (300 MHz, DMSO-d6): δ 10.58 (s, 1H); 8.45 (d, 1H, J=5.3 Hz); 7.94-7.83 (m, 2H); 7.53-7.31 (ovl. m, 7H); 6.53-6.40 (m, 3H); 4.31-4.19 (m, 4H); 3.95 (s, 3H); 3.78 (m, 3H); 3.61-3.55 (m, 4H); 3.20 (m, 8H); 2.40 (m, 4H); 1.99 (m, 2H); 1.33-1.11 (m, 6H)

### Example 174 [AXL22160]

### 4-ethoxy-N-[3-fluoro-4-[[6-methoxy-7-[3-[4-[(Z)-4-(4-methyl-1-piperidyl)-4-oxo-but-2-enoyl]piperazin-1-yl]propoxy]-4-quinolyl]oxy]phenyl]-1-(4-fluorophenyl)-2-oxo-pyridine-3-carboxamide

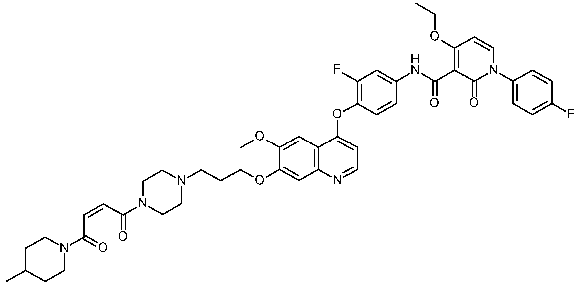

LCMS Anal. calculated for C₄₇H₅₀F₂N₆O₈: 864.4, found m/z: 863.6 [M-H]⁻, 865.6 [M+H]⁺, t_{R} = 2.73 min; peak area 100%. ¹H NMR (300 MHz, DMSO-d6): δ 10.58 (s, 1H); 8.45 (d, 1H, J=5.2 Hz); 7.92 (dd, 1H, J=13.1 Hz, J=1.51 Hz); 7.85 (d, 1H, J=7.6 Hz); 7.53 (m, 1H); 7.49-7.44 (m, 3H); 7.41-7.39 (m, 2H); 7.34 (m, 1H); 6.51 (d, 1H, J=8.0 Hz); 6.47-6.42 (m, 2H); 4.32-4.19 (m, 5H); 3.95 (s, 3H); 3.76 (m, 2H); 3.20 (m, 4H); 2.95 (m, 1H); 2.40 (m, 4H); 1.99 (m, 2H); 1.58 (m, 3H); 1.31 (t, 3H, J=6.9 Hz); 1.10-0.88 (m, 5H)

### Example 175 [AXL22161]

### N-[4-[[7-[3-[4-[(Z)-4-(azetidin-1-yl)-4-oxo-but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide

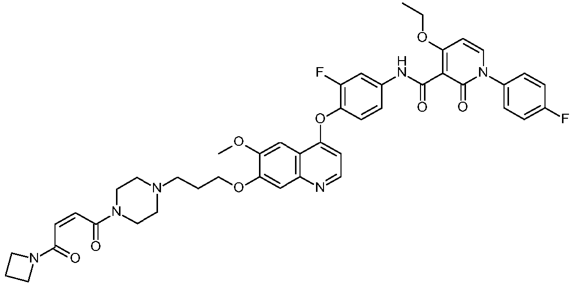

LCMS Anal. calculated for C₄₄H₄₄F₂N₆O₈: 822.3, found m/z: 821.6 [M-H]⁻, 823.6 [M+H]⁺, t_{R} = 3.46 min; peak area 97%.

### Example 176 [AXL22162]

### 4-ethoxy-N-[3-fluoro-4-[[6-methoxy-7-[3-[4-[(Z)-4-(3-methyl-1-piperidyl)-4-oxo-but-2-enoyl]piperazin-1-yl]propoxy]-4-quinolyl]oxy]phenyl]-1-(4-fluorophenyl)-2-oxo-pyridine-3-carboxamide

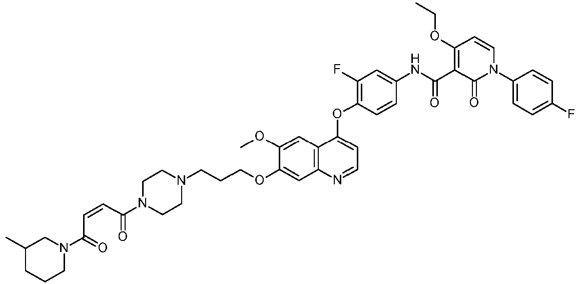

LCMS Anal. calculated for C₄₇H₅₀F₂N₆O₈: 864.5, found m/z: 863.7 [M-H]⁻, 865.7 [M+H]⁺, t_{R} = 3.83 min; peak area 98%. ¹H NMR (300 MHz, DMSO-d6): δ 11.58 (s, 1H); 8.45 (d, 1H, J=5.2 Hz); 7.92 (m, 1H); 7.85 (d, 1H, J=7.8 Hz); 7.53-7.51 (m, 1H); 7.48-7.44 (m, 3H); 7.40-7.33 (m, 3H); 6.53-6.43 (m, 4H); 4.30-4.15 (m, 5H); 3.95 (s, 3H); 3.67-3.56 (m, 4H); 2.39-2.25 (m, 5H); 3.21-3.11 (m, 4H); 1.99 (m, 2H); 1.62-1.57 (m, 4H); 1.31 (m, 4H); 1.11 (m, 1H); 0.86-0.81 (m, 3H)

### Example 177 [AXL22062]

### N-[3-fluoro-4-[[6-methoxy-7-[3-[4-[(E)-4-morpholinobut-2-enoyl]piperazin-1-yl]propoxy]-4-quinolyl]oxy]phenyl]-2-(4-fluorophenyl)-3-oxo-pyridazine-4-carboxamide

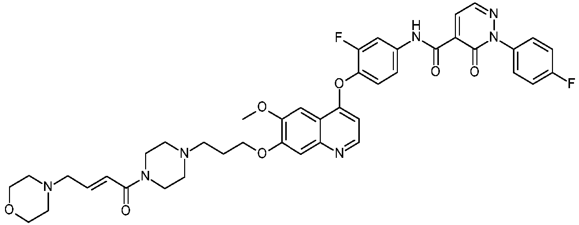

LCMS Anal. calculated for C₄₂H₄₃F₂N₇O₇: 795.3, found m/z: 794.3 [M-H]⁻, 796.3 [M+H]⁺, t_{R} = 2.61 min; peak area 100%.

### Example 178 [AXL22063]

### N-[4-[[7-[3-[4-[(E)-4-(dimethylamino)but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide

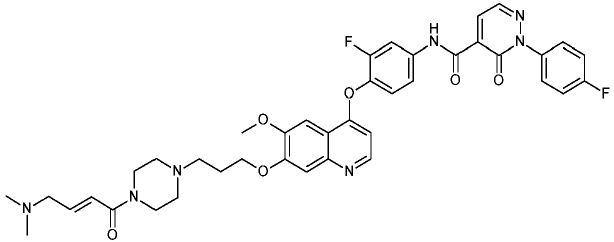

LCMS Anal. calculated for C₄₀H₄₁F₂N₇O₆: 753.3, found m/z: 752.3 [M-H]⁻, 754.3 [M+H]⁺, t_{R} = 2.55 min; peak area 100%.

### Example 179 [AXL22064]

### N-[3-fluoro-4-[[6-methoxy-7-[3-[4-[(E)-4-(1-piperidyl)but-2-enoyl]piperazin-1-yl]propoxy]-4-quinolyl]oxy]phenyl]-2-(4-fluorophenyl)-3-oxo-pyridazine-4-carboxamide

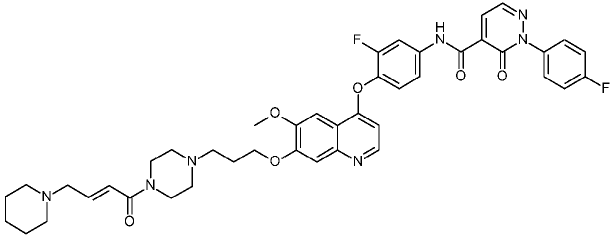

LCMS Anal. calculated for C₄₃H₄₅F₂N₇O₆: 793.3, found m/z: 792.3 [M-H]⁻, 794.4 [M+H]⁺, t_{R} = 2.69 min; peak area 100%.

### Example 180 [AXL22065]

### N-[3-fluoro-4-[[6-methoxy-7-[3-[4-[(E)-4-(4-methylpiperazin-1-yl)but-2-enoyl]piperazin-1-yl]propoxy]-4-quinolyl]oxy]phenyl]-2-(4-fluorophenyl)-3-oxo-pyridazine-4-carboxamide

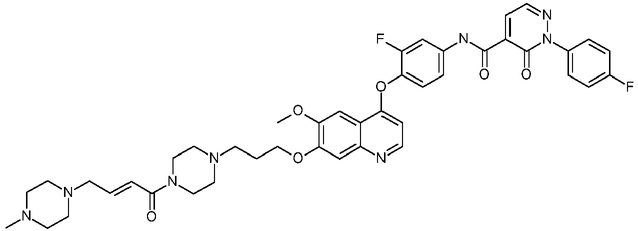

LCMS Anal. calculated for C₄₃H₄₆F₂N₈O₆: 808.4, found m/z: 807.3 [M-H]⁻, 809.3 [M+H]⁺, t_{R} = 2.65 min; peak area 97%.

### Example 181 [AXL22084]

### N-[3-fluoro-4-[[6-methoxy-7-[3-(4-prop-2-enoylpiperazin-1-yl)propoxy]-4-quinolyl]oxy]phenyl]-2-(4-fluorophenyl)-3-oxo-pyridazine-4-carboxamide

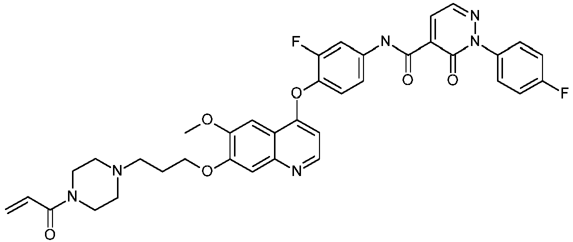

LCMS Anal. calculated for C₃₇H₃₄F₂N₆O₆: 696.3, found m/z: 695.3 [M-H]⁻, 697.3 [M+H]⁺, t_{R} = 2.97 min; peak area 99%.

### Example 182 [AXL22108]

### N-[4-[[7-[3-[4-[(E)-4-(dimethylamino)but-2-enoyl]piperazin-1-yl]propoxy]-6-methoxy-4-quinolyl]oxy]-3-fluoro-phenyl]-3-oxo-2-[4-(trifluoromethyl)phenyl]pyridazine-4-carboxamide

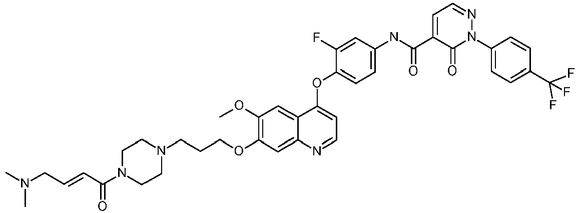

LCMS Anal. calculated for C₄₁H₄₁F₄N₇O₆: 803.3, found m/z: 802.4 [M-H]⁻, 804.5 [M+H]⁺, t_{R} = 2.76 min; peak area 97%.

### Example 183 [AXL22109]

### N-[3-fluoro-4-[[6-methoxy-7-[3-(4-prop-2-enoylpiperazin-1-yl)propoxy]-4-quinolyl]oxy]phenyl]-3-oxo-2-[4-(trifluoromethyl)phenyl]pyridazine-4-carboxamide

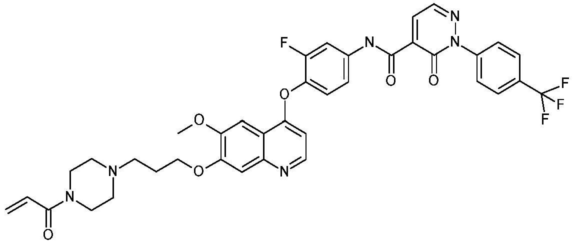

LCMS Anal. calculated for C₃₈H₃₄F₄N₆O₆: 746.2, found m/z: 745.4 [M-H]⁻, 747.4 [M+H]⁺, t_{R} = 3.08 min; peak area 99%.

### Example 184 [AXL22110]

### N-[3-fluoro-4-[[6-methoxy-7-[3-[4-[(E)-4-(1-piperidyl)but-2-enoyl]piperazin-1-yl]propoxy]-4-quinolyl]oxy]phenyl]-3-oxo-2-[4-(trifluoromethyl)phenyl]pyridazine-4-carboxamide

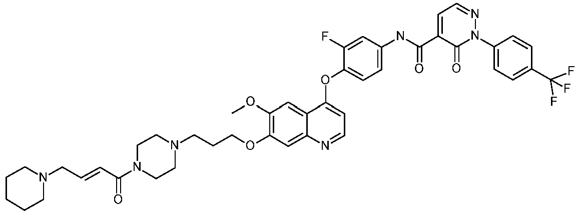

LCMS Anal. calculated for C₄₄H₄₅F₄N₇O₆: 843.3, found m/z: 842.5 [M-H]⁻, 844.5 [M+H]⁺, t_{R} = 2.81 min; peak area 98%.

### Example 185 [AXL22171]

### (Z)-4-[3-[[4-[2-fluoro-4-[[2-(4-fluorophenyl)-3-oxo-pyridazine-4-carbonyl]amino]phenoxy]-6-methoxy-7-quinolyl]oxy]propylamino]-4-oxo-but-2-enoic acid

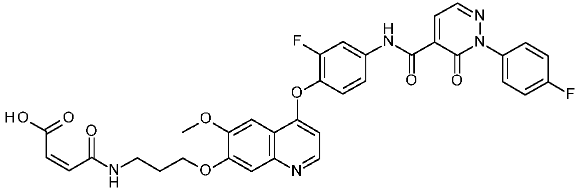

573 mg (1 mmol) N-(4-{[7-(3-aminopropoxy)-6-methoxyquinolin-4-yl]oxy}-3-fluorophenyl)-2-(4-fluorophenyl)-3-oxo-2,3-dihydropyridazine-4-carboxamide was dissolved in 20 ml anhydrous acetonitrile at 50-60 °C and 98mg (1 mmol) maleic anhydride was added to the solution, and refluxed for 5 mins. The solvent was removed under reduced pressure and the crude product was used in the next step without purification. LCMS Anal. calculated for C₃₄H₂₇F₂N₅O₈: 671.3, found m/z: 670.4 [M-H]⁻, 672.4 [M+H]⁺, t_{R} = 3.46 min; peak area 100%.

### Example 186 [AXL22172]

### (Z)-N-[3-[[4-[2-fluoro-4-[[2-(4-fluorophenyl)-3-oxo-pyridazine-4-carbonyl]amino]phenoxy]-6-methoxy-7-quinolyl]oxy]propyl]-N',N'-dimethyl-but-2-enediamide

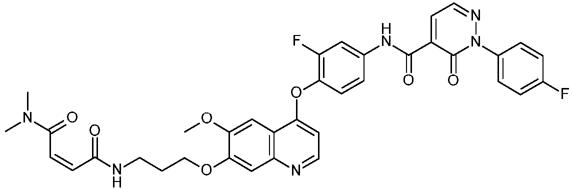

LCMS Anal. calculated for C₃₆H₃₂F₂N₆O₇: 698.3, found m/z: 697.5 [M-H]⁻, 699.5 [M+H]⁺, t_{R} = 3.29 min; peak area 100%. ¹H NMR (300 MHz, DMSO-d6): δ 11.68 (s, 1H); 8.48 (d, 1H, J=5.0 Hz); 8.37 (d, 1H, J=3.9 Hz); 8.29-8.25 (m, 2H); 8.02 (dd, 1H, J=11.0 Hz, J=1.9 Hz); 7.70-7.66 (m, 2H); 7.61-7.58 (m, 1H); 7.53 (s, 1H); 7.48 (m, 1H); 7.45-7.38 (m, 3H); 6.48 (d, 1H, J=4.9 Hz); 6.35 (d, 1H, J=11.9 Hz); 6.00 (d, 1H, J= Hz); 4.19 (t, 2H, J=5.8 Hz); 3.95 (s, 3H); 3.20 (m, 2H); 2.83-2.81 (m, 6H); 1.98 (m, 2H)

### Example 187 [AXL22175]

### N-[3-fluoro-4-[[6-methoxy-7-[3-[[(Z)-4-morpholino-4-oxo-but-2-enoyl]amino]propoxy]-4-quinolyl]oxy]phenyl]-2-(4-fluorophenyl)-3-oxo-pyridazine-4-carboxamide

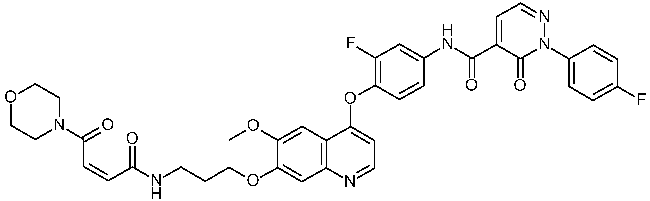

LCMS Anal. calculated for C₃₈H₃₄F₂N₆O₈: 740.3, found m/z: 739.5 [M-H]⁻, 741.5 [M+H]⁺, t_{R} = 3.30 min; peak area 100%. ¹H NMR (300 MHz, DMSO-d6): δ 11.68 (s, 1H); 8.48 (d, 1H, J=5.0 Hz); 8.37 (d, 1H, J=3.9 Hz); 8.29-8.25 (m, 2H); 8.02 (dd, 1H, J=11.0 Hz, J=1.9 Hz); 7.70-7.66 (m, 2H); 7.59 (m, 1H); 7.54 (s, 1H); 7.46 (m, 1H); 7.45-7.38 (m, 3H); 6.49 (d, 1H, J=5.0 Hz); 6.35 (d, 1H, J=11.9 Hz); 6.04 (d,1H, J=11.9 Hz); 4.29 (t, 2H, J=5.8 Hz); 3.95 (s, 3H); 3.56-3.49 (m, 5H); 3.45-3.42 (m, 3H); 3.20 (m, 2H); 1.98 (m,2H)

Example 188 [AXL22176]

### N-[3-fluoro-4-[[6-methoxy-7-[3-[[(Z)-4-oxo-4-(1-piperidyl)but-2-enoyl]amino]propoxy]-4-quinolyl]oxy]phenyl]-2-(4-fluorophenyl)-3-oxo-pyridazine-4-carboxamide

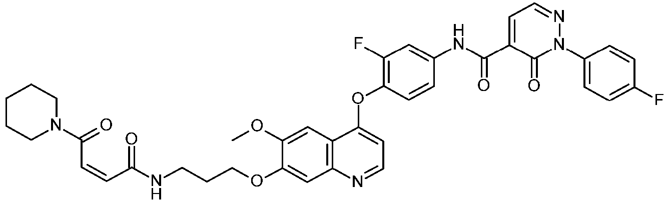

LCMS Anal. calculated for C₃₉H₃₆F₂N₆O₇: 738.3, found m/z: 737.6 [M-H]⁻, 739.6 [M+H]⁺, t_{R} = 3.54 min; peak area 100%. ¹H NMR (300 MHz, DMSO-d6): δ 11.68 (s, 1H); 8.48 (d, 1H, J=5.0 Hz); 8.37 (d, 1H, J=3.9 Hz); 8.26 (m, 1H,); 8.21 (m, 1H); 8.02 (dd, 1H, J=11.0 Hz, J=1.9 Hz); 7.71-7.66 (m, 2H); 7.59 (m, 1H); 7.54 (s, 1H); 7.48 (m, 1H); 7.45-7.38 (m, 3H); 6.50 (d, 1H, J=5.3 Hz); 6.34 (d, 1H, J=12.0 Hz); 6.00 (d, 1H, J=12.0 Hz); 4.19 (t, 1H, J=6.0 Hz); 3.95 (s, 3H); 3.40-3.35 (m, 2H); 3.25-3.23 (m, 2H); 1.98 (m, 2H); 1.50-1.43 (m, 6H)

### Example 189 [AXL22177]

### N-[3-fluoro-4-[[6-methoxy-7-[3-[[(Z)-4-(4-methylpiperazin-1-yl)-4-oxo-but-2-enoyl]amino]propoxy]-4-quinolyl]oxy]phenyl]-2-(4-fluorophenyl)-3-oxo-pyridazine-4-carboxamide

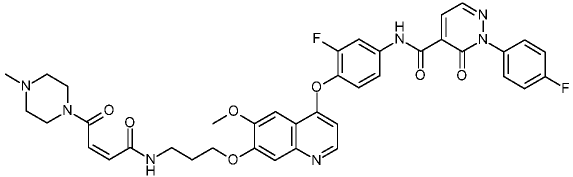

LCMS Anal. calculated for C₃₉H₃₇F₂N₇O₇: 753.4, found m/z: 752.6 [M-H]⁻, 754.6 [M+H]⁺, t_{R} = 2.95 min; peak area 98%. ¹H NMR (300 MHz, DMSO-d6): δ 11.68 (s, 1H); 8.48 (d, 2H, J=5.0 Hz); 8.37 (d, 1H, J=3.9 Hz); 8.26 (m, 2H,); 8.02 (dd, 1H, J=11.0 Hz, J=1.9 Hz); 7.71-7.66 (m, 2H); 7.59 (m, 1H); 7.53-7.38 (m, 5H); 6.48 (d, 1H, J=5.1 Hz); 6.34 (d, 1H, J= 11.8Hz); 4.18 (t, 2H, J=6.0 Hz); 3.95 (s, 3H); 2.45-2.33 (m, 6H); 2.31 (m, 4H); 2.19 (m, 3H); 1.98 (m, 2H)

Example 190 [AXL22188]

### N-[3-fluoro-4-[[6-methoxy-7-[3-[[(Z)-4-oxo-4-pyrrolidin-1-yl-but-2-enoyl]amino]propoxy]-4-quinolyl]oxy]phenyl]-2-(4-fluorophenyl)-3-oxo-pyridazine-4-carboxamide

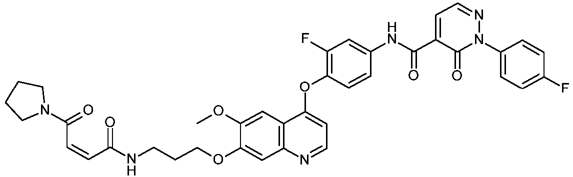

LCMS Anal. calculated for C₃₈H₃₄F₂N₆O₇: 724.2, found m/z: 723.5 [M-H]⁻, 725.5 [M+H]⁺, t_{R} = 3.31 min; peak area 100%. ¹H NMR (300 MHz, DMSO-d6): δ 11.68 (s, 1H); 8.47 (m, 2H); 8.37 (d, 1H, J=3.9 Hz); 8.26 (d, 1H, J=4.0 Hz); 8.02 (dd, 1H, J=11.0 Hz, J=1.9 Hz); 7.71-7.66 (m, 2H); 7.59 (m, 1H); 7.53-7.48 (m, 2H); 7.45-7.38 (m, 3H); 6.48 (d, 1H, J=5.4 Hz); 6.35 (d, 1H, J= 12.0 Hz); 6.02 (d, 1H, J= 12.3Hz); 4.19 (t, 2H, J=6.0 Hz);3.95 (s, 3H); 3.50 (m, 2H); 3.20 (m, 4H); 1.98 (m, 2H); 1.71-1.60 (m, 4H)

### Example 191 [AXL22189]

### (Z)-N',N'-diethyl-N-[3-[[4-[2-fluoro-4-[[2-(4-fluorophenyl)-3-oxo-pyridazine-4-carbonyl]amino]phenoxy]-6-methoxy-7-quinolyl]oxy]propyl]but-2-enediamide

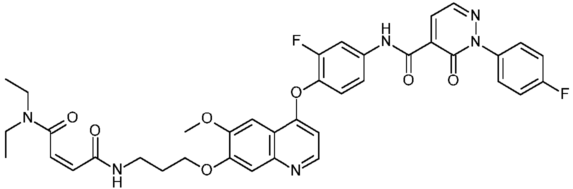

LCMS Anal. calculated for C₃₈H₃₆F₂N₆O₇: 726.2, found m/z: 725.5 [M-H]⁻, 727.5 [M+H]⁺, t_{R} = 3.38 min; peak area 100%. ¹H NMR (300 MHz, DMSO-d6): δ 11.68 (s, 1H); 8.48 (d, 1H, J=5.3 Hz); 8.37 (d, 1H, J=4.0 Hz); 8.26 (d, 1H, J=4.0 Hz); 8.02 (dd, 1H, J=11.0 Hz, J=1.9 Hz);7.71-7.66 (m, 2H); 7.59 (m, 1H); 7.54-7.48 (m, 2H); 7.45-7.38 (m, 3H); 6.40 (m, 1H); 6.51 (m, 1H); 6.49 (d, 1H, J=5.2 Hz); 4.19 (t, 2H, J= 6.0 Hz); 3.95 (s, 3H); 3.21-3.18 (m, 7H); 1.99 (m, 2H); 1.14-0.98 (m, 6H)

### Example 192 [AXL22190]

### N-[4-[[7-[3-[[(Z)-4-(azetidin-1-yl)-4-oxo-but-2-enoyl]amino]propoxy]-6-methoxy-4-quinolyl]oxy]-3-fluoro-phenyl]-2-(4-fluorophenyl)-3-oxo-pyridazine-4-carboxamide

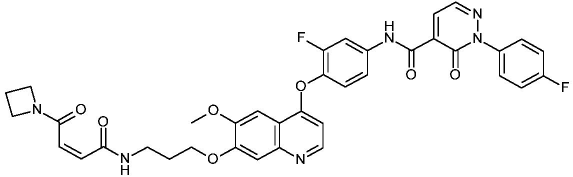

LCMS Anal. calculated for C₃₇H₃₂F₂N₆O₇: 710.2, found m/z: 709.5 [M-H]⁻, 711.5 [M+H]⁺, t_{R} = 3.24 min; peak area 99%. ¹H NMR (300 MHz, DMSO-d6): δ 11.68 (s, 1H); 8.47 (d, 1H, J=5.2 Hz); 8.37 (d, 1H, J=3.9 Hz); 8.26 (d, 1H, J=4.0 Hz); 8.02 (dd, 1H, J=11.0 Hz, J=1.9 Hz); 7.71-7.66 (m, 2H); 7.60-7.48 (m, 3H); 7.45-7.38 (m, 3H); 6.48 (m, 1H); 6.10 (m, 1H); 4.28-4.19 (m, 4H); 4.02-3.83 (m, 6H); 3.21 (m, 2H); 2.26-2.12 (m, 2H); 2.00 (m, 2H)

### Example 193 [AXL22191]

### N-[4-[[7-[3-[4-[(E)-4-(diethylamino)but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide

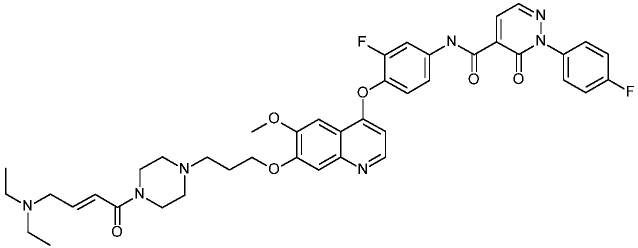

LCMS Anal. calculated for C₄₂H₄₅F₂N₇O₆: 781.5, found m/z: 780.6 [M-H]⁻, 782.6 [M+H]⁺, t_{R} = 2.54 min; peak area 100%. ¹H NMR (300 MHz, DMSO-d6): δ 11.68 (s, 1H); 8.47 (d, 1H, J=5.2 Hz); 8.37 (d, 1H, J=3.9 Hz); 8.26 (d, 1H, J=4.0 Hz); 8.02 (dd, 1H, J=11.0 Hz, J=1.9 Hz); 7.71-7.66 (m, 2H); 7.58 (m, 2H); 7.53-7.48 (m, 2H); 7.44-7.38 (m, 3H); 4.21 (t, 2H, J= 6.0Hz); 3.95 (s, 3H); 3.54-3.50 (m, 10H); 3.18 (m, 4H); 2.46-2.41 (m, 6H); 1.98 (m, 2H); 1.23-0.93 (m, 4H)

### Example 194 [AXL22192]

### (Z)-4-[4-[3-[[4-[2-fluoro-4-[[2-(4-fluorophenyl)-3-oxo-pyridazine-4-carbonyl]amino]phenoxy]-6-methoxy-7-quinolyl]oxy]propyl]piperazin-1-yl]-4-oxo-but-2-enoic acid

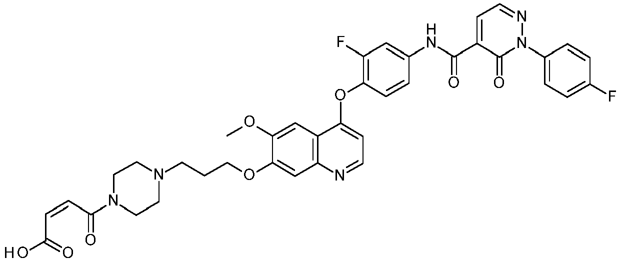

LCMS Anal. calculated for C₃₈H₃₄F₂N₆O₈: 740.2, found m/z: 739.5 [M-H]⁻, 741.5 [M+H]⁺, t_{R} = 2.78 min; peak area 100%. ¹H NMR (300 MHz, DMSO-d6): δ 11.68 (s, 1H); 8.47 (d, 1H, J=5.2 Hz); 8.37 (d, 1H, J=3.9 Hz); 8.26 (d, 1H, J=4.0 Hz); 8.02 (dd, 1H, J=11.0 Hz, J=1.9 Hz); 7.71-7.66 (m, 2H); 7.59 (m, 1H); 7.53-7.49 (m, 2H); 7.44-7.38 (m, 3H); 6.65 (m, 1H); 6.48 (d, 1H, J=4.9 Hz); 5.97 (m, 1H); 4.21 (t, 2H, J=6.0 Hz); 3.95 (s, 3H); 3.49-3.46 (m, 4H); 2.59 (m, 2H); 2.41 (m, 4H); 1.98 (t, 2H, J=6.3 Hz)

### Example 195 [AXL22193]

### N-[4-[[7-[3-[4-[(Z)-4-(dimethylamino)-4-oxo-but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide

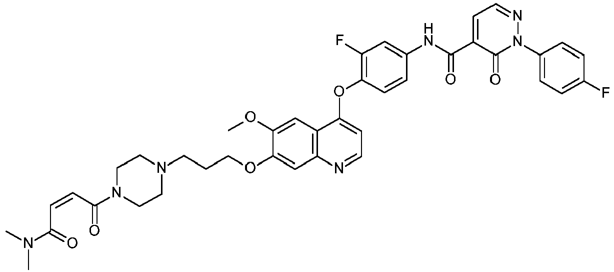

LCMS Anal. calculated for C₄₀H₃₉F₂N₇O₇: 767.4, found m/z: 766.6 [M-H]⁻, 768.6 [M+H]⁺, t_{R} = 2.78 min; peak area 100%. ¹H NMR (300 MHz, DMSO-d6): δ 11.68 (s, 1H); 8.47 (d, 1H, J=4.9 Hz); 8.37 (d, 1H); 8.26 (d, 1H); 8.02 (dd, 1H, J=10.8 Hz, J=1.9 Hz) 7.71 -7.66 (m, 2H); 760-7.51 (m, 2H); 7.45-7.38 (m, 4H); 6.48 (d, 1H); 6.45 (d, 1H); 4.21 (t, 2H, J=6.0 Hz); 3.94 (s, 3H); 2.93 (m, 3H); 2.82 (s, 3H); 2.39 (m, 6H); 1.98 (m, 2H)

### Example 196 [AXL22194]

### N-[4-[[7-[3-[4-[(Z)-4-(diethylamino)-4-oxo-but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide

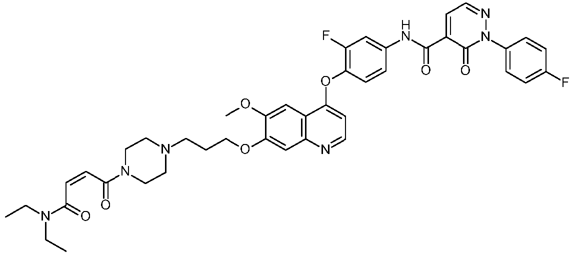

LCMS Anal. calculated for C₄₂H₄₃F₂N₇O₇: 795.3, found m/z: 794.6 [M-H]⁻, 796.6 [M+H]⁺, t_{R} = 2.91 min; peak area 100%.

### Example 197 [AXL22195]

### N-[3-fluoro-4-[[6-methoxy-7-[3-[4-[(Z)-4-morpholino-4-oxo-but-2-enoyl]piperazin-1-yl]propoxy]-4-quinolyl]oxy]phenyl]-2-(4-fluorophenyl)-3-oxo-pyridazine-4-carboxamide

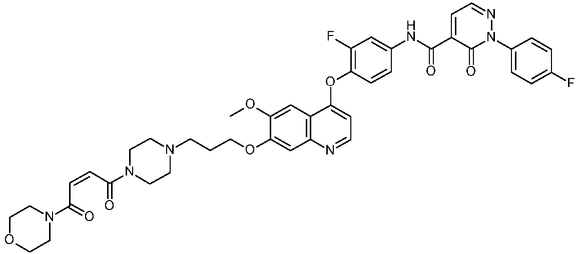

LCMS Anal. calculated for C₄₂H₄₁F₂N₇O₈: 809.3, found m/z: 808.5 [M-H]⁻, 810.6 [M+H]⁺, t_{R} = 2.78 min; peak area 100%. ¹H NMR (300 MHz, DMSO-d6): δ 11.68 (s, 1H); 8.47 (d, 1H, J=4.9 Hz); 8.37 (d, 1H); 8.26 (d, 1H); 8.02 (dd, 1H); 7.71-7.66 (m, 2H);7.51 (m, 3H); 7.45-7.38 (m, 3H); 6.49 (m, 2H); 4.21 (t, 2H); 3.95 (s, 3H); 3.62-3.54 (m, 7H); 3.21 (m, 6H); 2.40 (m, 4H); 1.99 (t, 2H)

### Example 198 [AXL22196]

### N-[3-fluoro-4-[[6-methoxy-7-[3-[4-[(Z)-4-(4-methylpiperazin-1-yl)-4-oxo-but-2-enoyl]piperazin-1-yl]propoxy]-4-quinolyl]oxy]phenyl]-2-(4-fluorophenyl)-3-oxo-pyridazine-4-carboxamide

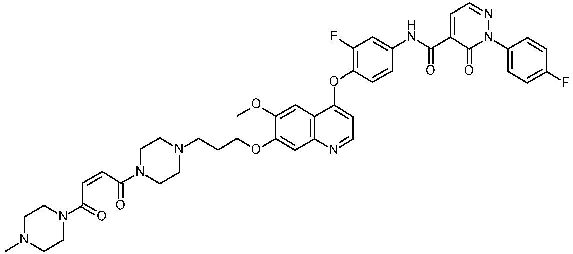

LCMS Anal. calculated for C₄₃H₄₄F₂N₂O₇: 822.3, found m/z: 821.6 [M-H]⁻, 823.6 [M+H]⁺, t_{R} = 2.50 min; peak area 100%. ¹H NMR (300 MHz, DMSO-d6): δ 11.68 (s, 1H); 8.47 (d, 1H, J=4.8 Hz); 8.38 (d, 1H); 8.26 (d, 1H); 8.02 (m, 1H); 7.71-7.66 (m, 2H);7.58 (m, 1H); 7.53-7.38 (m, 5H); 6.48 (d, 1H); 6.43 (m, 1H); 4.20 (t, 2H, J=6.2 Hz); 3.95 (s, 3H); 3.88 (s, 3H); 3.30-3.21 (m, 5H); 2.39-2.17 (m, 12H); 2.00 (m, 2H)

### Example 199 [AXL22197]

### N-[3-fluoro-4-[[6-methoxy-7-[3-[4-[(Z)-4-oxo-4-(1-piperidyl)but-2-enoyl]piperazin-1-yl]propoxy]-4-quinolyl]oxy]phenyl]-2-(4-fluorophenyl)-3-oxo-pyridazine-4-carboxamide

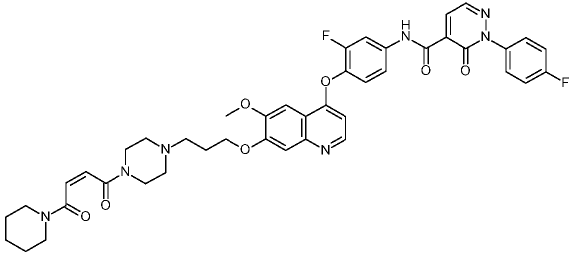

LCMS Anal. calculated for C₄₃H₄₃F₂N₇O₇: 807.3, found m/z: 806.6 [M-H]⁻, 808.6 [M+H]⁺, t_{R} = 2.92 min; peak area 100%. ¹H NMR (300 MHz, DMSO-d6): δ 11.68 (s, 1H); 8.47 (d, 1H, J=4.8 Hz); 8.38 (d, 1H); 8.26 (d, 1H); 8.02 (dd, 1H); 7.71-7.66 (m, 2H); 7.59 (d, 1H); 7.53-7.38 (m, 4H); 6.48 (d, 1H); 6.43 (m, 1H); 4.21 (t, 2H, J=6.0 Hz); 3.95 (s, 3H); 3.60-3.30 (m, 10H); 3.20 (m, 3H); 2.41 (m, 3H); 1.99 (m, 2H); 1.55-1.46 (m, 4H)

### Example 200 [AXL22198]

### N-[4-[[7-[3-[4-[(Z)-4-(azetidin-1-yl)-4-oxo-but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide

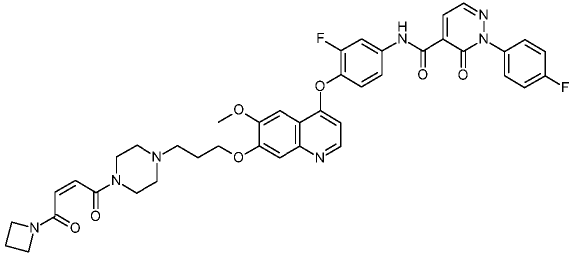

LCMS Anal. calculated for C₄₁H₃₉F₂N₇O₇: 779.3, found m/z: 778.6 [M-H]⁻, 780.6 [M+H]⁺, t_{R} = 2.82 min; peak area 97%. ¹H NMR (300 MHz, DMSO-d6): δ 11.68 (s, 1H); 8.47 (d, 1H, J=4.8 Hz); 8.37 (d, 1H); 8.26 (d, 1H); 8.02 (dd, 1H); 7.71-7.66 (m, 2H); 7.59 (d, 1H); 7.53-7.38 (m, 4H); 6.48 (d, 1H, J=5.2Hz); 6.43 (m, 1H); 4.21 (t, 2H, J=6.0 Hz); 3.94 (s, 3H); 3.21 (m, 8H); 2.51-2.40 (m, 9H); 1.99(t, 1H, J=6.0 Hz); 1.57-1.46 (m, 2H)

### Example 201 [AXL22199]

### N-[3-fluoro-4-[[6-methoxy-7-[3-[4-[(Z)-4-oxo-4-pyrrolidin-1-yl-but-2-enoyl]piperazin-1-yl]propoxy]-4-quinolyl]oxy]phenyl]-2-(4-fluorophenyl)-3-oxo-pyridazine-4-carboxamide

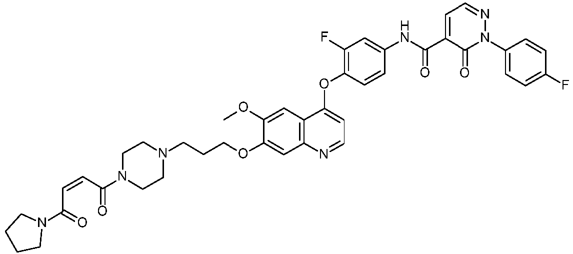

LCMS Anal. calculated for C₄₂H₄₁F₂N₇O₇: 793.3, found m/z: 792.6 [M-H]⁻, 794.6 [M+H]⁺, t_{R} = 2.85 min; peak area 99%. ¹H NMR (300 MHz, DMSO-d6): δ 11.68 (s, 1H); 8.47 (d, 1H); 8.37 (d, 1H); 8.26 (d, 1H); 8.02 (dd, 1H); 7.68 (m, 2H); 7.71-7.66(m, 2H); 7.61 (m, 3H); 6.48 (d, 1H, J=5.0 Hz); 6.37 (m, 1H); 4.21 (t, 2H, J=6.0 Hz); 3.95 (s, 3H); 3.57-3.41 (ov.m, 4H); 3.20 (m, 2H); 2.42 (m, 4H); 1.99 (t, 2H, J=6.0 Hz); 1.90-1.73 (m, 6H)

### Example 202 [AXL22200]

### N-[4-[[7-[3-[4-[(Z)-4-[(3R)-3-(dimethylamino)pyrrolidin-1-yl]-4-oxo-but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide

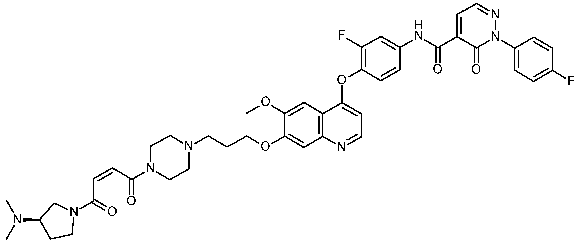

LCMS Anal. calculated for C₄₄H₄₆F₂N₈O₇: 836.3, found m/z: 835.6 [M-H]⁻, 837.6 [M+H]⁺, t_{R} = 2.52 min; peak area 100%. ¹H NMR (300 MHz, DMSO-d6): δ 11.68 (s, 1H); 8.47 (d, 1H, J=5.2 Hz); 8.37 (d, 1H, J=4.0Hz); 8.26 (d, 1H, J=4.2Hz); 8.02 (dd, 1H, J=10.0 Hz, J=1.8 Hz); 7.71-7.66 (m, 2H); 7.58 (m, 1H); 7.52-7.38 (m, 4H); 6.48 (d, 1H, J=5.2 Hz); 6.44-6.30 (m, 1H); 4.21 (t, 2H, J=5.7 Hz); 3.95 (s, 3H); 3.72-3.55 (m, 8H); 2.99 (s, 6H);2.66 (m, 2H); 2.39 (m, 4H); 2.15 (m, 5H); 1.98 (t, 2H)

### Example 203 [AXL22132]

### N-(3-fluoro-4-{[6-methoxy-7-(3-{[(2E)-4-piperidin-1-ylbut-2-enoyl]amino}propoxy)quinolin-4-yl]oxy}phenyl)-1-(4-fluorophenyl)-4-methoxy-2-oxo-1,2-dihydropyridine-3-carboxamide

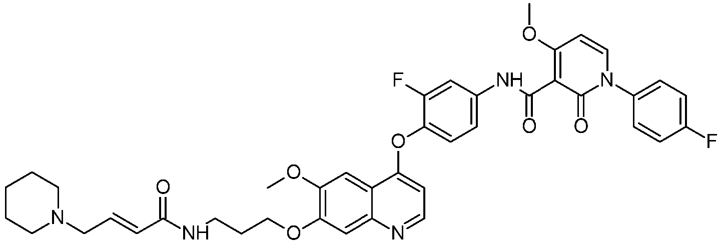

To a solution of 60 mg (0.98mmol) N-(4-{[7-(3-aminopropoxy)-6-methoxyquinolin-4-yl]oxy}-3-fluorophenyl)-4-ethoxy-1-(4-fluorophenyl)-2-oxo-1,2-dihydropyridine-3-carboxamide in anhydrous DMF (2 ml), 51 microliter DIPEA, 40mg HBTU (0.11 mmol) and (19 mg, 0.98 mmol) (2E)-4-piperidin-1-ylbut-2-enoic acid *HCl were added to the solution and stirred for 2 hours at room temperature. The reaction mixture was evaporated under pressure. The residue was taken up with aq. 20% K₂CO₃ and extracted with 2x10 ml EtOAc. The organic layer was separated and dried over sicc. Na₂SO₄. The oily residue was dissolved in methanol and evaporated onto 300 mg Kieselgel. The product was purified by column chromatograpy (chloroform/ methanol (6 N) ( 25:1) We have perceived that the ethoxy group was significantly replaced by methoxy group then title compound was purified by HPLC chromatography. t_{R} = 2.61 min [M-H]⁺ 754.4, M-H]⁻ 752.5

### Example 204 [AXL22024]

### N-[4-[[7-(3-[4-[(2E)-3-(3,4-dihydroxyphenyl)prop-2-enoyl]piperazin-1-yl]propoxy]-6-methoxyquinolin-4-yl]oxy]-3-fluorophenyl]-2-(trifluoromethyl)benzenesulfonamide

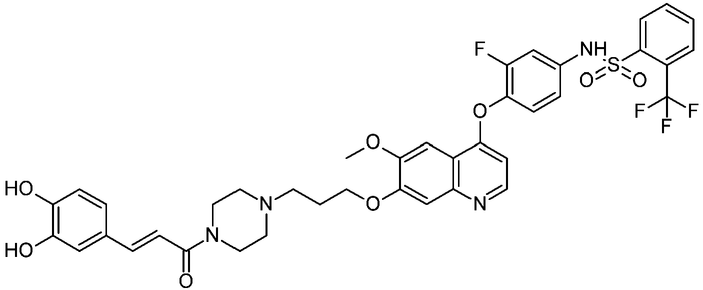

To the solution of caffeic acid (8.11 mg 0.045 mmol), N-[3-fluoro-4-[[6-methoxy-7-(3-piperazin-1-ylpropoxy)-4-quinolyl]oxy]phenyl]-2-(trifluoromethyl)benzenesulfonamide (I7B2, 22.74 mg, 0.036 mmol) and HBTU (32.26 mg, 0.085 mmol) in DMSO (1.6 mL) DIPEA (17.50 mg, 0.135 mmol) was added. The mixture was stirred for 48 hours at room temperature. The mixture was purified by reverse phase chromatography. Thus, 0.71 mg title compound was obtained as a white solid after lyophilization. Yield: 3%. LCMS Anal. calculated for C₃₉H₃₆F₄N₄O₈S: 796.2, found m/z: 795.2 [M-H]-, 797.2 [M+H]+, t_{R} = 2.98 min; peak area 96%.

### AXL in vitro kinase assay protocol

The compounds were dissolved in DMSO to make 5 mM stock solutions. 12-point dilution series were made from the stock solutions in 3-fold dilution steps (5 mM to 28.225 nM) using DMSO on 384-well polypropylene microplates (Greiner 784201). Reaction buffer was prepared in Eppendorf tubes, consisting of HEPES pH = 8 (20 mM final concentration), DTT (1 mM final concentration), MgCl₂ (10 mM final concentration) and Brij35 (0.01 V/V% final concentration) in H₂O. Substrate + ATP solution was prepared by adding a fluorescent Axl substrate (TAMRA-KKVVALYDYMPMN-NH2; 400 nM final concentration) and ATP (46.34 µM final concentration ~ ATP K_{Mapp} value) to the Reaction buffer. Enzyme solution was prepared by adding the AXL kinase (2.5 to 20 nM final concentration) to the reaction buffer. 6 µL of Substrate + ATP solution was added to each well of the assay plate (Greiner 784900 - black, small volume, polystyrene, flat bottom). 0.0125 nL from each concentration of the dilution series of each compound was added to the appropriate assay plate wells using a pin tool mounted on the RoMa gripper arm of a Tecan Freedom EVO workstation. DMSO without inhibitor was added to positive (MAX - "100% kinase activity") and negative (MIN - "0% kinase activity") control wells. 2 µL of Enzyme solution was added to each well except for the MIN wells to start the kinase reaction; 2 µL of Reaction buffer was added to the MIN wells. The assay plate was covered and the enzyme reaction was incubated on room temperature for 60 or 120 minutes (depending on final Axl concentration). The kinase reaction was stopped by addition of 10 µl IMAP detection mixture (17% IMAP Binding Buffer A, 3% IMAP Binding Buffer B, 0.25% IMAP Binding reagent in H₂O). Fluorescence Polarization (FP) and Fluorescence Intensity (FI) measurements were performed on Tecan Infinite M1000 Pro reader 60 minutes after addition of the IMAP detection mixture. IC₅₀ values were calculated by a curve fitting the FP data points using XLfit 5.1; FI was used to identify any compounds with fluorescent properties that may interfere with the detection that could would provide false data.

**Table: In vitro IC₅₀ values of the compounds on he AXL kinase.**

| Batch ID | Average IC₅₀ (nM) |
|---|---|
| Example 1 [AXL22001] | 2.8 |
| Example 2 [AXL22002] | 2.5 |
| Example 3 [AXL22005] | 6.2 |
| Example 4 [AXL22006] | 2.6 |
| Example 5 [AXL22007] | 11.6 |
| Example 6 [AXL22008] | 6.2 |
| Example 7 [AXL22009] | 4.7 |
| Example 8 [AXL22010] | 19.1 |
| Example 9 [AXL22012] | 4.2 |
| Example 10 [AXL22013] | 20.0 |
| Example 11 [AXL22016] | 13.7 |
| Example 12 [AXL22017] | 56.8 |
| Example 13 [AXL22018] | 39.6 |
| Example 14 [AXL22020] | 3.5 |
| Example 15 [AXL22021] | 7.2 |
| Example 16 [AXL22022] | 3.4 |
| Example 17 [AXL22023] | 86.2 |
| Example 204 AXL22024 | 25.0 |
| Example 18 [AXL22025] | 114.2 |
| Example 19 [AXL22026] | 65.7 |
| Example 20 [AXL22027] | 10.5 |
| Example 21 [AXL22028] | 6.8 |
| Example 22 [AXL22030] | 20.2 |
| Example 139 [AXL22031] | <1.25 |
| Example 23 [AXL22032] | 5.6 |
| Example 24 [AXL22033] | 15.2 |
| Example 25 [AXL22034] | 13.5 |
| Example 26 [AXL22035] | 110.9 |
| Example 27 [AXL22036] | 45.8 |
| Example 28 [AXL22037] | 73.5 |
| Example 29 [AXL22038] | 6.3 |
| Example 30 [AXL22039] | 16.5 |
| Example 31 [AXL22040] | 8.6 |
| Example 32 [AXL22041] | 18.4 |
| Example 140 [AXL22042] | <1.25 |
| Example 141 [AXL22043] | <1.25 |
| Example 33 [AXL22044] | 28.2 |
| Example 34 [AXL22045] | 72.6 |
| Example 35 [AXL22046] | 96.7 |
| Example 36 [AXL22047] | 163.3 |
| Example 37 [AXL22048] | 37.2 |
| Example 38 [AXL22049] | 12.5 |
| Example 39 [AXL22050] | 2.7 |
| Example 40 [AXL22051] | 417.9 |
| Example 41 [AXL22052] | 57.7 |
| Example 42 [AXL22053] | 447.9 |
| Example 43 [AXL22054] | 16.1 |
| Example 44 [AXL22055] | 135.3 |
| Example 142 [AXL22056] | <1.25 |
| Example 143 [AXL22057] | <1.25 |
| Example 45 [AXL22058] | 42.8 |
| Example 46 [AXL22059] | 41.0 |
| Example 47 [AXL22060] | 61.3 |
| Example 48 [AXL22061] | 255.1 |
| Example 177 [AXL22062] | <1.25 |
| Example 178 [AXL22063] | <1.25 |
| Example 179 [AXL22064] | <1.25 |
| Example 180 [AXL22065] | <1.25 |
| Example 49 [AXL22066] | 29.3 |
| Example 50 [AXL22067] | 55.9 |
| Example 51 [AXL22068] | 93.3 |
| Example 52 [AXL22069] | 12.3 |
| Example 53 [AXL22070] | 71.5 |
| Example 54 [AXL22071] | 209.2 |
| Example 55 [AXL22072] | 194.8 |
| Example 56 [AXL22074] | 9.6 |
| Example 57 [AXL22075] | 17.5 |
| Example 58 [AXL22076] | 347.1 |
| Example 59 [AXL22077] | 281.0 |
| Example 60 [AXL22078] | 87.7 |
| Example 61 [AXL22079] | 176.8 |
| Example 62 [AXL22080] | 709.2 |
| Example 63 [AXL22081] | 2774.4 |
| Example 64 [AXL22082] | 383.1 |
| Example 65 [AXL22083] | 3569.3 |
| Example 181 [AXL22084] | <1.25 |
| Example 66 [AXL22088] | 4.5 |
| Example 67 [AXL22089] | 24.0 |
| Example 144 [AXL22090] | <1.25 |
| Example 68 [AXL22091] | 1.6 |
| Example 69 [AXL22092] | 1.9 |
| Example 70 [AXL22093] | 1.9 |
| Example 71 [AXL22094] | 8.6 |
| Example 72 [AXL22095] | 9.9 |
| Example 73 [AXL22096] | 31.0 |
| Example 74 [AXL22097] | 4.7 |
| Example 75 [AXL22098] | 4.5 |
| Example 76 [AXL22099] | 9.4 |
| Example 77 [AXL22100] | 3.4 |
| Example 78 [AXL22101] | 1.8 |
| Example 79 [AXL22102] | 2.0 |
| Example 80 [AXL22103] | 6.4 |
| Example 81 [AXL22104] | 7.7 |
| Example 82 [AXL22105] | 1.5 |
| Example 83 [AXL22106] | 114.5 |
| Example 84 [AXL22107] | 53.4 |
| Example 182 [AXL22108] | 12.2 |
| Example 183 [AXL22109] | 16.8 |
| Example 184 [AXL22110] | 25.0 |
| Example 85 [AXL22111] | 66.8 |
| Example 86 [AXL22112] | 4.5 |
| Example 87 [AXL22113] | 63.1 |
| Example 88 [AXL22114] | 11.4 |
| Example 89 [AXL22115] | 10.8 |
| Example 90 [AXL22116] | 2.4 |
| Example 91 [AXL22117] | 11.1 |
| Example 92 [AXL22118] | 26.6 |
| Example 93 [AXL22119] | 2.1 |
| Example 94 [AXL22120] | <1.25 |
| Example 95 [AXL22121] | 34.8 |
| Example 96 [AXL22122] | 25.2 |
| Example 97 [AXL22123] | 14.4 |
| Example 98 [AXL22124] | 5.3 |
| Example 99 [AXL22125] | 59.9 |
| Example 100 [AXL22126] | 80.3 |
| Example 145 [AXL22127] | 5.3 |
| Example 146 [AXL22128] | <1.25 |
| Example 147 [AXL22129] | 1.5 |
| Example 148 [AXL22130] | 3.0 |
| Example 149 [AXL22131] | <1.25 |
| Example 203 AXL22132 | <1.25 |
| Example 151 [AXL22134] | 2.4 |
| Example 152 [AXL22136] | 4.5 |
| Example 153 [AXL22137] | 1.9 |
| Example 154 [AXL22139] | 3.1 |
| Example 155 [AXL22140] | <1.25 |
| Example 156 [AXL22141] | <1.25 |
| Example 157 [AXL22143] | 6.7 |
| Example 158 [AXL22144] | 3.7 |
| Example 159 [AXL22145] | 12.9 |
| Example 160 [AXL22146] | 6.6 |
| Example 161 [AXL22147] | 8.4 |
| Example 162 [AXL22148] | 1.5 |
| Example 163 [AXL22149] | 29.6 |
| Example 164 [AXL22150] | <1.25 |
| Example 165 [AXL22151] | <1.25 |
| Example 166 [AXL22152] | <1.25 |
| Example 167 [AXL22153] | <1.25 |
| Example 168 [AXL22154] | <1.25 |
| Example 169 [AXL22155] | <1.25 |
| Example 170 [AXL22156] | 3.5 |
| Example 171 [AXL22157] | <1.25 |
| Example 172 [AXL22158] | 3.4 |
| Example 173 [AXL22159] | <1.25 |
| Example 174 [AXL22160] | 2.8 |
| Example 175 [AXL22161] | <1.25 |
| Example 176 [AXL22162] | <1.25 |
| Example 101 [AXL22163] | 6.0 |
| Example 102 [AXL22164] | 20.9 |
| Example 103 [AXL22165] | 4.9 |
| Example 104 [AXL22166] | 95.9 |
| Example 105 [AXL22167] | 79.7 |
| Example 106 [AXL22168] | 44.6 |
| Example 107 [AXL22169] | 5.0 |
| Example 108 [AXL22170] | 34.2 |
| Example 185 [AXL22171] | <1.25 |
| Example 186 [AXL22172] | 2.0 |
| Example 109 [AXL22173] | 14.6 |
| Example 110 [AXL22174] | 12.3 |
| Example 187 [AXL22175] | 1.5 |
| Example 188 [AXL22176] | <1.25 |
| Example 189 [AXL22177] | 3.2 |
| Example 111 [AXL22178] | 32.0 |
| Example 112 [AXL22179] | 4.1 |
| Example 113 [AXL22180] | 37.2 |
| Example 114 [AXL22181] | 4.0 |
| Example 115 [AXL22182] | 5.1 |
| Example 116 [AXL22183] | 109.3 |
| Example 117 [AXL22184] | 5.4 |
| Example 118 [AXL22185] | 93.8 |
| Example 120 [AXL22187] | 5.1 |
| Example 190 [AXL22188] | <1.25 |
| Example 191 [AXL22189] | 2.3 |
| Example 192 [AXL22190] | <1.25 |
| Example 193 [AXL22191] | 2.9 |
| Example 194 [AXL22192] | <1.25 |
| Example 195 [AXL22193] | 1.8 |
| Example 196 [AXL22194] | 4.8 |
| Example 197 [AXL22195] | <1.25 |
| Example 198 [AXL22196] | <1.25 |
| Example 199 [AXL22197] | <1.25 |
| Example 200 [AXL22198] | <1.25 |
| Example 201 [AXL22199] | <1.25 |
| Example 202 [AXL22200] | <1.25 |
| Example 121 [AXL22202] | <1.25 |
| Example 122 [AXL22203] | 2.9 |
| Example 123 [AXL22204] | 1.5 |
| Example 124 [AXL22205] | <1.25 |
| Example 125 [AXL22206] | 3.3 |
| Example 126 [AXL22207] | 4.7 |
| Example 127 [AXL22208] | <1.25 |
| Example 128 [AXL22209] | 2.6 |
| Example 129 [AXL22210] | <1.25 |
| Example 130 [AXL22211] | 2.4 |
| Example 131 [AXL22212] | <1.25 |
| Example 132 [AXL22213] | 2.0 |
| Example 133 [AXL22214] | 2.2 |
| Example 134 [AXL22215] | <1.25 |
| Example 135 [AXL22216] | 3.5 |
| Example 136 [AXL22217] | 3.2 |
| Example 137 [AXL22218] | 79.1 |
| Example 138 [AXL22219] | 4.5 |
| 18d | 1.8 |
| 19h | <1.25 |
| BMS777607 | 1.5 |
| Foretinib | 2.9 |
| IM1331B | 4.4 |
| IM1330BT | 4.0 |

### MER in vitro kinase assay protocol

### Single-concentration-point residual activity measurements

The compounds were dissolved in DMSO to make 5 mM stock. Diluted solutions of 0.641 mM and 0.0641 mM concentration of the compounds were made from the 5 mM stock solutions. The 5 mM, 0.641 mM and 0.0641 mM solutions of each compound were added to 384-well polypropylene microplates (Greiner 784201). Reaction buffer was prepared in Eppendorf tubes, consisting of HEPES pH = 7.5 (20 mM final concentration), DTT (1 mM final concentration), MgCl₂ (3 mM final concentration), MnCl₂ (3 mM final concentration) and Tween20 (0.01 V/V% final concentration) in H₂O. Substrate + ATP solution was prepared by adding the substrate Poly Glu-Tyr (4:1) (0.05 mg/mL final concentration) and ATP (2.5 µM final concentration ~ ATP K_{Mapp} value) to the reaction buffer. Enzyme solution was prepared by adding MER kinase (8 nM final concentration) to the reaction buffer. 6 µL of Substrate + ATP solution was added to each well of the assay plate (Greiner 784900 - black, small volume, polystyrene, flat bottom). 0.0125 nL from the stock solutions of each compound was added to the appropriate assay plate wells using a pin tool mounted on the RoMa gripper arm of a Tecan Freedom EVO workstation. DMSO without inhibitor was added to positive (MAX - "100% kinase activity") and negative (MIN - "0% kinase activity") control wells. 2 µL of Enzyme solution was added to each well except for the MIN wells to start the kinase reaction. The assay plate was covered, and the enzyme reaction was incubated at room temperature for 60 minutes. The kinase reaction was stopped by addition of 8 µL detection mixture containing Transcreener ADP Alexa633 Tracer (3 nM final concentration), Transcreener ADP² Antibody (3.70 µg/ml final concentration), HEPES pH = 7.5 (20 mM final concentration), EDTA (40 mM final concentration) and Brij35 (0.02 V/V% final concentration). 2 µL of Enzyme solution was added to the MIN wells after the addition of the detection mixture. Fluorescence Polarization (FP) and Fluorescence Intensity (FI) measurements were performed on Tecan Infinite M1000 Pro reader 60 minutes after addition of the detection mixture. Percentage inhibition values of EGFR by the compounds in 7.8 µM, 1 µM and 0.1 µM concentrations were calculated using the results obtained for MAX and MIN control wells.

**Table: In vitro MER inhibition percentage values of the compounds in 7.8 µM, 1.0 µM and 0.1 µM concentrations.**

| AXL ID | MER Inhibition @ 7.8 uM (%) | MER Inhibition @ 1 uM (%) | MER Inhibition @ 0.1 uM (%) |
|---|---|---|---|
| Example 1 [AXL22001] | >99 | 88 | 61 |
| Example 2 [AXL22002] | >99 | 92 | 64 |
| Example 3 [AXL22005] | >99 | 92 | 40 |
| Example 4 [AXL22006] | 99 | 89 | 42 |
| Example 5 [AXL22007] | >99 | 87 | 51 |
| Example 6 [AXL22008] | 98 | 79 | 25 |
| Example 7 [AXL22009] | 99 | 79 | 37 |
| Example 8 [AXL22010] | 88 | 71 | 15 |
| Example 9 [AXL22012] | 98 | 86 | 38 |
| Example 10 [AXL22013] | >99 | 81 | 35 |
| Example 11 [AXL22016] | 93 | 67 | 5 |
| Example 12 [AXL22017] | 84 | 61 | 10 |
| Example 13 [AXL22018] | 74 | 52 | 8 |
| Example 14 [AXL22020] | >99 | 87 | 43 |
| Example 15 [AXL22021] | 97 | 89 | 49 |
| Example 16 [AXL22022] | 88 | 88 | 51 |
| Example 17 [AXL22023] | 86 | 74 | 26 |
| Example 204 AXL22024 | 87 | 80 | 36 |
| Example 18 [AXL22025] | 83 | 62 | 18 |
| Example 19 [AXL22026] | 83 | 73 | 23 |
| Example 20 [AXL22027] | 96 | 85 | 51 |
| Example 21 [AXL22028] | 88 | 91 | 41 |
| Example 22 [AXL22030] | 83 | 68 | 18 |
| Example 139 [AXL22031] | 81 | 93 | 92 |
| Example 23 [AXL22032] | 96 | 88 | 36 |
| Example 24 [AXL22033] | 92 | 80 | 26 |
| Example 25 [AXL22034] | 96 | 76 | 24 |
| Example 26 [AXL22035] | 64 | 33 | 2 |
| Example 27 [AXL22036] | 74 | 55 | 8 |
| Example 28 [AXL22037] | 66 | 52 | 11 |
| Example 29 [AXL22038] | 96 | 81 | 47 |
| Example 30 [AXL22039] | 87 | 79 | 32 |
| Example 31 [AXL22040] | 93 | 78 | 18 |
| Example 32 [AXL22041] | 91 | 51 | 12 |
| Example 140 [AXL22042] | >99 | 92 | 73 |
| Example 141 [AXL22043] | >99 | 96 | 92 |
| Example 33 [AXL22044] | 96 | 66 | 12 |
| Example 34 [AXL22045] | 92 | 50 | 13 |
| Example 35 [AXL22046] | 79 | 29 | 4 |
| Example 36 [AXL22047] | 91 | 26 | 1 |
| Example 37 [AXL22048] | 89 | 57 | 7 |
| Example 38 [AXL22049] | >99 | 66 | 16 |
| Example 39 [AXL22050] | >99 | 78 | 38 |
| Example 40 [AXL22051] | 62 | 28 | 2 |
| Example 41 [AXL22052] | 90 | 50 | 6 |
| Example 42 [AXL22053] | 58 | 21 | <1 |
| Example 43 [AXL22054] | 99 | 66 | 15 |
| Example 44 [AXL22055] | 60 | 23 | <1 |
| Example 142 [AXL22056] | >99 | 99 | 87 |
| Example 143 [AXL22057] | 94 | 94 | 93 |
| Example 45 [AXL22058] | 92 | 76 | 21 |
| Example 46 [AXL22059] | 89 | 61 | 15 |
| Example 47 [AXL22060] | 84 | 62 | 13 |
| Example 48 [AXL22061] | 68 | 34 | 5 |
| Example 177 [AXL22062] | 97 | 94 | 81 |
| Example 178 [AXL22063] | 97 | 95 | 75 |
| Example 179 [AXL22064] | >99 | 96 | 86 |
| Example 180 [AXL22065] | >99 | 97 | 87 |
| Example 49 [AXL22066] | 96 | 74 | 26 |
| Example 50 [AXL22067] | 91 | 60 | 16 |
| Example 51 [AXL22068] | 80 | 45 | 5 |
| Example 52 [AXL22069] | 97 | 89 | 57 |
| Example 53 [AXL22070] | 83 | 64 | 10 |
| Example 54 [AXL22071] | 62 | 35 | <1 |
| Example 55 [AXL22072] | 84 | 64 | 13 |
| Example 56 [AXL22074] | 95 | 87 | 42 |
| Example 57 [AXL22075] | 97 | 76 | 40 |
| Example 58 [AXL22076] | 57 | 43 | 4 |
| Example 59 [AXL22077] | 52 | 23 | 3 |
| Example 60 [AXL22078] | 78 | 59 | 14 |
| Example 61 [AXL22079] | 76 | 33 | 14 |
| Example 62 [AXL22080] | 53 | 19 | 2 |
| Example 63 [AXL22081] | 17 | <1 | <1 |
| Example 64 [AXL22082] | 51 | 21 | <1 |
| Example 65 [AXL22083] | 59 | 35 | 11 |
| Example 181 [AXL22084] | 94 | 96 | 83 |
| Example 66 [AXL22088] | 94 | 86 | 42 |
| Example 67 [AXL22089] | 91 | 77 | 22 |
| Example 144 [AXL22090] | >99 | 97 | 89 |
| Example 68 [AXL22091] | 99 | 95 | 63 |
| Example 69 [AXL22092] | >99 | 93 | 62 |
| Example 70 [AXL22093] | >99 | 89 | 51 |
| Example 71 [AXL22094] | 94 | 91 | 50 |
| Example 72 [AXL22095] | 97 | 85 | 55 |
| Example 73 [AXL22096] | 93 | 68 | 25 |
| Example 74 [AXL22097] | >99 | >99 | 59 |
| Example 75 [AXL22098] | 97 | >99 | 78 |
| Example 76 [AXL22099] | >99 | 86 | 40 |
| Example 77 [AXL22 100] | 91 | 94 | 45 |
| Example 78 [AXL22101] | >99 | 92 | 62 |
| Example 79 [AXL22102] | 96 | 96 | 67 |
| Example 80 [AXL22103] | >99 | 95 | 48 |
| Example 81 [AXL22104] | 92 | 98 | 48 |
| Example 82 [AXL22105] | 96 | 91 | 61 |
| Example 83 [AXL22106] | 99 | 74 | 29 |
| Example 84 [AXL22107] | 94 | 82 | 31 |
| Example 182 [AXL22108] | 91 | 87 | 48 |
| Example 183 [AXL22109] | >99 | 94 | 52 |
| Example 184 [AXL22110] | 99 | 94 | 37 |
| Example 85 [AXL22111] | 90 | 90 | 50 |
| Example 86 [AXL22112] | 95 | 91 | 57 |
| Example 87 [AXL22113] | 78 | 79 | 33 |
| Example 88 [AXL22114] | 99 | 88 | 36 |
| Example 89 [AXL22115] | >99 | 88 | 48 |
| Example 90 [AXL22116] | >99 | 97 | 58 |
| Example 91 [AXL22117] | 99 | 78 | 28 |
| Example 92 [AXL22118] | 94 | 87 | 43 |
| Example 93 [AXL22119] | >99 | 91 | 51 |
| Example 94 [AXL22120] | 94 | 87 | 55 |
| Example 95 [AXL22121] | 96 | 73 | 23 |
| Example 96 [AXL22122] | 94 | 83 | 39 |
| Example 97 [AXL22123] | >99 | 91 | 63 |
| Example 98 [AXL22124] | 97 | 97 | 59 |
| Example 99 [AXL22125] | 98 | 89 | 31 |
| Example 100 [AXL22126] | 95 | 93 | 47 |
| Example 145 [AXL22127] | 99 | 99 | 90 |
| Example 146 [AXL22128] | >99 | >99 | 93 |
| Example 147 [AXL22129] | 95 | 93 | 83 |
| Example 148 [AXL22130] | 99 | >99 | 88 |
| Example 149 [AXL22131] | 98 | 91 | 91 |
| Example 203 AXL22132 | 98 | >99 | 89 |
| Example 150 [AXL22133] | >99 | 95 | 92 |
| Example 151 [AXL22134] | 99 | 99 | 94 |
| Example 152 [AXL22136] | 89 | 92 | 65 |
| Example 153 [AXL22137] | 97 | 95 | 94 |
| Example 154 [AXL22139] | >99 | 97 | 84 |
| Example 155 [AXL22140] | 99 | >99 | 91 |
| Example 156 [AXL22141] | >99 | 92 | 91 |
| Example 157 [AXL22143] | 98 | 76 | 76 |
| Example 158 [AXL22144] | 96 | 94 | 66 |
| Example 159 [AXL22145] | 90 | 97 | 79 |
| Example 160 [AXL22146] | 96 | 93 | 64 |
| Example 161 [AXL22147] | 99 | 96 | 67 |
| Example 162 [AXL22148] | >99 | 99 | 89 |
| Example 163 [AXL22149] | >99 | 88 | 47 |
| Example 164 [AXL22150] | 98 | >99 | 93 |
| Example 165 [AXL22151] | 96 | >99 | 92 |
| Example 166 [AXL22152] | >99 | 98 | 92 |
| Example 167 [AXL22153] | 97 | >99 | 92 |
| Example 168 [AXL22154] | >99 | >99 | 97 |
| Example 169 [AXL22155] | >99 | >99 | 92 |
| Example 170 [AXL22156] | >99 | >99 | 93 |
| Example 171 [AXL22157] | >99 | >99 | 87 |
| Example 172 [AXL22158] | >99 | >99 | 96 |
| Example 174 [AXL22160] | 96 | >99 | 95 |
| Example 175 [AXL22161] | 98 | 83 | 96 |
| Example 176 [AXL22162] | 93 | 95 | 91 |
| Example 101 [AXL22163] | 97 | 80 | 34 |
| Example 102 [AXL22164] | 93 | 85 | 28 |
| Example 103 [AXL22165] | 96 | 82 | 43 |
| Example 104 [AXL22166] | 88 | 70 | 23 |
| Example 105 [AXL22167] | 94 | 72 | 16 |
| Example 106 [AXL22168] | 93 | 73 | 18 |
| Example 107 [AXL22169] | 99 | 81 | 29 |
| Example 108 [AXL22170] | 92 | 73 | 20 |
| Example 185 [AXL22171] | 98 | 93 | 92 |
| Example 186 [AXL22172] | 97 | 93 | 84 |
| Example 109 [AXL22173] | 96 | 86 | 36 |
| Example 110 [AXL22174] | 90 | 70 | 14 |
| Example 187 [AXL22175] | 96 | >99 | 90 |
| Example 188 [AXL22176] | >99 | 89 | 75 |
| Example 189 [AXL22177] | 98 | 97 | 90 |
| Example 111 [AXL22178] | 98 | 83 | 33 |
| Example 112 [AXL22179] | >99 | 75 | 24 |
| Example 113 [AXL22180] | 97 | 73 | 21 |
| Example 114 [AXL22181] | 97 | 76 | 28 |
| Example 115 [AXL22182] | >99 | 87 | 28 |
| Example 116 [AXL22183] | >99 | 68 | 23 |
| Example 117 [AXL22184] | >99 | 79 | 25 |
| Example 118 [AXL22185] | >99 | 78 | 29 |
| Example 119 [AXL22186] | 97 | 80 | 33 |
| Example 120 [AXL22187] | 95 | 82 | 36 |
| Example 190 [AXL22188] | >99 | 93 | 87 |
| Example 191 [AXL22189] | 97 | 93 | 86 |
| Example 192 [AXL22190] | 94 | 97 | 83 |
| Example 193 [AXL22191] | >99 | 95 | 88 |
| Example 194 [AXL22192] | 97 | 94 | 92 |
| Example 195 [AXL22193] | >99 | >99 | 88 |
| Example 196 [AXL22194] | 98 | 96 | 73 |
| Example 197 [AXL22195] | >99 | >99 | 93 |
| Example 198 [AXL22196] | 92 | 89 | 91 |
| Example 199 [AXL22197] | 93 | >99 | 83 |
| Example 200 [AXL22198] | 98 | 95 | 88 |
| Example 201 [AXL22199] | 99 | >99 | 89 |
| Example 202 [AXL22200] | 98 | >99 | 90 |
| Example 122 [AXL22203] | >99 | 91 | 63 |
| Example 123 [AXL22204] | >99 | 96 | 60 |
| Example 124 [AXL22205] | >99 | 99 | 71 |
| Example 125 [AXL22206] | 97 | 87 | 36 |
| Example 126 [AXL22207] | >99 | 95 | 59 |
| Example 127 [AXL22208] | >99 | 94 | 58 |
| Example 128 [AXL22209] | >99 | 95 | 54 |
| Example 129 [AXL22210] | 96 | 94 | 70 |
| Example 130 [AXL22211] | 97 | 98 | 75 |
| Example 131 [AXL22212] | >99 | 97 | 76 |
| Example 132 [AXL22213] | >99 | >99 | 79 |
| Example 133 [AXL22214] | 97 | 92 | 51 |
| Example 134 [AXL22215] | 98 | 91 | 59 |
| Example 135 [AXL22216] | 96 | 94 | 47 |
| Example 136 [AXL22217] | 95 | >99 | 68 |
| Example 137 [AXL22218] | >99 | 69 | 17 |
| Example 138 [AXL22219] | 90 | 71 | 22 |
| BMS777607 | 97 | 99 | 87 |
| Staurosporine | 97 | 95 | 86 |
| Foretinib | 98 | 99 | 81 |
| BSZ1431A | >99 | 95 | 71 |
| 19h | >99 | 92 | 63 |
| Gefitinib | 3 | <1 | <1 |

### IC₅₀ determination

The compounds were dissolved in DMSO to make 5 mM stock solutions. 12-point dilution series were made from the stock solutions in 3-fold dilution steps (5 mM to 28.225 nM) using DMSO on 384-well polypropylene microplates (Greiner 784201). Reaction buffer was prepared in Eppendorf tubes, consisting of HEPES pH = 7.5 (20 mM final concentration), DTT (1 mM final concentration), MgCl₂ (3 mM final concentration), MnCl₂ (3 mM final concentration) and Tween20 (0.01 V/V% final concentration) in H₂O. Substrate + ATP solution was prepared by adding the substrate Poly Glu-Tyr (4:1) (0.05 mg/mL final concentration) and ATP (2.5 µM final concentration ~ ATP K_{Mapp} value) to the reaction buffer. Enzyme solution was prepared by adding MER kinase (8 nM final concentration) to the reaction buffer. 6 µL of Substrate + ATP solution was added to each well of the assay plate (Greiner 784900 - black, small volume, polystyrene, flat bottom). 0.0125 nL from each concentration of the dilution series of each compound was added to the appropriate assay plate wells using a pin tool mounted on the RoMa gripper arm of a Tecan Freedom EVO workstation. DMSO without inhibitor was added to positive (MAX - "100% kinase activity") and negative (MIN - "0% kinase activity") control wells. Calibration (CAL) wells were prepared in a 12-point series containing a fixed 2.5 µM of total ATP+ADP concentration with different ATP/ADP ratios, dissolved in the reaction buffer containing the substrate Poly Glu-Tyr (4:1) (0.05 mg/mL final concentration). Each CAL solution was added to the assay plate in 6 µL volumes. 2 µL of Enzyme solution was added to each well except for the MIN and CAL wells to start the kinase reaction. The assay plate was covered and the enzyme reaction was incubated on room temperature for 60 minutes. The kinase reaction was stopped by addition of 8 µL detection mixture containing Transcreener ADP Alexa633 Tracer (3 nM final concentration), Transcreener ADP² Antibody (3.7 µg/ml final concentration), HEPES pH = 7.5 (20 mM final concentration), EDTA (40 mM final concentration) and Brij35 (0.02 V/V% final concentration). 2 µL of Enzyme solution was added to MIN and CAL wells after the addition of the detection mixture. Fluorescence Polarization (FP) and Fluorescence Intensity (FI) measurements were performed on Tecan Infinite M1000 Pro reader 60 minutes after addition of the detection mixture. IC₅₀ values were calculated by a curve fitting the FP data points using XLfit 5.1; FI was used to identify any compounds with fluorescent properties that may interfere with the detection that could provide false data.

**Table: In vitro IC₅₀ values of the compounds on MER kinase.**

| ID | MER Kinase IC50 (nM) |
|---|---|
| Example 139 [AXL22031] | <4 |
| Example 141 [AXL22043] | <4 |
| Example 142 [AXL22056] | 4.3 |
| Example 143 [AXL22057] | <4 |
| Example 179 [AXL22064] | 9.2 |
| Example 180 [AXL22065] | <4 |
| Example 56 [AXL22074] | 125.9 |
| Example 181 [AXL22084] | 22.0 |
| Example 144 [AXL22090] | <4 |
| Example 78 [AXL22101] | 36.5 |
| Example 182 [AXL22108] | 35.9 |
| Example 97 [AXL22123] | 16.5 |
| Example 145 [AXL22127] | <4 |
| Example 146 [AXL22128] | <4 |
| Example 147 [AXL22129] | 9.8 |
| Example 149 [AXL22131] | 6.1 |
| Example 203 AXL22132 | <4 |
| Example 150 [AXL22133] | <4 |
| Example 151 [AXL22134] | <4 |
| Example 153 [AXL22137] | <4 |
| Example 154 [AXL22139] | <4 |
| Example 155 [AXL22140] | <4 |
| Example 156 [AXL22141] | <4 |
| Example 162 [AXL22148] | 4.2 |
| Example 164 [AXL22150] | <4 |
| Example 165 [AXL22151] | <4 |
| Example 166 [AXL22152] | <4 |
| Example 167 [AXL22153] | <4 |
| Example 168 [AXL22154] | <4 |
| Example 169 [AXL22155] | <4 |
| Example 170 [AXL22156] | <4 |
| Example 172 [AXL22158] | <4 |
| Example 174 [AXL22160] | <4 |
| Example 175 [AXL22161] | <4 |
| Example 176 [AXL22162] | <4 |
| Example 185 [AXL22171] | <4 |
| Example 186 [AXL22172] | <4 |
| Example 187 [AXL22175] | <4 |
| Example 188 [AXL22176] | 5.5 |
| Example 189 [AXL22177] | <4 |
| Example 190 [AXL22188] | <4 |
| Example 191 [AXL22189] | 8.0 |
| Example 193 [AXL22191] | <4 |
| Example 194 [AXL22192] | <4 |
| Example 195 [AXL22193] | <4 |
| Example 197 [AXL22195] | <4 |
| Example 198 [AXL22196] | <4 |
| Example 200 [AXL22198] | <4 |
| Example 201 [AXL22199] | <4 |
| Example 202 [AXL22200] | <4 |
| 19h (JMC) | 25.0 |
| BMS777607 | 4.1 |

### EGFR in vitro kinase assay protocol

### Single concentration point residual activity measurement

The compounds were dissolved in DMSO to make 5 mM stock solutions , and were added to 384-well polypropylene microplates (Greiner 784201). Reaction buffer was prepared in Eppendorf tubes, consisting of HEPES pH = 7.5 (20 mM final concentration), DTT (1 mM final concentration), MgCl₂ (10 mM final concentration), MnCl₂ (2 mM final concentration) and NP40 (0.01 V/V% final concentration) in H₂O. Substrate + ATP solution was prepared by adding the substrate Poly Glu-Tyr (4:1) (0.01 mg/mL final concentration) and ATP (7.52 µM final concentration ~ ATP K_{Mapp} value) to the reaction buffer. Enzyme solution was prepared by adding EGFR kinase (8 nM final concentration) to the reaction buffer. 6 µL of Substrate + ATP solution was added to each well of the assay plate (Greiner 784900 - black, small volume, polystyrene, flat bottom). 0.0125 nL from the stock solutions of each compound was added to the appropriate assay plate wells using a pin tool mounted on the RoMa gripper arm of a Tecan Freedom EVO workstation. DMSO without inhibitor was added to positive (MAX - "100% kinase activity") and negative (MIN - "0% kinase activity") control wells. 2 µL of Enzyme solution was added to each well except for the MIN wells to start the kinase reaction. The assay plate was covered, and the enzyme reaction was incubated at room temperature for 60 minutes. The kinase reaction was stopped by addition of 8 µL detection mixture containing Transcreener ADP Alexa633 Tracer (3 nM final concentration), Transcreener ADP² Antibody (9.12 µg/ml final concentration), HEPES pH = 7.5 (20 mM final concentration), EDTA (40 mM final concentration) and Brij35 (0.02 V/V% final concentration). 2 µL of Enzyme solution was added to the MIN wells after the addition of the detection mixture. Fluorescence Polarization (FP) and Fluorescence Intensity (FI) measurements were performed on Tecan Infinite M1000 Pro reader 60 minutes after addition of the detection mixture. Percentage inhibition values of EGFR by the compounds in 7.8 µM concentration were calculated using the results obtained for MAX and MIN control wells.

**Table: In vitro EGFR inhibition percentage values of the compounds in 7.8 µM concentration.**

| ID | EGFR kinase inhibition at 7.8 µM (%) | ID | EGFR kinase inhibition at 7.8 µM (%) |
|---|---|---|---|
| 19h (JMC) | >99 | Example 90 [AXL22116] | 84 |
| Example 1 [AXL22001] | >99 | Example 91 [AXL22117] | 96 |
| Example 2 [AXL22002] | 88 | Example 92 [AXL22118] | 99 |
| Example 3 [AXL22005] | 72 | Example 93 [AXL22119] | >99 |
| Example 4 [AXL22006] | 87 | Example 94 [AXL22120] | 94 |
| Example 5 [AXL22007] | 54 | Example 95 [AXL22121] | >99 |
| Example 6 [AXL22008] | 57 | Example 96 [AXL22122] | 95 |
| Example 7 [AXL22009] | 67 | Example 97 [AXL22123] | 92 |
| Example 8 [AXL22010] | 23 | Example 98 [AXL22124] | 70 |
| Example 9 [AXL22012] | 85 | Example 99 [AXL22125] | 83 |
| Example 10 [AXL22013] | 59 | Example 100 [AXL22126] | 58 |
| Example 11 [AXL22016] | 67 | Example 145 [AXL22127] | 73 |
| Example 12 [AXL22017] | 18 | Example 146 [AXL22128] | 88 |
| Example 13 [AXL22018] | 46 | Example 147 [AXL22129] | 19 |
| Example 14 [AXL22020] | 85 | Example 148 [AXL22130] | 88 |
| Example 15 [AXL22021] | 49 | Example 149 [AXL22131] | 57 |
| Example 16 [AXL22022] | 63 | Example 203 AXL22132 | 57 |
| Example 17 [AXL22023] | 59 | Example 150 [AXL22133] | 38 |
| Example 204 AXL22024 | 62 | Example 151 [AXL22134] | 27 |
| Example 18 [AXL22025] | 21 | Example 152 [AXL22136] | <1 |
| Example 19 [AXL22026] | 29 | Example 153 [AXL22137] | 47 |
| Example 20 [AXL22027] | 43 | Example 154 [AXL22139] | 62 |
| Example 21 [AXL22028] | 47 | Example 155 [AXL22140] | 56 |
| Example 22 [AXL22030] | 39 | Example 156 [AXL22141] | 84 |
| Example 139 [AXL22031] | 71 | Example 157 [AXL22143] | 5 |
| Example 23 [AXL22032] | 49 | Example 158 [AXL22144] | 15 |
| Example 24 [AXL22033] | 32 | Example 159 [AXL22145] | 23 |
| Example 25 [AXL22034] | 49 | Example 160 [AXL22146] | 4 |
| Example 26 [AXL22035] | 11 | Example 161 [AXL22147] | 22 |
| Example 27 [AXL22036] | 10 | Example 162 [AXL22148] | 55 |
| Example 28 [AXL22037] | 10 | Example 163 [AXL22149] | 3 |
| Example 29 [AXL22038] | 47 | Example 164 [AXL22150] | 55 |
| Example 30 [AXL22039] | 42 | Example 165 [AXL22151] | 65 |
| Example 31 [AXL22040] | 42 | Example 166 [AXL22152] | 66 |
| Example 32 [AXL22041] | 60 | Example 167 [AXL22153] | 35 |
| Example 140 [AXL22042] | 66 | Example 168 [AXL22154] | 61 |
| Example 141 [AXL22043] | 35 | Example 169 [AXL22155] | 47 |
| Example 33 [AXL22044] | 67 | Example 170 [AXL22156] | 79 |
| Example 34 [AXL22045] | 32 | Example 171 [AXL22157] | 92 |
| Example 35 [AXL22046] | 20 | Example 172 [AXL22158] | 79 |
| Example 36 [AXL22047] | 44 | Example 174 [AXL22160] | 37 |
| Example 37 [AXL22048] | 56 | Example 175 [AXL22161] | 38 |
| Example 38 [AXL22049] | 83 | Example 176 [AXL22162] | 35 |
| Example 39 [AXL22050] | 96 | Example 101 [AXL22163] | 86 |
| Example 40 [AXL22051] | 4 | Example 102 [AXL22164] | 95 |
| Example 41 [AXL22052] | 31 | Example 103 [AXL22165] | 80 |
| Example 42 [AXL22053] | 1 | Example 104 [AXL22166] | 45 |
| Example 43 [AXL22054] | 57 | Example 105 [AXL22167] | 80 |
| Example 44 [AXL22055] | <1 | Example 106 [AXL22168] | 74 |
| Example 142 [AXL22056] | 73 | Example 107 [AXL22169] | 88 |
| Example 143 [AXL22057] | 78 | Example 108 [AXL22170] | 81 |
| Example 45 [AXL22058] | 44 | Example 185 [AXL22171] | <1 |
| Example 46 [AXL22059] | 38 | Example 186 [AXL22172] | <1 |
| Example 47 [AXL22060] | 33 | Example 109 [AXL22173] | 97 |
| Example 48 [AXL22061] | 18 | Example 110 [AXL22174] | 33 |
| Example 177 [AXL22062] | 35 | Example 187 [AXL22175] | <1 |
| Example 178 [AXL22063] | 12 | Example 188 [AXL22176] | 9 |
| Example 179 [AXL22064] | 35 | Example 189 [AXL22177] | <1 |
| Example 180 [AXL22065] | 10 | Example 111 [AXL22178] | 94 |
| Example 49 [AXL22066] | 74 | Example 112 [AXL22179] | >99 |
| Example 50 [AXL22067] | 54 | Example 113 [AXL22180] | 59 |
| Example 51 [AXL22068] | 13 | Example 114 [AXL22181] | 78 |
| Example 52 [AXL22069] | 85 | Example 115 [AXL22182] | >99 |
| Example 53 [AXL22070] | 27 | Example 116 [AXL22183] | 89 |
| Example 54 [AXL22071] | 16 | Example 117 [AXL22184] | >99 |
| Example 55 [AXL22072] | 37 | Example 118 [AXL22185] | >99 |
| Example 56 [AXL22074] | 64 | Example 119 [AXL22186] | 55 |
| Example 57 [AXL22075] | 67 | Example 120 [AXL22187] | 90 |
| Example 58 [AXL22076] | 17 | Example 190 [AXL22188] | <1 |
| Example 59 [AXL22077] | <1 | Example 191 [AXL22189] | 2 |
| Example 60 [AXL22078] | 18 | Example 192 [AXL22190] | <1 |
| Example 61 [AXL22079] | 23 | Example 193 [AXL22191] | <1 |
| Example 62 [AXL22080] | 17 | Example 194 [AXL22192] | <1 |
| Example 63 [AXL22081] | <1 | Example 195 [AXL22193] | <1 |
| Example 64 [AXL22082] | 3 | Example 196 [AXL22194] | <1 |
| Example 65 [AXL22083] | 11 | Example 197 [AXL22195] | <1 |
| Example 181 [AXL22084] | <1 | Example 198 [AXL22196] | <1 |
| Example 66 [AXL22088] | 79 | Example 199 [AXL22197] | 14 |
| Example 67 [AXL22089] | 65 | Example 200 [AXL22198] | 13 |
| Example 144 [AXL22090] | 69 | Example 201 [AXL22199] | 6 |
| Example 68 [AXL22091] | >99 | Example 202 [AXL22200] | 2 |
| Example 69 [AXL22092] | >99 | Example 122 [AXL22203] | >99 |
| Example 70 [AXL22093] | >99 | Example 123 [AXL22204] | >99 |
| Example 71 [AXL22094] | 89 | Example 124 [AXL22205] | >99 |
| Example 72 [AXL22095] | 82 | Example 125 [AXL22206] | 80 |
| Example 73 [AXL22096] | 83 | Example 126 [AXL22207] | >99 |
| Example 74 [AXL22097] | >99 | Example 127 [AXL22208] | >99 |
| Example 75 [AXL22098] | 84 | Example 128 [AXL22209] | >99 |
| Example 76 [AXL22099] | 98 | Example 129 [AXL22210] | 85 |
| Example 77 [AXL22 100] | 78 | Example 130 [AXL22211] | >99 |
| Example 78 [AXL22101] | >99 | Example 131 [AXL22212] | 75 |
| Example 79 [AXL22102] | 86 | Example 132 [AXL22213] | 96 |
| Example 80 [AXL22103] | >99 | Example 133 [AXL22214] | 77 |
| Example 81 [AXL22104] | 91 | Example 134 [AXL22215] | 52 |
| Example 82 [AXL22105] | 81 | Example 135 [AXL22216] | 44 |
| Example 83 [AXL22106] | 75 | Example 136 [AXL22217] | 88 |
| Example 84 [AXL22107] | 70 | Example 137 [AXL22218] | 99 |
| Example 182 [AXL22108] | <1 | Example 138 [AXL22219] | 36 |
| Example 183 [AXL22109] | 29 | BMS777607 | <1 |
| Example 184 [AXL22110] | <1 | BSZ1431A (ref) | 99 |
| Example 85 [AXL22111] | 2 | Foretinib | 33 |
| Example 86 [AXL22112] | 88 | Gefitinib | >99 |
| Example 87 [AXL22113] | >99 | Intermediate 1 | >99 |
| Example 88 [AXL22114] | 91 | Intermediate 2 | >99 |
| Example 89 [AXL22115] | >99 | Staurosporine | 78 |

### IC₅₀ determination

The compounds were dissolved in DMSO to make 5 mM stock solutions. 12-point dilution series were made from the stock solutions in 3-fold dilution steps (5 mM to 28.225 nM) using DMSO on 384-well polypropylene microplates (Greiner 784201). Reaction buffer was prepared in Eppendorf tubes, consisting of HEPES pH = 7.5 (20 mM final concentration), DTT (1 mM final concentration), MgCl₂ (10 mM final concentration), MnCl₂ (2 mM final concentration) and NP40 (0.01 V/V% final concentration) in H₂O. Substrate + ATP solution was prepared by adding the substrate Poly Glu-Tyr (4:1) (0.01 mg/mL final concentration) and ATP (7.52 µM final concentration ~ ATP K_{Mapp} value) to the reaction buffer. Enzyme solution was prepared by adding EGFR kinase (8 nM final concentration) to the reaction buffer. 6 µL of Substrate + ATP solution was added to each well of the assay plate (Greiner 784900 - black, small volume, polystyrene, flat bottom). 0.0125 nL from each concentration of the dilution series of each compound was added to the appropriate assay plate wells using a pin tool mounted on the RoMa gripper arm of a Tecan Freedom EVO workstation. DMSO without inhibitor was added to positive (MAX - "100% kinase activity") and negative (MIN - "0% kinase activity") control wells. Calibration (CAL) wells were prepared in a 12-point series containing a fixed 7.52 µM of total ATP+ADP concentration with different ATP/ADP ratios, dissolved in the reaction buffer containing the substrate Poly Glu-Tyr (4:1) (0.05 mg/mL final concentration). Each CAL solution was added to the assay plate in 6 µL volumes. 2 µL of Enzyme solution was added to each well except for the MIN and CAL wells to start the kinase reaction. The assay plate was covered and the enzyme reaction was incubated on room temperature for 60 minutes. The kinase reaction was stopped by addition of 8 µL detection mixture containing Transcreener ADP Alexa633 Tracer (3 nM final concentration), Transcreener ADP² Antibody (9.12 µg/ml final concentration), HEPES pH = 7.5 (20 mM final concentration), EDTA (40 mM final concentration) and Brij35 (0.02 V/V% final concentration). 2 µL of Enzyme solution was added to the MIN and CAL wells after the addition of the detection mixture. Fluorescence Polarization (FP) and Fluorescence Intensity (FI) measurements were performed on Tecan Infinite M1000 Pro reader 60 minutes after addition of the detection mixture. IC₅₀ values were calculated by curve fitting on FP data points using XLfit 5.1; FI was used to identify any compounds with fluorescent properties that may interfere with the detection that could provide false data.

**Table: In vitro IC₅₀ values of the compounds on EGFR kinase.**

| ID | EGFR Kinase IC50 (nM) | No. of experiments |
|---|---|---|
| Example 1 [AXL22001] | 52.9 | 2 |
| Example 39 [AXL22050] | 233.3 | 2 |
| Example 143 [AXL22057] | 1088.9 | 2 |
| Example 179 [AXL22064] | >7800 | 2 |
| Example 56 [AXL22074] | 1283.9 | 2 |
| Example 68 [AXL22091] | 46.4 | 2 |
| Example 69 [AXL22092] | 131.4 | 2 |
| Example 70 [AXL22093] | 133.1 | 2 |
| Example 71 [AXL22094] | 240.8 | 2 |
| Example 74 [AXL22097] | 71.2 | 2 |
| Example 76 [AXL22099] | 489.4 | 2 |
| Example 78 [AXL22101] | 169.5 | 2 |
| Example 80 [AXL22103] | 177.4 | 2 |
| Example 81 [AXL22104] | 351.7 | 2 |
| Example 182 [AXL22108] | >7800 | 2 |
| Example 87 [AXL22113] | 74.2 | 2 |
| Example 88 [AXL22114] | 182.9 | 2 |
| Example 89 [AXL22115] | 70.6 | 2 |
| Example 93 [AXL22119] | 114.7 | 2 |
| Example 94 [AXL22120] | 151.4 | 2 |
| Example 97 [AXL22123] | 233.9 | 2 |
| Example 146 [AXL22128] | 586.7 | 2 |
| Example 148 [AXL22130] | 458.7 | 2 |
| Example 151 [AXL22134] | 7700 | 2 |
| Example 154 [AXL22139] | >7800 | 2 |
| Example 156 [AXL22141] | 1958.2 | 2 |
| Example 170 [AXL22156] | 1462.5 | 2 |
| Example 171 [AXL22157] | 881.4 | 2 |
| Example 172 [AXL22158] | 970.5 | 2 |
| Example 174 [AXL22160] | 4558.6 | 2 |
| Example 102 [AXL22164] | 541.9 | 2 |
| Example 109 [AXL22173] | 324.9 | 2 |
| Example 188 [AXL22176] | >7800 | 2 |
| Example 111 [AXL22178] | 709.3 | 2 |
| Example 112 [AXL22179] | 510 | 2 |
| Example 115 [AXL22182] | 608.3 | 2 |
| Example 116 [AXL22183] | 909.7 | 2 |
| Example 117 [AXL22184] | 376.9 | 2 |
| Example 118 [AXL22185] | 211.5 | 2 |
| Example 120 [AXL22187] | 176.4 | 2 |
| Example 122 [AXL22203] | 155.2 | 2 |
| Example 123 [AXL22204] | 185.3 | 2 |
| Example 124 [AXL22205] | 107.6 | 2 |
| Example 126 [AXL22207] | 106.5 | 2 |
| Example 127 [AXL22208] | 272 | 2 |
| Example 128 [AXL22209] | 89.7 | 2 |
| Example 130 [AXL22211] | 115.9 | 2 |
| Example 132 [AXL22213] | 172.3 | 2 |
| Example 137 [AXL22218] | 1037 | 2 |
| Gefitinib | 4.9 | 4 |
| Staurosporine | 410.7 | 4 |

### In vitro b-Raf(WT) and b-Raf(V600E) inhibition of Example 78 [AXL22101]

Example 78 [AXL22101] was tested against the wild-type and the V600E mutant of b-Raf kinase in an *in vitro* biochemical assay. The assays were completed both with and without preincubation of the kinase with the test compound.

### B-Raf(WT) kinase assay protocol without preincubation

The compounds were dissolved in DMSO to make 5 mM stock solutions. 12-point dilution series were made from the stock solutions in 3-fold dilution steps (5 mM to 28.225 nM) using DMSO on 384-well polypropylene microplates (Greiner 784201). Reaction buffer was prepared in Eppendorf tubes, consisting of HEPES pH = 7.5 (50 mM final concentration), DTT (1 mM final concentration), MgCl₂ (10 mM final concentration), and Brij35 (0.01 V/V% final concentration) in H₂O. Substrate + ATP solution was prepared by adding the substrate MEK1(K97M) (0.01 mg/mL final concentration) and ATP (2.10 µM final concentration ~ ATP K_{Mapp} value) to the reaction buffer. Enzyme solution was prepared by adding wild-type b-Raf kinase (50 nM final concentration) to the reaction buffer. 6 µL of Substrate + ATP solution was added to each well of the assay plate (Greiner 784900 - black, small volume, polystyrene, flat bottom). 0.0125 nL from each concentration of the dilution series of each compound was added to the appropriate assay plate wells using a pin tool mounted on the RoMa gripper arm of a Tecan Freedom EVO workstation. DMSO without inhibitor was added to positive (MAX - "100% kinase activity") and negative (MIN - "0% kinase activity") control wells. Calibration (CAL) wells were prepared in a 12-point series containing a fixed 2.10 µM of total ATP+ADP concentration with different ATP/ADP ratios, dissolved in the reaction buffer. Each CAL solution was added to the assay plate in 6 µL volumes. 2 µL of enzyme solution was added to each well except for the MIN and CAL wells to start the kinase reaction. The assay plate was covered and the enzyme reaction was incubated at room temperature for 60 minutes. The kinase reaction was stopped by addition of 8 µL detection mixture containing Transcreener ADP Alexa633 Tracer (3 nM final concentration), Transcreener ADP² Antibody (12 µg/ml final concentration), HEPES pH = 7.5 (20 mM final concentration), EDTA (40 mM final concentration) and Brij35 (0.02 V/V% final concentration). 2 µL of enzyme solution was added to the MIN and CAL wells after the addition of the detection mixture. Fluorescence Polarization (FP) and Fluorescence Intensity (FI) measurements were performed on Tecan Infinite M1000 Pro reader 60 minutes after addition of the detection mixture. IC₅₀ values were calculated by a curve fitting the FP data points using XLfit 5.1; FI was used to identify any compounds with fluorescent properties that may interfere with the detection that could provide false data.

### B-Raf(V600E) kinase assay protocol without preincubation

The compounds were dissolved in DMSO to make 5 mM stock solutions. 12-point dilution series were made from the stock solutions in 3-fold dilution steps (5 mM to 28.225 nM) using DMSO on 384-well polypropylene microplates (Greiner 784201). Reaction buffer was prepared in Eppendorf tubes, consisting of HEPES pH = 7.5 (50 mM final concentration), DTT (1 mM final concentration), MgCl₂ (10 mM final concentration), and Brij35 (0.01 V/V% final concentration) in H₂O. Substrate + ATP solution was prepared by adding the substrate MEK1(K97M) (0.01 mg/mL final concentration) and ATP (3.60 µM final concentration ~ ATP K_{Mapp} value) to the reaction buffer. Enzyme solution was prepared by adding b-Raf(V600E) kinase (30 nM final concentration) to the reaction buffer. 6 µL of Substrate + ATP solution was added to each well of the assay plate (Greiner 784900 - black, small volume, polystyrene, flat bottom). 0.0125 nL from each concentration of the dilution series of each compound was added to the appropriate assay plate wells using a pin tool mounted on the RoMa gripper arm of a Tecan Freedom EVO workstation. DMSO without inhibitor was added to positive (MAX - "100% kinase activity") and negative (MIN - "0% kinase activity") control wells. Calibration (CAL) wells were prepared in a 12-point series containing a fixed 3.60 µM of total ATP+ADP concentration with different ATP/ADP ratios, dissolved in the reaction buffer. Each CAL solution was added to the assay plate in 6 µL volumes. 2 µL of enzyme solution was added to each well except for the MIN and CAL wells to start the kinase reaction. The assay plate was covered and the enzyme reaction was incubated at room temperature for 60 minutes. The kinase reaction was stopped by addition of 8 µL detection mixture containing Transcreener ADP Alexa633 Tracer (3 nM final concentration), Transcreener ADP² Antibody (12 µg/ml final concentration), HEPES pH = 7.5 (20 mM final concentration), EDTA (40 mM final concentration) and Brij35 (0.02 V/V% final concentration). 2 µL of enzyme solution was added to the MIN and CAL wells after the addition of the detection mixture. Fluorescence Polarization (FP) and Fluorescence Intensity (FI) measurements were performed on Tecan Infinite M1000 Pro reader 60 minutes after addition of the detection mixture. IC₅₀ values were calculated by a curve fitting the FP data points using XLfit 5.1; FI was used to identify any compounds with fluorescent properties that may interfere with the detection that could provide false data.

### B-Raf(WT) kinase assay protocol with preincubation

The compounds were dissolved in DMSO to make 5 mM stock solutions. 12-point dilution series were made from the stock solutions in 3-fold dilution steps (5 mM to 28.225 nM) using DMSO on 384-well polypropylene microplates (Greiner 784201). Reaction buffer was prepared in Eppendorf tubes, consisting of HEPES pH = 7.5 (50 mM final concentration), DTT (1 mM final concentration), MgCl₂ (10 mM final concentration), and Brij35 (0.01 V/V% final concentration) in H₂O. Substrate + ATP solution was prepared by adding the substrate MEK1(K97M) (0.01 mg/mL final concentration) and ATP (2.10 µM final concentration ~ ATP K_{Mapp} value) to the reaction buffer. Enzyme solution was prepared by adding wild-type b-Raf kinase (50 nM final concentration) to the reaction buffer. 6 µL of enzyme solution was added to each well except for the MIN and CAL wells of the assay plate (Greiner 784900 - black, small volume, polystyrene, flat bottom). 0.0125 nL from each concentration of the dilution series of each compound was added to the appropriate assay plate wells using a pin tool mounted on the RoMa gripper arm of a Tecan Freedom EVO workstation. DMSO without inhibitor was added to positive (MAX - "100% kinase activity") and negative (MIN - "0% kinase activity") control wells. The plate containing b-Raf(WT) and the test compounds was incubated for 90 minutes. 2 µL of Substrate + ATP solution was added to each well of the assay plate. The assay plate was covered and the enzyme reaction was incubated at room temperature for 60 minutes. Calibration (CAL) wells were prepared in a 12-point series containing a fixed 2.10 µM of total ATP+ADP concentration with different ATP/ADP ratios, dissolved in the reaction buffer. Each CAL solution was added to the assay plate in 6 µL volumes. The kinase reaction was stopped by addition of 8 µL detection mixture containing Transcreener ADP Alexa633 Tracer (3 nM final concentration), Transcreener ADP² Antibody (12 µg/ml final concentration), HEPES pH = 7.5 (20 mM final concentration), EDTA (40 mM final concentration) and Brij35 (0.02 V/V% final concentration). 6 µL of enzyme solution was added to the MIN and CAL wells after the addition of the detection mixture. Fluorescence Polarization (FP) and Fluorescence Intensity (FI) measurements were performed on Tecan Infinite M1000 Pro reader 60 minutes after addition of the detection mixture. IC₅₀ values were calculated by a curve fitting the FP data points using XLfit 5.1; FI was used to identify any compounds with fluorescent properties that may interfere with the detection that could provide false data.

### B-Raf(V600E) kinase assay protocol with preincubation

The compounds were dissolved in DMSO to make 5 mM stock solutions. 12-point dilution series were made from the stock solutions in 3-fold dilution steps (5 mM to 28.225 nM) using DMSO on 384-well polypropylene microplates (Greiner 784201). Reaction buffer was prepared in Eppendorf tubes, consisting of HEPES pH = 7.5 (50 mM final concentration), DTT (1 mM final concentration), MgCl₂ (10 mM final concentration), and Brij35 (0.01 V/V% final concentration) in H₂O. Substrate + ATP solution was prepared by adding the substrate MEK1(K97M) (0.01 mg/mL final concentration) and ATP (3.60 µM final concentration ~ ATP K_{Mapp} value) to the reaction buffer. Enzyme solution was prepared by adding b-Raf(V600E) kinase (35 nM final concentration) to the reaction buffer. 6 µL of enzyme solution was added to each well except for the MIN and CAL wells of the assay plate (Greiner 784900 - black, small volume, polystyrene, flat bottom). 0.0125 nL from each concentration of the dilution series of each compound was added to the appropriate assay plate wells using a pin tool mounted on the RoMa gripper arm of a Tecan Freedom EVO workstation. DMSO without inhibitor was added to positive (MAX - "100% kinase activity") and negative (MIN - "0% kinase activity") control wells. The plate containing b-Raf(WT) and the test compounds was incubated for 90 minutes. 2 µL of Substrate + ATP solution was added to each well of the assay plate. The assay plate was covered and the enzyme reaction was incubated at room temperature for 60 minutes. Calibration (CAL) wells were prepared in a 12-point series containing a fixed 3.60 µM of total ATP+ADP concentration with different ATP/ADP ratios, dissolved in the reaction buffer. Each CAL solution was added to the assay plate in 6 µL volumes. The kinase reaction was stopped by addition of 8 µL detection mixture containing Transcreener ADP Alexa633 Tracer (3 nM final concentration), Transcreener ADP² Antibody (12 µg/ml final concentration), HEPES pH = 7.5 (20 mM final concentration), EDTA (40 mM final concentration) and Brij35 (0.02 V/V% final concentration). 6 µL of enzyme solution was added to the MIN and CAL wells after the addition of the detection mixture. Fluorescence Polarization (FP) and Fluorescence Intensity (FI) measurements were performed on Tecan Infinite M1000 Pro reader 60 minutes after addition of the detection mixture. IC₅₀ values were calculated by a curve fitting the FP data points using XLfit 5.1; FI was used to identify any compounds with fluorescent properties that may interfere with the detection and could provide false data.

**Table: In vitro IC₅₀ values of the compounds on b-Raf(WT) and b-Raf(V600E) kinases.**

| | b-Raf(WT) [µM] | | b-Raf(V600E) [µM] | |
|---|---|---|---|---|
| Compound | w/o preincubation | w/ preincubation | w/o preincubation | w/ preincubation |
| Example 179 [AXL22064] | | | 3.743 | |
| Example 56 [AXL22074] | | | 0.509 | |
| Example 78 [AXL22101] | 0.384 | 0.239 | 0.213 | 0.138 |
| Example 182 [AXL22108] | | | >7.800 | |
| Example 97 [AXL22123] | | | 0.215 | |
| Example 151 [AXL22134] | | | 7.371 | |
| Example 154 [AXL22139] | | | >7.800 | |
| Example 174 [AXL22160] | | | 3.478 | |
| Example 188 [AXL22176] | | | >7.800 | |
| Vemurafenib | 0.503 | 0.332 | 0.358 | 0.167 |

### Primary cell viability screening

Two cell lines were used in primary screening: the AXL-expressing Hs578t and AXL-non-expressing MCF-7 cell lines. 1000 cells/well were seeded in 384-well plates in 30ul RPMI 1640 medium (Gibco). Medium was complemented with 10% FBS (Gibco), Penicillin/Streptomycin (Gibco) and incubated overnight. All compounds were dissolved in DMSO. Cells were treated for 72h at 1uM and 5uM final concentration, measurements were performed in duplicates. The following internal positive control compounds were used during the measurements: Foretinib, Cabozantinib, Staurosporine, BMS777607, IM_1309_T. The total DMSO concentration was set to 0.1% for all wells. Cell viability was detected using CellTiter-Glo^{®} Luminescent Cell Viability Assay (Promega, G7570) according to user manual. Measurements were performed on TECAN infinite M1000 Pro. Treated wells were correlated to average DMSO control and viability is presented in % (where 100% is the averaged DMSO control viability, 0% is minimum viability).

Compounds were considered as potentially active if showed decreased cell viability by at least 30% at 5uM treatment. These compounds were further tested for IC50 on both cell lines.

**IC50 measurements.** Potent compounds were filtered in primary screening as previously described. The Hs578t and MCF-7 cell lines were seeded at 1000 cells/well density in 384-well plates in 30 µL of medium supplemented with 10% FBS + Penicillin/Streptomycin and incubated overnight. A ten point two- or threefold dilution series were prepared, and cells were treated for 72h. Foretinib, Cabozantinib, Staurosporine, BMS777607 and IM_1309_T were used as internal positive control compounds. Cell viability was tested using the CellTiter-Glo^{®} Luminescent Cell Viability Assay (Promega, G7570) according to the user manual. Luminescence signal was detected on TECAN infinite M1000 Pro. Treated wells were correlated to average DMSO control, and viability was calculated and presented in %. Values were log transformed and IC50 curve was prepared using 4 parameter fitting (constrain set to constant zero). Z' values were calculated in order to validate if signal noise ratio was appropriate in the assay. DMSO treated control and signal intensity in empty wells were used for calculation.

**Table: Viability measurement with the Hs578t and MCF-7 cell lines.**

| | Hs578t @ 1µM | | Hs578t @ 5µM | | MCF-7 @ 1µM | | MCF-7 @ 5µM | |
|---|---|---|---|---|---|---|---|---|
| ID | Viability (%) | SD | Viability (%) | SD | Viability (%) | SD | Viability (%) | SD |
| Example 1 [AXL22001] | 65.0% | 10.5% | 3.6% | 1.7% | 148% | 9% | 126% | 26% |
| Example 2 [AXL22002] | 75.4% | 16.6% | 33.6% | 45.8% | 159% | 5% | 77% | 47% |
| Example 3 [AXL22005] | 42.6% | 3.3% | 39.8% | 32.4% | 131% | 2% | 154% | 29% |
| Example 4 [AXL22006] | 101.4% | 3.7% | 83.7% | 34.6% | 145% | 34% | 141% | 15% |
| Example 5 [AXL22007] | 86.6% | 1.8% | 61.7% | 61.6% | 145% | 1% | 123% | 37% |
| Example 6 [AXL22008] | 65.9% | 1.0% | 69.7% | 28.1% | 149% | 12% | 121% | 32% |
| Example 7 [AXL22009] | 96.6% | 2.9% | 60.9% | 1.8% | 118% | 10% | 89% | 4% |
| Example 8 [AXL22010] | 103.6% | 3.8% | 86.7% | 3.5% | 142% | 4% | 99% | 34% |
| Example 9 [AXL22012] | 92.0% | 0.8% | 58.7% | 13.8% | 127% | 5% | 110% | 8% |
| Example 10 [AXL22013] | 99.2% | 1.1% | 63.4% | 1.9% | 158% | 4% | 100% | 0% |
| Example 11 [AXL22016] | 96.9% | 0.3% | 73.1% | 10.7% | 143% | 9% | 94% | 1% |
| Example 12 [AXL22017] | 84.3% | 45.3% | 86.9% | 7.2% | 92% | 9% | 88% | 7% |
| Example 13 [AXL22018] | 103.9% | 1.8% | 2.3% | 0.5% | 161% | 0% | 108% | 22% |
| Example 14 [AXL22020] | 81.1% | 8.1% | 53.9% | 4.7% | 150% | 2% | 125% | 3% |
| Example 15 [AXL22021] | 95.1% | 0.7% | 66.7% | 0.3% | 138% | 2% | 95% | 2% |
| Example 16 [AXL22022] | 103.4% | 13.9% | 71.4% | 0.6% | 130% | 18% | 138% | 25% |
| Example 17 [AXL22023] | 107.3% | 10.8% | 81.2% | 2.4% | 201% | 79% | 111% | 3% |
| Example 204 AXL22024 | 109.5% | 2.3% | 72.3% | 22% | 93% | 9% | 82% | 13% |
| Example 18 [AXL22025] | 105.3% | 0.3% | 95.6% | 11.3% | 161% | 16% | 116% | 10% |
| Example 19 [AXL22026] | 107.7% | 0.7% | 86.0% | 8.1% | 165% | 8% | 102% | 3% |
| Example 20 [AXL22027] | 104.9% | 8.6% | 66.8% | 2.5% | 149% | 5% | 100% | 2% |
| Example 21 [AXL22028] | 95.7% | 5.8% | 79.6% | 1.0% | 151% | 2% | 103% | 1% |
| Example 22 [AXL22030] | 100.2% | 0.2% | 82.1% | 0.1% | 150% | 5% | 115% | 18% |
| Example 139 [AXL22031] | 98.7% | 8.8% | 1.6% | 0.5% | 123% | 10% | 79% | 11% |
| Example 23 [AXL22032] | 105.1% | 2.4% | 60.9% | 2.1% | 155% | 4% | 120% | 1% |
| Example 24 [AXL22033] | 100.2% | 4.2% | 84.5% | 1.9% | 106% | 2% | 90% | 6% |
| Example 25 [AXL22034] | 88.8% | 0.8% | 2.6% | 0.4% | 125% | 7% | 134% | 64% |
| Example 26 [AXL22035] | 117.1% | 3.8% | 96.9% | 0.3% | 138% | 21% | 110% | 74% |
| Example 27 [AXL22036] | 110.1% | 4.8% | 92.5% | 1.6% | 138% | 11% | 146% | 43% |
| Example 28 [AXL22037] | 115.8% | 1.0% | 99.6% | 0.8% | 132% | 16% | 129% | 2% |
| Example 29 [AXL22038] | 102.7% | 7.4% | 74.3% | 1.7% | 132% | 14% | 126% | 13% |
| Example 30 [AXL22039] | 97.1% | 7.6% | 76.1% | 4.5% | 153% | 36% | 109% | 8% |
| Example 31 [AXL22040] | 94.0% | 5.5% | 38.5% | 8.4% | 117% | 4% | 135% | 32% |
| Example 32 [AXL22041] | 117.7% | 8.3% | 91.5% | 0.8% | 123% | 2% | 114% | 5% |
| Example 140 [AXL22042] | 94.6% | 1.3% | 26.0% | 7.6% | 88% | 1% | 52% | 4% |
| Example 141 [AXL22043] | 87.9% | 9.8% | 20.3% | 1.6% | 78% | 23% | 65% | 8% |
| Example 33 [AXL22044] | 113.8% | 2.7% | 95.5% | 6.0% | 132% | 31% | 142% | 7% |
| Example 34 [AXL22045] | 117.8% | 2.6% | 102.2% | 0.9% | 133% | 20% | 130% | 4% |
| Example 35 [AXL22046] | 118.0% | 5.1% | 104.2% | 2.4% | 142% | 1% | 128% | 3% |
| Example 36 [AXL22047] | 113.8% | 5.4% | 104.7% | 3.3% | 138% | 4% | 138% | 4% |
| Example 37 [AXL22048] | 116.4% | 16.4% | 111.8% | 7.7% | 118% | 13% | 126% | 1% |
| Example 38 [AXL22049] | 129.0% | 0.9% | 112.3% | 2.5% | 134% | 7% | 127% | 6% |
| Example 39 [AXL22050] | 125.0% | 4.7% | 98.8% | 0.1% | 127% | 15% | 133% | 8% |
| Example 40 [AXL22051] | 120.4% | 6.9% | 117.9% | 3.9% | 140% | 12% | 128% | 7% |
| Example 41 [AXL22052] | 120.6% | 0.3% | 108.9% | 6.4% | 135% | 7% | 128% | 7% |
| Example 42 [AXL22053] | 120.5% | 3.3% | 114.3% | 6.6% | 137% | 3% | 121% | 3% |
| Example 43 [AXL22054] | 122.5% | 8.2% | 122.8% | 6.7% | 145% | 2% | 137% | 4% |
| Example 44 [AXL22055] | 113.1% | 7.1% | 105.6% | 7.6% | 128% | 4% | 109% | 2% |
| Example 142 [AXL22056] | 109.0% | 14.1% | 41.2% | 43.8% | 85% | 11% | 37% | 6% |
| Example 143 [AXL22057] | 97.4% | 2.3% | 57.5% | 5.9% | 90% | 8% | 43% | 10% |
| Example 45 [AXL22058] | 116.6% | 10.4% | 103.9% | 1.3% | 126% | 9% | 116% | 7% |
| Example 46 [AXL22059] | 123.2% | 0.5% | 113.3% | 0.6% | 129% | 2% | 123% | 2% |
| Example 47 [AXL22060] | 121.4% | 3.1% | 102.8% | 6.7% | 129% | 2% | 130% | 1% |
| Example 48 [AXL22061] | 105.7% | 7.5% | 99.1% | 4.9% | 134% | 4% | 121% | 7% |
| Example 177 [AXL22062] | 99.2% | 10.6% | 82.4% | 7.6% | 82% | 16% | 74% | 15% |
| Example 178 [AXL22063] | 1.5% | 0.2% | 116.5% | 4.9% | 2% | 0% | 114% | 4% |
| Example 179 [AXL22064] | 113.4% | 1.3% | 67.2% | 2.6% | 102% | 2% | 67% | 1% |
| Example 180 [AXL22065] | 128.4% | 39.1% | 74.4% | 17.7% | 103% | 11% | 70% | 1% |
| Example 49 [AXL22066] | 122.3% | 0.3% | 112.6% | 1.3% | 124% | 2% | 117% | 7% |
| Example 50 [AXL22067] | 116.7% | 5.2% | 108.5% | 2.5% | 125% | 8% | 128% | 1% |
| Example 51 [AXL22068] | 108.7% | 1.0% | 106.9% | 10.8% | 107% | 8% | 95% | 7% |
| Example 52 [AXL22069] | 110.2% | 0.9% | 92.4% | 3.3% | 113% | 2% | 105% | 3% |
| Example 53 [AXL22070] | 104.7% | 2.3% | 91.5% | 3.5% | 108% | 7% | 86% | 26% |
| Example 54 [AXL22071] | 109.0% | 1.7% | 106.0% | 7.0% | 104% | 1% | 91% | 6% |
| Example 55 [AXL22072] | 110.3% | 2.0% | 110.4% | 7.8% | 107% | 3% | 101% | 2% |
| Example 56 [AXL22074] | 100.1% | 2.3% | 67.2% | 1.7% | 101% | 5% | 57% | 0% |
| Example 57 [AXL22075] | 100.4% | 3.1% | 65.7% | 0.9% | 102% | 2% | 69% | 5% |
| Example 58 [AXL22076] | 112.1% | 1.3% | 118.7% | 6.8% | 106% | 2% | 98% | 3% |
| Example 59 [AXL22077] | 115.3% | 7.7% | 106.4% | 1.6% | 106% | 3% | 58% | 22% |
| Example 60 [AXL22078] | 100.4% | 6.1% | 79.6% | 7.8% | 73% | 33% | 63% | 2% |
| Example 61 [AXL22079] | 111.6% | 1.9% | 111.9% | 1.8% | 109% | 1% | 96% | 0% |
| Example 62 [AXL22080] | 109.0% | 3.5% | 119.5% | 2.9% | 102% | 1% | 101% | 5% |
| Example 63 [AXL22081] | 111.5% | 4.5% | 105.9% | 1.3% | 106% | 1% | 82% | 6% |
| Example 64 [AXL22082] | 115.7% | 6.5% | 61.6% | 8.7% | 106% | 6% | 58% | 6% |
| Example 65 [AXL22083] | 109.4% | 3.5% | 97.3% | 0.4% | 107% | 4% | 90% | 3% |
| Example 181 [AXL22084] | 54.6% | 0.2% | 39.6% | 0.3% | 61% | 2% | 42% | 0% |
| Example 66 [AXL22088] | 92.6% | 1.5% | 49.9% | 3.4% | 96% | 3% | 85% | 3% |
| Example 67 [AXL22089] | 106.5% | 7.4% | 86.3% | 2.6% | 110% | 3% | 102% | 4% |
| Example 144 [AXL22090] | 104.1% | 1.0% | 67.1% | 8.3% | 112% | 4% | 84% | 1% |
| Example 68 [AXL22091] | 84.2% | 1.2% | 38.7% | 2.1% | 81% | 5% | 92% | 3% |
| Example 69 [AXL22092] | 70.4% | 0.5% | 27.5% | 8.8% | 81% | 0% | 50% | 2% |
| Example 70 [AXL22093] | 114.2% | 2.1% | 99.6% | 1.7% | 107% | 1% | 107% | 4% |
| Example 71 [AXL22094] | 111.1% | 2.5% | 86.3% | 0.9% | 111% | 0% | 104% | 3% |
| Example 72 [AXL22095] | 84.3% | 1.9% | 57.9% | 1.4% | 83% | 2% | 69% | 0% |
| Example 73 [AXL22096] | 106.4% | 1.3% | 83.4% | 7.1% | 112% | 1% | 106% | 8% |
| Example 74 [AXL22097] | 89.9% | 7.3% | 44.1% | 5.7% | 110% | 7% | 86% | 4% |
| Example 75 [AXL22098] | 97.0% | 1.7% | 41.0% | 9.4% | 88% | 5% | 63% | 2% |
| Example 76 [AXL22099] | 113.0% | 3.8% | 93.7% | 5.9% | 105% | 5% | 93% | 9% |
| Example 77 [AXL22100] | 103.9% | 0.4% | 68.4% | 3.6% | 104% | 2% | 83% | 4% |
| Example 78 [AXL22101] | 94.3% | 6.2% | 37.5% | 2.2% | 106% | 3% | 94% | 2% |
| Example 79 [AXL22102] | 95.5% | 2.5% | 30.5% | 2.0% | 94% | 8% | 62% | 5% |
| Example 80 [AXL22103] | 110.1% | 1.8% | 86.7% | 3.7% | 111% | 4% | 113% | 4% |
| Example 81 [AXL22104] | 104.1% | 2.3% | 74.5% | 2.8% | 112% | 1% | 104% | 6% |
| Example 82 [AXL22105] | 85.1% | 3.5% | 55.7% | 1.4% | 87% | 5% | 69% | 4% |
| Example 83 [AXL22106] | 87.2% | 5.1% | 64.4% | 9.4% | 122% | 6% | 101% | 6% |
| Example 84 [AXL22107] | 80.9% | 4.2% | 74.6% | 0.2% | 110% | 7% | 93% | 24% |
| Example 182 [AXL22108] | 98.8% | 5.1% | 1.4% | 0.1% | 95% | 6% | 2% | 1% |
| Example 183 [AXL22109] | 56.9% | 2.2% | 45.9% | 5.6% | 76% | 2% | 67% | 3% |
| Example 184 [AXL22110] | 97.6% | 1.1% | 1.4% | 0.3% | 90% | 1% | 2% | 0% |
| Example 85 [AXL22111] | 109.9% | 1.7% | 1.3% | 0.2% | 130% | 2% | 23% | 31% |
| Example 86 [AXL22112] | 107.1% | 3.7% | 50.9% | 1.9% | 128% | 2% | 78% | 21% |
| Example 87 [AXL22113] | 105.9% | 2.1% | 109.2% | 18.7% | 124% | 17% | 100% | 13% |
| Example 88 [AXL22114] | 109.5% | 1.9% | 103.7% | 7.7% | 122% | 1% | 110% | 7% |
| Example 89 [AXL22115] | 105.0% | 3.8% | 45.4% | 2.2% | 124% | 9% | 105% | 7% |
| Example 90 [AXL22116] | 100.6% | 4.2% | 51.0% | 5.6% | 124% | 2% | 86% | 7% |
| Example 91 [AXL22117] | 105.4% | 3.0% | 97.5% | 3.2% | 122% | 0% | 99% | 9% |
| Example 92 [AXL22118] | 124.3% | 1.6% | 103.8% | 7.0% | 132% | 4% | 124% | 14% |
| Example 93 [AXL22119] | 107.6% | 0.7% | 51.8% | 16.0% | 125% | 2% | 97% | 6% |
| Example 94 [AXL22120] | 94.4% | 2.4% | 48.6% | 1.1% | 133% | 6% | 92% | 12% |
| Example 95 [AXL22121] | 103.3% | 0.9% | 104.9% | 3.9% | 136% | 5% | 114% | 4% |
| Example 96 [AXL22122] | 107.5% | 1.4% | 107.3% | 1.0% | 144% | 6% | 124% | 26% |
| Example 97 [AXL22123] | 73.4% | 5.7% | 1.2% | 0.3% | 106% | 11% | 7% | 4% |
| Example 98 [AXL22124] | 100.1% | 1.2% | 1.1% | 0.0% | 111% | 5% | 8% | 10% |
| Example 99 [AXL22125] | 76.6% | 0.0% | 79.6% | 33.6% | 115% | 4% | 83% | 15% |
| Example 100 [AXL22126] | 75.9% | 7.1% | 72.9% | 21.6% | 107% | 1% | 76% | 8% |
| Example 145 [AXL22127] | 28.3% | 1.2% | 31.9% | 6.3% | 44% | 7% | 48% | 3% |
| Example 146 [AXL22128] | 2.6% | 0.6% | 0.8% | 0.1% | 25% | 8% | 3% | 1% |
| Example 147 [AXL22129] | 64.1% | 0.8% | 63.8% | 3.8% | 49% | 4% | 50% | 1% |
| Example 148 [AXL22130] | 42.6% | 5.5% | 1.0% | 0.3% | 47% | 1% | 28% | 4% |
| Example 149 [AXL22131] | 39.1% | 2.4% | 3.6% | 0.5% | 47% | 7% | 32% | 3% |
| AXL22132 | 57.8% | 81.3% | 38.7% | 54.3% | 50% | 70% | 29% | 40% |
| Example 150 [AXL22133] | 44.2% | 1.5% | 4.1% | 0.8% | 44% | 9% | 32% | 5% |
| Example 151 [AXL22134] | 43.0% | 60.4% | 22.2% | 31.2% | 12% | 16% | 18% | 25% |
| Example 152 [AXL22136] | 86.9% | 21.0% | 90.2% | 13.1% | 90% | 16% | 71% | 3% |
| Example 153 [AXL22137] | 93.0% | 0.6% | 49.1% | 52.9% | 65% | 13% | 48% | 30% |
| Example 154 [AXL22139] | 79.0% | 24.0% | 25.8% | 35.5% | 74% | 27% | 45% | 12% |
| Example 155 [AXL22140] | 97.5% | 3.3% | 18.9% | 9.8% | 61% | 12% | 30% | 7% |
| Example 156 [AXL22141] | 106.6% | 18.7% | 6.0% | 7.1% | 63% | 6% | 42% | 13% |
| Example 157 [AXL22143] | 90.7% | 0.5% | 53.6% | 62.5% | 73% | 15% | 47% | 21% |
| Example 158 [AXL22144] | 85.8% | 17.1% | 51.3% | 42.6% | 75% | 13% | 52% | 25% |
| Example 159 [AXL22145] | 95.7% | 13.4% | 103.6% | 3.0% | 87% | 4% | 63% | 3% |
| Example 160 [AXL22146] | 62.7% | 55.2% | 48.1% | 67.2% | 101% | 20% | 46% | 28% |
| Example 161 [AXL22147] | 47.0% | 63.7% | 47.6% | 66.6% | 72% | 10% | 47% | 32% |
| Example 162 [AXL22148] | 44.9% | 63.2% | 44.9% | 63.1% | 38% | 53% | 36% | 50% |
| Example 163 [AXL22149] | 88.6% | 2.9% | 86.0% | 7.7% | 120% | 54% | 50% | 14% |
| Example 164 [AXL22150] | 45.3% | 63.6% | 6.1% | 8.6% | 28% | 39% | 25% | 35% |
| Example 165 [AXL22151] | 87.9% | 3.4% | 10.5% | 2.9% | 50% | 6% | 46% | 12% |
| Example 166 [AXL22152] | 96.9% | 27.8% | 8.0% | 4.4% | 50% | 10% | 40% | 2% |
| Example 167 [AXL22153] | 105.1% | 4.3% | 47.4% | 58.3% | 82% | 6% | 48% | 1% |
| Example 168 [AXL22154] | 106.7% | 8.2% | 8.2% | 9.8% | 62% | 1% | 37% | 3% |
| Example 169 [AXL22155] | 101.2% | 2.4% | 1.3% | 0.2% | 64% | 14% | 36% | 4% |
| Example 170 [AXL22156] | 59.7% | 8.3% | 46.9% | 5.7% | 41% | 7% | 23% | 0% |
| Example 171 [AXL22157] | 77.0% | 1.9% | 10.6% | 3.2% | 35% | 0% | 20% | 1% |
| Example 172 [AXL22158] | 36.2% | 0.5% | 4.6% | 3.7% | 49% | 2% | 27% | 1% |
| Example 173 [AXL22159] | 69.6% | 4.6% | 1.3% | 1.4% | 41% | 0% | 20% | 1% |
| Example 174 [AXL22160] | 131.4% | 3.1% | 162.4% | 0.7% | 87% | 1% | 81% | 2% |
| Example 175 [AXL22161] | 92.2% | 31.4% | 2.7% | 2.4% | 55% | 12% | 36% | 4% |
| Example 176 [AXL22162] | 93.8% | 16.3% | 1.5% | 1.1% | 50% | 16% | 32% | 8% |
| Example 101 [AXL22163] | 145.0% | 62.2% | 88.1% | 119.2% | 105% | 54% | 95% | 79% |
| Example 102 [AXL22164] | 168.5% | 18.4% | 166.8% | 12.8% | 135% | 16% | 138% | 9% |
| Example 103 [AXL22165] | 102.0% | 8.7% | 104.9% | 21.3% | 106% | 9% | 106% | 7% |
| Example 104 [AXL22166] | 89.0% | 0.2% | 93.3% | 1.8% | 106% | 14% | 92% | 3% |
| Example 105 [AXL22167] | 106.6% | 149.6% | 75.1% | 105.6% | 72% | 101% | 12% | 17% |
| Example 106 [AXL22168] | 187.1% | 2.9% | 173.7% | 15.4% | 147% | 4% | 121% | 37% |
| Example 107 [AXL22169] | 89.9% | 3.4% | 90.3% | 2.7% | 107% | 1% | 109% | 0% |
| Example 108 [AXL22170] | 186.1% | 48.3% | 198.4% | 2.5% | 149% | 11% | 146% | 1% |
| Example 185 [AXL22171] | 185.6% | 0.6% | 129.4% | 71.6% | 135% | 4% | 117% | 41% |
| Example 186 [AXL22172] | 121.6% | 36.8% | 87.5% | 4.9% | 113% | 66% | 72% | 24% |
| Example 109 [AXL22173] | 127.1% | 68.7% | 137.4% | 82.6% | 110% | 37% | 100% | 70% |
| Example 110 [AXL22174] | 181.4% | 37.0% | 193.3% | 25.7% | 140% | 13% | 137% | 6% |
| Example 187 [AXL22175] | 59.8% | 1.0% | 51.5% | 7.8% | 43% | 3% | 31% | 0% |
| Example 188 [AXL22176] | 57.4% | 12.3% | 49.6% | 11.6% | 52% | 5% | 32% | 4% |
| Example 189 [AXL22177] | 54.7% | 1.1% | 43.2% | 6.9% | 61% | 5% | 44% | 2% |
| Example 111 [AXL22178] | 91.2% | 3.2% | 88.2% | 11.7% | 111% | 10% | 110% | 5% |
| Example 112 [AXL22179] | 143.5% | 28.3% | 133.9% | 26.0% | 85% | 4% | 87% | 10% |
| Example 113 [AXL22180] | 100.4% | 4.7% | 100.5% | 10.6% | 102% | 0% | 102% | 8% |
| Example 114 [AXL22181] | 81.8% | 1.7% | 96.8% | 5.7% | 99% | 8% | 99% | 1% |
| Example 115 [AXL22182] | 50.4% | 1.4% | 42.2% | 0.3% | 26% | 3% | 97% | 3% |
| Example 116 [AXL22183] | 143.3% | 14.8% | 117.1% | 6.6% | 94% | 2% | 83% | 2% |
| Example 117 [AXL22184] | 92.6% | 3.6% | 103.9% | 0.8% | 101% | 8% | 108% | 5% |
| Example 118 [AXL22185] | 105.7% | 2.6% | 106.8% | 8.2% | 113% | 4% | 112% | 2% |
| Example 119 [AXL22186] | 102.4% | 0.9% | 79.1% | 0.7% | 117% | 0% | 111% | 5% |
| Example 120 [AXL22187] | 58.2% | 1.9% | 60.8% | 0.1% | 32% | 7% | 23% | 4% |
| Example 190 [AXL22188] | 30.8% | 43.2% | 46.8% | 65.8% | 35% | 49% | 23% | 33% |
| Example 191 [AXL22189] | 60.8% | 0.0% | 47.1% | 3.9% | 46% | 4% | 34% | 2% |
| Example 192 [AXL22190] | 66.2% | 18.2% | 55.4% | 0.8% | 51% | 2% | 31% | 0% |
| Example 193 [AXL22191] | 108.7% | 19.5% | 42.2% | 56.6% | 85% | 29% | 49% | 12% |
| Example 194 [AXL22192] | 109.9% | 20.6% | 34.3% | 45.9% | 90% | 6% | 58% | 2% |
| Example 195 [AXL22193] | 78.9% | 3.4% | 85.9% | 5.9% | 66% | 22% | 45% | 13% |
| Example 196 [AXL22194] | 50.8% | 0.4% | 32.3% | 1.6% | 43% | 2% | 19% | 2% |
| Example 197 [AXL22195] | 56.3% | 0.7% | 44.7% | 0.9% | 33% | 1% | 23% | 1% |
| Example 198 [AXL22196] | 64.2% | 11.9% | 46.6% | 13.6% | 55% | 1% | 32% | 7% |
| Example 199 [AXL22197] | 104.1% | 1.0% | 112.9% | 3.7% | 43% | 3% | 29% | 2% |
| Example 200 [AXL22198] | 102.4% | 5.5% | 116.6% | 3.0% | 32% | 1% | 28% | 1% |
| Example 201 [AXL22199] | 92.0% | 8.3% | 104.3% | 15.0% | 41% | 2% | 29% | 1% |
| Example 202 [AXL22200] | 69.2% | 9.7% | 69.2% | 1.6% | 67% | 10% | 62% | 1% |
| Example 121 [AXL22202] | 89.3% | 2.2% | 69.7% | 3.3% | 97% | 0% | 76% | 4% |
| Example 122 [AXL22203] | 99.0% | 3.0% | 57.2% | 6.3% | 91% | 5% | 77% | 2% |
| Example 123 [AXL22204] | 91.0% | 4.1% | 56.3% | 2.5% | 89% | 0% | 64% | 1% |
| Example 124 [AXL22205] | 149.6% | 39.5% | 81.6% | 5.2% | 74% | 58% | 70% | 43% |
| Example 125 [AXL22206] | 158.7% | 1.8% | 98.6% | 21.9% | 132% | 5% | 88% | 1% |
| Example 126 [AXL22207] | 108.0% | 8.6% | 78.6% | 0.9% | 96% | 6% | 77% | 0% |
| Example 127 [AXL22208] | 100.1% | 2.9% | 75.6% | 7.9% | 101% | 18% | 67% | 2% |
| Example 128 [AXL22209] | 105.2% | 4.3% | 68.4% | 8.9% | 98% | 1% | 73% | 4% |
| Example 129 [AXL22210] | 86.1% | 3.3% | 59.4% | 2.7% | 72% | 1% | 53% | 2% |
| Example 130 [AXL22211] | 86.6% | 0.0% | 56.2% | 2.5% | 91% | 1% | 73% | 11% |
| Example 131 [AXL22212] | 76.8% | 0.9% | 61.0% | 1.4% | 77% | 0% | 63% | 0% |
| Example 132 [AXL22213] | 80.1% | 8.5% | 58.0% | 0.5% | 90% | 5% | 68% | 8% |
| Example 133 [AXL22214] | 130.5% | 33.7% | 96.6% | 16.7% | 133% | 1% | 92% | 2% |
| Example 134 [AXL22215] | 93.9% | 3.3% | 83.5% | 27.8% | 96% | 12% | 60% | 3% |
| Example 135 [AXL22216] | 95.0% | 0.9% | 65.9% | 1.4% | 91% | 3% | 69% | 2% |
| Example 136 [AXL22217] | 84.0% | 8.5% | 1.2% | 8.1% | 69% | 9% | 63% | 5% |
| Example 137 [AXL22218] | 100.2% | 0.5% | 100.6% | 8.8% | 115% | 2% | 113% | 5% |
| Example 138 [AXL22219] | 96.4% | 0.7% | 93.2% | 0.7% | 114% | 0% | 91% | 3% |

**Table: Cellular IC₅₀ values on the Hs578t and MCF-7 cell lines.**

| | Hs578t | | MCF-7 | |
|---|---|---|---|---|
| ID | IC₅₀ [µM] | z' | IC₅₀ [µM] | z' |
| Example 1 [AXL22001] | 3.1 | 0.46 | 22.3 | 0.83 |
| Example 2 [AXL22002] | 4.6 | 0.46 | 5.9 | 0.83 |
| Example 3 [AXL22005] | 2.2 | 0.46 | 16.6 | 0.83 |
| Example 5 [AXL22007] | 6.5 | 0.46 | 9.1 | 0.83 |
| Example 6 [AXL22008] | 2.6 | 0.68 | >20 | 0.65 |
| Example 7 [AXL22009] | 7.1 | 0.46 | 11.1 | 0.83 |
| Example 9 [AXL22012] | 6.2 | 0.46 | 9.0 | 0.83 |
| Example 10 [AXL22013] | 9.9 | 0.46 | 14.0 | 0.83 |
| Example 13 [AXL22018] | 1.8 | 0.46 | 1.5 | 0.83 |
| Example 14 [AXL22020] | 1.6 | 0.46 | 22.6 | 0.83 |
| Example 15 [AXL22021] | 29.9 | 0.46 | 7.5 | 0.83 |
| Example 20 [AXL22027] | 14.4 | 0.46 | 11.6 | 0.83 |
| Example 139 [AXL22031] | 4.2 | 0.46 | 0.8 | 0.83 |
| Example 23 [AXL22032] | 2.9 | 0.46 | 7.6 | 0.83 |
| Example 25 [AXL22034] | 1.9 | 0.46 | 9.3 | 0.83 |
| Example 31 [AXL22040] | 2.6 | 0.46 | 10.8 | 0.83 |
| Example 140 [AXL22042] | 3.1 | 0.46 | 0.4 | 0.83 |
| Example 141 [AXL22043] | 4.9 | 0.46 | 0.3 | 0.83 |
| Example 142 [AXL22056] | 5.9 | 0.46 | 1.1 | 0.83 |
| Example 143 [AXL22057] | 5.0 | 0.46 | 1.2 | 0.83 |
| Example 178 [AXL22063] | 15.5 | 0.65 | >30 | 0.54 |
| Example 179 [AXL22064] | 4.3 | 0.46 | 1.9 | 0.83 |
| Example 56 [AXL22074] | 5.7 | 0.79 | 9.9 | 0.76 |
| Example 57 [AXL22075] | 7.9 | 0.79 | 12.5 | 0.76 |
| Example 64 [AXL22082] | 2.6 | 0.79 | 2.9 | 0.76 |
| Example 181 [AXL22084] | 0.9 | 0.79 | 0.4 | 0.76 |
| Example 66 [AXL22088] | 1.8 | 0.79 | 55.0 | 0.76 |
| Example 144 [AXL22090] | 10.8 | 0.79 | 41.1 | 0.76 |
| Example 68 [AXL22091] | 2.9 | 0.75 | 17.0 | 0.81 |
| Example 69 [AXL22092] | 2.7 | 0.79 | 7.8 | 0.76 |
| Example 72 [AXL22095] | 2.8 | 0.79 | 10.6 | 0.76 |
| Example 74 [AXL22097] | 2.9 | 0.79 | 29.5 | 0.76 |
| Example 75 [AXL22098] | 2.9 | 0.79 | 9.4 | 0.76 |
| Example 77 [AXL22 100] | 9.4 | 0.79 | 23.5 | 0.76 |
| Example 78 [AXL22101] | 1.8 | 0.79 | 22.1 | 0.76 |
| Example 79 [AXL22102] | 2.5 | 0.79 | 9.0 | 0.76 |
| Example 81 [AXL22104] | 8.7 | 0.79 | 15.8 | 0.76 |
| Example 82 [AXL22105] | 4.1 | 0.79 | 8.7 | 0.76 |
| Example 83 [AXL22106] | 23.1 | 0.68 | #N/A | |
| Example 182 [AXL22108] | 2.9 | 0.79 | 4.1 | 0.76 |
| Example 183 [AXL22109] | 1.4 | 0.79 | 21.6 | 0.76 |
| Example 184 [AXL22110] | 7.7 | 0.79 | 3.1 | 0.76 |
| Example 85 [AXL22111] | 2.6 | 0.68 | 6.1 | 0.87 |
| Example 86 [AXL22112] | 8.2 | 0.68 | >20 | 0.87 |
| Example 89 [AXL22115] | 18.9 | 0.68 | >30 | 0.76 |
| Example 90 [AXL22116] | 13.5 | 0.68 | >30 | 0.76 |
| Example 93 [AXL22119] | 15.8 | 0.68 | >30 | 0.76 |
| Example 94 [AXL22120] | 15.3 | 0.68 | >20 | 0.87 |
| Example 97 [AXL22123] | 2.4 | 0.68 | 12.3 | 0.87 |
| Example 98 [AXL22124] | 2.7 | 0.68 | 5.6 | 0.87 |
| Example 145 [AXL22127] | 1.9 | 0.75 | 1.8 | 0.76 |
| Example 146 [AXL22128] | 0.7 | 0.75 | 0.4 | 0.76 |
| Example 147 [AXL22129] | 15.6 | 0.75 | 2.7 | 0.76 |
| Example 148 [AXL22130] | 0.1 | 0.75 | 0.4 | 0.81 |
| Example 149 [AXL22131] | 0.1 | 0.75 | 0.5 | 0.81 |
| Example 203 AXL22132 | 2.5 | 0.68 | 6.5 | 0.76 |
| Example 150 [AXL22133] | 0.0 | 0.75 | 0.4 | 0.81 |
| Example 151 [AXL22134] | 0.1 | 0.68 | 0.4 | 0.76 |
| Example 153 [AXL22137] | 1.2 | 0.75 | 0.9 | 0.76 |
| Example 154 [AXL22139] | 0.7 | 0.75 | 1.4 | 0.76 |
| Example 157 [AXL22143] | 14.9 | 0.75 | 4.3 | 0.76 |
| Example 158 [AXL22144] | >30 | 0.75 | 4.4 | 0.76 |
| Example 160 [AXL22146] | 0.2 | 0.75 | 0.1 | 0.76 |
| Example 161 [AXL22147] | 18.2 | 0.75 | 8.2 | 0.76 |
| Example 162 [AXL22148] | 7.6 | 0.75 | 0.4 | 0.54 |
| Example 164 [AXL22150] | 0.9 | 0.75 | 0.4 | 0.54 |
| Example 165 [AXL22151] | 1.3 | 0.75 | 0.3 | 0.54 |
| Example 166 [AXL22152] | 1.4 | 0.75 | 0.2 | 0.54 |
| Example 167 [AXL22153] | 1.0 | 0.75 | 0.3 | 0.54 |
| Example 168 [AXL22154] | 0.4 | 0.75 | 0.2 | 0.54 |
| Example 169 [AXL22155] | 0.8 | 0.75 | 0.4 | 0.54 |
| Example 170 [AXL22156] | 0.4 | 0.65 | 0.4 | 0.72 |
| Example 171 [AXL22157] | 0.0 | 0.65 | 0.3 | 0.72 |
| Example 172 [AXL22158] | 0.0 | 0.75 | 0.2 | 0.81 |
| Example 173 [AXL22159] | 0.1 | 0.65 | 0.2 | 0.72 |
| Example 174 [AXL22160] | 0.1 | 0.65 | 0.2 | 0.72 |
| Example 175 [AXL22161] | 0.5 | 0.75 | 0.3 | 0.54 |
| Example 176 [AXL22162] | 0.6 | 0.75 | 0.2 | 0.54 |
| Example 103 [AXL22165] | 22.5 | 0.75 | >30 | 0.81 |
| Example 104 [AXL22166] | 15.1 | 0.75 | 20.2 | 0.81 |
| Example 107 [AXL22169] | 14.2 | 0.75 | >30 | 0.81 |
| Example 187 [AXL22175] | 3.3 | 0.79 | 1.3 | 0.78 |
| Example 188 [AXL22176] | 4.3 | 0.79 | 3.3 | 0.78 |
| Example 189 [AXL22177] | 7.4 | 0.79 | 4.7 | 0.78 |
| Example 111 [AXL22178] | 10.2 | 0.75 | >30 | 0.81 |
| Example 112 [AXL22179] | >30 | 0.65 | >30 | 0.72 |
| Example 113 [AXL22180] | >30 | 0.75 | 16.6 | 0.81 |
| Example 114 [AXL22181] | 12.0 | 0.75 | 21.1 | 0.81 |
| Example 115 [AXL22182] | >30 | 0.79 | 29.0 | 0.78 |
| Example 116 [AXL22183] | >30 | 0.65 | >30 | 0.72 |
| Example 117 [AXL22184] | 25.1 | 0.75 | >30 | 0.81 |
| Example 118 [AXL22185] | >30 | 0.75 | >30 | 0.81 |
| Example 119 [AXL22186] | 18.3 | 0.75 | >30 | 0.81 |
| Example 120 [AXL22187] | >30 | 0.65 | >30 | 0.72 |
| Example 190 [AXL22188] | 11.7 | 0.75 | 1.1 | 0.54 |
| Example 191 [AXL22189] | 6.2 | 0.79 | 1.3 | 0.78 |
| Example 192 [AXL22190] | 5.2 | 0.79 | 2.6 | 0.78 |
| Example 193 [AXL22191] | 1.6 | 0.65 | 1.2 | 0.54 |
| Example 194 [AXL22192] | 4.9 | 0.75 | 1.9 | 0.54 |
| Example 196 [AXL22194] | 0.3 | 0.65 | 1.0 | 0.72 |
| Example 197 [AXL22195] | 1.6 | 0.79 | 0.5 | 0.78 |
| Example 198 [AXL22196] | 3.5 | 0.79 | 3.0 | 0.78 |
| Example 199 [AXL22197] | 2.8 | 0.79 | 1.0 | 0.78 |
| Example 200 [AXL22198] | 3.2 | 0.79 | #N/A | |
| Example 201 [AXL22199] | 2.3 | 0.79 | 0.9 | 0.78 |
| Example 202 [AXL22200] | 0.6 | 0.65 | 0.7 | 0.72 |
| Example 121 [AXL22202] | 16.2 | 0.79 | 17.5 | 0.78 |
| Example 122 [AXL22203] | 5.5 | 0.79 | 16.8 | 0.78 |
| Example 123 [AXL22204] | 4.6 | 0.79 | 8.5 | 0.78 |
| Example 126 [AXL22207] | >30 | 0.79 | 24.5 | 0.78 |
| Example 127 [AXL22208] | 13.6 | 0.79 | 16.1 | 0.78 |
| Example 128 [AXL22209] | 10.4 | 0.79 | 17.9 | 0.78 |
| Example 129 [AXL22210] | 7.9 | 0.79 | 12.7 | 0.78 |
| Example 130 [AXL22211] | 9.6 | 0.79 | 15.0 | 0.78 |
| Example 131 [AXL22212] | 12.6 | 0.79 | >30 | 0.78 |
| Example 132 [AXL22213] | 9.3 | 0.79 | >30 | 0.78 |
| Example 134 [AXL22215] | 11.1 | 0.79 | 11.0 | 0.78 |
| Example 135 [AXL22216] | 13.2 | 0.79 | 11.5 | 0.78 |
| Example 136 [AXL22217] | 2.5 | 0.75 | 6.0 | 0.78 |
| Example 137 [AXL22218] | 30.0 | 0.75 | >30 | 0.81 |
| Example 138 [AXL22219] | >30 | 0.75 | 25.1 | 0.81 |

**IC50 measurements on glioblastoma cell lines.** The most active compounds on the Hs578t cells were tested on two further AXL-expressing glioblastoma cell lines, namely, A172 and U87MG. Concentrations of 1uM and 5uM were applied on cells and IC50 values were determined using 10-point serial dilutions as described previously. Foretinib, Cabozantinib, Staurosporine, BMS777607 and IM_1309_T were used as internal positive control compounds. Cell viability was tested using the CellTiter-Glo^{®} Luminescent Cell Viability Assay (Promega, G7570) according to the user manual. Luminescence signal was detected on TECAN infinite M1000 Pro. Treated wells were correlated to average DMSO control, and viability was calculated and presented in %. Values were log transformed, and IC50 curve was prepared using a 4-parameter fitting (constrain set to constant zero).

Z' values were calculated in order to validate if signal noise ratio was appropriate in the assay. DMSO treated control and signal intensity in empty wells were used for calculation.

**Table: Inhibitory effect of the compounds on the A172 and U87MG cell lines shown via cell viability.**

| | A172 @ 1µM | | A172 @ 5µM | | U87MG @ 1µM | | U87MG @ 5µM | |
|---|---|---|---|---|---|---|---|---|
| ID | Viability (%) | SD | Viability (%) | SD | Viability (%) | SD | Viability (%) | SD |
| AXL22001 [Example 1] | 91% | 4% | 50% | 0% | 98% | 6% | 61% | 5% |
| AXL22002 [Example 2] | 85% | 1% | 28% | 1% | 85% | 0% | 67% | 7% |
| AXL22005 [Example 3] | 82% | 0% | 44% | 6% | 90% | 10% | 60% | 11% |
| AXL22007 [Example 5] | 87% | 3% | 63% | 2% | 92% | 3% | 81% | 10% |
| AXL22008 [Example 6] | 84% | 3% | 56% | 7% | 92% | 2% | 61% | 11% |
| AXL22009 [Example 7] | 94% | 5% | 56% | 4% | 92% | 4% | 55% | 12% |
| AXL22012 [Example 9] | 96% | 2% | 62% | 4% | 97% | 3% | 46% | 3% |
| AXL22013 [Example 10] | 105% | 5% | 67% | 5% | 101% | 5% | 54% | 3% |
| AXL22018 [Example 13] | 10% | 2% | 2% | 0% | 54% | 13% | 2% | 0% |
| AXL22020 [Example 14] | 93% | 1% | 56% | 1% | 96% | 7% | 60% | 0% |
| AXL22031 [Example 139] | 57% | 1% | 3% | 0% | 56% | 0% | 20% | 1% |
| AXL22032 [Example 23] | 96% | 3% | 60% | 3% | 90% | 3% | 51% | 2% |
| AXL22034 [Example 25] | 62% | 3% | 11% | 0% | 89% | 0% | 32% | 2% |
| AXL22040 [Example 31] | 68% | 7% | 8% | 4% | 87% | 6% | 40% | 14% |
| AXL22042 [Example 140] | 55% | 4% | 22% | 1% | 64% | 1% | 21% | 0% |
| AXL22043 [Example 141] | 65% | 4% | 35% | 1% | 75% | 7% | 34% | 1% |
| AXL22056 [Example 142] | 62% | 1% | 2% | 0% | 67% | 2% | 12% | 14% |
| AXL22057 [Example 143] | 53% | 3% | 21% | 1% | 61% | 1% | 27% | 3% |
| AXL22064 [Example 179] | 80% | 6% | 3% | 0% | 77% | 2% | 60% | 3% |
| AXL22074 [Example 56] | 102% | 5% | 64% | 2% | 109% | 1% | 71% | 1% |
| AXL22075 [Example 57] | 104% | 3% | 66% | 2% | 109% | 9% | 79% | 2% |
| AXL22082 [Example 64] | 104% | 5% | 4% | 1% | 97% | 5% | 33% | 5% |
| AXL22084 [Example 181] | 62% | 5% | 29% | 1% | 66% | 2% | 44% | 0% |
| AXL22088 [Example 66] | 100% | 4% | 63% | 0% | 99% | 4% | 64% | 6% |
| AXL22090 [Example 144] | 95% | 6% | 76% | 2% | 90% | 8% | 70% | 1% |
| AXL22091 [Example 68] | 101% | 1% | 53% | 0% | 94% | 3% | 54% | 6% |
| AXL22092 [Example 69] | 70% | 2% | 46% | 2% | 83% | 2% | 60% | 3% |
| AXL22095 [Example 72] | 92% | 0% | 67% | 1% | 90% | 1% | 70% | 3% |
| AXL22097 [Example 74] | 97% | 3% | 61% | 2% | 102% | 0% | 65% | 3% |
| AXL22098 [Example 75] | 90% | 7% | 51% | 2% | 85% | 3% | 71% | 2% |
| AXL22100 [Example 77] | 100% | 4% | 77% | 2% | 108% | 1% | 85% | 4% |
| AXL22101 [Example 78] | 95% | 7% | 48% | 5% | 97% | 6% | 51% | 1% |
| AXL22102 [Example 79] | 84% | 2% | 14% | 6% | 92% | 5% | 65% | 2% |
| AXL22104 [Example 81] | 102% | 3% | 94% | 6% | 102% | 4% | 83% | 4% |
| AXL22105 [Example 82] | 88% | 6% | 60% | 2% | 97% | 1% | 74% | 6% |
| AXL22108 [Example 182] | 87% | 3% | 26% | 21% | 90% | 3% | 72% | 4% |
| AXL22109 [Example 183] | 65% | 6% | 52% | 3% | 66% | 5% | 46% | 4% |
| AXL22110 [Example 184] | 93% | 7% | 22% | 11% | 88% | 3% | 68% | 4% |
| AXL22111 [Example 85] | 84% | 8% | 39% | 1% | 92% | 2% | 77% | 2% |
| AXL22112 [Example 86] | 103% | 2% | 75% | 0% | 99% | 5% | 89% | 5% |
| AXL22116 [Example 90] | 106% | 3% | 96% | 4% | 103% | 2% | 101% | 3% |
| AXL22123 [Example 97] | 90% | 0% | 48% | 0% | 100% | 4% | 54% | 4% |
| AXL22124 [Example 98] | 72% | 3% | 15% | 5% | 86% | 2% | 62% | 0% |
| AXL22127 [Example 145] | 64% | 4% | 48% | 1% | 70% | 2% | 45% | 3% |
| AXL22128 [Example 146] | 62% | 1% | 2% | 1% | 71% | 1% | 22% | 4% |
| AXL22130 [Example 148] | 63% | 7% | 32% | 6% | 69% | 2% | 21% | 1% |
| AXL22131 [Example 149] | 63% | 2% | 34% | 2% | 72% | 1% | 30% | 2% |
| AXL22132 [Example 203] | 73% | 4% | 60% | 5% | 77% | 7% | 42% | 0% |
| AXL22134 [Example 151] | 65% | 1% | 47% | 0% | 71% | 2% | 38% | 2% |
| AXL22137 [Example 153] | 59% | 3% | 30% | 1% | 63% | 4% | 26% | 0% |
| AXL22139 [Example 154] | 51% | 3% | 2% | 1% | 66% | 3% | 2% | 1% |
| AXL22146 [Example 160] | 4% | 0% | 1% | 0% | 13% | 3% | 1% | 0% |
| AXL22148 [Example 162] | 69% | 1% | 54% | 3% | 71% | 0% | 67% | 5% |
| AXL22150 [Example 164] | 65% | 3% | 28% | 2% | 63% | 5% | 26% | 1% |
| AXL22151 [Example 165] | 61% | 2% | 22% | 1% | 64% | 1% | 27% | 1% |
| AXL22152 [Example 166] | 58% | 3% | 29% | 1% | 63% | 0% | 26% | 1% |
| AXL22153 [Example 167] | 60% | 1% | 23% | 3% | 66% | 1% | 22% | 0% |
| AXL22154 [Example 168] | 57% | 3% | 8% | 10% | 59% | 2% | 21% | 2% |
| AXL22155 [Example 169] | 43% | 25% | 24% | 5% | 55% | 15% | 30% | 1% |
| AXL22156 [Example 170] | 71% | 2% | 53% | 2% | 70% | 5% | 53% | 1% |
| AXL22157 [Example 171] | 66% | 5% | 49% | 1% | 65% | 0% | 28% | 0% |
| AXL22158 [Example 172] | 64% | 1% | 37% | 8% | 64% | 1% | 24% | 1% |
| AXL22159 [Example 173] | 61% | 3% | 26% | 1% | 65% | 3% | 29% | 4% |
| AXL22160 [Example 174] | 34% | 4% | 0% | 0% | 33% | 6% | 1% | 0% |
| AXL22161 [Example 175] | 35% | 3% | 13% | 4% | 31% | 4% | 13% | 16% |
| AXL22162 [Example 176] | 63% | 4% | 23% | 2% | 35% | 1% | 21% | 7% |
| AXL22175 [Example 187] | 41% | 3% | 31% | 0% | 61% | 2% | 57% | 5% |
| AXL22176 [Example 188] | 36% | 3% | 31% | 4% | 48% | 3% | 45% | 3% |
| AXL22177 [Example 189] | 45% | 1% | 28% | 2% | 72% | 15% | 63% | 3% |
| AXL22178 [Example 111] | 90% | 5% | 74% | 11% | 102% | 10% | 85% | 14% |
| AXL22181 [Example 114] | 87% | 4% | 83% | 0% | 96% | 1% | 79% | 34% |
| AXL22188 [Example 190] | 62% | 1% | 50% | 3% | 78% | 0% | 73% | 9% |
| AXL22189 [Example 191] | 58% | 1% | 47% | 1% | 82% | 1% | 65% | 13% |
| AXL22190 [Example 192] | 64% | 11% | 61% | 0% | 52% | 0% | 49% | 12% |
| AXL22191 [Example 193] | 67% | 0% | 55% | 1% | 87% | 14% | 71% | 9% |
| AXL22192 [Example 194] | 57% | 1% | 45% | 6% | 77% | 7% | 62% | 1% |
| AXL22194 [Example 196] | 53% | 1% | 37% | 1% | 72% | 6% | 61% | 1% |
| AXL22195 [Example 197] | 83% | 1% | 76% | 4% | 55% | 17% | 77% | 22% |
| AXL22196 [Example 198] | 119% | 0% | 83% | 10% | 70% | 25% | 29% | 19% |
| AXL22197 [Example 199] | 52% | 1% | 40% | 2% | 72% | 1% | 58% | 4% |
| AXL22198 [Example 200] | 45% | 7% | 36% | 2% | 75% | 13% | 56% | 4% |
| AXL22199 [Example 201] | 46% | 3% | 36% | 2% | 64% | 3% | 52% | 2% |
| AXL22200 [Example 202] | 61% | 1% | 42% | 2% | 71% | 7% | 69% | 7% |
| AXL22203 [Example 122] | 92% | 1% | 51% | 0% | 107% | 12% | 64% | 1% |
| AXL22204 [Example 123] | 86% | 4% | 56% | 7% | 99% | 6% | 72% | 0% |
| AXL22209 [Example 128] | 95% | 2% | 60% | 4% | 95% | 15% | 75% | 2% |
| AXL22210 [Example 129] | 102% | 1% | 84% | 0% | 92% | 48% | 64% | 20% |
| AXL22211 [Example 130] | 83% | 1% | 59% | 2% | 99% | 2% | 78% | 1% |
| AXL22212 [Example 131] | 100% | 2% | 80% | 12% | 43% | 5% | 34% | 0% |
| AXL22213 [Example 132] | 75% | 3% | 60% | 5% | 99% | 6% | 80% | 7% |
| AXL22215 [Example 134] | 39% | 4% | 49% | 0% | 31% | 7% | 32% | 4% |
| AXL22216 [Example 135] | 101% | 5% | 66% | 2% | 105% | 7% | 82% | 13% |
| AXL22217 [Example 136] | 97% | 0% | 59% | 2% | 104% | 8% | 78% | 6% |

The compounds were initially screened at one concentration at first and the best inhibitors were selected. We determined the IC50 values on both A172 and U87MG cells for the best inhibitors.

**Table: IC50 values for a selected set of inhibitors on A172 and U87MG cells.**

| | A172 | | U87MG | |
|---|---|---|---|---|
| ID | IC₅₀ [µM] | z' | IC₅₀ [µM] | z' |
| AXL22001 [Example 1 ] | 3.31 | 0.683613 | 6.325 | 0.545163 |
| AXL22002 [Example 2 ] | 2.91 | 0.683613 | 9.298 | 0.545163 |
| AXL22005 [Example 3 ] | 4.34 | 0.683613 | 8.619 | 0.545163 |
| AXL22007 [Example 5 ] | 15.12 | 0.683613 | 29.46 | 0.545163 |
| AXL22008 [Example 6 ] | 10.54 | 0.683613 | 8.965 | 0.545163 |
| AXL22009 [Example 7 ] | 18.73 | 0.683613 | 11.28 | 0.545163 |
| AXL22012 [Example 9 ] | 20.16 | 0.683613 | 9.798 | 0.545163 |
| AXL22013 [Example 10 ] | 9.59 | 0.683613 | 9.941 | 0.545163 |
| AXL22018 [Example 13 ] | 0.52 | 0.683613 | 0.9196 | 0.545163 |
| AXL22020 [Example 14 ] | 9.77 | 0.683613 | 8.883 | 0.545163 |
| AXL22031 [Example 139 ] | 0.08 | 0.683613 | 0.2996 | 0.545163 |
| AXL22032 [Example 23 ] | 5.24 | 0.683613 | 11.29 | 0.545163 |
| AXL22034 [Example 25 ] | 0.51 | 0.683613 | 1.818 | 0.545163 |
| AXL22040 [Example 31 ] | 0.75 | 0.683613 | 3.192 | 0.545163 |
| AXL22042 [Example 140 ] | 0.40 | 0.683613 | 0.1657 | 0.545163 |
| AXL22043 [Example 141 ] | 0.34 | 0.683613 | 2.373 | 0.545163 |
| AXL22056 [Example 142 ] | 0.19 | 0.683613 | 1.193 | 0.545163 |
| AXL22057 [Example 143 ] | 0.38 | 0.683613 | 1.163 | 0.545163 |
| AXL22064 [Example 179 ] | 1.18 | 0.683613 | 3.546 | 0.545163 |
| AXL22074 [Example 56 ] | 12.10 | 0.683613 | 9.173 | 0.545163 |
| AXL22075 [Example 57 ] | 7.93 | 0.739278 | 12.89 | 0.590457 |
| AXL22082 [Example 64 ] | 3.99 | 0.739278 | 3.616 | 0.590457 |
| AXL22084 [Example 181 ] | 1.58 | 0.739278 | 4.073 | 0.590457 |
| AXL22088 [Example 66 ] | 28.21 | 0.739278 | 28.64 | 0.590457 |
| AXL22090 [Example 144 ] | 14.09 | 0.461733 | 7.816 | 0.532724 |
| AXL22091 [Example 68 ] | 7.42 | 0.461733 | >30 | 0.590457 |
| AXL22092 [Example 69 ] | 6.00 | 0.461733 | 12.49 | 0.532724 |
| AXL22095 [Example 72 ] | 14.21 | 0.461733 | 20.2 | 0.532724 |
| AXL22097 [Example 74 ] | 10.04 | 0.461733 | 19.88 | 0.590457 |
| AXL22098 [Example 75 ] | 5.10 | 0.461733 | 11.66 | 0.590457 |
| AXL22100 [Example 77 ] | >30 | 0.461733 | >30 | 0.590457 |
| AXL22101 [Example 78 ] | 4.55 | 0.461733 | 5.318 | 0.590457 |
| AXL22102 [Example 79 ] | 2.88 | 0.461733 | 6.847 | 0.590457 |
| AXL22105 [Example 82 ] | 9.70 | 0.461733 | 21.79 | 0.532724 |
| AXL22108 [Example 182 ] | 3.09 | 0.461733 | 1.951 | 0.532724 |
| AXL22109 [Example 183 ] | 6.23 | 0.461733 | 3.797 | 0.590457 |
| AXL22110 [Example 184 ] | ~ 8.248 | 0.461733 | 4.339 | 0.532724 |
| AXL22111 [Example 85 ] | 2.00 | 0.461733 | 2.582 | 0.532724 |
| AXL22112 [Example 86 ] | 8.60 | 0.461733 | >30 | 0.590457 |
| AXL22123 [Example 97 ] | 3.87 | 0.461733 | 2.52 | 0.590457 |
| AXL22124 [Example 98 ] | 2.20 | 0.461733 | 12.95 | 0.532724 |
| AXL22127 [Example 145 ] | 1.41 | 0.461733 | 1.567 | 0.590457 |
| AXL22128 [Example 146] | 1.02 | 0.461733 | 1.023 | 0.590457 |
| AXL22130 [Example 148 ] | 0.85 | 0.461733 | 0.2793 | 0.590457 |
| AXL22131 [Example 149 ] | 0.35 | 0.461733 | 0.7072 | 0.590457 |
| AXL22132 [Example 203 ] | 7.66 | 0.461733 | 3.967 | 0.532724 |
| AXL22133 [Example 150 ] | 0.42 | 0.693548 | 1.298 | 0.618657 |
| AXL22134 [Example 151 ] | 1.61 | 0.772488 | 3.196 | 0.532724 |
| AXL22137 [Example 153 ] | 0.44 | 0.772488 | 0.7787 | 0.532724 |
| AXL22139 [Example 154 ] | 0.14 | 0.772488 | 1.006 | 0.532724 |
| AXL22146 [Example 160 ] | 0.07 | 0.772488 | 0.9389 | 0.532724 |
| AXL22148 [Example 162] | 7.55 | 0.772488 | 15.64 | 0.532724 |
| AXL22150 [Example 164 ] | 0.72 | 0.772488 | 0.4897 | 0.532724 |
| AXL22151 [Example 165 ] | 0.29 | 0.772488 | 1.287 | 0.532724 |
| AXL22152 [Example 166 ] | 0.77 | 0.739278 | 0.8198 | 0.532724 |
| AXL22153 [Example 167 ] | 0.27 | 0.739278 | 1.56 | 0.532724 |
| AXL22154 [Example 168 ] | 0.11 | 0.739278 | >30 | 0.532724 |
| AXL22155 [Example 169 ] | 0.52 | 0.772488 | >30 | 0.532724 |
| AXL22156 [Example 170 ] | 4.19 | 0.772488 | 3.612 | 0.532724 |
| AXL22157 [Example 171 ] | 2.07 | 0.772488 | 20.9 | 0.532724 |
| AXL22158 [Example 172 ] | 0.56 | 0.739278 | 0.9997 | 0.532724 |
| AXL22159 [Example 173 ] | 0.47 | 0.739278 | 0.732 | 0.590457 |
| AXL22160 [Example 174 ] | 0.60 | 0.739278 | 0.3801 | 0.618657 |
| AXL22161 [Example 175 ] | 0.23 | 0.772488 | 0.5689 | 0.618657 |
| AXL22162 [Example 176 ] | 1.11 | 0.772488 | 0.8501 | 0.618657 |
| AXL22175 [Example 187 ] | 3.58 | 0.772488 | >30 | 0.590457 |
| AXL22176 [Example 188 ] | 6.51 | 0.772488 | 5.336 | 0.590457 |
| AXL22177 [Example 189 ] | 6.36 | 0.772488 | >30 | 0.590457 |
| AXL22178 [Example 111 ] | - 29.70 | 0.772488 | 13.2 | 0.547766 |
| AXL22181 [Example 114 ] | >30 | 0.789376 | 17.41 | 0.547766 |
| AXL22188 [Example 190 ] | 2.52 | 0.772488 | >30 | 0.547766 |
| AXL22189 [Example 191 ] | 3.60 | 0.789376 | 19.38 | 0.547766 |
| AXL22190 [Example 192 ] | 1.94 | 0.789376 | 3.618 | 0.547766 |
| AXL22191 [Example 193 ] | 3.07 | 0.789376 | 3.92 | 0.547766 |
| AXL22192 [Example 194 ] | 5.07 | 0.789376 | >30 | 0.547766 |
| AXL22194 [Example 196 ] | 2.94 | 0.789376 | >30 | 0.547766 |
| AXL22195 [Example 197 ] | 1.13 | 0.789376 | >30 | 0.547766 |
| AXL22196 [Example 198 ] | 2.76 | 0.789376 | >30 | 0.547766 |
| AXL22197 [Example 199 ] | 1.47 | 0.789376 | 25.74 | 0.547766 |
| AXL22198 [Example 200 ] | 1.21 | 0.789376 | >30 | 0.547766 |
| AXL22199 [Example 201 ] | 1.02 | 0.789376 | 15.51 | 0.547766 |
| AXL22200 [Example 202 ] | 2.17 | 0.789376 | 5.745 | 0.547766 |
| AXL22203 [Example 122 ] | 9.83 | 0.789376 | 16.9 | 0.547766 |
| AXL22204 [Example 123 ] | 10.99 | 0.789376 | 27.87 | 0.547766 |
| AXL22209 [Example 128 ] | 11.53 | 0.789376 | 29.42 | 0.547766 |
| AXL22210 [Example 129 ] | 12.38 | 0.789376 | >30 | 0.547766 |
| AXL22211 [Example 130 ] | 13.74 | 0.789376 | >30 | 0.547766 |
| AXL22212 [Example 131 ] | 10.98 | 0.789376 | >30 | 0.547766 |
| AXL22213 [Example 132 ] | 5.99 | 0.789376 | >30 | 0.547766 |
| AXL22215 [Example 134 ] | 14.41 | 0.789376 | >30 | 0.547766 |
| AXL22216 [Example 135 ] | 13.25 | 0.789376 | >30 | 0.547766 |
| AXL22217 [Example 136 ] | 5.99 | 0.789376 | 14.16 | 0.618657 |

### In vivo toxicity studies

8-12 weeks old female BALB/C mice were treated with the test compounds in the toxicity experiments. 9 compounds were administered in 3-3 doses (10 mg/kg, 1 mg/kg, 0.1 mg/kg). During the 14-day experimental period, general health, and appearance of the animals was examined daily, as well as the changes in their body weights. Body masses were recorded for 2 weeks on every weekday. For further studies, including toxicity studies, the compounds in the following Table were selected.

**Table: Mean half-life values and intrinsic clearance rates (number of measurements: n=2).**

| **Example no.** | |
|---|---|
| Example 56 [AXL22074] | 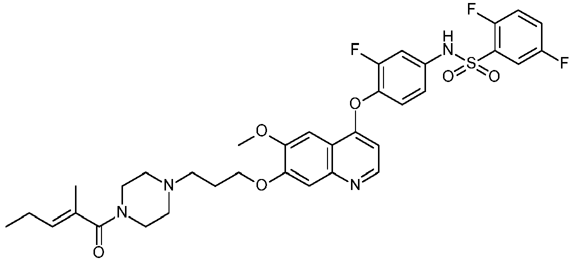 |
| Example 78 [AXL22101] | 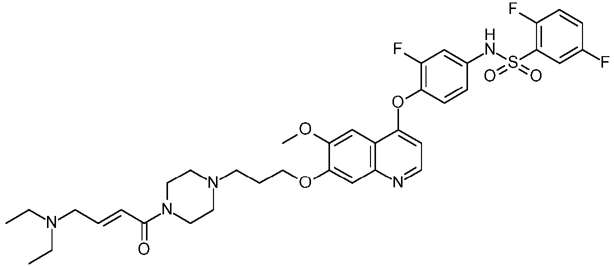 |
| Example 97 [AXL22123] | 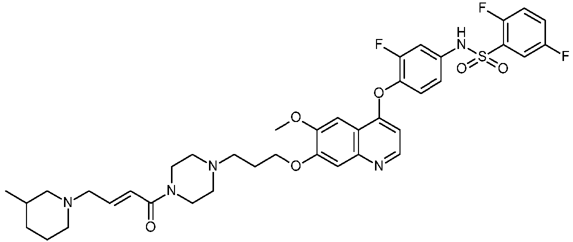 |
| Example 151 [AXL22134] | 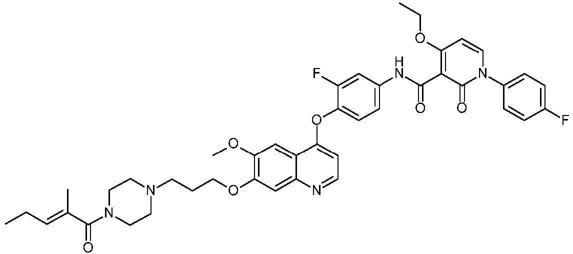 |
| Example 154 **[AXL22139]** | 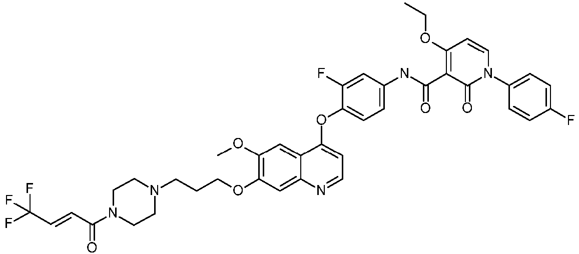 |
| Example 174 [AXL22160] | 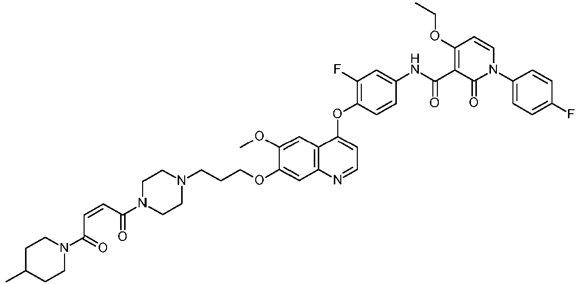 |
| Example 179 **[AXL22064]** | 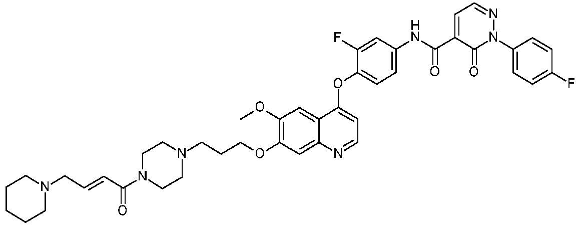 |
| Example 182 **[AXL22108]** | 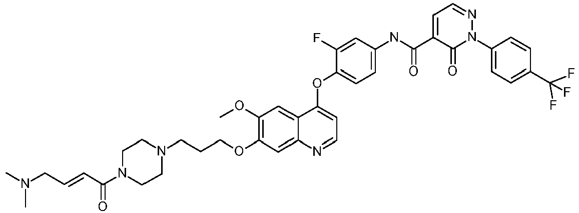 |
| Example 188 [AXL22176] | 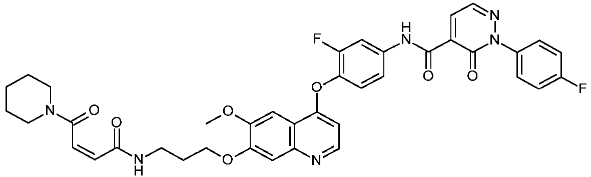 |

### Acute toxicity

3 mice in each group were treated in parallel once intraperitoneally on day 1. There was no adverse effect registered during the 2-week window of the experiment, neither in behaviour/outlook nor body mass of the animals. Based on the results, a single dose of 10mg/kg treatment intraperitoneally (i.p.) should be safe.

**Table: Results of the acute toxicity study with Example 179 [AXL22064] (See Figure 5)**

| Example 179 [AXL22064] | 10mg/kg | | 1mg/kg | | 0.1mg/kg | |
|---|---|---|---|---|---|---|
| Day | body | relative body | body mass | relative body | body | relative body |
| | mass (g) | mass (%) | (g) | mass (%) | mass (g) | mass (%) |
| 0 | 24.0 | 100.00 | 23.9 | 100.00 | 24.5 | 100.00 |
| 1 | 24.1 | 100.54 | 23.9 | 100.16 | 24.7 | 101.06 |
| 2 | 23.9 | 99.72 | 23.5 | 98.47 | 24.2 | 99.03 |
| 3 | 23.7 | 98.61 | 23.0 | 96.20 | 24.2 | 98.87 |
| 4 | 24.1 | 100.55 | 23.4 | 98.19 | 24.1 | 98.61 |
| 7 | 23.4 | 97.50 | 22.6 | 94.66 | 23.4 | 95.46 |
| 8 | 23.8 | 99.01 | 22.6 | 94.86 | 23.8 | 97.11 |
| 9 | 24.2 | 100.80 | 23.6 | 98.92 | 23.9 | 97.68 |
| 10 | 23.6 | 98.47 | 22.8 | 95.37 | 23.3 | 95.36 |
| 11 | 23.2 | 96.66 | 22.7 | 95.01 | 23.4 | 95.49 |
| 14 | 23.6 | 98.30 | 23.0 | 96.38 | 23.2 | 94.84 |

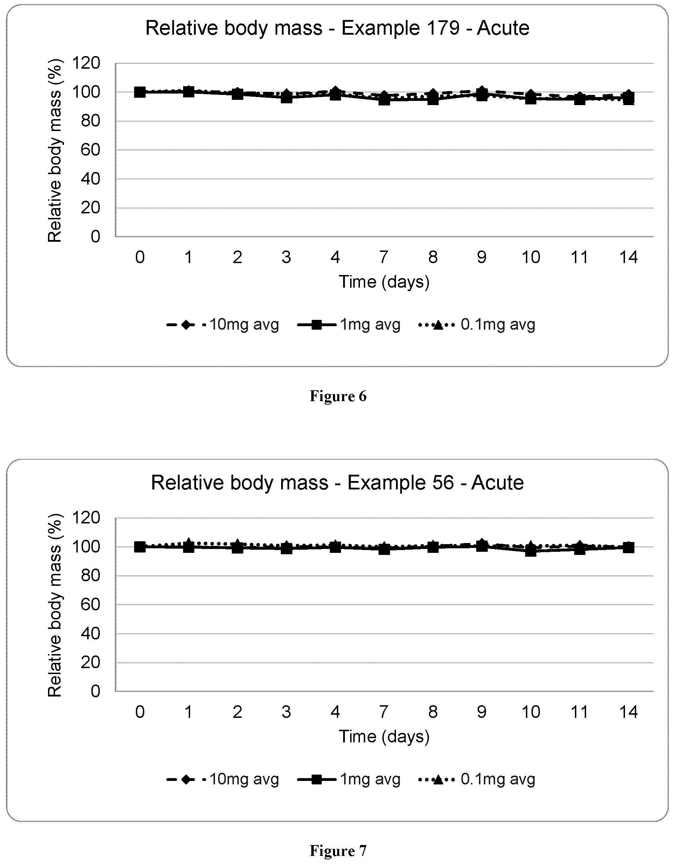

**Table: Results of the acute toxicity study with Example 56 [AXL22074] (See also Figure 6)**

| Example 56 [AXL22074] | 10mg/kg | | 1mg/kg | | 0.1mg/kg | |
|---|---|---|---|---|---|---|
| Day | body mass | relative body | body mass | relative body | body | relative body |
| | (g) | mass (%) | (g) | mass (%) | mass (g) | mass (%) |
| 0 | 22.9 | 100.00 | 24.3 | 100.00 | 24.8 | 100.00 |
| 1 | 22.9 | 99.97 | 24.3 | 99.73 | 25.4 | 102.56 |
| 2 | 22.7 | 99.24 | 24.1 | 99.18 | 25.2 | 101.75 |
| 3 | 22.9 | 100.14 | 24.0 | 98.78 | 24.9 | 100.67 |
| 4 | 22.8 | 99.85 | 24.2 | 99.61 | 25.1 | 101.21 |
| 7 | 22.7 | 99.11 | 23.9 | 98.24 | 24.7 | 99.87 |
| 8 | 22.9 | 100.28 | 24.3 | 99.73 | 25.0 | 100.81 |
| 9 | 23.4 | 102.18 | 24.4 | 100.29 | 24.9 | 100.41 |
| 10 | 22.7 | 99.40 | 23.6 | 97.01 | 24.9 | 100.55 |
| 11 | 23.0 | 100.42 | 23.9 | 98.09 | 25.0 | 100.96 |
| 14 | 22.9 | 100.12 | 24.2 | 99.48 | 24.7 | 99.74 |

**Table: Results of the acute toxicity study with Example 78 [AXL22101] (See also Figure 7)**

| Example 78 [AXL22101] | 10mg/kg | | 1mg/kg | | 0.1mg/kg | |
|---|---|---|---|---|---|---|
| Day | body mass | relative body | body mass | relative body | body | relative body |
| | (g) | mass (%) | (g) | mass (%) | mass (g) | mass (%) |
| 0 | 21.9 | 100.00 | 24.3 | 100.00 | 24.1 | 100.00 |
| 1 | 22.3 | 101.51 | 24.3 | 100.01 | 24.3 | 100.78 |
| 2 | 21.9 | 99.81 | 24.1 | 99.02 | 23.8 | 98.81 |
| 3 | 21.8 | 99.48 | 23.9 | 98.22 | 24.1 | 100.07 |
| 4 | 22.4 | 102.23 | 24.5 | 100.66 | 24.4 | 101.37 |
| 7 | 22.4 | 101.92 | 24.2 | 99.30 | 23.9 | 99.10 |
| 8 | 22.6 | 102.82 | 24.2 | 99.31 | 24.0 | 99.42 |
| 9 | 22.4 | 102.17 | 24.1 | 98.90 | 23.8 | 98.90 |
| 10 | 22.3 | 101.45 | 24.0 | 98.73 | 23.5 | 97.35 |
| 11 | 22.7 | 103.44 | 24.2 | 99.57 | 23.6 | 97.74 |
| 14 | 22.3 | 101.50 | 24.7 | 101.39 | 24.1 | 99.85 |

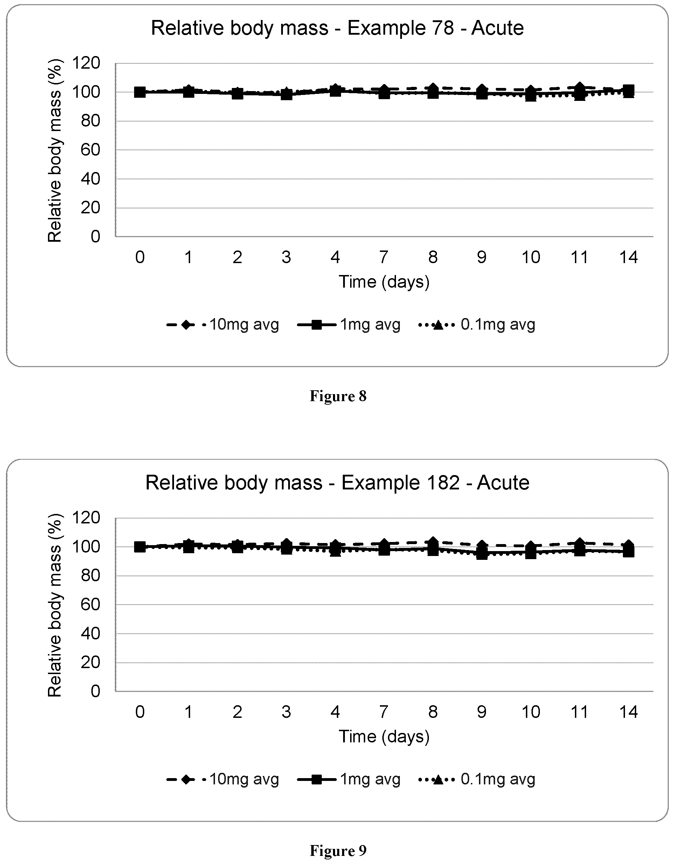

**Table: Results of the acute toxicity study with Example 182 [AXL22108] (See also Figure 8)**

| Example 182 [AXL22108] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 23.1 | 100.00 | 24.2 | 100.00 | 25.6 | 100.00 |
| 1 | 23.5 | 101.88 | 24.4 | 100.69 | 25.4 | 99.35 |
| 2 | 23.5 | 101.57 | 24.3 | 100.42 | 25.4 | 99.39 |
| 3 | 23.6 | 102.22 | 24.1 | 99.74 | 25.1 | 98.37 |
| 4 | 23.4 | 101.38 | 24.0 | 99.18 | 24.8 | 96.93 |
| 7 | 23.6 | 102.10 | 23.7 | 97.79 | 25.1 | 98.09 |
| 8 | 23.9 | 103.43 | 23.9 | 98.76 | 24.9 | 97.59 |
| 9 | 23.3 | 101.04 | 23.2 | 95.86 | 24.2 | 94.84 |
| 10 | 23.2 | 100.58 | 23.3 | 96.27 | 24.4 | 95.36 |
| 11 | 23.7 | 102.56 | 23.6 | 97.51 | 24.8 | 97.16 |
| 14 | 23.4 | 101.24 | 23.4 | 96.69 | 24.7 | 96.52 |

**Table: Results of the acute toxicity study with Example 97 [AXL22123] (See also Figure 9)**

| Example 97 [AXL22123] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 23.1 | 100.00 | 24.7 | 100.00 | 23.9 | 100.00 |
| 1 | 23.0 | 99.71 | 24.5 | 99.34 | 23.5 | 98.31 |
| 2 | 22.5 | 97.59 | 24.4 | 99.08 | 22.9 | 95.80 |
| 3 | 22.8 | 98.90 | 24.4 | 99.01 | 23.1 | 96.65 |
| 4 | 22.7 | 98.45 | 24.0 | 97.19 | 23.6 | 98.83 |
| 7 | 22.8 | 98.89 | 24.2 | 98.11 | 23.1 | 96.72 |
| 8 | 22.7 | 98.30 | 24.3 | 98.57 | 23.1 | 96.86 |
| 9 | 22.5 | 97.29 | 24.1 | 97.39 | 23.0 | 96.47 |
| 10 | 22.4 | 97.14 | 23.9 | 96.59 | 23.2 | 97.04 |
| 11 | 22.9 | 99.16 | 24.3 | 98.35 | 23.4 | 98.03 |
| 14 | 23.6 | 102.18 | 25.5 | 103.15 | 23.8 | 99.79 |

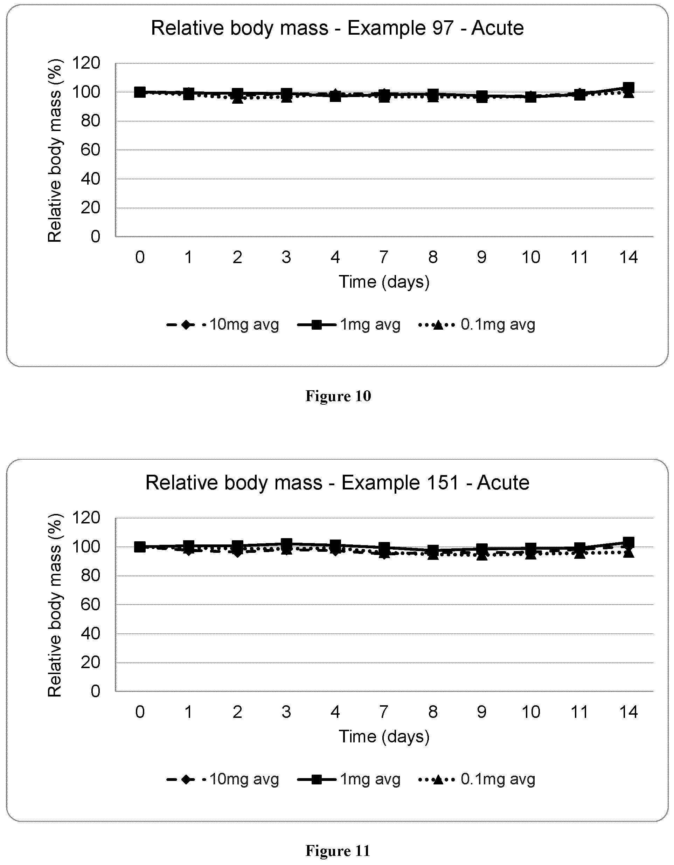

**Table: Results of the acute toxicity study with Example 151 [AXL22134] (See also Figure 10)**

| Example 151 [AXL22134] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 23.8 | 100.00 | 23.3 | 100.00 | 26.4 | 100.00 |
| 1 | 23.2 | 97.57 | 23.5 | 100.71 | 26.2 | 99.20 |
| 2 | 22.9 | 96.34 | 23.5 | 100.72 | 26.0 | 98.69 |
| 3 | 23.3 | 98.03 | 23.8 | 102.01 | 26.0 | 98.47 |
| 4 | 23.1 | 97.33 | 23.6 | 101.01 | 26.1 | 99.13 |
| 7 | 22.6 | 95.07 | 23.2 | 99.46 | 25.3 | 95.97 |
| 8 | 22.8 | 96.05 | 22.7 | 97.45 | 25.0 | 94.73 |
| 9 | 22.8 | 95.81 | 23.0 | 98.45 | 24.9 | 94.36 |
| 10 | 22.9 | 96.22 | 23.1 | 98.86 | 25.1 | 95.07 |
| 11 | 23.4 | 98.30 | 23.1 | 99.15 | 25.2 | 95.54 |
| 14 | 23.8 | 100.27 | 24.0 | 103.02 | 25.3 | 96.18 |

**Table: Results of the acute toxicity study with Example 154 [AXL22139] (See also Figure 11)**

| Example 154 [AXL22139] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 23.8 | 100.00 | 24.9 | 100.00 | 25.5 | 100.00 |
| 1 | 23.5 | 98.61 | 25.1 | 100.67 | 25.6 | 100.27 |
| 2 | 23.4 | 98.17 | 25.1 | 100.79 | 25.5 | 99.88 |
| 3 | 23.5 | 98.73 | 25.0 | 100.22 | 25.5 | 99.74 |
| 4 | 23.5 | 98.47 | 24.8 | 99.56 | 25.6 | 100.27 |
| 7 | 22.9 | 96.22 | 24.9 | 100.22 | 25.2 | 98.83 |
| 8 | 22.9 | 96.09 | 24.8 | 99.76 | 25.6 | 100.12 |
| 9 | 22.7 | 95.37 | 24.7 | 99.27 | 25.3 | 99.08 |
| 10 | 22.3 | 93.73 | 24.5 | 98.38 | 25.3 | 99.22 |
| 11 | 22.5 | 94.43 | 24.6 | 98.94 | 25.4 | 99.34 |
| 14 | 23.4 | 98.31 | 25.3 | 101.93 | 25.8 | 101.04 |

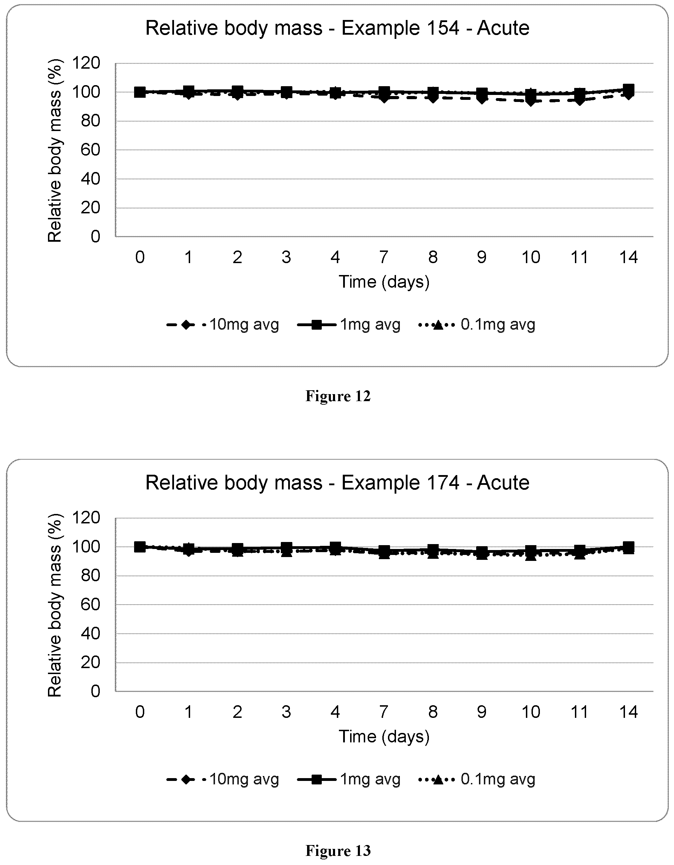

**Table: Results of the acute toxicity study with Example 174 [AXL22160] (See also Figure 12)**

| Example 174 [AXL22160] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 23.9 | 100.00 | 23.0 | 100.00 | 25.9 | 100.00 |
| 1 | 23.1 | 96.95 | 22.7 | 98.53 | 25.7 | 99.25 |
| 2 | 23.1 | 96.66 | 22.7 | 98.74 | 25.1 | 96.91 |
| 3 | 23.1 | 96.65 | 22.8 | 99.32 | 25.1 | 96.86 |
| 4 | 23.3 | 97.47 | 22.9 | 99.63 | 25.3 | 97.87 |
| 7 | 22.7 | 95.25 | 22.4 | 97.30 | 24.7 | 95.30 |
| 8 | 23.0 | 96.22 | 22.5 | 98.07 | 24.7 | 95.56 |
| 9 | 22.7 | 95.09 | 22.2 | 96.56 | 24.5 | 94.77 |
| 10 | 23.9 | 95.51 | 22.4 | 97.26 | 24.4 | 94.15 |
| 11 | 22.8 | 95.52 | 22.4 | 97.57 | 24.6 | 94.94 |
| 14 | 23.6 | 98.98 | 23.0 | 100.00 | 25.6 | 98.72 |

**Table: Results of the acute toxicity study with Example 188 [AXL22176] (See also Figure 13)**

| Example 188 [AXL22176] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 23.5 | 100.00 | 22.8 | 100.00 | 25.8 | 100.00 |
| 1 | 23.7 | 100.98 | 22.8 | 100.07 | 25.6 | 99.38 |
| 2 | 23.2 | 99.00 | 22.8 | 99.97 | 25.2 | 97.72 |
| 3 | 23.2 | 98.90 | 22.8 | 99.94 | 25.0 | 97.18 |
| 4 | 22.9 | 97.76 | 22.6 | 99.39 | 25.0 | 96.93 |
| 7 | 22.9 | 97.47 | 22.6 | 99.29 | 24.6 | 95.40 |
| 8 | 22.9 | 97.61 | 22.3 | 98.10 | 24.6 | 95.38 |
| 9 | 22.3 | 95.04 | 22.3 | 97.99 | 24.5 | 95.26 |
| 10 | 22.4 | 95.61 | 22.6 | 99.27 | 24.6 | 95.55 |
| 11 | 23.0 | 98.02 | 22.6 | 99.47 | 24.8 | 96.41 |
| 14 | 23.6 | 100.59 | 23.2 | 101.79 | 25.2 | 97.82 |

### Chronic toxicity

3 mice in each group were treated six times in intraperitoneally (i.p.) on days 0, 2, 4, 7, 9, and 11. There was no adverse effect registered during the 2-week window of the experiment, neither in behaviour/outlook nor body mass of the animals. Based on the results, repeated doses of 10mg/kg treatment three times a week i.p. should be safe.

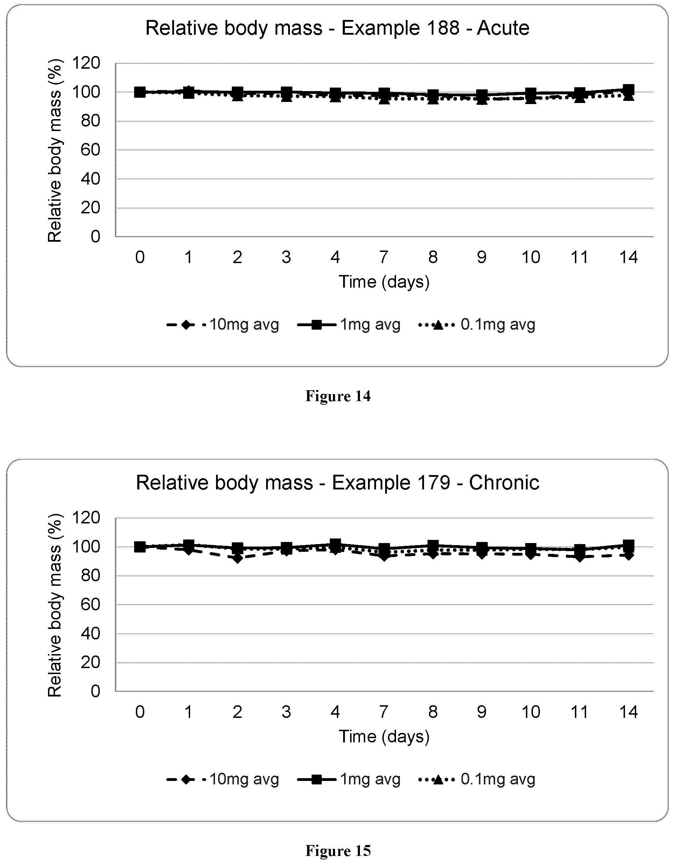

**Table: Results of the chronic toxicity study with Example 179 [AXL22064] (See Figure 14)**

| Example 179 [AXL22064] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 24.1 | 100.00 | 23.6 | 100.00 | 23.7 | 100.00 |
| 1 | 23.6 | 97.93 | 23.8 | 101.17 | 24.0 | 101.41 |
| 2 | 22.3 | 92.25 | 23.4 | 99.15 | 23.4 | 98.61 |
| 3 | 23.4 | 97.14 | 23.4 | 99.31 | 23.3 | 98.33 |
| 4 | 23.6 | 98.03 | 24.0 | 101.72 | 23.6 | 99.75 |
| 7 | 22.6 | 93.70 | 23.2 | 98.60 | 22.8 | 96.21 |
| 8 | 23.0 | 95.21 | 23.8 | 100.84 | 23.1 | 97.63 |
| 9 | 22.9 | 95.11 | 23.4 | 99.38 | 23.2 | 97.76 |
| 10 | 22.8 | 94.70 | 23.3 | 98.77 | 23.3 | 98.33 |
| 11 | 22.4 | 93.05 | 23.1 | 97.94 | 23.3 | 98.18 |
| 14 | 22.7 | 94.22 | 23.8 | 101.20 | 23.7 | 99.86 |

**Table: Results of the chronic toxicity study with Example 56 [AXL22074] (See Figure 15)**

| Example 56 [AXL22074] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 23.4 | 100.00 | 23.9 | 100.00 | 25.3 | 100.00 |
| 1 | 23.4 | 99.99 | 24.3 | 101.80 | 25.3 | 99.87 |
| 2 | 23.1 | 98.57 | 23.7 | 99.41 | 24.6 | 97.37 |
| 3 | 23.2 | 98.87 | 23.9 | 100.22 | 24.9 | 98.28 |
| 4 | 23.5 | 100.30 | 24.2 | 101.20 | 25.1 | 99.33 |
| 7 | 22.7 | 96.74 | 23.3 | 97.61 | 24.7 | 97.75 |
| 8 | 23.0 | 98.31 | 23.6 | 98.90 | 25.3 | 99.99 |
| 9 | 22.9 | 97.75 | 23.5 | 98.35 | 25.1 | 99.34 |
| 10 | 22.9 | 97.89 | 23.8 | 99.93 | 25.5 | 100.66 |
| 11 | 22.5 | 96.18 | 23.8 | 99.74 | 25.1 | 99.22 |
| 14 | 22.7 | 96.88 | 24.2 | 101.41 | 25.0 | 98.96 |

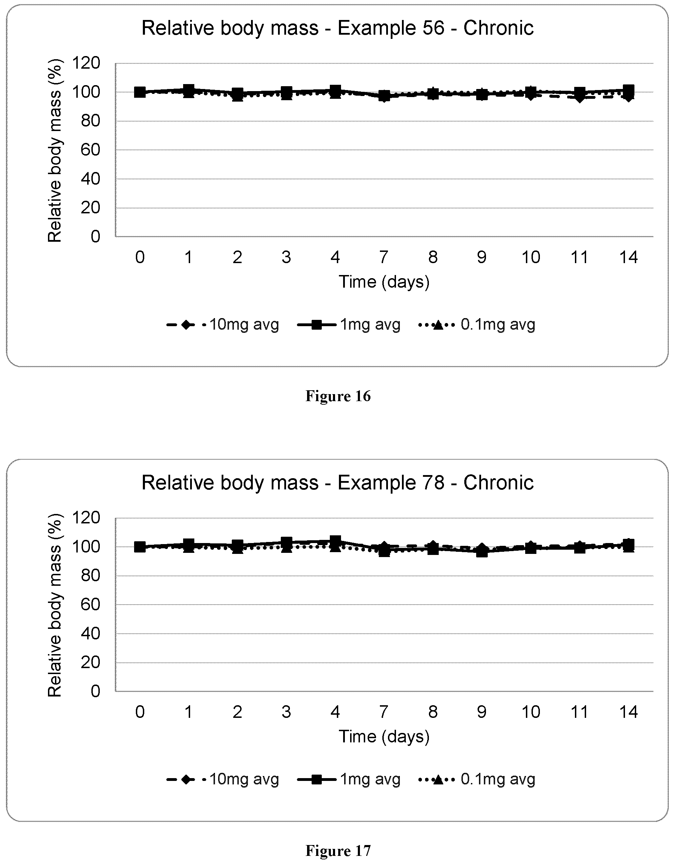

**Table: Results of the chronic toxicity study with Example 78 [AXL22101] (See Figure 16)**

| Example 78 [AXL22101] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 21.8 | 100.00 | 24.6 | 100.00 | 24.2 | 100.00 |
| 1 | 21.8 | 99.97 | 25.0 | 101.76 | 24.1 | 99.57 |
| 2 | 21.8 | 100.29 | 24.9 | 101.06 | 23.9 | 98.85 |
| 3 | 22.3 | 102.56 | 25.3 | 102.95 | 24.1 | 99.75 |
| 4 | 22.2 | 101.99 | 25.6 | 104.00 | 24.2 | 100.15 |
| 7 | 21.8 | 100.14 | 24.1 | 97.92 | 23.4 | 96.67 |
| 8 | 21.9 | 100.78 | 24.3 | 98.60 | 23.8 | 98.25 |
| 9 | 21.6 | 99.08 | 23.8 | 96.60 | 23.7 | 98.13 |
| 10 | 21.9 | 100.48 | 24.4 | 99.01 | 23.9 | 98.81 |
| 11 | 21.9 | 100.54 | 24.4 | 99.16 | 24.1 | 99.50 |
| 14 | 22.2 | 102.21 | 25.0 | 101.55 | 24.1 | 99.83 |

**Table: Results of the chronic toxicity study with Example 182 [AXL22108] (See Figure 17)**

| Example 182 [AXL22108] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 23.0 | 100.00 | 24.0 | 100.00 | 25.6 | 100.00 |
| 1 | 22.9 | 99.69 | 24.1 | 100.28 | 25.6 | 99.99 |
| 2 | 22.9 | 99.83 | 23.8 | 99.18 | 25.4 | 98.96 |
| 3 | 22.3 | 97.09 | 23.9 | 99.45 | 25.3 | 98.68 |
| 4 | 22.7 | 98.83 | 24.4 | 101.52 | 25.8 | 100.77 |
| 7 | 22.4 | 97.37 | 23.8 | 98.89 | 24.7 | 96.27 |
| 8 | 22.3 | 97.21 | 23.9 | 99.32 | 24.9 | 97.32 |
| 9 | 21.9 | 95.34 | 23.4 | 97.23 | 24.9 | 97.22 |
| 10 | 22.0 | 95.61 | 23.4 | 97.51 | 25.0 | 97.67 |
| 11 | 22.0 | 95.92 | 23.6 | 98.20 | 24.7 | 96.52 |
| 14 | 22.8 | 99.12 | 23.7 | 98.48 | 25.4 | 99.26 |

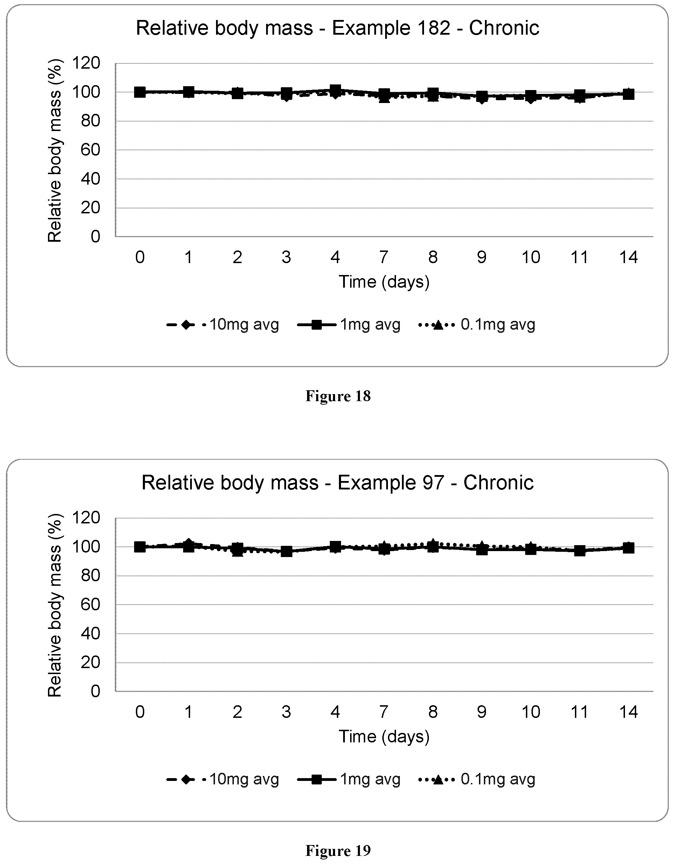

**Table: Results of the chronic toxicity study with Example 97 [AXL22123] (See Figure 18)**

| Example 97 [AXL22123] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 22.3 | 100.00 | 24.9 | 100.00 | 24.2 | 100.00 |
| 1 | 22.8 | 102.25 | 24.8 | 99.84 | 24.3 | 100.35 |
| 2 | 22.2 | 99.41 | 24.6 | 98.99 | 23.5 | 96.97 |
| 3 | 21.6 | 96.88 | 24.1 | 96.68 | 23.4 | 96.67 |
| 4 | 22.1 | 99.11 | 24.9 | 100.12 | 24.1 | 99.80 |
| 7 | 21.8 | 97.75 | 24.4 | 98.47 | 24.2 | 100.26 |
| 8 | 22.2 | 99.55 | 24.8 | 99.89 | 24.7 | 102.18 |
| 9 | 21.9 | 98.05 | 24.3 | 97.93 | 24.3 | 100.34 |
| 10 | 22.1 | 99.24 | 24.3 | 98.13 | 24.1 | 99.78 |
| 11 | 21.7 | 97.44 | 24.1 | 97.18 | 23.5 | 97.12 |
| 14 | 22.3 | 99.85 | 24.6 | 99.08 | 24.0 | 99.09 |

**Table: Results of the chronic toxicity study with Example 151 [AXL22134] (See Figure 19)**

| Example 151 [AXL22134] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 22.0 | 100.00 | 23.7 | 100.00 | 26.0 | 100.00 |
| 1 | 21.9 | 99.42 | 23.9 | 100.59 | 26.3 | 100.98 |
| 2 | 21.8 | 98.80 | 23.6 | 99.32 | 25.6 | 98.55 |
| 3 | 21.4 | 97.10 | 23.4 | 98.49 | 25.6 | 98.42 |
| 4 | 21.6 | 98.02 | 23.6 | 99.33 | 25.6 | 98.54 |
| 7 | 21.5 | 97.68 | 22.9 | 96.60 | 25.1 | 96.47 |
| 8 | 21.5 | 97.68 | 23.2 | 97.88 | 25.3 | 97.28 |
| 9 | 21.4 | 96.98 | 23.0 | 96.94 | 25.1 | 96.33 |
| 10 | 21.7 | 98.25 | 23.1 | 97.49 | 25.2 | 96.99 |
| 11 | 21.6 | 97.79 | 23.7 | 99.75 | 25.2 | 96.84 |
| 14 | 22.5 | 102.02 | 24.7 | 104.25 | 25.8 | 99.21 |

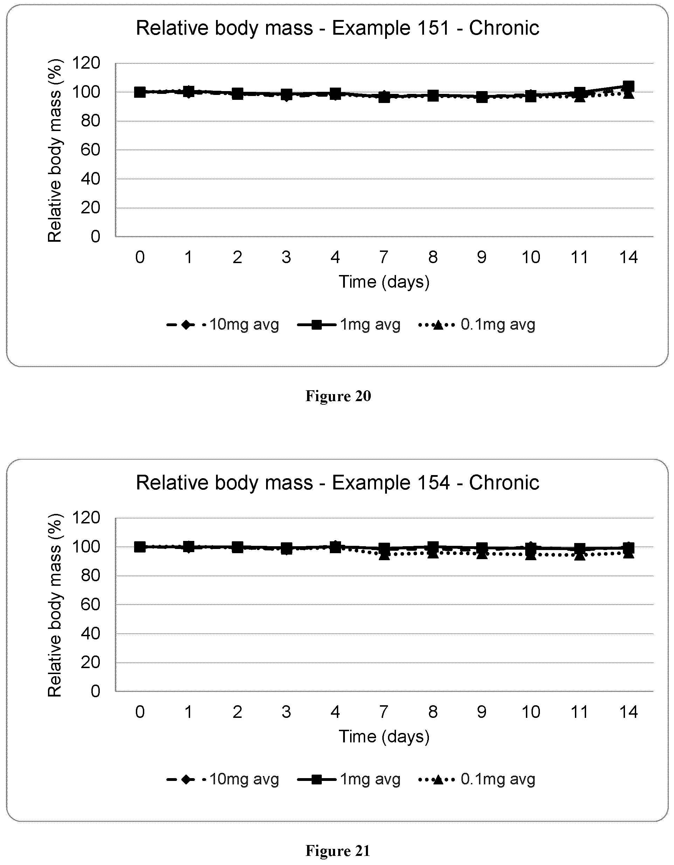

**Table: Results of the chronic toxicity study with Example 154 [AXL22139] (See Figure 20)**

| Example 154 [AXL22139] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 22.2 | 100.00 | 25.5 | 100.00 | 26.4 | 100.00 |
| 1 | 22.0 | 99.25 | 25.5 | 100.04 | 26.4 | 100.29 |
| 2 | 22.0 | 99.42 | 25.5 | 99.99 | 26.2 | 99.29 |
| 3 | 21.7 | 97.92 | 25.3 | 99.20 | 26.0 | 98.50 |
| 4 | 22.3 | 100.76 | 25.5 | 99.95 | 26.2 | 99.41 |
| 7 | 21.8 | 98.22 | 25.2 | 98.83 | 24.9 | 94.63 |
| 8 | 21.8 | 98.35 | 25.5 | 99.98 | 25.3 | 95.89 |
| 9 | 21.6 | 97.44 | 25.3 | 99.18 | 25.1 | 95.27 |
| 10 | 22.2 | 100.02 | 25.2 | 98.83 | 24.9 | 94.64 |
| 11 | 21.6 | 97.60 | 25.2 | 98.74 | 24.9 | 94.34 |
| 14 | 22.1 | 99.83 | 25.3 | 99.07 | 25.3 | 95.87 |

**Table: Results of the chronic toxicity study with Example 174 [AXL22160] (See Figure 21)**

| Example 174 [AXL22160] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 21.5 | 100.00 | 23.4 | 100.00 | 25.5 | 100.00 |
| 1 | 21.2 | 98.74 | 23.5 | 100.24 | 25.7 | 100.51 |
| 2 | 20.7 | 96.43 | 23.1 | 98.68 | 25.3 | 99.18 |
| 3 | 20.2 | 94.10 | 22.7 | 97.16 | 24.7 | 96.69 |
| 4 | 21.3 | 99.23 | 23.2 | 98.95 | 25.3 | 98.96 |
| 7 | 21.2 | 98.50 | 22.9 | 97.76 | 24.9 | 97.43 |
| 8 | 21.3 | 98.93 | 23.4 | 99.86 | 25.3 | 98.86 |
| 9 | 20.9 | 96.99 | 23.0 | 98.32 | 25.0 | 97.97 |
| 10 | 21.7 | 100.92 | 23.0 | 98.50 | 25.2 | 98.77 |
| 11 | 21.3 | 99.10 | 23.1 | 98.67 | 25.3 | 99.20 |
| 14 | 22.1 | 102.60 | 23.7 | 101.51 | 26.1 | 102.27 |

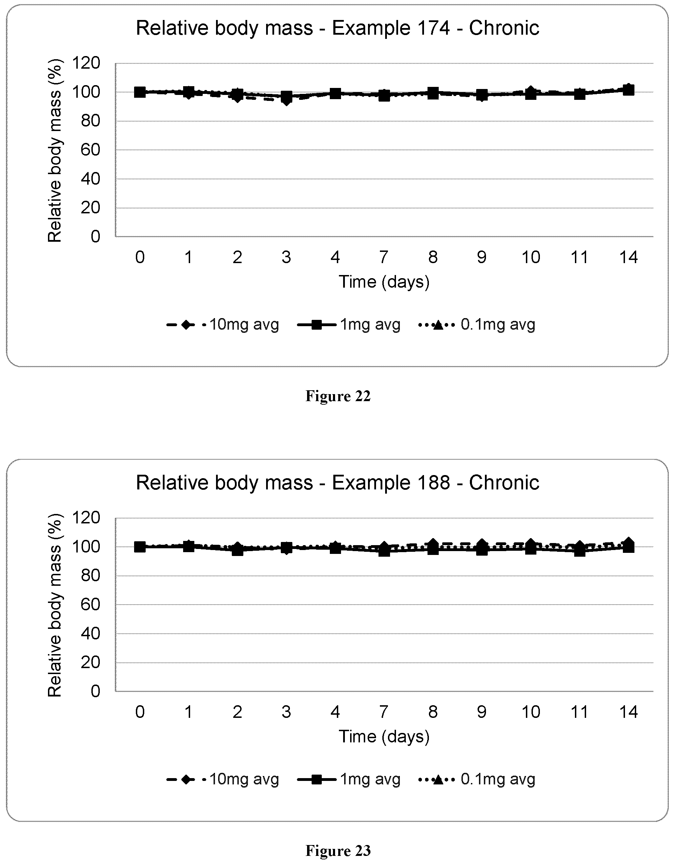

**Table: Results of the chronic toxicity study with Example 188 [AXL22176] (See Figure 22)**

| Example 188 [AXL22176] | 10mg/kg | | 1mg/kg | | 0.1mg/kg | |
|---|---|---|---|---|---|---|
| Day | body mass (g) | relative body mass (%) | body mass (g) | relative body mass (%) | body mass (g) | relative body mass (%) |
| 0 | 21.1 | 100.00 | 23.4 | 100.00 | 24.8 | 100.00 |
| 1 | 21.3 | 100.95 | 23.4 | 100.04 | 25.1 | 101.07 |
| 2 | 21.1 | 99.83 | 22.8 | 97.48 | 24.7 | 99.46 |
| 3 | 20.7 | 98.12 | 23.3 | 99.47 | 24.8 | 99.73 |
| 4 | 21.1 | 100.15 | 23.2 | 98.89 | 24.9 | 100.40 |
| 7 | 21.1 | 100.19 | 22.7 | 96.88 | 24.6 | 99.04 |
| 8 | 21.5 | 102.08 | 23.0 | 98.12 | 24.8 | 99.84 |
| 9 | 21.5 | 101.95 | 22.9 | 97.82 | 24.7 | 99.45 |
| 10 | 21.5 | 102.12 | 23.1 | 98.47 | 25.0 | 100.51 |
| 11 | 21.3 | 100.85 | 22.7 | 96.97 | 24.7 | 99.43 |
| 14 | 21.7 | 103.08 | 23.3 | 99.56 | 25.2 | 101.32 |

### Effects of the compounds on the growth of tumor xenografts

The anti-tumor effect of Example 179 [AXL22064], Example 182 [AXL22108] and Example 154 [AXL22139] were evaluated on the 4T1 cell tumor xenografts in mice. 8-10 weeks old female Balb/C mice were subcutaneously injected in the right flank with 200,000 4T1 cells suspended in 100 µL of saline. Tumor size was measured 7-10 days after injection using an electronic caliper. The volume of the xenograft was calculated using the following formula: Volume = width² (mm) × length (mm) × 0.5. When the tumor volume reached 60 mm³, the mice were divided into experimental groups, and drug administration was started. Each experimental group contained 7 mice, while control group contained 8 mice. Subcutaneous injections were given using an insulin syringe with a 26G x 1/2" needle, administering 100 µL of drug solution at a dose of 50 mg/kg body weight. The injections were given near the xenograft site. The drug was administered on days 2, 4, 7, 9, and 11. Thus,, the injections were performed on alternate days (Mon-Wed-Fri; Mon-Wed-Fri). Tumor size was measured on days 5, 12, 19 and 26 to monitor changes in tumor volume in response to treatment. On day 26, 4 mice of each group were euthanized, and the xenografts were excised, measured, and weighed. The tumors were then fixed in 4% paraformaldehyde (PFA) for 24 hours, after which the PFA was replaced with 70% ethanol for storage.

**Table: Terminal measurements of the excised tumors.**

| | Tumor volume (mm³) | Tumor weight (mg) |
|---|---|---|
| Control | 726.8 | 457.5 |
| Example 179 [AXL22064] | 202.1 | 127.5 |
| Example 182 [AXL22108] | 241.4 | 150.0 |
| Example 154 [AXL22139] | 0.0 | 0.0 |

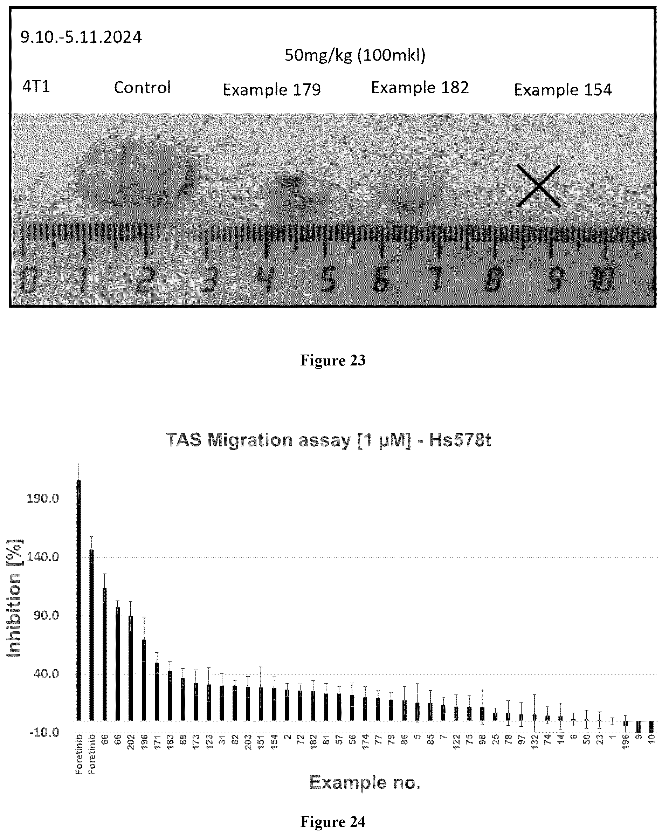

Figure 23 shows the tumours excised from mice of the each experimental group.

### Microsomal stability

Intrinsic clearance values in human microsomes in a 0.1 mg/mL concentration were obtained. Microsomes were incubated along with test compounds for 0, 15, 30, 45, and 60 minutes at 37 °C. Compound quantities were measured by HPLC-MS/MS, and peak area was used to calculate the percentage of the remaining compound, and consequently used to determine apparent intrinsic clearance rate.

**Table: Mean half-life values and intrinsic clearance rates of compounds (n=2).**

| **Example no.** | **Concentration (M)** | **Mean half-life (min)** | **Intrinsic clearance (µl/min/mg)** |
|---|---|---|---|
| Example 56 [AXL22074] | 1.00E-07 | 3.9 | 1774.8 |
| Example 78 [AXL22101] | 1.00E-07 | 58.9 | 117.7 |
| Example 97 [AXL22123] | 1.00E-07 | 10.6 | 653.5 |
| Example 151 [AXL22134] | 1.00E-07 | 8.0 | 871.3 |
| Example 154 [AXL22139] | 1.00E-07 | 43.9 | 158.0 |
| Example 174 [AXL22160] | 1.00E-07 | 5.1 | 1365.7 |
| Example 179 [AXL22064] | 1.00E-07 | 12.3 | 567.7 |
| Example 182 [AXL22108] | 1.00E-07 | 45.1 | 153.8 |
| Example 188 [AXL22176] | 1.00E-07 | 6.0 | 1160.5 |
| Imipramine | 1.00E-07 | >120 | <57.8 |
| Propranolol | 1.00E-07 | >120 | <57.8 |
| Terfenadine | 1.00E-07 | 13.2 | 524.9 |
| Verapamil | 1.00E-07 | 33.8 | 205.1 |

### Western blot

Endogenous AXL expression in the Hs-578T, MCF-7, A172, and U87MG cell lines were detected using the anti-AXL antibody (ProteinTech 68108-1-Ig). All four cell lines expressed AXL of 138kDa. MCF-7 showed low levels of AXL expression.

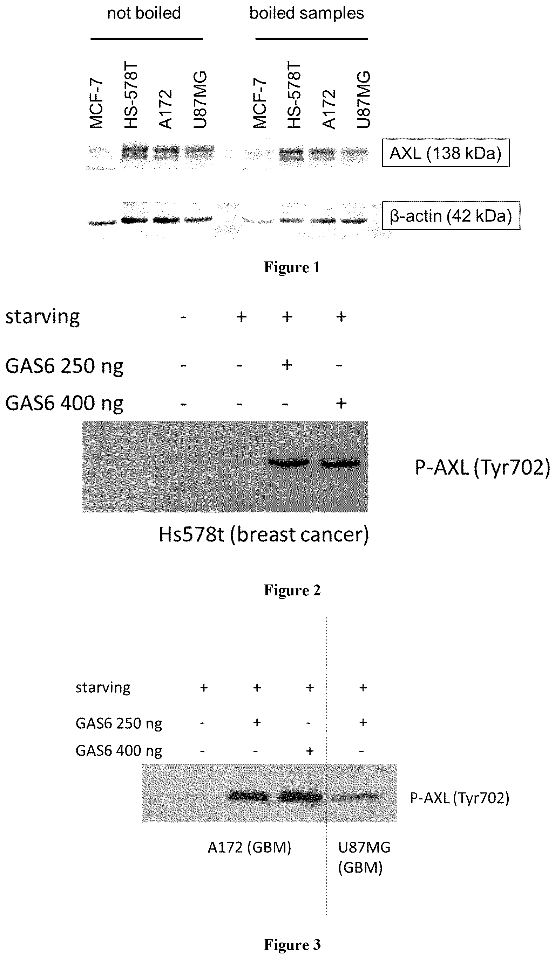

Figure 1 shows the result of the Western blot measurements for cell lines Hs-578T, MCF-7, A172, and U87MG cell lines were detected using the anti-AXL antibody (ProteinTech 68108-1-Ig).

The expression of GAS6-induced phospho-AXL was studied in the Hs-578T, A172, and U87MG cell lines. All three cell lines expressed phospho-AXL following the stimulation of the starved cells with 250ng/µl and 400ng/µl GAS6. The treatment conditions were deemed suitable for testing AXL activation inhibitors. Figure 2 shows the expression of GAS6-induced phospho-AXL studied in the Hs-578T cell line whereas figure 3 shows the expression of GAS6-induced phospho-AXL studied in the A172 and U87MG cell lines; the starved cells were stimulated with 250ng/µl and 400ng/µl GAS6 .

The effects of the inhibitors on GAS6-induced AXL phosphorylation in Hs-578T cell culture were studied in 1000 nM, 100 nM, and 10 nM concentrations. The inhibition of AKT phosphorylation by the compounds was also measured, as well as total AXL and total AKT expression.

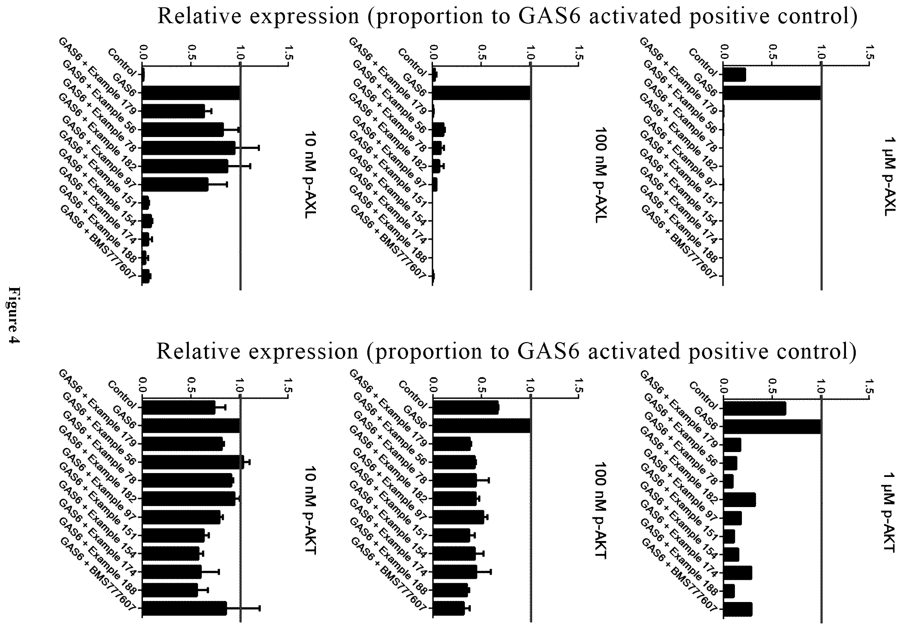

Figure 4 shows the effects of the inhibitors on GAS6-induced AXL phosphorylation in Hs-578T cell culture.

The effect of all inhibitors was detected and was found to act in a dose-dependent manner. While treatment with the inhibitors in a 1 µM concentration resulted in complete inhibition of GAS6-induced AXL activation, depending on the compound, all compounds were found to have weaker effect when applied in smaller concentrations. Similarly, response of p-AKT expression was experienced to act in a dose-dependent manner. Notably, the commercial compound BMS777607 also caused similar expression patterns as the most effective patent examples.

### Experimental details:

### Cell lines: HS-578T breast carcinoma

### Stimulation: GAS6 (250ng/µl) R&D Systems, 885-GSB

After cell seeding, all cells were serum-starved overnight, then a 15-minute inhibitor treatment and a subsequent 15-minute inhibitor+GAS6 treatment were applied to the respective wells on a 6-well plate.

Cell lysates were collected from wells of 6-well plates, grown to 80% confluency. Medium removal and wash with PBS was followed with the addition of 0.8ml 6% trichloroacetic acid solution, and incubation at 4°C for 60 minutes for protein precipitation. Next, the samples were scraped and pipetted to a centrifuge tube. The samples were centrifuged for 15 minutes at room temperature at 13000 × g. Supernatants were discarded and the pellets containing all proteins were resuspended in 40 µl SSP buffer (0.02% bromophenol blue (Merck, Germany, Darmstadt), 10% glycerol (Merck, Germany, Darmstadt), 2% SDS (sodium-dodecylsulfate) (Merck, Germany, Darmstadt), 100 mM dithiothreitol (DTT) (Merck, Germany, Darmstadt), 5 mM EDT (Merck, Germany, Darmstadt) A, 125 mg/mL urea (Merck, Germany, Darmstadt), 90 mM Tris-HCl, pH 7.9). When needed, pH was adjusted by the addition of 0-8ul of 1.7M TRIS-HCL (pH 8.8) buffer until the solution stabilized blue color.

Protein concentration was measured using the Qubit 4 instrument and a Qubit Protein Assay kit (Invitrogen Q33212), following the manufacturer's instructions.

*Gel electrophoresis:* SDS-PAGE with hand-casted 10% polyacrylamide gels.

*Transfer:* Bio-Rad Wet blot system was used to transfer the proteins to a PVDF membrane (250 mA for 80 minutes, kept on ice).

*Blocking:* Bio-Rad Everyblot blocking solution, rinsed for 5 minutes at room temperature.

### Primary antibody:

Total Axl: ProteinTech 68108-1-Ig Mouse mAb, 1:2500 in TBS-tween with 5% nonfat dry milk phospho-Axl (Tyr702): Cell Signaling Rabbit mAb #5724; 1:250 in TBS Tween with 5% BSA,
Total Akt: Cell Signaling, #9272, mouse mAb, 1:500 in TBS Tween with 5% BSA
phospho-Akt: Cell Signaling, #9272, rabbit mAb, 1:500 in TBS Tween with 5% BSA

Staining was performed overnight, at 4°C, with gentle rinse.
Wash: 3x15min, TBS-tween
Secondary antibody: Anti-mouse and anti-rabbit IgG HRP conjugated antibodies (Cell Signaling Technologies), used in 1:5000 dilution in TBS-Tween with 5% nonfat dry milk, gentle rinse for 60 minutes, at room temperature.
Wash: 3x15min, TBS-tween

Signals were visualized using Advansta Western Bright ECL system, and captured by the Uvitec Alliance Q9 imaging system.

*Protein concentrations by Qubit 4 Protein measurement kit:* Standard curve consisted of samples equivalent to 2 mg/ml and 4 mg/ml concentrations. The curve fitting was only done in range of 0.1-6 mg/ml samples. Sample concentrations were calculated by the instrument, following the built-in measurement and reagent protocol. Sample protein concentrations varied between 1 µg/µl and 4 µg/µl, Thus,,, the calculated equal protein in the 25 µl total volume varied from 30 µg to 60 µg per lane.

### TAS migration assay

The TAS migration assay was performed on the most active compounds in the cell in accordance with the recent publication [Veres-Székely, A. et al. "Transient Agarose Spot (TAS) Assay: A New Method to Investigate Cell Migration." Int. J. Mol. Sci. (2022) 23, 2119.]

**Table: TAS migration assay**

| ID | mean migr. inhib. at 1 µM [%] | SD [%], n=10 |
|---|---|---|
| Example 1 [AXL22001] | -0.5 | 3 |
| Example 2 [AXL22002] | 26.4 | 5.7 |
| Example 5 [AXL22007] | 15.3 | 16.4 |
| Example 6 [AXL22008] | 1.5 | 5.2 |
| Example 7 [AXL22009] | 13 | 6.6 |
| Example 9 [AXL22012] | -12.7 | 12.6 |
| Example 10 [AXL22013] | -15.3 | 7.8 |
| Example 14 [AXL22020] | 3.9 | 11.1 |
| Example 23 [AXL22032] | 0.5 | 7.2 |
| Example 25 [AXL22034] | 6.9 | 3.9 |
| Example 31 [AXL22040] | 30.2 | 9.9 |
| Example 56 [AXL22074] | 22.3 | 10.1 |
| Example 57 [AXL22075] | 23.1 | 6.4 |
| Example 66 [AXL22088] | 105.25 | 8.8 |
| Example 69 [AXL22092] | 36.3 | 8.5 |
| Example 72 [AXL22095] | 25.8 | 5.5 |
| Example 74 [AXL22097] | 4.5 | 7.3 |
| Example 75 [AXL22098] | 12 | 9.2 |
| Example 77 [AXL22100] | 19.3 | 6.9 |
| Example 78 [AXL22101] | 6.5 | 10.7 |
| Example 79 [AXL22102] | 18.2 | 5.9 |
| Example 81 [AXL22104] | 23.2 | 8.9 |
| Example 82 [AXL22105] | 30.2 | 4.4 |
| Example 182 [AXL22108] | 25.2 | 9.1 |
| Example 183 [AXL22109] | 42.4 | 8.6 |
| Example 85 [AXL22111] | 15.1 | 10.9 |
| Example 86 [AXL22112] | 17.3 | 11.9 |
| Example 97 [AXL22123] | 5.4 | 10.4 |
| Example 98 [AXL22124] | 11.5 | 14.8 |
| Example 203 AXL22132 | 28.7 | 9 |
| Example 151 [AXL22134] | 28.5 | 17.5 |
| Example 154 [AXL22139] | 27.8 | 9.9 |
| Example 171 [AXL22157] | 49.5 | 8.9 |
| Example 173 [AXL22159] | 32.2 | 11 |
| Example 174 [AXL22160] | 20 | 9.3 |
| Example 196 [AXL22194] | 32.55 | 13.85 |
| Example 202 [AXL22200] | 89.3 | 12.4 |
| Example 122 [AXL22203] | 12.3 | 10.4 |
| Example 123 [AXL22204] | 30.9 | 14.6 |
| Example 132 [AXL22213] | 5.2 | 17.1 |
| BMS777607 | 1.1 | 7.6 |
| Foretinib | 175.95 | 15.9 |

### Transient agarose spot (TAS)

We have determined the inhibition of the FBS induced enhanced migration of the Hs578t, A172, and U87MG cells at 1 µM inhibitor concentration in a transient agarose spot assay. Values higher than 100% can be linked to toxicity of the compound.

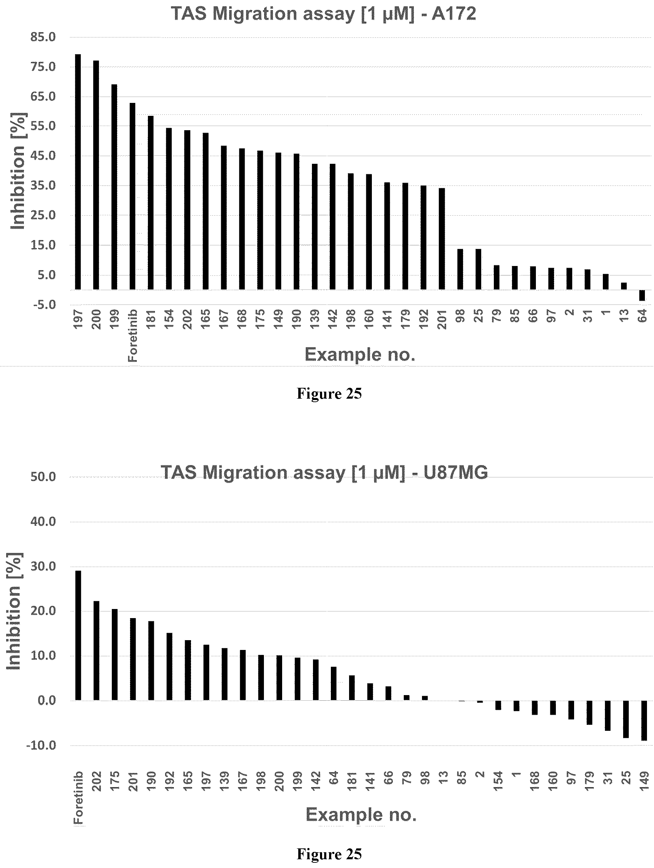

Figures 24, 25 and 26 show the Transient agarose spot (TAS) migration assays with the Hs578t, A172, and U87MG cells at 1 µM inhibitor concentration. The graphs show the percentage inhibition of the FBS induced enhanced migration of the cells.

Experimental protocol:
- Cell lines: Hs578t, A172, U87MG
- Control: starved cells - 0.1% FBS (minimal migration) - technical control
- FBS: feeded cells -10% FBS as a reference for compound treated cell samples
- Compounds: 10% FBS feeded cells treated with compounds in 1 µM concentration

### Water solubility of the compounds

Samples of 5 mM of compounds dissolved in DMSO were diluted using MilliQ water (pH = 7.0) to obtain solutions with a 120 µM final concentration. DMSO was used as control. The samples were incubated for 24 hours at room temperature followed by a 30 minutes centrifugation at 3700 rpm. 40 µL of the supernatants were injected into HPLC (gradient elution, eluent A 0.1 % formic acid in water, eluent B ACN, column XBridge C18 3.5 µm 4.6X50mm) and the AUC values (measured on a sample-specific wavelength) of the buffered samples were divided by the AUC values (same wavelength as of the buffered samples) of the DMSO control samples.

| **ID** | **Solubility pH = 7.0 (µM; max 120 µM)** |
|---|---|
| Example 139 [AXL22031] | 90.4 |
| Example 141 [AXL22043] | 130.3 |
| Example 142 [AXL22056] | 89.6 |
| Example 143 [AXL22057] | 91.7 |
| Example 177 [AXL22062] | 128.0* |
| Example 178 [AXL22063] | 134.0* |
| Example 179 [AXL22064] | 40.3 |
| Example 180 [AXL22065] | 112.3 |
| Example 56 [AXL22074] | 51.8 |
| Example 68 [AXL22091] | 91.0 |
| Example 78 [AXL22101] | 58.2 |
| Example 82 [AXL22105] | 105.5 |
| Example 182 [AXL22108] | 75.0 |
| Example 94 [AXL22120] | 78.9 |
| Example 97 [AXL22123] | 42.3 |
| Example 150 [AXL22133] | 84.4 |
| Example 151 [AXL22134] | 103.5 |
| Example 154 [AXL22139] | 64.0 |
| Example 164 [AXL22150] | 96.7 |
| Example 165 [AXL22151] | 89.5 |
| Example 171 [AXL22157] | 160.2* |
| Example 174 [AXL22160] | 75.5 |
| Example 175 [AXL22161] | 104.8 |
| Example 188 [AXL22176] | 33.8 |
| Example 192 [AXL22190] | 92.6 |
| Example 197 [AXL22195] | 55.9 |
| Example 198 [AXL22196] | 127.7* |
| Example 200 [AXL22198] | 152.7* |
| Example 201 [AXL22199] | 117.5 |
| Example 202 [AXL22200] | 66.3 |
| Example 121 [AXL22202] | 28.2 |
| Example 123 [AXL22204] | 96.6 |
| Example 124 [AXL22205] | 71.7 |
| Example 127 [AXL22208] | 98.2 |
| Example 129 [AXL22210] | 61.8 |
| Example 131 [AXL22212] | 71.5 |
| Example 134 [AXL22215] | 82.1 |

| | |
|---|---|
| • Values above 120 µM are showing better solubility in water than in DMSO | |

### Solubilization of the compounds by cyclodextrin complex formation

Enhancement of the solubility of small molecules with a molecular weight over 500 Da is often desired. Solubilization with cyclodextrins is a common method for this purpose. Multiple types of cyclodextrin are commercially available and often used in the pharmaceutical industry. 2-hydroxypropyl-β-cyclodextrin (2-HP-β-CD) has improved water solubility over α-, β- and γ-cyclodextrin, and has been shown to be well tolerated and safe when administered orally. 2-HP-β-CD is also eliminated rapidly, almost completely unmetabolized, mainly through renal excretion, with a short biological half-life [Gould, S and Scott, Robert C. "2-Hydroxypropyl-β-cyclodextrin (HP-β-CD): A toxicology review", Food and Chemical Toxicology, Volume 43, Issue 10, 2005, Pages 1451-1459]. Due to the above properties, the test compounds were solubilized with 2-HP-β-CD.

### General procedure:

Test compounds with a basic function were isolated as hydrochloride salts, while Example 188 [AXL22176] was used in a neutral form. To 10 mg of test compound, 1 mL of MilliQ water (pH = 7.0) was added. 2-HP-β-CD was added in 2-5 mg portions, and the mixture was sonicated after every added portion. Addition of the cyclodextrin was continued until the initially opalescent mixtures turned into a clear solution.

**Table: Molar ratio of 2-HP-β-CD and the test compounds upon obtaining a clear solution (calculated with an average molecular weight of 1400 for 2-HP-β-CD).**

| ID | Salt | Eq. 2-HP-β-CD |
|---|---|---|
| Example 179 [AXL22064] | 2HCl | 3 |
| Example 56 [AXL22074]* | 1HCl | - |
| Example 78 [AXL22101] | 2HCl | 2.2 |
| Example 182 [AXL22108] | 2HCl | 2 |
| Example 97 [AXL22123] | 2HCl | 3.2 |
| Example 151 [AXL22134] | 1HCl | 2.0 |
| Example 154 [AXL22139] | 1HCl | 2.5 |
| Example 174 [AXL22160] | 1HCl | 2.7 |
| Example 188 [AXL22176] | - | - |

The Example 56 (AXL22074) was dissolved by adding 2.1 eq. Sulfobutyl ether-β-CD.

## Claims

1. A Quinolinyloxyphenyl compound represented by general formula (I) or a pharmaceutically acceptable salt thereof 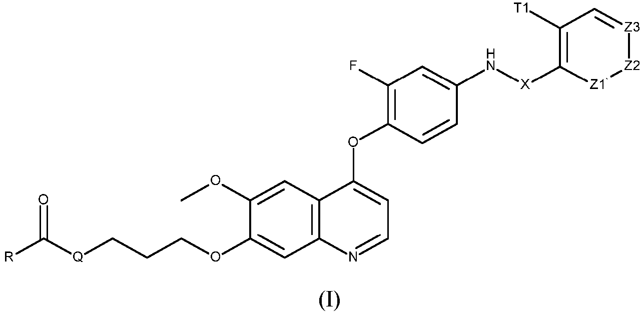 wherein
R is -CR¹=CR²R³, -C=CH, -C≡C-C₁₋₂ alkyl, -CH₂halogen, -CH₂-CH₂-halogen, -CH₂-C≡N, -CH₂-CH₂-C≡N, -CH₂-CH₂-O-C₁₋₂ alkyl, -CH₂-CH₂-CH₂-O-C₁₋₂ alkyl, -CH₂-CH₂-R⁴, -CH₂-CH₂-CH₂-R⁴, -CH₂-CH₂-C(O)R⁴ or -CH₂-CH₂-CH₂-C(O)R⁴;
R¹ is H, halogen or -C₁₋₂ alkyl;
R² is H, halogen or -C₁₋₂ alkyl;
R³ is H; -C₁₋₂ alkyl; -CF₃; -CH₂-R⁴; phenyl optionally substituted with 1 or 2 substituents selected independently from hydroxy, alkoxy, halogen or -CF₃; 5-membered heteroaryl containing 1 or 2 heteroatom selected from O, N and S; -C(O)-phenyl, -CH₂-O-benzotriazol, -C(O)R⁴
R⁴ is -OH, -NC₁₋₃ alkyl₂, -NH-OH, -Nallyl₂, or a N-containing 4-, 5- or 6-membered saturated heterocyclic ring which may further comprise an additional heteroatom selected from N, O or S, said heterocyclic ring is optionally substituted with 1 or 2 substituent selected from hidroxy, methyl, allyl, -NC₁₋₂ alkyl₂, said heterocyclic ring is connected to the rest of the compound through the N-atom;
Q is 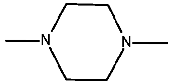 or -NH-;
X is -C(O)- or -S(O)₂-;
Z¹ is -C(O)- or C-H;
Z² is N-T³ or C-T²; as valency permits
the bond between Z¹ and Z² is a double bond when Z¹ is C-H and Z² is C-T² or a single bond when Z¹ is -C(O)- and Z² is N-T³;
Z³ is C-H or N;
T¹ is H, F, CF₃, methoxy or ethoxy; preferably H, F, CF₃ or ethoxy;
T² is H or F;
T³ is phenyl substituted in para position with F or CF₃.

2. The compound according to claim 1 or a pharmaceutically acceptable salt thereof, wherein
R is -CR¹=CR²R³, -C≡C-C₁₋₂ alkyl, -CH₂halogen, -CH₂-C≡N, -CH₂-CH₂-C(O)R⁴ or -CH₂-CH₂-CH₂-C(O)R⁴, preferably -CR¹=CR²R³, -C=C-Me, -CH₂-Cl, -CH₂-C≡N, -CH₂-CH₂-C(O)R⁴ or -CH₂-CH₂-CH₂-C(O)R⁴;
R¹ is H or methyl, preferably H;
R² is H or methyl, preferably H;
R³ is H; -C₁₋₂ alkyl; -CF₃; -CH₂-R⁴; phenyl optionally substituted with 1 or 2 substituents selected independently from hydroxy, methoxy, chloro or -CF₃; 5-membered heteroaryl containing 1 or 2 heteroatom selected from N and S, preferably the heteroaryl is selected from thienyl and 1H-imidazolyl; -C(O)-phenyl, -CH₂-O-benzotriazol, -C(O)R⁴; and/or R⁴ is -OH, -NMe₂, -NEt₂, -NH-OH, -Nallyl₂, or a N-containing heterocyclic ring selected from azetidinyl, pyrrolidinyl, piperidynyl, morpholinyl, thiomorpholinyl, or piperazinyl; said heterocyclic ring is optionally substituted with 1 or 2 substituent selected from hidroxy, methyl, allyl, -NMe₂, -NEt₂, said heterocyclic ring is connected to the rest of the compound through the N-atom.

3. The compound according to claim 1 or 2 or a pharmaceutically acceptable salt thereof, wherein
R⁴ is -OH, -NMe₂, -NEt₂, -NH-OH, -Nallyl₂, or a N-containing heterocyclic ring selected from azetidinyl, pyrrolidinyl, 3-hydroxypyrrolidinyl, 3-(dimethylamino)pyrrolidinyl, piperidynyl, 3-methylpiperidyl, 4-methylpiperidyl, thiomorpholinyl, morpholinyl, 2-methylmorpholinyl, methylpiperazinyl, allylpiperazinyl, 4-(diethylamino)piperidyl; said heterocyclic ring is connected to the rest of the compound through the N-atom.

4. The compound of any one of the previous claims or a pharmaceutically acceptable salt thereof, wherein R³ is H; -C₁₋₂ alkyl; -CF₃; -CH₂-NMe₂; -CH₂-NEt₂; -CH₂-piperidine; -CH₂-piperidine substituted with methyl in position 3; -CH₂-morpholine; -CH₂-piperazine; -CH₂-piperazine substituted with methyl in para position; phenyl; 2-hydroxyphenyl, 3,4-dihydroxyphenyl; 2-methoxyphenyl; 2,3-dimethoxyphenyl; 2,5-dimethoxyphenyl; 2-chlorophenyl; 3-(trifluoromethyl)phenyl; thienyl; 1H-imidazolyl; -C(O)-phenyl, -CH₂-O-benzotriazol, -C(O)R⁴.

5. The compound of any one of the previous claims or a pharmaceutically acceptable salt thereof, wherein R is -CR¹=CR²R³, -CH₂-CH₂-C(O)R⁴ or -CH₂-CH₂-CH₂-C(O)R⁴; preferably -CR¹=CR²R³.

6. The compound of any one of the previous claims or a pharmaceutically acceptable salt thereof, wherein
X is -S(O)₂-, Z¹ is C-H, Z² is C-T² and Z³ is C-H, or
X is -C(O)-, Z¹ is -C(O)-, Z² is N-T³ and Z³ is C-H or N.

7. The compound according to claim 6 or a pharmaceutically acceptable salt thereof, wherein
X is -S(O)₂-; T¹ is F and T² is F or T¹ is CF₃ and T² is H.

8. The compound according to claim 6 or a pharmaceutically acceptable salt thereof, wherein
X is -C(O)-; T¹ is ethoxy, Z² is N-T³, T³ is phenyl substituted in para position with F, and Z³ is C-H.

9. The compound according to claim 8 or a pharmaceutically acceptable salt thereof, wherein
Q is

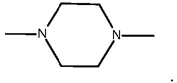

10. The compound according to claim 6 or a pharmaceutically acceptable salt thereof, wherein
X is -C(O)-; T¹ is H, Z² is N-T³, T³ is phenyl substituted in para position with F or CF₃, and Z³ is N.

11. The compound according to claim 1 or a pharmaceutically acceptable salt thereof, said compound being selected from the group consisting of:
| | IUPAC Name |
|---|---|
| Example 1 [AXL22001] | N-[4-[[7-[3-[4-[(E)-4-(dimethylamino)but-2-enoyl]piperazin-1-yl]propoxy]-6-methoxy-4-quinolyl]oxy]-3-fluoro-phenyl]-2,5-difluoro-benzenesulfonamide |
| Example 2 [AXL22002] | N-[4-[[7-[3-[4-[(E)-4-(dimethylamino)but-2-enoyl]piperazin-1-yl]propoxy]-6-methoxy-4-quinolyl]oxy]-3-fluoro-phenyl]-2-(trifluoromethyl)benzenesulfonamide |
| Example 3 [AXL22005] | 2,5-difluoro-N-[3-fluoro-4-[[6-methoxy-7-[3-(4-prop-2-enoylpiperazin-1-yl)propoxy]-4-quinolyl]oxyphenyl]benzenesulfonamide |
| Example 4 [AXL22006] | N-[4-[[7-[3-[4-(2-chloroacetyl)piperazin-1-yl]propoxy]-6-methoxy-4-quinolyl]oxy]-3-fluoro-phenyl]-2,5-difluoro-benzenesulfonamide |
| Example 5 [AXL22007] | N-[4-[[7-[3-[4-(2-chloroacetyl)piperazin-1-yl]propoxy]-6-methoxy-4-quinolyl]oxy]-3-fluoro-phenyl]-2-(trifluoromethyl)benzenesulfonamide |
| Example 6 [AXL22008] | N-[4-[[7-[3-(4-but-2-ynoylpiperazin-1-yl)propoxy]-6-methoxy-4-quinolyl]oxy]-3-fluoro-phenyl]-2,5-difluoro-benzenesulfonamide |
| Example 7 [AXL22009] | 2,5-difluoro-N-[3-fluoro-4-[[6-methoxy-7-[3-[4-(3-methylbut-2-enoyl)piperazin-1-yl]propoxy]-4-quinolyl]oxy]phenyl]benzenesulfonamide |
| Example 8 [AXL22010] | 2,5-difluoro-N-[3-fluoro-4-[[6-methoxy-7-[3-[4-[(E)-3-(2-thienyl)prop-2-enoyl]piperazin-1-yl]propoxy]-4-quinolyl]oxy]phenyl]benzenesulfonamide |
| Example 9 [AXL22012] | 2,5-difluoro-N-[3-fluoro-4-[[6-methoxy-7-[3-[4-[(E)-2-methylbut-2-enoyl]piperazin-1-yl]propoxy]-4-quinolyl]oxy]phenyl]benzenesulfonamide |
| Example 10 [AXL22013] | 2,5-difluoro-N-[3-fluoro-4-[[7-[3-[4-[(E)-3-(1H-imidazol-5-yl)prop-2-enoyl]piperazin-1-yl]propoxy]-6-methoxy-4-quinolyl]oxy]phenyl]benzenesulfonamide |
| Example 11 [AXL22016] | 2,5-difluoro-N-[3-fluoro-4-[[6-methoxy-7-[3-[4-[(E)-4,4,4-trifluoro-3-methyl-but-2-enoyl]piperazin-1-yl]propoxy]-4-quinolyl]oxy]phenyl]benzenesulfonamide |
| Example 12 [AXL22017] | 2,5-difluoro-N-[3-fluoro-4-[[6-methoxy-7-[3-[4-[(E)-3-phenylprop-2-enoyl]piperazin-1-yl]propoxy]-4-quinolyl]oxy]phenyl]benzenesulfonamide |
| Example 13 [AXL22018] | 2,5-difluoro-N-[3-fluoro-4-[[6-methoxy-7-[3-[4-[(E)-4-oxo-4-phenyl-but-2-enoyl]piperazin-1-yl]propoxy]-4-quinolyl]oxy]phenyl]benzenesulfonamide |
| Example 14 [AXL22020] | 2,5-difluoro-N-[3-fluoro-4-[[6-methoxy-7-[3-[4-[(E)-4-morpholinobut-2-enoyl]piperazin-1-yl]propoxy]-4-quinolyl]oxy]phenyl]benzenesulfonamide |
| Example 15 [AXL22021] | N-[3-fluoro-4-[[6-methoxy-7-[3-[4-(3-methylbut-2-enoyl)piperazin-1-yl]propoxy]-4-quinolyl]oxy]phenyl]-2-(trifluoromethyl)benzenesulfonamide |
| Example 16 [AXL22022] | N-[3-fluoro-4-[[6-methoxy-7-[3-(4-prop-2-enoylpiperazin-1-yl)propoxy]-4-quinolyl]oxy]phenyl]-2-(trifluoromethyl)benzenesulfonamide |
| Example 17 [AXL22023] | N-[3-fluoro-4-[[7-[3-[4-[(E)-3-(2-hydroxyphenyl)prop-2-enoyl]piperazin-1-yl]propoxy]-6-methoxy-4-quinolyl]oxy]phenyl]-2-(trifluoromethyl)benzenesulfonamide |
| Example 18 [AXL22025] | N-[3-fluoro-4-[[6-methoxy-7-[3-[4-[(E)-3-(2-methoxyphenyl)prop-2-enoyl]piperazin-1-yl]propoxy]-4-quinolyl]oxy]phenyl]-2-(trifluoromethyl)benzenesulfonamide |
| Example 19 [AXL22026] | N-[4-[[7-[3-[4-[(E)-3-(2,3-dimethoxyphenyl)prop-2-enoyl]piperazin-1-yl]propoxy]-6-methoxy-4-quinolyl]oxy]-3-fluoro-phenyl]-2-(trifluoromethyl)benzenesulfonamide |
| Example 20 [AXL22027] | N-[3-fluoro-4-[[6-methoxy-7-[3-[4-[(E)-2-methylbut-2-enoyl]piperazin-1-yl]propoxy]-4-quinolyl]oxy]phenyl]-2-(trifluoromethyl)benzenesulfonamide |
| Example 21 [AXL22028] | N-[3-fluoro-4-[[6-methoxy-7-[3-[4-[(E)-2-methylpent-2-enoyl]piperazin-1-yl]propoxy]-4-quinolyl]oxy]phenyl]-2-(trifluoromethyl)benzenesulfonamide |
| Example 22 [AXL22030] | N-[3-fluoro-4-[[6-methoxy-7-[3-[4-[(E)-2-methyl-3-phenyl-prop-2-enoyl]piperazin-1-yl]propoxy]-4-quinolyl]oxy]phenyl]-2-(trifluoromethyl)benzenesulfonamide |
| Example 23 [AXL22032] | N-[4-[[7-[3-[4-[(E)-but-2-enoyl]piperazin-1-yl]propoxy]-6-methoxy-4-quinolyl]oxy]-3-fluoro-phenyl]-2,5-difluoro-benzenesulfonamide |
| Example 24 [AXL22033] | N-[4-[[7-[3-[4-[(E)-4-(benzotriazol-1-yloxy)but-2-enoyl]piperazin-1-yl]propoxy]-6-methoxy-4-quinolyl]oxy]-3-fluoro-phenyl]-2,5-difluoro-benzenesulfonamide |
| Example 25 [AXL22034] | 2,5-difluoro-N-[3-fluoro-4-[[6-methoxy-7-[3-[4-[(E)-4,4,4-trifluorobut-2-enoyl]piperazin-1-yl]propoxy]-4-quinolyl]oxy]phenyl]benzenesulfonamide |
| Example 26 [AXL22035] | 2,5-difluoro-N-[3-fluoro-4-[[6-methoxy-7-[3-[4-[(E)-3-[3-(trifluoromethyl)phenyl]prop-2-enoyl]piperazin-1-yl]propoxy]-4-quinolyl]oxy]phenyl]benzenesulfonamide |
| Example 27 [AXL22036] | N-[4-[[7-[3-[4-[(E)-3-(2,5-dimethoxyphenyl)prop-2-enoyl]piperazin-1-yl]propoxy]-6-methoxy-4-quinolyl]oxy]-3-fluoro-phenyl]-2,5-difluoro-benzenesulfonamide |
| Example 28 [AXL22037] | N-[4-[[7-[3-[4-[(E)-3-(2-chlorophenyl)prop-2-enoyl]piperazin-1-yl]propoxy]-6-methoxy-4-quinolyl]oxy]-3-fluoro-phenyl]-2,5-difluoro-benzenesulfonamide |
| Example 29 [AXL22038] | N-[4-[[7-[3-[4-[(E)-but-2-enoyl]piperazin-1-yl]propoxy]-6-methoxy-4-quinolyl]oxy]-3-fluoro-phenyl]-2-(trifluoromethyl)benzenesulfonamide |
| Example 30 [AXL22039] | N-[4-[[7-[3-[4-[(E)-4-(benzotriazol-1-yloxy)but-2-enoyl]piperazin-1-yl]propoxy]-6-methoxy-4-quinolyl]oxy]-3-fluoro-phenyl]-2-(trifluoromethyl)benzenesulfonamide |
| Example 31 [AXL22040] | N-[3-fluoro-4-[[6-methoxy-7-[3-[4-[(E)-4,4,4-trifluorobut-2-enoyl]piperazin-1-yl]propoxy]-4-quinolyl]oxy]phenyl]-2-(trifluoromethyl)benzenesulfonamide |
| Example 32 [AXL22041] | (E)-N-[3-[[4-[4-[(2,5-difluorophenyl)sulfonylamino]-2-fluoro-phenoxy]-6-methoxy-7-quinolyl]oxy]propyl]but-2-enamide |
| Example 33 [AXL22044] | N-[3-[[4-[4-[(2,5-difluorophenyl)sulfonylamino]-2-fluoro-phenoxy]-6-methoxy-7-quinolyl]oxy]propyl]prop-2-enamide |
| Example 34 [AXL22045] | (E)-N-[3-[[4-[4-[(2,5-difluorophenyl)sulfonylamino]-2-fluoro-phenoxy]-6-methoxy-7-quinolyl]oxy]propyl]-2-methyl-but-2-enamide |
| Example 35 [AXL22046] | (E)-N-[3-[[4-[4-[(2,5-difluorophenyl)sulfonylamino]-2-fluoro-phenoxy]-6-methoxy-7-quinolyl]oxy]propyl]-4,4,4-trifluoro-but-2-enamide |
| Example 36 [AXL22047] | (E)-N-[3-[[4-[4-[(2,5-difluorophenyl)sulfonylamino]-2-fluoro-phenoxy]-6-methoxy-7-quinolyl]oxy]propyl]-2-methyl-pent-2-enamide |
| Example 37 [AXL22048] | N-[3-[[4-[4-[(2,5-difluorophenyl)sulfonylamino]-2-fluoro-phenoxy]-6-methoxy-7-quinolyl]oxy]propyl]but-2-ynamide |
| Example 38 [AXL22049] | 2-chloro-N-[3-[[4-[4-[(2,5-difluorophenyl)sulfonylamino]-2-fluoro-phenoxy]-6-methoxy-7-quinolyl]oxy]propyl]acetamide |
| Example 39 [AXL22050] | (E)-N-[3-[[4-[4-[(2,5-difluorophenyl)sulfonylamino]-2-fluoro-phenoxy]-6-methoxy-7-quinolyl]oxy]propyl]-4-(dimethylamino)but-2-enamide |
| Example 40 [AXL22051] | (E)-N-[3-[[4-[4-[(2,5-difluorophenyl)sulfonylamino]-2-fluoro-phenoxy]-6-methoxy-7-quinolyl]oxy]propyl]-3-(2-hydroxyphenyl)prop-2-enamide |
| Example 41 [AXL22052] | N-[3-[[4-[4-[(2,5-difluorophenyl)sulfonylamino]-2-fluoro-phenoxy]-6-methoxy-7-quinolyl]oxy]propyl]-3-methyl-but-2-enamide |
| Example 42 [AXL22053] | (E)-N-[3-[[4-[4-[(2,5-difluorophenyl)sulfonylamino]-2-fluoro-phenoxy]-6-methoxy-7-quinolyl]oxy]propyl]-3-(2-thienyl)prop-2-enamide |
| Example 43 [AXL22054] | (E)-N-[3-[[4-[4-[(2,5-difluorophenyl)sulfonylamino]-2-fluoro-phenoxy]-6-methoxy-7-quinolyl]oxy]propyl]-3-(1H-imidazol-5-yl)prop-2-enamide |
| Example 44 [AXL22055] | (E)-N-[3-[[4-[4-[(2,5-difluorophenyl)sulfonylamino]-2-fluoro-phenoxy]-6-methoxy-7-quinolyl]ox y]propyl]-4,4,4-trifluoro-3-methyl-but-2-enamide |
| Example 45 [AXL22058] | N-[3-[[4-[2-fluoro-4-[[2-(trifluoromethyl)phenyl]sulfonylamino]phenoxy]-6-methoxy-7-quinolyl]oxy]propyl]prop-2-enamide |
| Example 46 [AXL22059] | (E)-N-[3-[[4-[2-fluoro-4-[[2-(trifluoromethyl)phenyl]sulfonylamino]phenoxy]-6-methoxy-7-quinolyl]oxy]propyl]but-2-enamide |
| Example 47 [AXL22060] | (E)-N-[3-[[4-[2-fluoro-4-[[2-(trifluoromethyl)phenyl]sulfonylamino]phenoxy]-6-methoxy-7-quinolyl]oxy]propyl]-2-methyl-but-2-enamide |
| Example 48 [AXL22061] | (E)-4,4,4-trifluoro-N-[3-[[4-[2-fluoro-4-[[2-(trifluoromethyl)phenyl] sulfonylamino]phenoxy]-6-methoxy-7-quinolyl]oxy]propyl]but-2-enamide |
| Example 49 [AXL22066] | 2-chloro-N-[3-[[4-[2-fluoro-4-[[2-(trifluoromethyl)phenyl]sulfonylamino]phenoxy]-6-methoxy-7-quinolyl]oxy]propyl]acetamide |
| Example 50 [AXL22067] | N-[3-[[4-[2-fluoro-4-[[2-(trifluoromethyl)phenyl]sulfonylamino]phenoxy]-6-methoxy-7-quinolyl]oxy]propyl]but-2-ynamide |
| Example 51 [AXL22068] | (E)-N-[3-[[4-[2-fluoro-4-[[2-(trifluoromethyl)phenyl]sulfonylamino]phenoxy]-6-methoxy-7-quinolyl]oxy]propyl]-2-methyl-pent-2-enamide |
| Example 52 [AXL22069] | (E)-4-(dimethylamino)-N-[3-[[4-[2-fluoro-4-[[2-(trifluoromethyl)phenyl] sulfonylamino]phenoxy]-6-methoxy-7-quinolyl]oxy]propyl]but-2-enamide |
| Example 53 [AXL22070] | N-[3-[[4-[2-fluoro-4-[[2-(trifluoromethyl)phenyl]sulfonylamino]phenoxy]-6-methoxy-7-quinolyl]oxy]propyl]-3-methyl-but-2-enamide |
| Example 54 [AXL22071] | (E)-N-[3-[[4-[2-fluoro-4-[[2-(trifluoromethyl)phenyl]sulfonylamino]phenoxy]-6-methoxy-7-quinolyl]oxy]propyl]-3-(2-thienyl)prop-2-enamide |
| Example 55 [AXL22072] | (E)-N-[3-[[4-[2-fluoro-4-[[2-(trifluoromethyl)phenyl]sulfonylamino]phenoxy]-6-methoxy-7-quinolyl]oxy]propyl]-3-(2-hydroxyphenyl)prop-2-enamide |
| Example 56 [AXL22074] | 2,5-difluoro-N-[3-fluoro-4-[[6-methoxy-7-[3-[4-[(E)-2-methylpent-2-enoyl]piperazin-1-yl]propoxy]-4-quinolyl]oxy]phenyl]benzenesulfonamide |
| Example 57 [AXL22075] | N-[4-[[7-[3-(4-but-2-ynoylpiperazin-1-yl)propoxy]-6-methoxy-4-quinolyl]oxy]-3-fluoro-phenyl]-2-(trifluoromethyl)benzenesulfonamide |
| Example 58 [AXL22076] | (E)-N-[3-[[4-[4-[(2,5-difluorophenyl)sulfonylamino]-2-fluoro-phenoxy]-6-methoxy-7-quinolyl]oxy]propyl]-3-phenyl-prop-2-enamide |
| Example 59 [AXL22077] | (E)-N-[3-[[4-[4-[(2,5-difluorophenyl)sulfonylamino]-2-fluoro-phenoxy]-6-methoxy-7-quinolyl]oxy]propyl]-2-methyl-3-phenyl-prop-2-enamide |
| Example 60 [AXL22078] | N-[4-[[7-[3-[4-[(E)-3-(2,5-dimethoxyphenyl)prop-2-enoyl]piperazin-1-yl]propoxy]-6-methoxy-4-quinolyl]oxy]-3-fluoro-phenyl]-2-(trifluoromethyl)benzenesulfonamide |
| Example 61 [AXL22079] | (E)-N-[3-[[4-[4-[(2,5-difluorophenyl)sulfonylamino]-2-fluoro-phenoxy]-6-methoxy-7-quinolyl]oxy]propyl]-3-(2,5-dimethoxyphenyl)prop-2-enamide |
| Example 62 [AXL22080] | (E)-3-(2-chlorophenyl)-N-[3-[[4-[4-[(2,5-difluorophenyl)sulfonylamino]-2-fluoro-phenoxy]-6-methoxy-7-quinolyl]oxy]propyl]prop-2-enamide |
| Example 63 [AXL22081] | (E)-N-[3-[[4-[4-[(2,5-difluorophenyl)sulfonylamino]-2-fluoro-phenoxy]-6-methoxy-7-quinolyl]oxy]propyl]-3-[4-(trifluoromethyl)phenyl]prop-2-enamide |
| Example 64 [AXL22082] | (E)-N-[3-[[4-[4-[(2,5-difluorophenyl)sulfonylamino]-2-fluoro-phenoxy]-6-methoxy-7-quinolyl]oxy]propyl]-4-oxo-4-phenyl-but-2-enamide |
| Example 65 [AXL22083] | (E)-4,4,4-trifluoro-N-[3-[[4-[2-fluoro-4-[[2-(trifluoromethyl)phenyl] sulfonylamino]phenoxy]-6-methoxy-7-quinolyl]oxy]propyl]-3-methyl-but-2-enamide |
| Example 66 [AXL22088] | N-[4-[[7-[3-[4-(2-cyanoacetyl)piperazin-1-yl]propoxy]-6-methoxy-4-quinolyl]oxy]-3-fluoro-phenyl]-2,5-difluoro-benzenesulfonamide |
| Example 67 [AXL22089] | 2-cyano-N-[3-[[4-[2-fluoro-4-[[2-(trifluoromethyl)phenyl]sulfonylamino]phenoxy]-6-methoxy-7-quinolyl]oxy]propyl]acetamide |
| Example 68 [AXL22091] | 2,5-difluoro-N-[3-fluoro-4-[[6-methoxy-7-[3-[4-[(E)-4-(1-piperidyl)but-2-enoyl]piperazin-1-yl]propoxy]-4-quinolyl]oxy]phenyl]benzenesulfonamide |
| Example 69 [AXL22092] | N-[3-fluoro-4-[[6-methoxy-7-[3-[4-[(E)-4-(1-piperidyl)but-2-enoyl]piperazin-1-yl]propoxy]-4-quinolyl]oxy]phenyl]-2-(trifluoromethyl)benzenesulfonamide |
| Example 70 [AXL22093] | (E)-N-[3-[[4-[4-[(2,5-difluorophenyl)sulfonylamino]-2-fluoro-phenoxy]-6-methoxy-7-quinolyl]oxy]propyl]-4-(1-piperidyl)but-2-enamide |
| Example 71 [AXL22094] | (E)-N-[3-[[4-[2-fluoro-4-[[2-(trifluoromethyl)phenyl]sulfonylamino]phenoxy]-6-methoxy-7-quinolyl]oxy]propyl]-4-(1-piperidyl)but-2-enamide |
| Example 72 [AXL22095] | N-[4-[[7-[3-[4-(2-cyanoacetyl)piperazin-1-yl]propoxy]-6-methoxy-4-quinolyl]oxy]-3-fluoro-phenyl]-2-(trifluoromethyl)benzenesulfonamide |
| Example 73 [AXL22096] | 2-cyano-N-[3-[[4-[4-[(2,5-difluorophenyl)sulfonylamino]-2-fluoro-phenoxy]-6-methoxy-7-quinolyl]oxy]propyl]acetamide |
| Example 74 [AXL22097] | 2,5-difluoro-N-[3-fluoro-4-[[6-methoxy-7-[3-[4-[(E)-4-(4-methylpiperazin-1-yl)but-2-enoyl]piperazin-1-yl]propoxy]-4-quinolyl]oxy]phenyl]benzenesulfonamide |
| Example 75 [AXL22098] | N-[3-fluoro-4-[[6-methoxy-7-[3-[4-[(E)-4-(4-methylpiperazin-1-yl)but-2-enoyl]piperazin-1-yl]propoxy]-4-quinolyl]oxy]phenyl]-2-(trifluoromethyl)benzenesulfonamide |
| Example 76 [AXL22099] | (E)-N-[3-[[4-[4-[(2,5-difluorophenyl)sulfonylamino]-2-fluoro-phenoxy]-6-methoxy-7-quinolyl]oxy]propyl]-4-(4-methylpiperazin-1-yl)but-2-enamide |
| Example 77 [AXL22100] | (E)-N-[3-[[4-[2-fluoro-4-[[2-(trifluoromethyl)phenyl]sulfonylamino]phenoxy]-6-methoxy-7-quinolyl]oxy]propyl]-4-(4-methylpiperazin-1-yl)but-2-enamide |
| Example 78 [AXL22101] | N-[4-[[7-[3-[4-[(E)-4-(diethylamino)but-2-enoyl]piperazin-1-yl]propoxy]-6-methoxy-4-quinolyl]oxy]-3-fluoro-phenyl]-2,5-difluoro-benzenesulfonamide |
| Example 79 [AXL22102] | N-[4-[[7-[3-[4-[(E)-4-(diethylamino)but-2-enoyl]piperazin-1-yl]propoxy]-6-methoxy-4-quinolyl]oxy]-3-fluoro-phenyl]-2-(trifluoromethyl)benzenesulfonamide |
| Example 80 [AXL22103] | (E)-4-(diethylamino)-N-[3-[[4-[4-[(2,5-difluorophenyl)sulfonylamino]-2-fluoro-phenoxy]-6-methoxy-7-quinolyl]oxy]propyl]but-2-enamide |
| Example 81 [AXL22104] | (E)-4-(diethylamino)-N-[3-[[4-[2-fluoro-4-[[2-(trifluoromethyl)phenyl] sulfonylamino]phenoxy] -6-methoxy-7-quinolyl]oxy]propyl]but-2-enamide |
| Example 82 [AXL22105] | N-[3-fluoro-4-[[6-methoxy-7-[3-[4-[(E)-4-morpholinobut-2-enoyl]piperazin-1-yl]propoxy]-4-quinolyl]oxy]phenyl]-2-(trifluoromethyl)benzenesulfonamide |
| Example 83 [AXL22106] | (E)-N-[3-[[4-[4-[(2,5-difluorophenyl)sulfonylamino]-2-fluoro-phenoxy]-6-methoxy-7-quinolyl]oxy]propyl]-4-morpholino-but-2-enamide |
| Example 84 [AXL22107] | (E)-N-[3-[[4-[2-fluoro-4-[[2-(trifluoromethyl)phenyl]sulfonylamino]phenoxy]-6-methoxy-7-quinolyl]oxy]propyl] -4-morpholino-but-2-enamide |
| Example 85 [AXL22111] | (Z)-4-[4-[3-[[4-[4-[(2,5-difluorophenyl)sulfonylamino]-2-fluoro-phenoxy]-6-methoxy-7-quinolyl]oxy]propyl]piperazin-1-yl]-4-oxo-but-2-enoic acid |
| Example 86 [AXL22112] | (Z)-4-[4-[3-[[4-[2-fluoro-4-[[2-(trifluoromethyl)phenyl]sulfonylamino]phenoxy]-6-methoxy-7-quinolyl]oxy]propyl]piperazin-1-yl]-4-oxo-but-2-enoic acid |
| Example 87 [AXL22113] | (Z)-4-[3-[[4-[4-[(2,5-difluorophenyl)sulfonylamino]-2-fluoro-phenoxy]-6-methoxy-7-quinolyl]oxy]propylamino]-4-oxo-but-2-enoic acid |
| Example 88 [AXL22114] | (Z)-4-[3-[[4-[2-fluoro-4-[[2-(trifluoromethyl)phenyl]sulfonylamino]phenoxy]-6-methoxy-7-quinolyl]oxy]propylamino]-4-oxo-but-2-enoic acid |
| Example 89 [AXL22115] | 4-[4-[3-[[4-[4-[(2,5-difluorophenyl)sulfonylamino]-2-fluoro-phenoxy]-6-methoxy-7-quinolyl]oxy]propyl]piperazin-1-yl]-4-oxo-butanoic acid |
| Example 90 [AXL22116] | 4-[4-[3-[[4-[2-fluoro-4-[[2-(trifluoromethyl)phenyl]sulfonylamino]phenoxy]-6-methoxy-7-quinolyl]oxypropyl]piperazin-1-yl]-4-oxo-butanoic acid |
| Example 91 [AXL22117] | 4-[3-[[4-[4-[(2,5-difluorophenyl)sulfonylamino]-2-fluoro-phenoxy]-6-methoxy-7-quinolyl]oxy]propylamino]-4-oxo-butanoic acid |
| Example 92 [AXL22118] | 4-[3-[[4-[2-fluoro-4-[[2-(trifluoromethyl)phenyl]sulfonylamino]phenoxy]-6-methoxy-7-quinolyl]oxy]propylamino]-4-oxo-butanoic acid |
| Example 93 [AXL22119] | 5-[4-[3-[[4-[4-[(2,5-difluorophenyl)sulfonylamino]-2-fluoro-phenoxy]-6-methoxy-7-quinolyl]oxy]propyl]piperazin-1-yl]-5-oxo-pentanoic acid |
| Example 94 [AXL22120] | 5-[4-[3-[[4-[2-fluoro-4-[[2-(trifluoromethyl)phenyl]sulfonylamino]phenoxy]-6-methoxy-7-quinolyl]oxypropyl]piperazin-1-yl]-5-oxo-pentanoic acid |
| Example 95 [AXL22121] | 5-[3-[[4-[4-[(2,5-difluorophenyl)sulfonylamino]-2-fluoro-phenoxy]-6-methoxy-7-quinolyl]oxy]propylamino]-5-oxo-pentanoic acid |
| Example 96 [AXL22122] | 5-[3-[[4-[2-fluoro-4-[[2-(trifluoromethyl)phenyl]sulfonylamino]phenoxy]-6-methoxy-7-quinolyl]oxy]propylamino]-5-oxo-pentanoic acid |
| Example 97 [AXL22123] | 2,5-difluoro-N-[3-fluoro-4-[[6-methoxy-7-[3-[4-[(E)-4-(3-methyl-1-piperidyl)but-2-enoyl]piperazin-1-yl]propoxy]-4-quinolyl]oxy]phenyl]benzenesulfonamide |
| Example 98 [AXL22124] | N-[3-fluoro-4-[[6-methoxy-7-[3-[4-[(E)-4-(3-methyl-1-piperidyl)but-2-enoyl]piperazin-1-yl]propoxy]-4-quinolyl]oxy]phenyl]-2-(trifluoromethyl)benzenesulfonamide |
| Example 99 [AXL22125] | (E)-N-[3-[[4-[4-[(2,5-difluorophenyl)sulfonylamino]-2-fluoro-phenoxy]-6-methoxy-7-quinolyl]oxy]propyl]-4-(3-methyl-1-piperidyl)but-2-enamide |
| Example 100 [AXL22126] | (E)-N-[3-[[4-[2-fluoro-4-[[2-(trifluoromethyl)phenyl]sulfonylamino]phenoxy]-6-methoxy-7-quinolyl]oxy]propyl]-4-(3-methyl-1-piperidyl)but-2-enamide |
| Example 101 [AXL22163] | (Z)-N-[3-[[4-[2-fluoro-4-[[2-(trifluoromethyl)phenyl]sulfonylamino]phenoxy]-6-methoxy-7-quinolyl]oxy]propyl]-N',N'-dimethyl-but-2-enediamide |
| Example 102 [AXL22164] | (Z)-N',N'-diethyl-N-[3-[[4-[2-fluoro-4-[[2-(trifluoromethyl)phenyl] sulfonylamino]phenoxy]-6-methoxy-7-quinolyl]oxy]propyl]but-2-enediamide |
| Example 103 [AXL22165] | (Z)-N-[3-[[4-[2-fluoro-4-[[2-(trifluoromethyl)phenyl]sulfonylamino]phenoxy]-6-methoxy-7-quinolyl]oxy]propyl]-4-morpholino-4-oxo-but-2-enamide |
| Example 104 [AXL22166] | (Z)-N-[3-[[4-[2-fluoro-4-[[2-(trifluoromethyl)phenyl]sulfonylamino]phenoxy]-6-methoxy-7-quinolyl]oxy]propyl]-4-oxo-4-pyrrolidin-1-yl-but-2-enamide |
| Example 105 [AXL22167] | (Z)-N-[3-[[4-[2-fluoro-4-[[2-(trifluoromethyl)phenyl]sulfonylamino]phenoxy]-6-methoxy-7-quinolyl]oxy]propyl]-4-oxo-4-(1-piperidyl)but-2-enamide |
| Example 106 [AXL22168] | (Z)-N-[3-[[4-[2-fluoro-4-[[2-(trifluoromethyl)phenyl]sulfonylamino]phenoxy]-6-methoxy-7-quinolyl]oxy]propyl]-4-(3-methyl-1-piperidyl)-4-oxo-but-2-enamide |
| Example 107 [AXL22169] | (Z)-N-[3-[[4-[2-fluoro-4-[[2-(trifluoromethyl)phenyl]sulfonylamino]phenoxy]-6-methoxy-7-quinolyl]oxy]propyl]-4-(4-methylpiperazin-1-yl)-4-oxo-but-2-enamide |
| Example 108 [AXL22170] | (Z)-N-[3-[[4-[2-fluoro-4-[[2-(trifluoromethyl)phenyl]sulfonylamino]phenoxy]-6-methoxy-7-quinolyl]oxy]propyl]-4-oxo-4-thiomorpholino-but-2-enamide |
| Example 109 [AXL22173] | (Z)-4-[4-(diethylamino)-1-piperidyl]-N-[3-[[4-[2-fluoro-4-[[2-(trifluoromethyl)phenyl] sulfonylamino]phenoxy]-6-methoxy-7-quinolyl]oxy]propyl]-4-oxo-but-2-enamide |
| Example 110 [AXL22174] | (Z)-N',N'-diallyl-N-[3-[[4-[2-fluoro-4-[[2-(trifluoromethyl)phenyl]sulfonylamino] phenoxy]-6-methoxy-7-quinolyl]oxy]propyl]but-2-enediamide |
| Example 111 [AXL22178] | (Z)-N-[3-[[4-[4-[(2,5-difluorophenyl)sulfonylamino]-2-fluoro-phenoxy]-6-methoxy-7-quinolyl]oxy]propyl]-N',N'-dimethyl-but-2-enediamide |
| Example 112 [AXL22179] | (Z)-N-[3-[[4-[4-[(2,5-difluorophenyl)sulfonylamino]-2-fluoro-phenoxy]-6-methoxy-7-quinolyl]oxy]propyl]-N',N'-diethyl-but-2-enediamide |
| Example 113 [AXL22180] | (Z)-N',N'-diallyl-N-[3-[[4-[4-[(2,5-difluorophenyl)sulfonylamino]-2-fluoro-phenoxy]-6-methoxy-7-quinolyl]oxy]propyl]but-2-enediamide |
| Example 114 [AXL22181] | (Z)-4-(azetidin-1-yl)-N-[3-[[4-[4-[(2,5-difluorophenyl)sulfonylamino]-2-fluoro-phenoxy]-6-methoxy-7-quinolyl]oxy]propyl]-4-oxo-but-2-enamide |
| Example 115 [AXL22182] | (Z)-N-[3-[[4-[4-[(2,5-difluorophenyl)sulfonylamino]-2-fluoro-phenoxy]-6-methoxy-7-quinolyl]oxy]propyl]-4-oxo-4-pyrrolidin-1-yl-but-2-enamide |
| Example 116 [AXL22183] | (Z)-N-[3-[[4-[4-[(2,5-difluorophenyl)sulfonylamino]-2-fluoro-phenoxy]-6-methoxy-7-quinolyl]oxy]propyl]-4-oxo-4-(1-piperidyl)but-2-enamide |
| Example 117 [AXL22184] | (Z)-N-[3-[[4-[4-[(2,5-difluorophenyl)sulfonylamino]-2-fluoro-phenoxy]-6-methoxy-7-quinolyl]oxy]propyl]-4-morpholino-4-oxo-but-2-enamide |
| Example 118 [AXL22185] | (Z)-N-[3-[[4-[4-[(2,5-difluorophenyl)sulfonylamino]-2-fluoro-phenoxy]-6-methoxy-7-quinolyl]oxy]propyl]-4-(4-methylpiperazin-1-yl)-4-oxo-but-2-enamide |
| Example 119 [AXL22186] | (Z)-4-(azetidin-1-yl)-N-[3-[[4-[2-fluoro-4-[[2-(trifluoromethyl)phenyl] sulfonylamino]phenoxy]-6-methoxy-7-quinolyl]oxy]propyl]-4-oxo-but-2-enamide |
| Example 120 [AXL22187] | (Z)-4-(4-allylpiperazin-1-yl)-N-[3-[[4-[2-fluoro-4-[[2-(trifluoromethyl)phenyl] sulfonylamino]phenoxy]-6-methoxy-7-quinolyl]oxy]propyl]-4-oxo-but-2-enamide |
| Example 121 [AXL22202] | 4-[4-[3-[[4-[4-[(2,5-difluorophenyl)sulfonylamino]-2-fluoro-phenoxy]-6-methoxy-7-quinolyl]oxy]propyl]piperazin-1-yl]-N,N-diethyl-4-oxo-butanamide |
| Example 122 [AXL22203] | N-[4-[[7-[3-[4-[5-(azetidin-1-yl)-5-oxo-pentanoyl]piperazin-1-yl]propoxy]-6-methoxy-4-quinolyl]oxy]-3-fluoro-phenyl]-2,5-difluoro-benzenesulfonamide |
| Example 123 [AXL22204] | 2,5-difluoro-N-[3-fluoro-4-[[6-methoxy-7-[3-[4-(5-morpholino-5-oxo-pentanoyl)piperazin-1-yl]propoxy]-4-quinolyl]oxy]phenyl]benzenesulfonamide |
| Example 124 [AXL22205] | 2,5-difluoro-N-[3-fluoro-4-[[6-methoxy-7-[3-[4-[5-(4-methylpiperazin-1-yl)-5-oxo-pentanoyl]piperazin-1-yl]propoxy]-4-quinolyl]oxy]phenyl]benzenesulfonamide |
| Example 125 [AXL22206] | 2,5-difluoro-N-[3-fluoro-4-[[6-methoxy-7-[3-[4-[5-(3-methyl-1-piperidyl)-5-oxo-pentanoyl]piperazin-1-yl]propoxy]-4-quinolyl]oxy]phenyl]benzenesulfonamide |
| Example 126 [AXL22207] | 4-[4-[3-[[4-[4-[(2,5-difluorophenyl)sulfonylamino]-2-fluoro-phenoxy]-6-methoxy-7-quinolyl]oxy]propyl]piperazin-1-yl]-N,N-dimethyl-4-oxo-butanamide |
| Example 127 [AXL22208] | 2,5-difluoro-N-[3-fluoro-4-[[6-methoxy-7-[3-[4-(4-oxo-4-pyrrolidin-1-yl-butanoyl)piperazin-1-yl]propoxy]-4-quinolyl]oxy]phenyl]benzenesulfonamide |
| Example 128 [AXL22209] | N-[4-[[7-[3-[4-[4-(4-allylpiperazin-1-yl)-4-oxo-butanoyl]piperazin-1-yl]propoxy]-6-methoxy-4-quinolyl]oxy]-3-fluoro-phenyl]-2,5-difluoro-benzenesulfonamide |
| Example 129 [AXL22210] | 4-[4-[3-[[4-[2-fluoro-4-[[2-(trifluoromethyl)phenyl]sulfonylamino]phenoxy]-6-methoxy-7-quinolyl]oxy]propyl]piperazin-1-yl]-N,N-dimethyl-4-oxo-butanamide |
| Example 130 [AXL22211] | N-[4-[[7-[3-[4-[4-(azetidin-1-yl)-4-oxo-butanoyl]piperazin-1-yl]propoxy]-6-methoxy-4-quinolyl]oxy]-3-fluoro-phenyl]-2-(trifluoromethyl)benzenesulfonamide |
| Example 131 [AXL22212] | N-[3-fluoro-4-[[6-methoxy-7-[3-[4-(4-morpholino-4-oxo-butanoyl)piperazin-1-yl]propoxy]-4-quinolyl]oxy]phenyl]-2-(trifluoromethyl)benzenesulfonamide |
| Example 132 [AXL22213] | N-[3-fluoro-4-[[6-methoxy-7-[3-[4-[4-(4-methylpiperazin-1-yl)-4-oxo-butanoyl]piperazin-1-yl]propoxy]-4-quinolyl]oxy]phenyl]-2-(trifluoromethyl)benzenesulfonamide |
| Example 133 [AXL22214] | N,N-diethyl-5-[4-[3-[[4-[2-fluoro-4-[[2-(trifluoromethyl)phenyl]sulfonylamino] phenoxy]-6-methoxy-7-quinolyl]oxy]propyl]piperazin-1-yl]-5-oxo-pentanamide |
| Example 134 [AXL22215] | N-[3-fluoro-4-[[6-methoxy-7-[3-[4-(5-oxo-5-pyrrolidin-1-yl-pentanoyl)piperazin-1-yl]propoxy]-4-quinolyl]oxy]phenyl]-2-(trifluoromethyl)benzenesulfonamide |
| Example 135 [AXL22216] | N-[3-fluoro-4-[[6-methoxy-7-[3-[4-[5-oxo-5-(1-piperidyl)pentanoyl]piperazin-1-yl]propoxy]-4-quinolyl]oxy]phenyl]-2-(trifluoromethyl)benzenesulfonamide |
| Example 136 [AXL22217] | N-[4-[[7-[3-[4-[5-(4-allylpiperazin-1-yl)-5-oxo-pentanoyl]piperazin-1-yl]propoxy]-6-methoxy-4-quinolyl]oxy]-3-fluoro-phenyl]-2-(trifluoromethyl)benzenesulfonamide |
| Example 137 [AXL22218] | (Z)-4-(4-allylpiperazin-1-yl)-N-[3-[[4-[4-[(2,5-difluorophenyl)sulfonylamino]-2-fluoro-phenoxy]-6-methoxy-7-quinolyl]oxy]propyl]-4-oxo-but-2-enamide |
| Example 138 [AXL22219] | (Z)-N-[3-[[4-[4-[(2,5-difluorophenyl)sulfonylamino]-2-fluoro-phenoxy]-6-methoxy-7-quinolyl]oxy]propyl]-4-(3-methyl-1-piperidyl)-4-oxo-but-2-enamide |
| Example 139 [AXL22031] | N-[4-[[7-[3-[4-[(E)-4-(dimethylamino)but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide |
| Example 140 [AXL22042] | 4-ethoxy-N-[3-fluoro-4-[[6-methoxy-7-[3-[4-[(E)-4-morpholinobut-2-enoyl]piperazin-1-yl]propoxy]-4-quinolyl]oxy]phenyl]-1-(4-fluorophenyl)-2-oxo-pyridine-3-carboxamide |
| Example 141 [AXL22043] | 4-ethoxy-N-[3-fluoro-4-[[6-methoxy-7-[3-(4-prop-2-enoylpiperazin-1-yl)propoxy]-4-quinolyl]oxy]phenyl]-1-(4-fluorophenyl)-2-oxo-pyridine-3-carboxamide |
| Example 142 [AXL22056] | 4-ethoxy-N-[3-fluoro-4-[[6-methoxy-7-[3-[4-[(E)-4-(1-piperidyl)but-2-enoyl]piperazin-1-yl]propoxy]-4-quinolyl]oxy]phenyl]-1-(4-fluorophenyl)-2-oxo-pyridine-3-carboxamide |
| Example 143 [AXL22057] | 4-ethoxy-N-[3-fluoro-4-[[6-methoxy-7-[3-[4-[(E)-4-(4-methylpiperazin-1-yl)but-2-enoyl]piperazin-1-yl]propoxy]-4-quinolyl]oxy]phenyl]-1-(4-fluorophenyl)-2-oxo-pyridine-3-carboxamide |
| Example 144 [AXL22090] | (Z)-4-[3-[[4-[4-[[4-ethoxy-1-(4-fluorophenyl)-2-oxo-pyridine-3-carbonyl]amino]-2-fluoro-phenoxy]-6-methoxy-7-quinolyl]oxy]propylamino]-4-oxo-but-2-enoic acid |
| Example 145 [AXL22127] | (Z)-4-[4-[3-[[4-[4-[[4-ethoxy-1-(4-fluorophenyl)-2-oxo-pyridine-3-carbonyl]amino]-2-fluoro-phenoxy]-6-methoxy-7-quinolyl]oxy]propyl]piperazin-1-yl]-4-oxo-but-2-enoic acid |
| Example 146 [AXL22128] | 4-ethoxy-N-[3-fluoro-4-[[6-methoxy-7-[3-[4-[(E)-4-(3-methyl-1-piperidyl)but-2-enoyl]piperazin-1-yl]propoxy]-4-quinolyl]oxy]phenyl]-1-(4-fluorophenyl)-2-oxo-pyridine-3-carboxamide |
| Example 147 [AXL22129] | 4-ethoxy-N-[3-fluoro-4-[[6-methoxy-7-[3-[4-[(E)-3-(2-thienyl)prop-2-enoyl]piperazin-1-yl]propoxy]-4-quinolyl]oxy]phenyl]-1-(4-fluorophenyl)-2-oxo-pyridine-3-carboxamide |
| Example 148 [AXL22130] | N-[4-[[7-[3-[4-[(E)-4-(diethylamino)but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide |
| Example 149 [AXL22131] | 4-ethoxy-N-[3-fluoro-4-[[6-methoxy-7-[3-[4-[(E)-2-methylbut-2-enoyl]piperazin-1-yl]propoxy]-4-quinolyl]oxy]phenyl]-1-(4-fluorophenyl)-2-oxo-pyridine-3-carboxamide |
| Example 150 [AXL22133] | N-[4-[[7-[3-(4-but-2-ynoylpiperazin-1-yl)propoxy]-6-methoxy-4-quinolyl]oxy]-3-fluoro-phenyl]-4-ethoxy-1-(4-fluorophenyl)-2-oxo-pyridine-3-carboxamide |
| Example 151 [AXL22134] | 4-ethoxy-N-[3-fluoro-4-[[6-methoxy-7-[3-[4-[(E)-2-methylpent-2-enoyl]piperazin-1-yl]propoxy]-4-quinolyl]oxy]phenyl]-1-(4-fluorophenyl)-2-oxo-pyridine-3-carboxamide |
| Example 152 [AXL22136] | N-[4-[[7-[3-[4-[(E)-3-(2,5-dimethoxyphenyl)prop-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide |
| Example 153 [AXL22137] | N-[4-[[7-[3-[4-[(E)-but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide |
| Example 154 [AXL22139] | 4-ethoxy-N-[3-fluoro-4-[[6-methoxy-7-[3-[4-[(E)-4,4,4-trifluorobut-2-enoyl]piperazin-1-yl]propoxy]-4-quinolyl]oxy]phenyl]-1-(4-fluorophenyl)-2-oxo-pyridine-3-carboxamide |
| Example 155 [AXL22140] | 4-ethoxy-N-[3-fluoro-4-[[6-methoxy-7-[3-[4-(3-methylbut-2-enoyl)piperazin-1-yl]propoxy]-4-quinolyl]oxy]phenyl]-1-(4-fluorophenyl)-2-oxo-pyridine-3-carboxamide |
| Example 156 [AXL22141] | 4-ethoxy-N-[3-fluoro-4-[[7-[3-[4-[(E)-3-(1H-imidazol-4-yl)prop-2-enoyl]piperazin-1-yl]propoxy]-6-methoxy-4-quinolyl]oxy]phenyl]-1-(4-fluorophenyl)-2-oxo-pyridine-3-carboxamide |
| Example 157 [AXL22143] | 4-ethoxy-N-[3-fluoro-4-[[6-methoxy-7-[3-[4-[(E)-3-phenylprop-2-enoyl]piperazin-1-yl]propoxy]-4-quinolyl]oxy]phenyl]-1-(4-fluorophenyl)-2-oxo-pyridine-3-carboxamide |
| Example 158 [AXL22144] | 4-ethoxy-N-[3-fluoro-4-[[6-methoxy-7-[3-[4-[(E)-2-methyl-3-phenyl-prop-2-enoyl]piperazin-1-yl]propoxy]-4-quinolyl]oxy]phenyl]-1-(4-fluorophenyl)-2-oxo-pyridine-3-carboxamide |
| Example 159 [AXL22145] | N-[4-[[7-[3-[4-[(E)-3-(2,3-dimethoxyphenyl)prop-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide |
| Example 160 [AXL22146] | 4-ethoxy-N-[3-fluoro-4-[[6-methoxy-7-[3-[4-[(E)-4-oxo-4-phenyl-but-2-enoyl]piperazin-1-yl]propoxy]-4-quinolyl]oxy]phenyl]-1-(4-fluorophenyl)-2-oxo-pyridine-3-carboxamide |
| Example 161 [AXL22147] | 4-ethoxy-N-[3-fluoro-4-[[6-methoxy-7-[3-[4-[(E)-3-(2-methoxyphenyl)prop-2-enoyl]piperazin-1-yl]propoxy]-4-quinolyl]oxy]phenyl]-1-(4-fluorophenyl)-2-oxo-pyridine-3-carboxamide |
| Example 162 [AXL22148] | 4-ethoxy-N-[3-fluoro-4-[[7-[3-[4-[(E)-3-(2-hydroxyphenyl)prop-2-enoyl]piperazin-1-yl]propoxy]-6-methoxy-4-quinolyl]oxy]phenyl]-1-(4-fluorophenyl)-2-oxo-pyridine-3-carboxamide |
| Example 163 [AXL22149] | 4-ethoxy-N-[3-fluoro-4-[[6-methoxy-7-[3-[4-[(E)-3-[3-(trifluoromethyl)phenyl]prop-2-enoyl]piperazin-1-yl]propoxy]-4-quinolyl]oxy]phenyl]-1-(4-fluorophenyl)-2-oxo-pyridine-3-carboxamide |
| Example 164 [AXL22150] | 4-ethoxy-N-[3-fluoro-4-[[6-methoxy-7-[3-[4-[(Z)-4-morpholino-4-oxo-but-2-enoyl]piperazin-1-yl]propoxy]-4-quinolyl]oxy]phenyl]-1-(4-fluorophenyl)-2-oxo-pyridine-3-carboxamide |
| Example 165 [AXL22151] | N-[4-[[7-[3-[4-[(Z)-4-(dimethylamino)-4-oxo-but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide |
| Example 166 [AXL22152] | N-[4-[[7-[3-[4-[(Z)-4-(diethylamino)-4-oxo-but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide |
| Example 167 [AXL22153] | 4-ethoxy-N-[3-fluoro-4-[[6-methoxy-7-[3-[4-[(Z)-4-oxo-4-pyrrolidin-1-yl-but-2-enoyl]piperazin-1-yl]propoxy]-4-quinolyl]oxy]phenyl]-1-(4-fluorophenyl)-2-oxo-pyridine-3-carboxamide |
| Example 168 [AXL22154] | 4-ethoxy-N-[3-fluoro-4-[[6-methoxy-7-[3-[4-[(Z)-4-(4-methylpiperazin-1-yl)-4-oxo-but-2-enoyl]piperazin-1-yl]propoxy]-4-quinolyl]oxy]phenyl]-1-(4-fluorophenyl)-2-oxo-pyridine-3-carboxamide |
| Example 169 [AXL22155] | 4-ethoxy-N-[3-fluoro-4-[[6-methoxy-7-[3-[4-[(Z)-4-oxo-4-(1-piperidyl)but-2-enoyl]piperazin-1-yl]propoxy]-4-quinolyl]oxy]phenyl]-1-(4-fluorophenyl)-2-oxo-pyridine-3-carboxamide |
| Example 170 [AXL22156] | 4-ethoxy-N-[3-fluoro-4-[[7-[3-[4-[(Z)-4-[(3R)-3-hydroxypyrrolidin-1-yl]-4-oxo-but-2-enoyl]piperazin-1-yl]propoxy]-6-methoxy-4-quinolyl]oxy]phenyl]-1-(4-fluorophenyl)-2-oxo-pyridine-3-carboxamide |
| Example 171 [AXL22157] | N-[4-[[7-[3-[4-[(Z)-4-[(3R)-3-(dimethylamino)pyrrolidin-1-yl]-4-oxo-but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide |
| Example 172 [AXL22158] | 4-ethoxy-N-[3-fluoro-4-[[7-[3-[4-[(Z)-4-(hydroxyamino)-4-oxo-but-2-enoyl]piperazin-1-yl]propoxy]-6-methoxy-4-quinolyl]oxy]phenyl]-1-(4-fluorophenyl)-2-oxo-pyridine-3-carboxamide |
| Example 173 [AXL22159] | 4-ethoxy-N-[3-fluoro-4-[[6-methoxy-7-[3-[4-[(Z)-4-[(3R)-3-methylmorpholin-4-yl]-4-oxo-but-2-enoyl]piperazin-1-yl]propoxy]-4-quinolyl]oxy]phenyl]-1-(4-fluorophenyl)-2-oxo-pyridine-3-carboxamide |
| Example 174 [AXL22160] | 4-ethoxy-N-[3-fluoro-4-[[6-methoxy-7-[3-[4-[(Z)-4-(4-methyl-1-piperidyl)-4-oxo-but-2-enoyl]piperazin-1-yl]propoxy]-4-quinolyl]oxy]phenyl]-1-(4-fluorophenyl)-2-oxo-pyridine-3-carboxamide |
| Example 175 [AXL22161] | N-[4-[[7-[3-[4-[(Z)-4-(azetidin-1-yl)-4-oxo-but-2-enoyl]piperazin-1-yl]propoxy]-6-methoxy-4-quinolyl]oxy]-3-fluoro-phenyl]-4-ethoxy-1-(4-fluorophenyl)-2-oxo-pyridine-3-carboxamide |
| Example 176 [AXL22162] | 4-ethoxy-N-[3-fluoro-4-[[6-methoxy-7-[3-[4-[(Z)-4-(3-methyl-1-piperidyl)-4-oxo-but-2-enoyl]piperazin-1-yl]propoxy]-4-quinolyl]oxy]phenyl]-1-(4-fluorophenyl)-2-oxo-pyridine-3-carboxamide |
| Example 177 [AXL22062] | N-[3-fluoro-4-[[6-methoxy-7-[3-[4-[(E)-4-morpholinobut-2-enoyl]piperazin-1-yl]propoxy]-4-quinolyl]oxy]phenyl]-2-(4-fluorophenyl)-3-oxo-pyridazine-4-carboxamide |
| Example 178 [AXL22063] | N-[4-[[7-[3-[4-[(E)-4-(dimethylamino)but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide |
| Example 179 [AXL22064] | N-[3-fluoro-4-[[6-methoxy-7-[3-[4-[(E)-4-(1-piperidyl)but-2-enoyl]piperazin-1-yl]propoxy]-4-quinolyl]oxy]phenyl]-2-(4-fluorophenyl)-3-oxo-pyridazine-4-carboxamide |
| Example 180 [AXL22065] | N-[3-fluoro-4-[[6-methoxy-7-[3-[4-[(E)-4-(4-methylpiperazin-1-yl)but-2-enoyl]piperazin-1-yl]propoxy]-4-quinolyl]oxy]phenyl]-2-(4-fluorophenyl)-3-oxo-pyridazine-4-carboxamide |
| Example 181 [AXL22084] | N-[3-fluoro-4-[[6-methoxy-7-[3-(4-prop-2-enoylpiperazin-1-yl)propoxy]-4-quinolyl]oxy]phenyl]-2-(4-fluorophenyl)-3-oxo-pyridazine-4-carboxamide |
| Example 182 [AXL22108] | N-[4-[[7-[3-[4-[(E)-4-(dimethylamino)but-2-enoyl]piperazin-1-yl]propoxy]-6-methoxy-4-quinolyl]oxy]-3-fluoro-phenyl]-3-oxo-2-[4-(trifluoromethyl)phenyl]pyridazine-4-carboxamide |
| Example 183 [AXL22109] | N-[3-fluoro-4-[[6-methoxy-7-[3-(4-prop-2-enoylpiperazin-1-yl)propoxy]-4-quinolyl]oxy]phenyl]-3-oxo-2-[4-(trifluoromethyl)phenyl]pyridazine-4-carboxamide |
| Example 184 [AXL22110] | N-[3-fluoro-4-[[6-methoxy-7-[3-[4-[(E)-4-(1-piperidyl)but-2-enoyl]piperazin-1-yl]propoxy]-4-quinolyl]oxy]phenyl]-3-oxo-2-[4-(trifluoromethyl)phenyl]pyridazine-4-carboxamide |
| Example 185 [AXL22171] | (Z)-4-[3-[[4-[2-fluoro-4-[[2-(4-fluorophenyl)-3-oxo-pyridazine-4-carbonyl]amino]phenoxy]-6-methoxy-7-quinolyl]oxy]propylamino]-4-oxo-but-2-enoic acid |
| Example 186 [AXL22172] | (Z)-N-[3-[[4-[2-fluoro-4-[[2-(4-fluorophenyl)-3-oxo-pyridazine-4-carbonyl]amino]phenoxy]-6-methoxy-7-quinolyl]oxy]propyl]-N',N'-dimethyl-but-2-enediamide |
| Example 187 [AXL22175] | N-[3-fluoro-4-[[6-methoxy-7-[3-[[(Z)-4-morpholino-4-oxo-but-2-enoyl]amino]propoxy]-4-quinolyl]oxy]phenyl]-2-(4-fluorophenyl)-3-oxo-pyridazine-4-carboxamide |
| Example 188 [AXL22176] | N-[3-fluoro-4-[[6-methoxy-7-[3-[[(Z)-4-oxo-4-(1-piperidyl)but-2-enoyl]amino]propoxy]-4-quinolyl]oxy]phenyl]-2-(4-fluorophenyl)-3-oxo-pyridazine-4-carboxamide |
| Example 189 [AXL22177] | N-[3-fluoro-4-[[6-methoxy-7-[3-[[(Z)-4-(4-methylpiperazin-1-yl)-4-oxo-but-2-enoyl]amino]propoxy]-4-quinolyl]oxy]phenyl]-2-(4-fluorophenyl)-3-oxo-pyridazine-4-carboxamide |
| Example 190 [AXL22188] | N-[3-fluoro-4-[[6-methoxy-7-[3-[[(Z)-4-oxo-4-pyrrolidin-1-yl-but-2-enoyl]amino]propoxy]-4-quinolyl]oxy]phenyl]-2-(4-fluorophenyl)-3-oxo-pyridazine-4-carboxamide |
| Example 191 [AXL22189] | (Z)-N',N'-diethyl-N-[3-[[4-[2-fluoro-4-[[2-(4-fluorophenyl)-3-oxo-pyridazine-4-carbonyl]amino]phenoxy]-6-methoxy-7-quinolyl]oxy]propyl]but-2-enediamide |
| Example 192 [AXL22190] | N-[4-[[7-[3-[[(Z)-4-(azetidin-1-yl)-4-oxo-but-2-enoyl]amino]propoxy]-6-methoxy-4-quinolyl]oxy]-3-fluoro-phenyl]-2-(4-fluorophenyl)-3-oxo-pyridazine-4-carboxamide |
| Example 193 [AXL22191] | N-[4-[[7-[3-[4-[(E)-4-(diethylamino)but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide |
| Example 194 [AXL22192] | (Z)-4-[4-[3-[[4-[2-fluoro-4-[[2-(4-fluorophenyl)-3-oxo-pyridazine-4-carbonyl]amino]phenoxy]-6-methoxy-7-quinolyl]oxy]propyl]piperazin-1-yl]-4-oxo-but-2-enoic acid |
| Example 195 [AXL22193] | N-[4-[[7-[3-[4-[(Z)-4-(dimethylamino)-4-oxo-but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide |
| Example 196 [AXL22194] | N-[4-[[7-[3-[4-[(Z)-4-(diethylamino)-4-oxo-but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide |
| Example 197 [AXL22195] | N-[3-fluoro-4-[[6-methoxy-7-[3-[4-[(Z)-4-morpholino-4-oxo-but-2-enoyl]piperazin-1-yl]propoxy]-4-quinolyl]oxy]phenyl]-2-(4-fluorophenyl)-3-oxo-pyridazine-4-carboxamide |
| Example 198 [AXL22196] | N-[3-fluoro-4-[[6-methoxy-7-[3-[4-[(Z)-4-(4-methylpiperazin-1-yl)-4-oxo-but-2-enoyl]piperazin-1-yl]propoxy]-4-quinolyl]oxy]phenyl]-2-(4-fluorophenyl)-3-oxo-pyridazine-4-carboxamide |
| Example 199 [AXL22197] | N-[3-fluoro-4-[[6-methoxy-7-[3-[4-[(Z)-4-oxo-4-(1-piperidyl)but-2-enoyl]piperazin-1-yl]propoxy]-4-quinolyl]oxy]phenyl]-2-(4-fluorophenyl)-3-oxo-pyridazine-4-carboxamide |
| Example 200 [AXL22198] | N-[4-[[7-[3-[4-[(Z)-4-(azetidin-1-yl)-4-oxo-but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide |
| Example 201 [AXL22199] | N-[3-fluoro-4-[[6-methoxy-7-[3-[4-[(Z)-4-oxo-4-pyrrolidin-1-yl-but-2-enoyl]piperazin-1-yl]propoxy]-4-quinolyl]oxy]phenyl]-2-(4-fluorophenyl)-3-oxo-pyridazine-4-carboxamide |
| Example 202 [AXL22200] | N-[4-[[7-[3-[4-[(Z)-4-[(3R)-3-(dimethylamino)pyrrolidin-1-yl]-4-oxo-but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide |
| Example 203 [AXL22132] | N-(4-{[7-(3-{4-[(2E)-3-(3,4-dihydroxyphenyl)prop-2-enoyl]piperazin-1-yl}propoxy)-6-methoxyquinolin-4-yl]oxy}-3-fluorophenyl)-2-(trifluoromethyl)benzenesulfonamide |
| Example 204 [AXL22024] | N-[4-[[7-[3-[4-[(Z)-4-[(3R)-3-(dimethylamino)pyrrolidin-1-yl]-4-oxo-but-2-enoyl]piperazin-1-yl]propoxy]-6-methoxy-4-quinolyl]oxy]-3-fluoro-phenyl]-2-(4-fluorophenyl)-3-oxo-pyridazine-4-carboxamide |
| preferably said compound being selected from the group consisting of | |
|---|---|
| Example 56 [AXL22074] | 2,5-difluoro-N-[3-fluoro-4-[[6-methoxy-7-[3-[4-[(E)-2-methylpent-2-enoyl]piperazin-1-yl]propoxy]-4-quinolyl]oxy]phenyl]benzenesulfonamide |
| Example 78 [AXL22101] | N-[4-[[7-[3-[4-[(E)-4-(diethylamino)but-2-enoyl]piperazin-1-yl]propoxy]-6-methoxy-4-quinolyl]oxy]-3-fluoro-phenyl]-2,5-difluoro-benzenesulfonamide |
| Example 97 [AXL22123] | 2,5-difluoro-N-[3-fluoro-4-[[6-methoxy-7-[3-[4-[(E)-4-(3-methyl-1-piperidyl)but-2-enoyl]piperazin-1-yl]propoxy]-4-quinolyl]oxy]phenyl]benzenesulfonamide |
| Example 151 [AXL22134] | 4-ethoxy-N-[3-fluoro-4-[[6-methoxy-7-[3-[4-[(E)-2-methylpent-2-enoyl]piperazin-1-yl]propoxy]-4-quinolyl]oxy]phenyl]-1-(4-fluorophenyl)-2-oxo-pyridine-3-carboxamide |
| Example 154 [AXL22139] | 4-ethoxy-N-[3-fluoro-4-[[6-methoxy-7-[3-[4-[(E)-4,4,4-trifluorobut-2-enoyl]piperazin-1-yl]propoxy]-4-quinolyl]oxy]phenyl]-1-(4-fluorophenyl)-2-oxo-pyridine-3-carboxamide |
| Example 174 [AXL22160] | 4-ethoxy-N-[3-fluoro-4-[[6-methoxy-7-[3-[4-[(Z)-4-(4-methyl-1-piperidyl)-4-oxo-but-2-enoyl]piperazin-1-yl]propoxy]-4-quinolyl]oxy]phenyl]-1-(4-fluorophenyl)-2-oxo-pyridine-3-carboxamide |
| Example 179 [AXL22064] | N-[3-fluoro-4-[[6-methoxy-7-[3-[4-[(E)-4-(1-piperidyl)but-2-enoyl]piperazin-1-yl]propoxy]-4-quinolyl]oxy]phenyl]-2-(4-fluorophenyl)-3-oxo-pyridazine-4-carboxamide |
| Example 182 [AXL22108] | N-[4-[[7-[3-[4-[(E)-4-(dimethylamino)but-2-enoyl]piperazin-1-yl]propoxy]-6-methoxy-4-quinolyl]oxy]-3-fluoro-phenyl]-3-oxo-2-[4-(trifluoromethyl)phenyl]pyridazine-4-carboxamide |
| Example 188 [AXL22176] | N-[3-fluoro-4-[[6-methoxy-7-[3-[[(Z)-4-oxo-4-(1-piperidyl)but-2-enoyl]amino]propoxy]-4-quinolyl]oxy]phenyl]-2-(4-fluorophenyl)-3-oxo-pyridazine-4-carboxamide |
| more preferably said compound being selected from the group consisting of | |
|---|---|
| Example 154 [AXL22139] | 4-ethoxy-N-[3-fluoro-4-[[6-methoxy-7-[3-[4-[(E)-4,4,4-trifluorobut-2-enoyl]piperazin-1-yl]propoxy]-4-quinolyl]oxy]phenyl]-1-(4-fluorophenyl)-2-oxo-pyridine-3-carboxamide |
| Example 179 [AXL22064] | N-[3-fluoro-4-[[6-methoxy-7-[3-[4-[(E)-4-(1-piperidyl)but-2-enoyl]piperazin-1-yl]propoxy]-4-quinolyl]oxy]phenyl]-2-(4-fluorophenyl)-3-oxo-pyridazine-4-carboxamide |
| Example 182 [AXL22108] | N-[4-[[7-[3-[4-[(E)-4-(dimethylamino)but-2-enoyl]piperazin-1-yl]propoxy]-6-methoxy-4-quinolyl]oxy]-3-fluoro-phenyl]-3-oxo-2-[4-(trifluoromethyl)phenyl]pyridazine-4-carboxamide. |

12. The compound according to any of claims 1 to 11 or a pharmaceutically acceptable salt thereof, wherein said compound is inhibitor of (capable of inhibiting) a TAM-family receptor tyrosine kinase, preferably a TAM-family receptor tyrosine kinase selected from the group consisting of AXL and MER, preferably at least AXL.

13. The compound according to any of claims 1 to 12, wherein said compound is an inhibitor of the AXL receptor tyrosine kinase (the AXL kinase), and preferably is also an inhibitor of (capable of inhibiting) the MER receptor tyrosine kinase.

14. A pharmaceutical composition comprising compound according to any of claims 1 to 13, or a pharmaceutically acceptable salt thereof as a pharmaceutically active ingredient, as well as at least one pharmaceutically acceptable carrier, excipient and/or diluent,
for use in the treatment of a hyperproliferative disorder associated with, or induced by a TAM-family receptor tyrosine kinase selected from the group consisting of AXL and MER
wherein preferably said pharmaceutical composition is formulated for oral administration, for parental administration or for intravenous administration.

15. The compound according to any of claims 1 to 13 or the pharmaceutical composition according to claim 14 use in the treatment of a cell proliferative disease, in a subject; preferably cancer or malignant tumour in said subject; preferably in the treatment of a patient having a diagnosed cell proliferative disease, preferably cancer, or malignant tumour.
